# EUROPÄISCHE PATENTSCHRIFT

(11) **EP 2 188 251 B1**
(45) Veröffentlichungstag und Bekanntmachung des Hinweises auf die Patenterteilung: **29.04.2015**
(21) Anmeldenummer: 08787128.1
(22) Anmeldetag: 12.08.2008
(51) Int. Cl.: C07D 205/04, C07D 207/06, C07D 295/14, C07D 295/18, C07D 403/04

(54) **ARYLSULFONAMIDE MIT ANALGETISCHER WIRKUNG**
ARYLSULFONAMIDES WITH ANALGESIC ACTIVITY
ARYLSULFONAMIDES AYANT ACTIVITÉ ANALGÉSIQUE

(30) Priorität: 14.08.2007 WO PCT/EP2007/058408; 26.02.2008 EP 08102047
(43) Veröffentlichungstag der Anmeldung: 26.05.2010
(73) Patentinhaber: Boehringer Ingelheim International GmbH, 55216 Ingelheim am Rhein (DE)
(72) Erfinder: HAUEL, Norbert, 55216 Ingelheim am Rhein (DE); CECI, Angelo, 55216 Ingelheim am Rhein (DE); DOODS, Henri, 55216 Ingelheim am Rhein (DE); KAUFFMANN-HEFNER, Iris, 55216 Ingelheim am Rhein (DE); KONETZKI, Ingo, 55216 Ingelheim am Rhein (DE); SCHULER-METZ, Annette, 55216 Ingelheim am Rhein (DE); WALTER, Rainer, 55216 Ingelheim am Rhein (DE)
(74) Vertreter: Simon, Elke Anna Maria
(86) Internationale Anmeldenummer: PCT/EP2008/060564
(87) Internationale Veröffentlichungsnummer: WO 2009/021946

(56) Entgegenhaltungen:
- WO-A-02/053516
- WO-A-03/106428
- WO-A-2006/036664
- WO-A-2008/022945

## Beschreibung

Gegenstand der vorliegenden Erfindung sind Verbindungen der allgemeinen Formel I 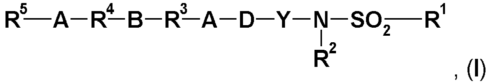 in der **A**, **B**, **D**, **Y**, **R**¹, **R**², **R**³, **R**⁴ und **R**⁵ wie in den Verbindungen des Anspruchs 1 definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

### STAND DER TECHNIK

Im Stand der Technik werden strukturell ähnliche Analgetika beschrieben. In der WO 02/053516 werden Verbindungen beschrieben, welche einen Chlor-Substituenten in ortho-Stellung zur Sulfonamid-Gruppe erfordern, während die Verbindungen der vorliegenden Anmeldung an dieser Stelle eine Methylgruppe aufweisen. Die WO 03/106428 umfasst Verbindungen, die an der Stelle von **R³** eine Phenylgruppe aufweisen, wohingegen die Verbindungen der vorliegenden Anmeldung hier eine Cycloalkylgruppe tragen. Die WO 2006/036664 beschreibt Verbindungen, in denen eine Etherbrücke nicht vorgesehen ist.

### DETAILLIERTE BESCHREIBUNG DER ERFINDUNG

Als ganz besonders bevorzugte Verbindungen der obigen allgemeinen Formel **I** seien beispielsweise folgende genannt:

| **Beispiel** | **Struktur** |
|---|---|
| (651) | 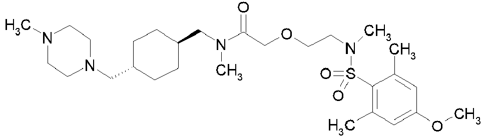 |
| (652) | 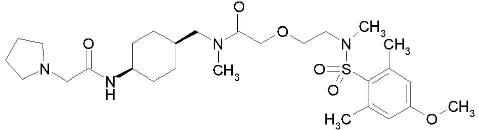 |
| (653) | 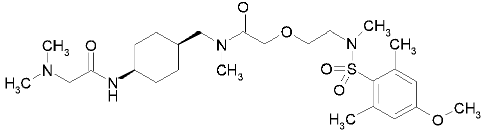 |
| (654) | 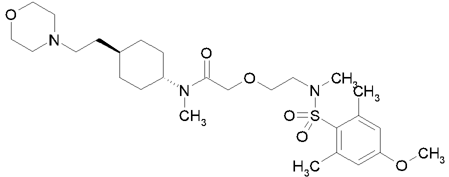 |
| (655) | 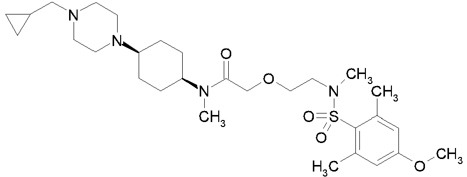 |
| (656) | 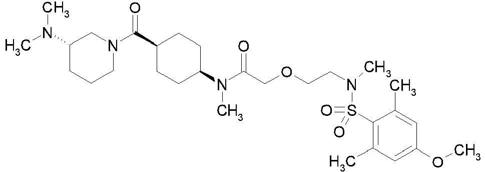 |
| (657) | 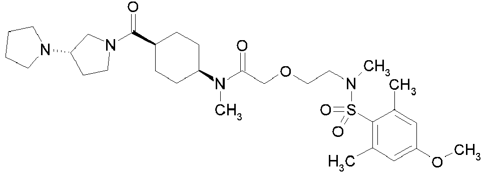 |
| (658) | 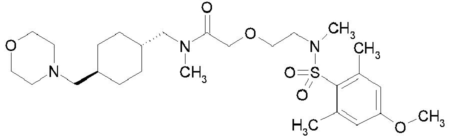 |
| (659) | 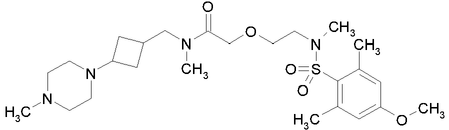 |
| (660) | 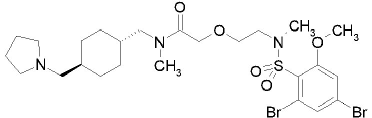 |
| (661) | 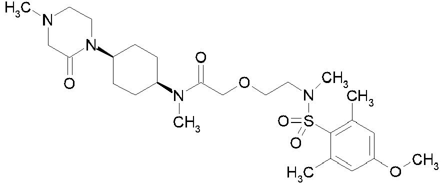 |
| (662) | 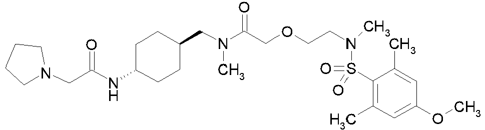 |
| (663) | 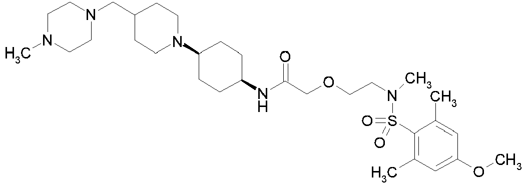 |
| (664) | 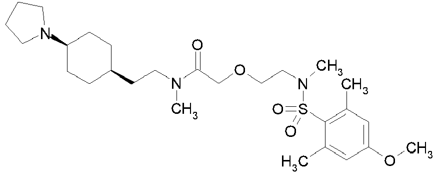 |
| (665) | 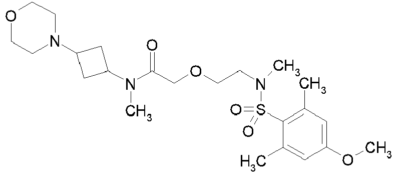 |
| (666) | 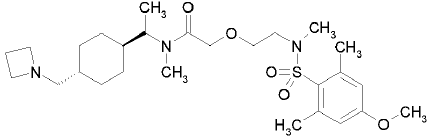 |
| (667) | 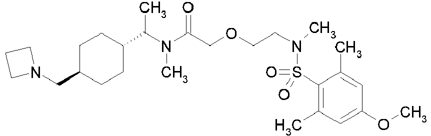 |
| (668) | 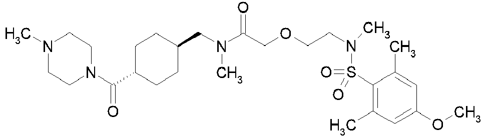 |
| (669) | 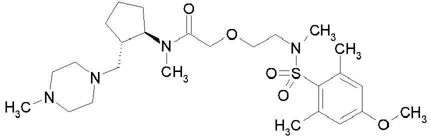 |
| (670) | 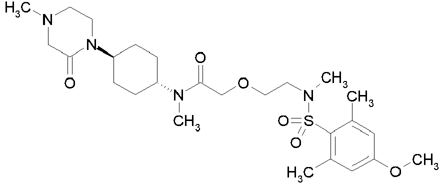 |
| (671) | 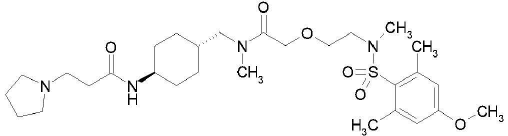 |
| (672) | 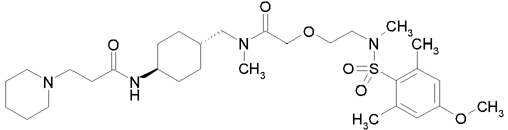 |
| (673) | 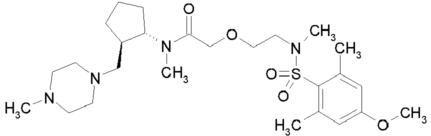 |
| (674) | 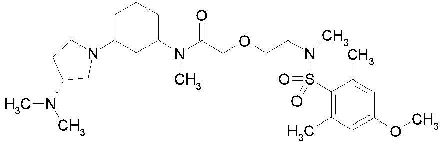 |
| (675) | 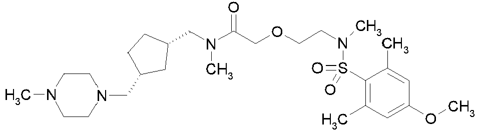 |
| (677) | 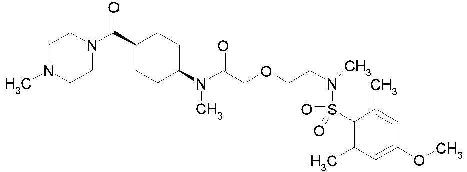 |
| (678) | 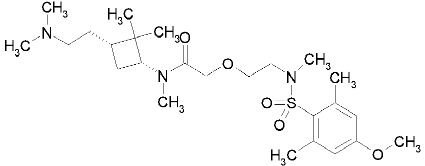 |
| (679) | 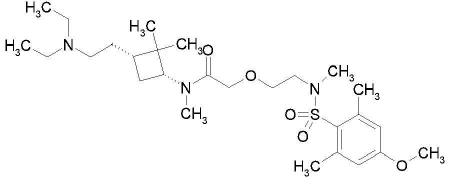 |
| (680) | 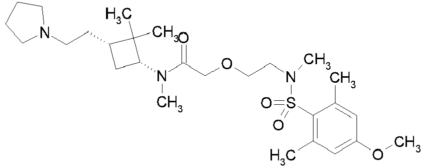 |
| (681) | 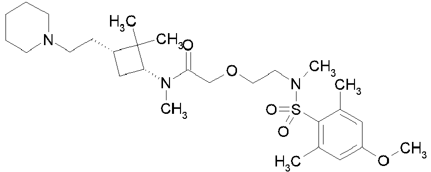 |
| (682) | 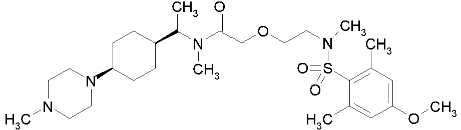 |
| (683) | 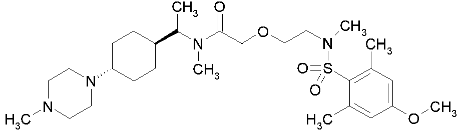 |
| (684) | 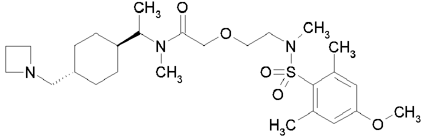 |
| (685) | 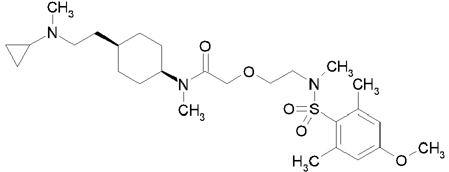 |
| (686) | 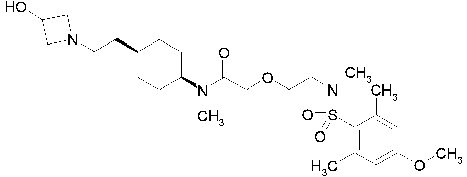 |
| (687) | 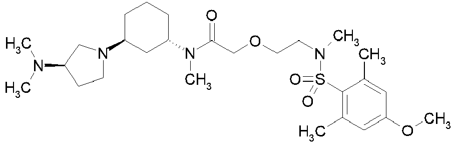 |
| (688) | 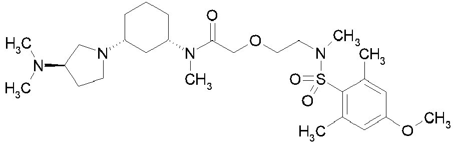 |
| (689) | 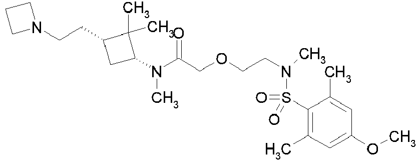 |
| (690) | 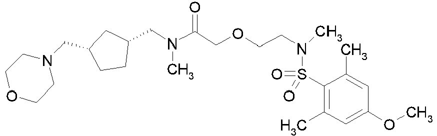 |
| (691) | 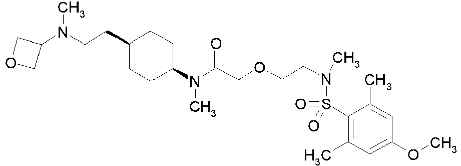 |
| (693) | 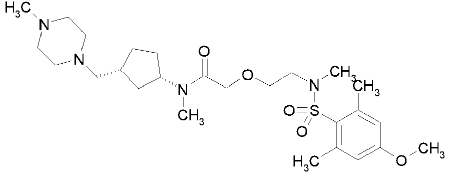 |
| (694) | 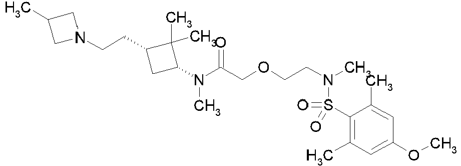 |
| (695) | 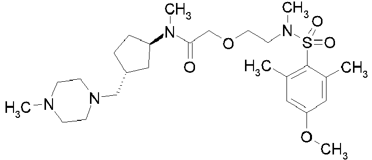 |
| (696) | 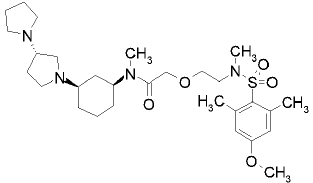 |
| (697) | 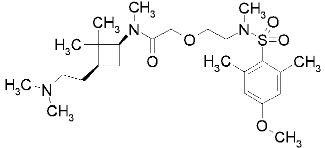 |
| (698) | 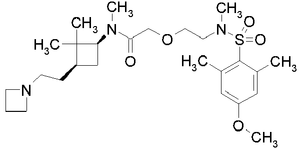 |
| (699) | 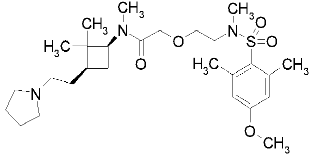 |
| (700) | 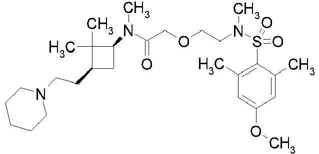 |
| (701) | 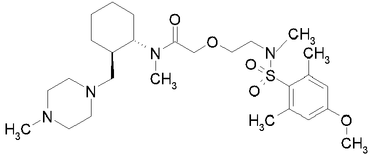 |
| (702) | 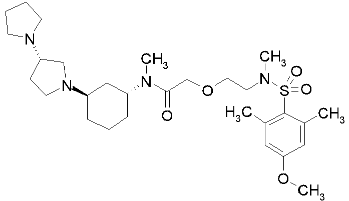 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Sollte in den voranstehend oder nachfolgend genannten Strukturformeln bei einem Substituenten eine Valenz nicht belegt sein und aus dem Namen nichts anderes hervorgehen, wird diese Valenz durch ein Proton besetzt, soweit das chemisch sinnvoll ist. Das gilt insbesondere bei Stickstoffsubstituenten.

Sollte in den voranstehend oder nachfolgend genannten Strukturformeln bei einem Substituenten eine Bindung nicht belegt sein und aus dem Namen nichts anderes hervorgehen, wird diese Bindung durch eine Methylgruppe besetzt, soweit das chemisch sinnvoll ist. Als Beispiele hierfür werden genannt:

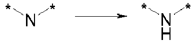

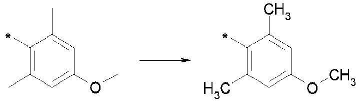

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" werden verzweigte und unverzweigte Alkylgruppen mit 1 bis 3 Kohlenstoffatomen und unter dem Begriff "C₁₋₆-Alkyl" verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, *n*-Pentyl, *iso*-Pentyl, *neo*-Pentyl und Hexyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert-*Butyl etc..

Weiterhin umfassen die voranstehend genannten Begriffe auch solche Reste, in denen jede Methylengruppe mit bis zu zwei und jede Methylgruppe mit bis zu drei Fluoratomen substituiert sein kann.

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel **I**, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Erfindungsgemäß erhält man die Verbindungen der allgemeinen Formel **I** nach an sich bekannten Verfahren, beispielsweise nach folgenden Verfahren:

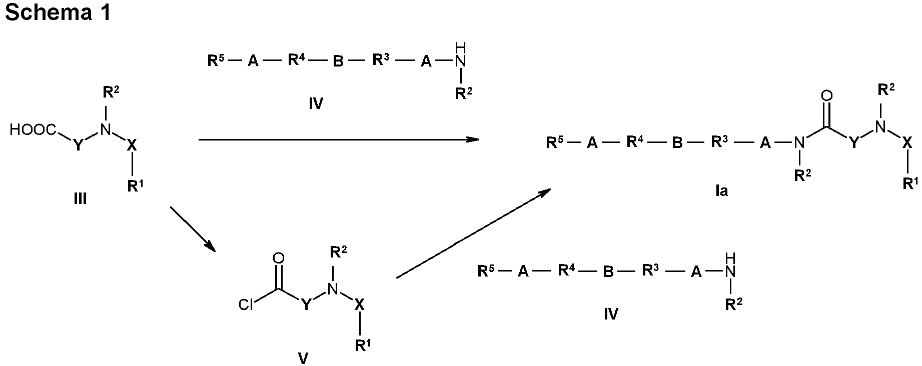

Die in Schema 1 dargestellte Verknüpfung von Carbonsäuren der allgemeinen Formel **III**, in der alle Reste wie in den in Anspruch 1 genannten Verbindungen definiert sind, mit Aminen der allgemeinen Formel **IV**, in der alle Reste wie in den in Anspruch 1 genannten Verbindungen definiert sind, unter Bildung von Carbonsäureamiden der allgemeinen Formel **Ia**, in der alle Reste wie in den in Anspruch 1 genannten Verbindungen definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, *O*-(1*H*-Benzotriazol-1-yl)-*N,N-N',N'*-tetramethyluronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran (THF), Acetonitril, Dimethylformamid (DMF), Dimethylacetamid (DMA), *N*-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase Diisopropylethylamin (DIPEA) (Hünig-Base) bevorzugt.

Eine alternative Methode zur Verknüpfung besteht in der Überführung einer Carbonsäure der allgemeinen Formel **III**, in der alle Reste wie in den in Anspruch 1 genannten Verbindungen definiert sind, in ein Carbonsäurechlorid der allgemeinen Formel V**,** in der alle Reste wie in den in Anspruch 1 genannten Verbindungen definiert sind, und anschließender Umsetzung mit einem Amin der allgemeinen Formel **IV**, in der alle Reste wie in den in Anspruch 1 genannten Verbindungen definiert sind. Die Synthese eines Carbonsäurechlorids der allgemeinen Formel **V** erfolgt nach literaturbekannten Verfahren (siehe z. B. Houben-Weyl, Methoden der Organischen Chemie, Bd. E5/1).

Die als Ausgangsstoffe verwendeten Carbonsäuren der allgemeinen Formel **III**, in der alle Reste wie in den in Anspruch 1 genannten Verbindungen definiert sind, erhält man nach an sich literaturbekannten Verfahren, beispielsweise durch die in Schema 2 bis 7 dargestellten Synthesewege.

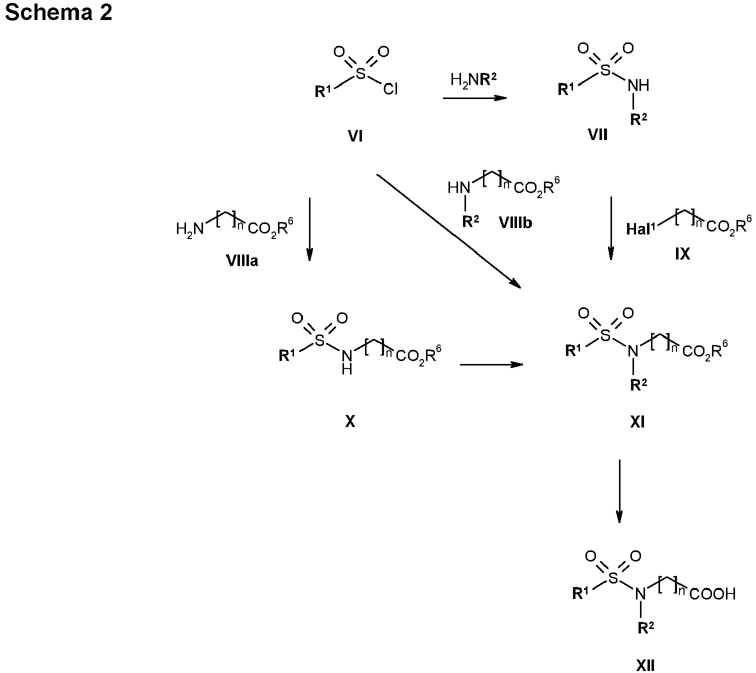

Die Sulfonsäurechloride der allgemeinen Formel **VI**, in der **R¹** wie in den in Anspruch 1 genannten Verbindungen definiert ist, sind entweder literaturbekannt oder käuflich erwerbbar. Sie werden nach Standard-Reaktionsbedingungen mit einem Amin der allgemeinen Formeln H₂N-**R²**, **VIIIa** oder **VIIIb** zu Sulfonsäureamiden der allgemeinen Formeln **VII**, **X** oder **XI** umgesetzt, wobei **R¹** und **R²** wie in den in Anspruch 1 genannten Verbindungen definiert sind und n eine Zahl 1, 2, 3 oder 4 und **R⁶** einen C₁₋₆-Alkylrest bedeuten. Die Umsetzung erfolgt gegebenenfalls in Gegenwart einer Base wie Triethylamin, DIPEA oder Pyridin und einem inerten Lösungsmittel wie Dichlormethan oder Tetrahydrofuran bei einer Temperatur von 0°C bis 100°C mit einer typischen Reaktionsdauer von einer bis 24 Stunden.

Die Umsetzung der Sulfonsäureamide der allgemeinen Formel **VII** mit einem Halogenid der allgemeinen Formel **IX**, in der **Hal¹** Chlor oder Brom bedeutet, erfolgt nach literaturbekannten Verfahren, beispielsweise mit Hilfe einer Base wie Kalium- oder Natriumcarbonat in Dimethylformamid oder Tetrahydrofuran bei 0°C bis 100°C.

Die Hydrolyse der Carbonsäurester der allgemeinen Formel **XI**, in der **R¹** und **R²** wie in den in Anspruch 1 genannten Verbindungen definiert sind, n eine Zahl 1, 2, 3 oder 4 und **R⁶** eine C₁₋₃-Alkylgruppe bedeuten, zu Carbonsäuren der allgemeinen Formel **XII**, in der **R¹** und **R²** wie in den in Anspruch 1 genannten Verbindungen definiert sind und n eine Zahl 1, 2, 3 oder 4 und **R⁶** eine C₁₋₃-Alkylgruppe bedeutet, wird unter bekannten Bedingungen durchgeführt, beispielsweise mit Lithium- oder Natriumcarbonat und Wasser in Methanol und/oder Tetrahydrofuran.

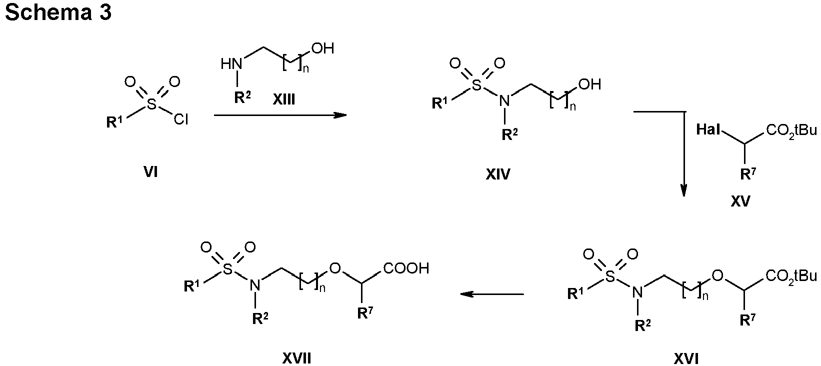

Die Herstellung von Sulfonsäureamiden der allgemeinen Formel **XIV** erfolgt wie unter Schema 2 beschrieben. Die Alkylierung der Hydroxylfunktion der Sulfonsäureamide der allgemeinen Formel **XIV**, in der **R¹** und **R²** wie in den in Anspruch 1 genannten Verbindungen definiert sind mit der Maßgabe, dass **R²** kein Wasserstoffatom darstellt, und **n** eine Zahl 1, 2, 3 oder 4 und **R⁶** eine C₁₋₃-Alkylgruppe bedeutet, wird unter literaturbekannten Reaktionsbedingungen durchgeführt, beispielsweise unter 2-Phasen-Bedingungen mit Hilfe eines Phasentransfer-Katalysators in Gegenwart einer starken anorganischen Base wie Natronlauge oder Kalilauge und in einem inerten Lösungsmittel wie Toluol bei 0°C bis 100°C.

Die Spaltung des tert-Butylesters der allgemeinen Formel **XVI**, in der **R¹** und **R²** wie in den in Anspruch 1 genannten Verbindungen definiert sind, n eine Zahl 1, 2, 3 oder 4 und **R⁶** eine C₁₋₃-Alkylgruppe und **R⁷** ein Wasserstoffatom oder eine C₁₋₃-Alkylgruppe bedeuten, erfolgt nach literaturbekannten Verfahren (siehe z.B. Philip J. Kocieriski, Protecting Groups, 3rd Edition, 2005, Georg Thieme Verlag).

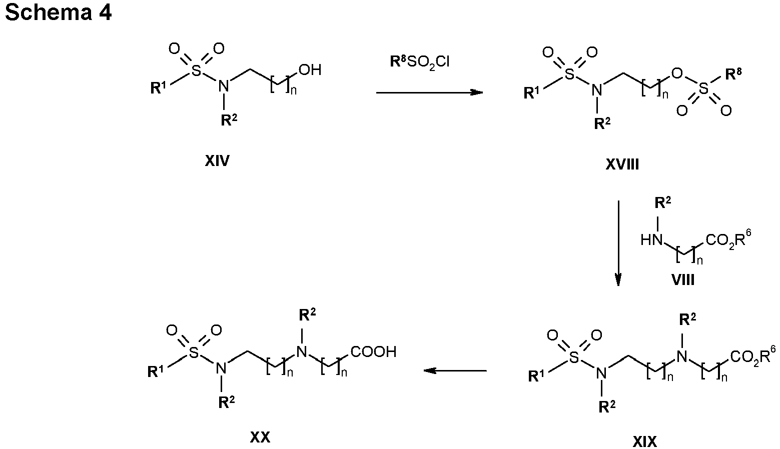

Die Sulfonierung der Hydroxylfunktion einer Verbindung der allgemeinen Formel **XIV**, in der **R¹** und **R²** wie in den in Anspruch 1 genannten Verbindungen definiert sind mit der Maßgabe, dass **R²** kein Wasserstoffatom darstellt, und n eine Zahl 1, 2, 3 oder 4 und **R⁶** eine C₁₋₃-Alkylgruppe bedeutet, mit einem Sulfonsäurechlorid der allgemeinen Formel **R⁸**SO₂Cl, in der **R⁸** eine C₁₋₃-Alkylgruppe oder eine ggf. durch C₁₋₃-Alkylgruppe substituierte Phenylgruppe darstellt, zu Verbindungen der allgemeinen Formel **XVIII**, in der alle Rest wie in den in Anspruch 1 genannten Verbindungen definiert sind, wird unter Standard-Reaktionsbedingungen durchgeführt, typischerweise in Gegenwart einer Base wie DMAP und/oder Pyridin und einem inerten Lösungsmittel wie Dichlormethan oder THF bei -5°C bis 35°C. Eine flüssige Base wie Pyridin kann als Base und gleichzeitig als Lösungsmittel verwendet werden.

Die nachfolgende Alkylierung der Amine mit der allgemeinen Formel **VII** zu Verbindungen der allgemeinen Formel **XIX**, in der **R¹** und **R² wie** in den in Anspruch 1 genannten Verbindungen definiert sind, **n** eine Zahl 1, 2, 3 oder 4 und **R⁶** eine C₁₋₃-Alkylgruppe und **R⁶** eine C₁₋₆-Alkylgruppe bedeuten, wird zweckmäßigerweise in einem Lösungsmittel wie Toluol, Chlorbenzol, Dimethylformamid, Dimethylsulfoxid (DMSO), Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XIX** zu Carbonsäuren der allgemeinen Formel **XX** erfolgt wie unter Schema 2 beschrieben.

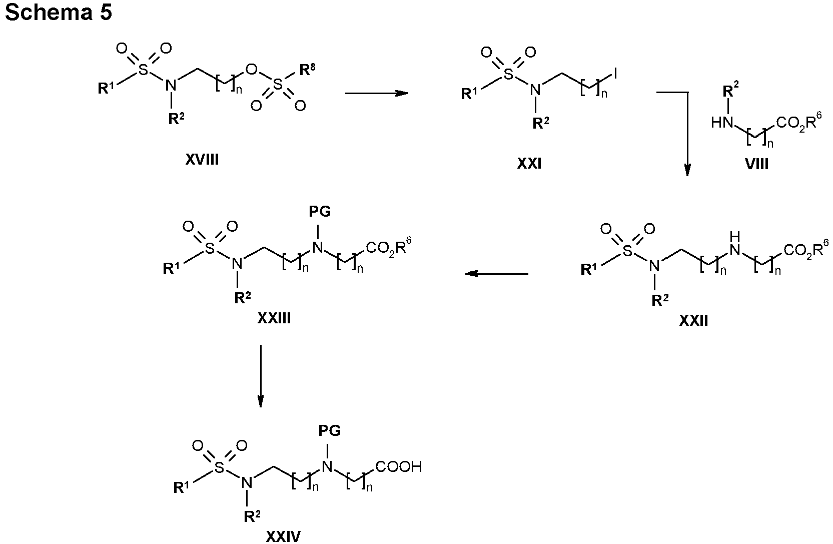

Die Finkelsteinstein-Reaktion von Verbindungen der allgemeinen Formel **XVIII**, in der **R¹** und **R²** wie in den in Anspruch 1 genannten Verbindungen definiert sind, n eine Zahl 1, 2, 3 oder 4 und **R⁶** eine C₁₋₃-Alkylgruppe und **R⁸** eine C₁₋₃-Alkylgruppe oder eine ggf. durch C₁₋₃-Alkylgruppe substituierte Phenylgruppe bedeuten, zu Halogeniden der allgemeinen Formel **XXI**, in der **R¹** und **R²** wie in den in Anspruch 1 genannten Verbindungen definiert sind und n eine Zahl 1, 2, 3 oder 4 und **R⁶** eine C₁₋₃-Alkylgruppe bedeutet, erfolgt unter bekannten Reaktionsbedingungen (siehe z.B. H. Finkelstein, Berichte der Deutschen Chemischen Gesellschaft 43, 1910, 1528).

Die anschließende Alkylierung des Glycinesters wird wie unter Schema 4 beschrieben durchgeführt (**R²** ≠ H).

Die Aminofunktion in den Verbindungen der allgemeinen Formel **XXIII** wird durch eine konventionelle Schutzgruppe **PG** nach bekannten Verfahren geschützt. Die ausgewählte Schutzgruppe ist eine, die unter nicht-hydrogenolytischen Bedingungen abgespalten werden kann. Eine bevorzugte Schutzgruppe ist die Boc-Gruppe. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocieński, Protecting Groups, 3rd Edition, 2005, Georg Thieme Verlag.

Die Spaltung der Carbonsäureester der allgemeinen Formel **XXIII** zu Carbonsäuren der allgemeinen Formel **XXIV** erfolgt wie unter Schema 2 beschrieben.

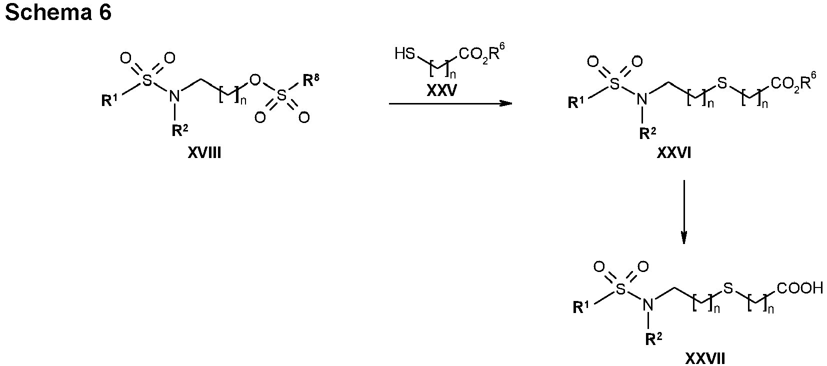

Die Alkylierung eines Thiols der allgemeinen Formel **XXV**, in der n eine Zahl 1, 2, 3 oder 4 und **R⁶** eine C₁₋₆-Alkylgruppe bedeutet, zu Verbindungen der allgemeinen Formel **XXVI**, in der **R¹** und **R²** wie in den in Anspruch 1 genannten Verbindungen definiert sind, n eine Zahl 1, 2, 3 oder 4 und **R⁶** eine C₁₋₆-Alkylgruppe bedeuten, wird zweckmäßigerweise in einem Lösungsmittel wie Toluol, Chlorbenzol, DMF, DMSO, Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XXVI** zu Carbonsäuren der allgemeinen Formel **XXVII**, in der alle Reste wie in den in Anspruch 1 genannten Verbindungen definiert sind, erfolgt wie unter Schema 2 beschrieben.

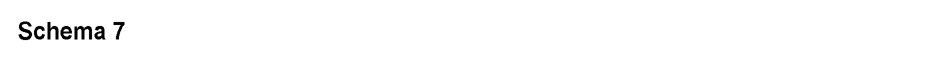

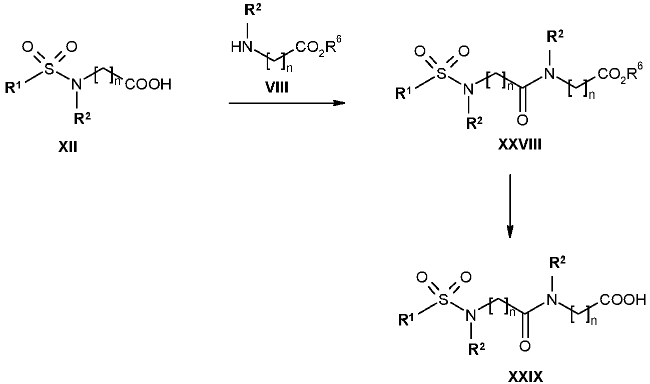

Die Amidknüpfung von Carbonsäuren der allgemeinen Formel **XII**, in der **R¹** und **R²** wie in den in Anspruch 1 genannten Verbindungen definiert sind und **n** eine Zahl 1, 2, 3 oder 4 bedeutet, und Aminosäuren der allgemeinen Formel **VIII**, in der **R¹** und **R²** wie in den in Anspruch 1 genannten Verbindungen definiert sind, **n** eine Zahl 1, 2, 3 oder 4 und **R⁶** eine C₁₋₆-Alkylgruppe bedeuten, zu Carbonsäureamiden der allgemeinen Formel **XXVIII**, in der **R¹** und **R²** wie in den in Anspruch 1 genannten Verbindungen definiert sind, n eine Zahl 1, 2, 3 oder 4 und **R⁶** eine C₁₋₆-Alkylgruppe bedeuten, wird wie unter Schema 1 beschrieben durchgeführt.

Wie unter Schema 2 ausgeführt, wird der Carbonsäureester der allgemeinen Formel **XXVIII** zu Carbonsäure der allgemeinen Formel **XXIX**, in der **R¹** und **R²** wie in den in Anspruch 1 genannten Verbindungen definiert sind und **n** eine Zahl 1, 2, 3 oder 4 bedeutet, gespalten.

Die als Ausgangsstoffe verwendeten Amine der allgemeinen Formel **IV** sind entweder käuflich erwerbbar, oder man erhält sie nach an sich literaturbekannten Verfahren, beispielsweise durch die in Schema 8 bis 12 dargestellten Synthesewege, wobei **R^{1.1}** wie in den in Anspruch 1 genannten Verbindungen definiert ist, **Hal¹** ein Chlor- oder Bromatom und **Hal²** ein Fluor-, Chlor- oder Bromatom oder einen Rest **R⁹** bedeuten.

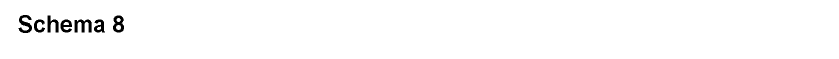

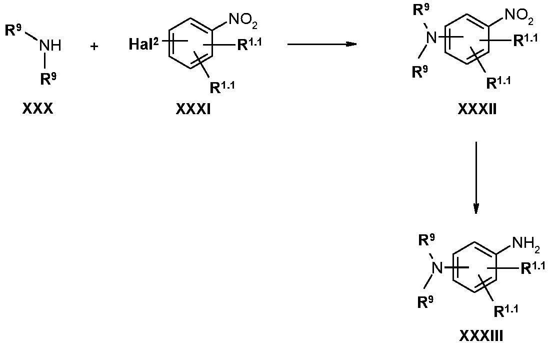

Die Reaktion eines Amins der allgemeinen Formel **XXX**, in der **R⁹** eine C₁₋₃-Alkylgruppe bedeutet, mit einem Halogen-nitrobenzol der allgemeinen Formel **XXXI**, in der **R^{1.1}** wie in den in Anspruch **1** genannten Verbindungen definiert ist und **Hal²** ein Fluor-, Chlor- oder Bromatom oder einen Rest **R⁹** bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer passenden Base wie Triethylamin oder Kaliumcarbonat, bei einer Temperatur von 20°C bis 160°C. Ist das Amin der allgemeinen Formel **XXX** flüssig, kann die Reaktion auch ohne Lösungsmittel und zusätzlicher Base durchgeführt werden.

Die Reduktion der Nitrogruppe zu Anilinen der allgemeinen Formel **XXXIII**, in der **R^{1.1}** wie in den in Anspruch 1 genannten Verbindungen definiert ist und **R⁹** eine C₁₋₃-Alkylgruppe bedeutet, erfolgt nach Standard-Reaktionsbedingungen (siehe z.B. Richard C. Larock, Comprehensive Organic Transformations, 1989, VCH), vorzugsweise nach Standard-Bedingungen der katalytischen Hydrogenolyse mit einem Katalysator wie Palladium auf Kohle oder Raney-Nickel in einem Lösungsmittel wie Methanol oder Ethanol.

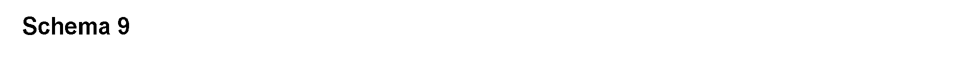

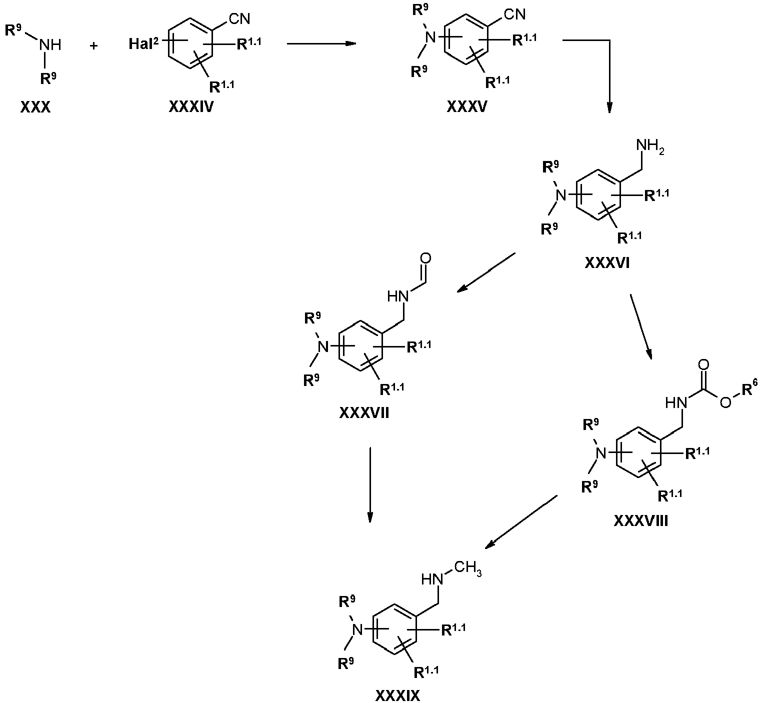

Die Umsetzung von Verbindungen der allgemeinen Formeln **XXX**, in der **R⁹** eine C₁₋₃-Alkylgruppe bedeutet, mit einer Verbindung der allgemeinen Formel **XXXIV**, in der **R^{1.1}** wie in den in Anspruch 1 genannten Verbindungen definiert ist und **Hal²** ein Fluor-, Chlor- oder Bromatom oder einen Rest **R⁹** bedeutet, zu Verbindungen mit der allgemeinen Formel **XXXV**, in der **R^{1.1}** wie in den in Anspruch 1 genannten Verbindungen definiert ist und **R⁹** eine C₁₋₃-Alkylgruppe bedeutet, wird wie unter Schema 8 beschrieben durchgeführt.

Die Reduktion eines Nitrils der allgemeinen Formel **XXXV** zu einem Amin der allgemeinen Formel **XXXVI**, in der **R^{1.1}** wie in den in Anspruch 1 genannten Verbindungen definiert ist und **R⁹** eine C₁₋₃-Alkylgruppe bedeutet, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungmittel wie Tetrahydrofuran, gegebenenfalls in Gegenwart von Aluminiumchlorid, durchgeführt werden.

Die Formylierung eines Amins der allgemeinen Formel **XXXVI** zu einer Verbindung der allgemeinen Formel **XXXVII**, in der **R^{1.1}** wie in den in Anspruch 1 genannten Verbindungen definiert ist und **R⁹** eine C₁₋₃-Alkylgruppe bedeutet, erfolgt zweckmäßigerweise in einem Lösungsmittel wie Dichlormethan, beispielsweise bei Temperaturen von 40°C bis 70°C und in Gegenwart von Essigsäureanhydrid und Ameisensäure.

Die Carbamatbildung zu Verbindungen der allgemeinen Formel **XXXVIII**, in der **R^{1.1}** wie in den in Anspruch 1 genannten Verbindungen definiert ist, **R⁶** eine C_{1.6}-Alkyl- und **R⁹** eine C₁₋₃-Alkylgruppe bedeutet, wird nach bekannten Verfahren durchgeführt, beispielsweise mit einem Chlorameisensäureester oder Boc-Anhydrid in Gegenwart einer Base wie Triethylamin oder Natronlauge und einem Lösungsmittel wie THF oder Dioxan.

Die Reduktion des Formyls bzw. des Carbamates zu Verbindungen der allgemeinen Formel **XXXIX**, in der **R^{1.1}** wie in den in Anspruch 1 genannten Verbindungen definiert ist und **R⁹** eine C₁₋₃-Alkylgruppe bedeutet, erfolgt unter Standard-Reaktionsbedingungen, vorzugsweise mit einem Reduktionsmittel wie Lithiumaluminiumhydrid und in einem Lösungsmittel wie Tetrahydrofuran bei einer Temperatur von 50°C bis 100°C.

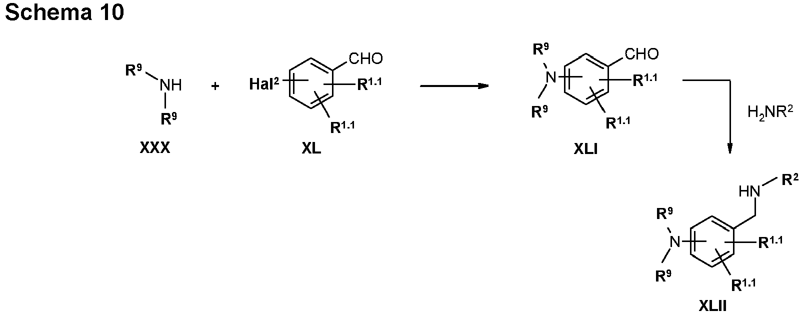

Der Halogen-Stickstoff-Austausch in Verbindungen der allgemeinen Formeln **XXX**, in der **R⁹** eine C₁₋₃-Alkylgruppe bedeutet, und **XL**, in der **R^{1.1}** wie in den in Anspruch 1 genannten Verbindungen definiert ist und **Hal²** ein Fluor-, Chlor- oder Bromatom oder einen Rest **R⁹** bedeutet, zur Herstellung von Verbindungen der allgemeinen Formel **XLI**, in der **R^{1.1}** wie in den in Anspruch 1 genannten Verbindungen definiert ist und **R⁹** eine C₁₋₃-Alkylgruppe bedeutet, wird wie unter Schema 8 beschrieben durchgeführt.

Die Reaktion von Benzaldehyden der allgemeinen Formel XLI, in der **R^{1.1}** wie in den in Anspruch 1 genannten Verbindungen definiert ist und **R⁹** eine C₁₋₃-Alkylgruppe bedeutet, mit einem Amin der allgemeinen Formel H₂N**R²,** in der **R²** wie in den in Anspruch 1 genannten Verbindungen definiert ist, zu einer Verbindung der allgemeinen Formel **XLII**, in der **R¹¹** und **R²** wie in den in Anspruch 1 genannten Verbindungen definiert sind und **R⁹** eine C₁₋₃-Alkylgruppe bedeutet, ist eine reduktive Aminierung. Sie erfolgt nach bekannten Verfahren, beispielsweise mit einem Reduktionsmittel wie Natriumtriacetoxyborhydrid, Natriumborhydrid oder Natriumcyanoborhydrid, zweckmäßigerweise in einem Lösungsmittel wie Tetrahydrofuran oder Dichlormethan, gegebenenfalls unter Zusatz von Essigsäure.

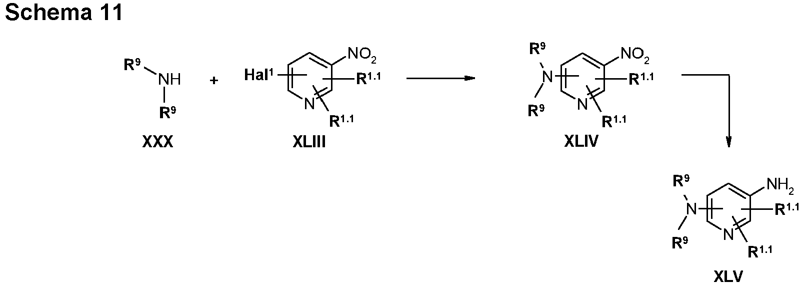

Die Reaktion eines Amins der allgemeinen Formel **XXX**, in der **R⁹** eine C₁₋₃-Alkylgruppe bedeutet, mit einem Halogen-nitropyridin der allgemeinen Formel **XLIII**, in der **R^{1.1}** wie in den in Anspruch 1 genannten Verbindungen definiert ist und **Hal¹** ein Chlor- oder Bromatom bedeutet, erfolgt nach bekannten Verfahren, beispielsweise in einem Lösungsmittel wie Tetrahydrofuran, Dichlormethan, Methanol oder DMSO und zweckmäßigerweise in Gegenwart einer passenden Base wie Triethylamin, Natronlauge oder Kaliumcarbonat und bei einer Temperatur von 20°C bis 100°C.

Die anschließende Reduktion der Nitrogruppe einer Verbindung der allgemeinen Formel **XLIV**, in der **R^{1.1}** wie in den in Anspruch 1 genannten Verbindungen definiert ist und **R⁹** eine C₁₋₃-Alkylgruppe bedeutet, zu Verbindungen der allgemeinen Formel **XLV**, in der **R^{1.1}** wie in den in Anspruch 1 genannten Verbindungen definiert ist und **R⁹** eine C₁₋₃-Alkylgruppe bedeutet, wird wie unter Schema 8 beschrieben durchgeführt.

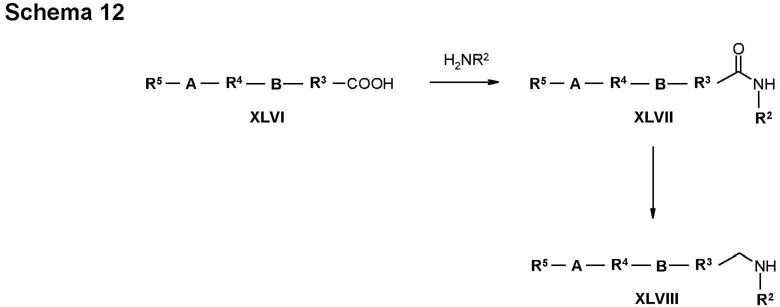

Die Amidknüpfung von Carbonsäuren der allgemeinen Formel **XLVI**, in der alle Reste wie in den in Anspruch 1 genannten Verbindungen definiert sind, und Aminen der allgemeinen Formel H₂N**R²,** in der **R²** wie in den in Anspruch 1 genannten Verbindungen definiert ist, zu Carbonsäureamiden der allgemeinen Formel **XLVII**, in der alle Reste wie in den in Anspruch 1 genannten Verbindungen definiert sind, wird wie unter Schema 1 beschrieben durchgeführt.

Die Reduktion von Carbonsäureamiden der allgemeinen Formel **XLVII** zu Aminen der allgemeinen Formel **XLVIII**, in der alle Reste wie in den in Anspruch 1 genannten Verbindungen definiert sind, erfolgt nach Standard-Reaktionsbedingungen, vorzugsweise in Gegenwart eines Reduktionsmittels wie Lithiumaluminiumhydrid und einem Lösungsmittel wie Tetrahydrofuran bei 40°C bis 100°C.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.

Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(I) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin.
Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblockerwie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirlukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207.
NK1 und NK2 Antagonisten wie z.B. DA-5018, R-116301, CP-728663, ZD-2249.
NMDA Antagonisten wie z.B. NER-MD-11, CNS-5161, EAA-090, AZ-756, CNP-3381. Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser.

Soweit nicht anders vermerkt sind die bei den Aufarbeitungen der Reaktionslösungen verwendeten Säure-, Basen- und Salzlösungen wässrige Systeme der angegebenen Konzentrationen.

Zu chromatographischen Reinigungen wird Kieselgel der Firma Millipore (MATREX*^{™}*, 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:

| | |
|---|---|
| CDI | 1,1'-Carbonyldiimidazol |
| DC | Dünnschichtchromatogramm |
| DIPEA | Diisopropylethylamin |
| DMAP | 4-Dimethylaminopyridin |
| DMF | Dimethylformamid |
| DMSO | Dimethylsulfoxid |
| HATU | *O*-(7-Azabenzotriazol-1-yl)-*N,N,N',N'*-tetramethyluroniumhexafluorphosphat |
| *tert* | tertiär |
| TBTU | 2-(1*H*-Benzotriazol-1-yl)-1,1,3,3-tetramethyluronium-tetrafluorborat |
| THF | Tetrahydrofuran |

Folgende analytische HPLC-Methoden wurden verwendet:
Methode 1:
Säule: XTerra^{™} MS C18, 2.5 µM, 4.6 x 30 mm
Detektion: 210 - 420 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 3.1 | 2.0 | 98.0 | 1.0 |
| 4.5 | 2.0 | 98.0 | 1.0 |
| 5.0 | 95.0 | 5.0 | 1.0 |

Methode 2:
Säule: Microsorb C18, 3 µM, 4.6 x 50 mm
Detektion: 220 - 320 nm
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 95.0 | 5.0 | 1.5 |
| 0.5 | 95.0 | 5.0 | 1.5 |
| 3.8 | 2.0 | 98.0 | 1.5 |
| 4.3 | 2.0 | 98.0 | 1.5 |
| 4.35 | 95.0 | 5.0 | 1.5 |
| 4.6 | 95.0 | 5.0 | 1.5 |

Methode 3:
Säule: XTerra^{™} MS C18, 3.5 µM, 4.6 x 50 mm
Detektion: 210 - 420 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 7.1 | 2.0 | 98.0 | 1.0 |
| 7.9 | 2.0 | 98.0 | 1.0 |
| 8.0 | 95.0 | 5.0 | 1.0 |

Methode 4:
Säule: Zorbax Stable Bond C18, 3.5 µM, 4.6 x 75 mm
Detektion: 230 - 360 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

Methode 5:
Säule: Interchim Strategy C18, 5 µM, 4.6 x 50 mm
Detektion: 220 - 320 nm
Fließmittel A: Wasser / 0.1 % TFA
Fließmittel B: Acetonitril
Gradient:

| Zeit in min | %A | %B | Flussrate in mL/min |
|---|---|---|---|
| 0.0 | 95.0 | 5.0 | 3.0 |
| 0.3 | 95.0 | 5.0 | 3.0 |
| 2.0 | 2.0 | 98.0 | 3.0 |
| 2.4 | 2.0 | 98.0 | 3.0 |
| 2.45 | 95.0 | 5.0 | 3.0 |
| 2.8 | 95.0 | 5.0 | 3.0 |

Methode 6:
Säule: Merck Cromolith Speed ROD RP18e, 4.6 x 50 mm
Detektion: 190 - 400 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 90.0 | 10.0 | 1.5 |

Methode 7:
Säule: Waters SunFire C18, 3.5 µM, 4.6 x 50 mm
Detektion: 210 - 500 nm
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 95.0 | 5.0 | 1.5 |
| 2.0 | 2.0 | 98.0 | 1.5 |
| 3.0 | 2.0 | 98.0 | 1.5 |
| 3.4 | 95.0 | 5.0 | 1.5 |

Methode 8:
Säule: Waters XBridge C18, 3.5 µM, 4.6 x 50 mm
Detektion: 210 - 500 nm
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 5.1 | 2.0 | 98.0 | 1.0 |
| 6.5 | 2.0 | 98.0 | 1.0 |
| 7.0 | 95.0 | 5.0 | 1.0 |

Methode 9:
Säule: Merck Chromolith^{™} Flash RP18e, 4.6 x 25 mm
Detektion: 190 - 400 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

Methode 10:
Säule: Merck Chromolith^{™} Flash RP18e, 4.6 x 25 mm
Detektion: 210 - 400 nm
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 95.0 | 5.0 | 2.5 |
| 0.2 | 95.0 | 5.0 | 2.5 |
| 1.5 | 2.0 | 98.0 | 2.5 |
| 1.7 | 2.0 | 98.0 | 2.5 |
| 1.9 | 95.0 | 5.0 | 2.5 |
| 2.2 | 95.0 | 5.0 | 2.5 |

Methode 11:
Säule: Waters XBridge C18, 3.5 µM, 4.6 x 50 mm
Detektion: 210 - 500 nm
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 3.0 | 0.0 | 100.0 | 1.5 |
| 3.4 | 95.0 | 5.0 | 1.5 |

Methode 12:
Säule: YMC-Pack ODS-AQ, 3.0 µM, 4.6 x 75 mm
Detektion: 230 - 360 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.6 |
| 4.5 | 10.0 | 90.0 | 1.6 |
| 5.0 | 10.0 | 90.0 | 1.6 |
| 5.5 | 90.0 | 10.0 | 1.6 |

Methode 13:
Säule: Varian C18 Pursuit XRs, 10 µM, 50 x 250 mm
Detektion: UV gesteuert
Fließmittel A: Wasser / 0.2 % Ammoniak
Fließmittel B: Acetonitril
Gradient:

| Zeit in min | %A | %B | Flussrate in mL/min |
|---|---|---|---|
| 0.0 | 90.0 | 10.0 | 100.00 |
| 1.0 | 90.0 | 10.0 | 100.00 |
| 1.5 | 90.0 | 10.0 | 180.00 |
| 13.0 | 0 | 100.0 | 180.00 |
| 15.5 | 0 | 100.0 | 180.00 |
| 15.75 | 90.0 | 10.0 | 180.00 |
| 19.0 | 90.0 | 10.0 | 180.00 |

Methode 14:
Säule: Waters Xbridge C18, 2.5 µM, 3.0 x 30 mm
Detektion: 230 - 360 nm
Fließmittel A: Wasser / 0.1 % Ammoniak
Fließmittel B: Acetonitril / 0.1 % Ammoniak
Gradient:

| Zeit in min | %A | %B | Flussrate in mL/min |
|---|---|---|---|
| 0.0 | 95.0 | 5.0 | 1.4 |
| 1.6 | 50.0 | 5.0 | 1.4 |
| 1.8 | 10.0 | 90.0 | 1.4 |
| 2.0 | 10.0 | 90.0 | 1.4 |
| 2.2 | 95 | 10 | 1.4 |

Folgende Mikrowellen-Apparatur wurde verwendet: Biotage EmrysOptimizer^{™}

### Herstellung der Endverbindungen

### Beispiel 1 (Referenzbeispiel)

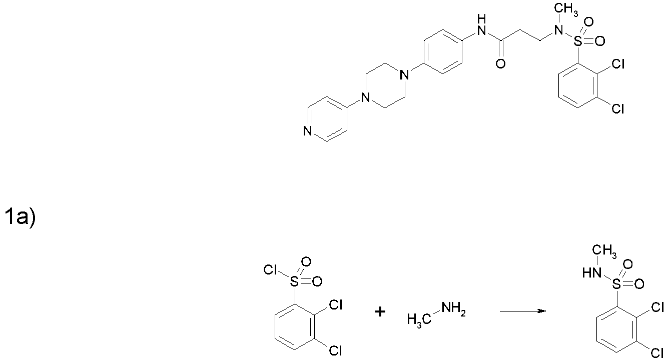

Eine Mischung aus 1.0 g (4.07 mMol) 2,3-Dichlorbenzolsulfonsäurechlorid, 0.33 g (4.89 mMol) Methylamin Hydrochlorid, 2.73 ml (19.55 mMol) Triethylamin und 20 ml Dichlormethan wird über Nacht bei Raumtemperatur gerührt. Die Reaktionsmischung wird anschließend je einmal mit 1 N HCl, gesättigter Natriumhydrogencarbonat-Lösung, Wasser und gesättigter Natriumchloridlösung gewaschen, über Natriumsulfat getrocknet und dann bis zur Trockne eingeengt.
C₇H₇Cl₂NO₂S (240.11)
[M+H]+ = 240/242/244
DC: Kieselgel, Petrolether / Ethylacetat 2:1, Rf-Wert = 0.50

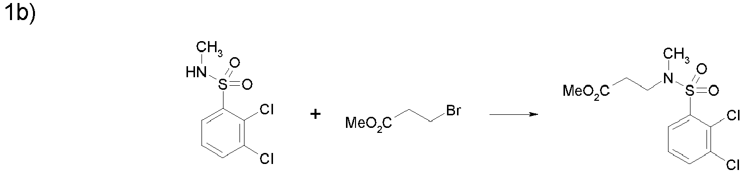

Eine Mischung aus 0.9 g (3.75 mMol) Produkt aus 1a und 20 ml DMF wird vorgelegt und mit 1.55 g (11.24 mMol) Kaliumcarbonat und 0.49 ml (4.50 mMol) 3-Brompropionsäureethylester versetzt. Die Reaktionsmischung wird über Nacht bei Raumtemperatur gerührt und dann mit Wasser versetzt. Man extrahiert zweimal mit Ethylacetat. Die organischen Extrakte werden dreimal mit Wasser und einmal mit gesättigter Natriumchloridlösung gewaschen, über Natriumsulfat getrocknet und zur Trockne eingeengt.
C₁₁H₁₃Cl₂NO₄S (326.20)
[M+H]+ = 326/328/330
DC: Kieselgel, Petrolether / Ethylacetat 2:1, Rf-Wert = 0.45

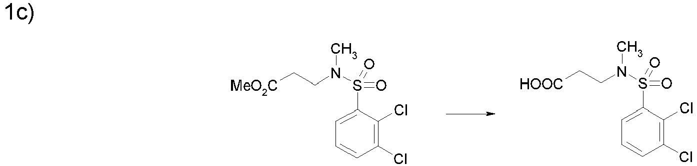

Eine Mischung aus 1.15 g (3.53 mMol) Produkt aus 1b, 0.74 g (17.63 mMol) Lithiumhydroxid Monohydrat, 15 ml THF und 15 ml Wasser wird eine Stunde bei Raumtemperatur gerührt. Danach wird das THF im Vakuum entfernt und der Rückstand mit konzentrierter HCl sauer gestellt. Die Reaktionsmischung wird anschließend dreimal mit Ethylacetat extrahiert. Die organischen Extrakte werden mit gesättigter Natriumchloridlösung gewaschen, über Natriumsulfat getrocknet und zur Trockne eingeengt. Das Rohprodukt wird mit Diethylether verrieben und abgesaugt.
C₁₀H₁₁Cl₂NO₄S (312.17)
[M+H]+ = 310/312/314
DC: Kieselgel, Petrolether / Ethylacetat 2:1, Rf-Wert = 0.03

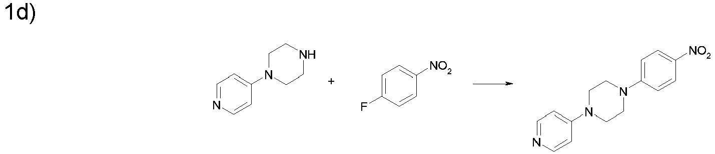

Eine Mischung aus 5.0 g (30.63 mMol) 1-Pyridin-4-yl-piperazin, 4.32 g (30.63 mMol) 1-Fluor-4-nitrobenzol (Aldrich), 10.62 ml (76.59 mMol) Triethylamin und 100 ml DMF wird 50 min unter Rückfluß erhitzt und anschließend bis zur Trockene eingeengt. Das so erhaltene Rohprodukt wird durch Säulenchromatographie über Kieselgel (Elutionsmittel: Dichlormethan / Ethanol / Ammoniak 12:1:0.1 bis 10:1:0.1) gereinigt.
C₁₅H₁₆N₄O₂ (284.31)
[M+H]+ = 285
DC: Kieselgel, Dichlormethan / Methanol / Ammoniak 9:1:0.1, Rf-Wert = 0.52

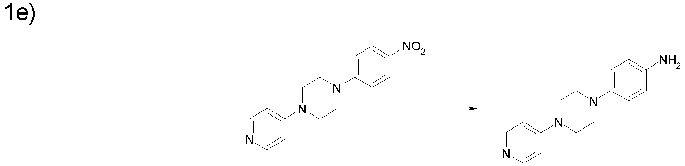

Eine Mischung aus 4.95 g (17.41 mMol) Produkt aus 1d, 0.6 g Palladium auf Kohle (10%), 120 ml Dichlormethan und 20 ml Methanol wird fünf Stunden im Autoklaven bei Raumtemperatur hydriert. Anschließend wird abgesaugt und der Filterkuchen noch sechsmal mit Dichlormethan / Methanol 1:1 aufgekocht und wieder abgesaugt. Die vereinigten Filtrate werden im Vakuum bis zur Trockene eingeengt.
C₁₅H₁₈N₄ (254.33)
[M+H]+ = 255

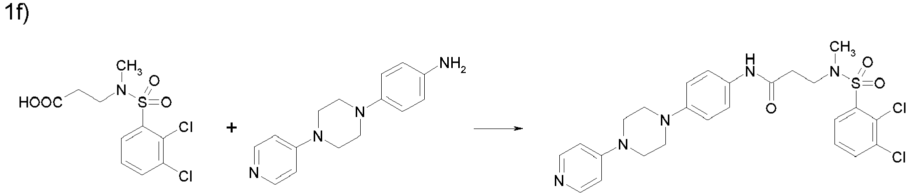

Eine Mischung aus 1.25 g (4.00 mMol) Produkt aus 1c, 2.0 ml (14.34 mMol) Triethylamin, 1.28 g (4.00 mMol) TBTU und 7 ml DMF wird 45 min bei Raumtemperatur gerührt. Danach wird 1.0 g (3.93 mMol) Produkt aus 1e hinzugefügt und über Nacht weiter bei Raumtemperatur gerührt. Anschließend wird die Reaktionsmischung in Wasser geschüttet und mit Dichlormethan extrahiert. Die organischen Extrakte werden mit Wasser gewaschen, über Na₂SO₄ getrocknet und bis zur Trockene eingeengt. Das so erhaltene Rohprodukt wurde durch Säulenchromatographie über Kieselgel (Elutionsmittel: Dichlormethan mit 5-20 % Methanol) gereinigt.
C₂₅H₂₇CI₂N₅O₃S (548.49)
[M+H]+ = 548/550/552
DC: Kieselgel, Dichlormethan / Methanol 4:1, Rf-Wert = 0.65

### Beispiel 2 (Referenzbeispiel)

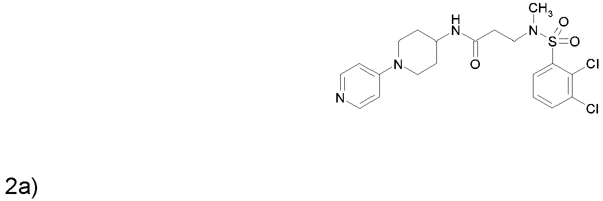

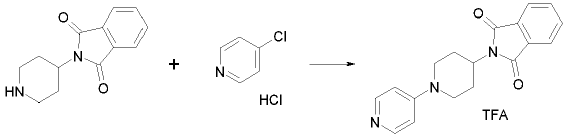

Eine Mischung aus 0.5 g (2.17 mMol) *N*-(1-Benzylpiperidin-4-yl)-phthalimid (Bioorg. Med. Chem. Lett. 11, 2001, 2325-2330), 0.33 g (2.17 mMol) 4-Chlorpyridin Hydrochlorid, 1.2 ml (8.69 mMol) Triethylamin und 2.4 ml Ethanol absolut wird eine Stunde in der Mikrowelle auf 150°C erhitzt. Die Reaktionsmischung wird anschließend mit Ethanol verdünnt, der entstandene Niederschlag wird abfiltriert. Man engt das Filtrat bis zur Trockene ein und reinigt das Rohprodukt durch präparative HPLC.
C₁₈H₁₇N₃O₂ x C₂HF₃O₂ (421.37)
[M+H]+ = 308
HPLC (Methode 1): Retentionszeit = 2.07 min

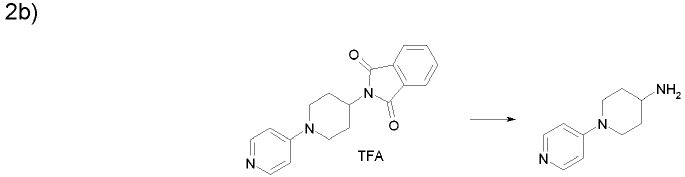

Eine Mischung aus 0.3 g (0.71 mMol) Produkt aus 2a, 0.09 g (1.42 mMol) Hydrazinhydrat 80%ig und 6 ml Ethanol absolut wird vier Stunden unter Rückfluß erhitzt. Die Reaktionsmischung wird anschließend auf 0°C abgekühlt, der entstandene Niederschlag wird abfiltriert. Man engt das Filtrat bis zur Trockene ein.
C₁₀H₁₅N₃ (177.25)
[M+H]+ = 178

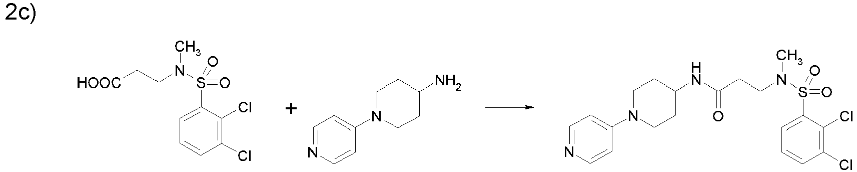

Beispiel 2 wird analog zu 1f aus 0.22 g (0.71 mMol) Produkt aus 1c, 0.24 g (1.35 mMol) Produkt aus 2b, 0.3 ml (2.13 mMol) Triethylamin und 0.23 g (0.71 mMol) TBTU in 5.5 ml DMF hergestellt.
C₂₀H₂₄Cl₂N₄O₃S (471.40)
[M+H]+ = 471/473/475
DC: Kieselgel, Dichlormethan / Methanol 9:1, Rf-Wert = 0.2

### Beispiel 3 (Referenzbeispiel)

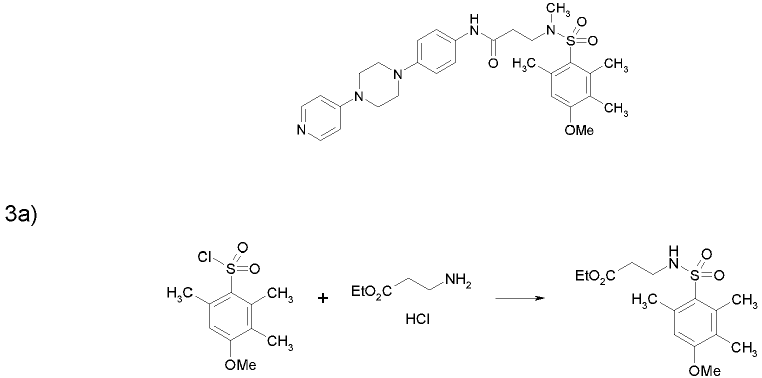

Eine Mischung aus 0.99 g (4.00 mMol) 4-Methoxy-2,3,6-trimethyl-benzolsulfonylchlorid, 0.69 g (4.51 mMol) β-Alaninethylester Hydrochlorid, 2.23 ml (15.98 mMol) Triethylamin und 20 ml Dichlormethan wird über Nacht bei Raumtemperatur gerührt. Die Reaktionsmischung wird anschließend mit 0.5 M HCl, gesättigter Natriumhydrogencarbonatlösung, Wasser und gesättigter Natrium-chloridlösung gewaschen, über Natriumsulfat getrocknet und im Vakuum bis zur Trockene eingeengt.
C₁₅H₂₃NO₅S (329.41)
[M+H]+ = 330
DC: Kieselgel, Petrolether / Ethylacetat 2:1, Rf-Wert = 0.43

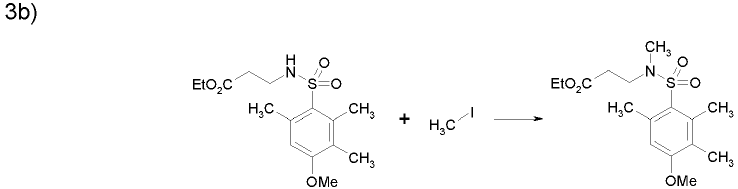

Eine Mischung aus 1.24 g (3.76 mMol) Produkt aus 3a, 0.84 ml (13.55 mMol) Methyliodid, 1.04 g (7.53 mMol) Kaliumcarbonat wasserfrei und 10 ml DMF wird fünf Stunden bei Raumtemperatur gerührt. Die Reaktionsmischung wird anschließend im Vakuum bis zur Trockene eingeengt, der Rückstand wird in Ethylacetat aufgenommen. Man wäscht mit Wasser, gesättigter Natriumhydrogencarbonatlösung und gesättigter Natriumchloridlösung, trocknet über Natriumsulfat und engt im Vakuum bis zur Trockene ein.
C₁₆H₂₅NO₅S (343.44)
[M+H]+ = 344
DC: Kieselgel, Petrolether / Ethylacetat 2:1, Rf-Wert = 0.52

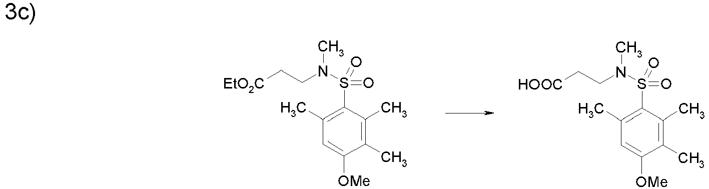

Die Säure wird analog zu 1 c aus 1.29 g (3.76 mMol) Produkt aus 3b, 0.79 g (18.80 mMol) Lithiumhydroxid Monohydrat, 15 ml THF und 15 ml Wasser hergestellt.
C₁₄H₂₁NO₅S (315.39)
[M+H]+ = 316
DC: Kieselgel, Petrolether / Ethylacetat 2:1, Rf-Wert = 0.07

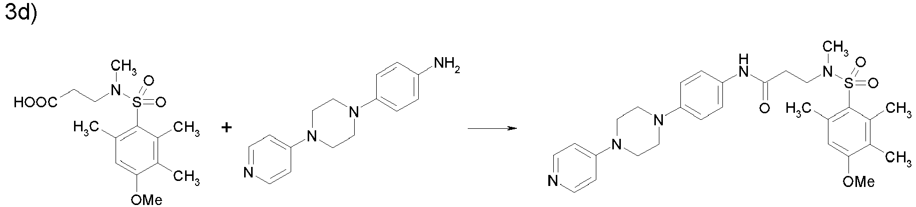

Beispiel 3 wird analog zu 1f aus 0.15 g (0.47 mMol) Produkt aus 3c, 0.12 g (0.47 mMol) Produkt aus 1e, 0.2 ml (1.43 mMol) Triethylamin und 0.15 g (0.48 mMol) TBTU in 8 ml DMF hergestellt.
C₂₉H₃₇N₅O₄S (551.70)
[M+H]+ = 552
DC: Kieselgel, Dichlormethan / Methanol 9:1, Rf-Wert = 0.38

### Beispiel 4 (Referenzbeispiel)

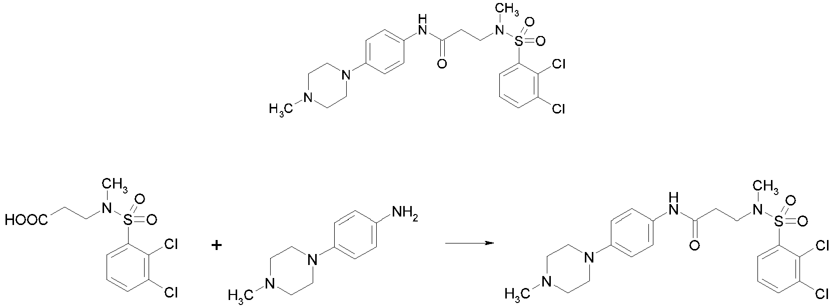

Beispiel 4 wird analog zu 1f aus 0.39 g (1.26 mMol) Produkt aus 1c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt.
C₂₁H₂₆CL₂N₄O₃S (485.43)
[M+H]+ = 485/487/489
HPLC (Methode 2): Retentionszeit = 2.64 min

### Beispiel 5 (Referenzbeispiel)

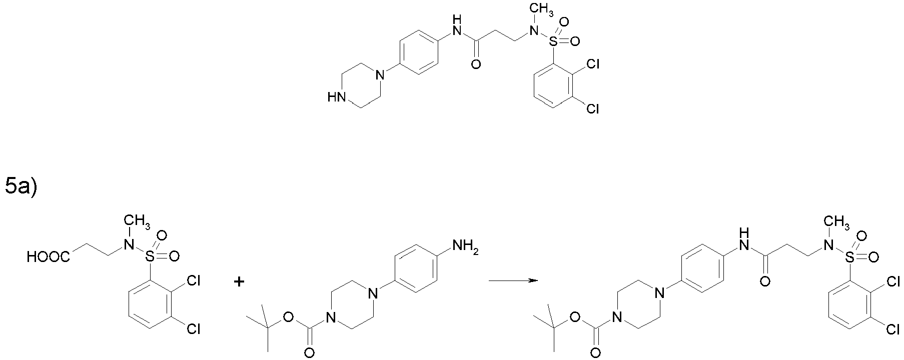

5a wird analog zu 1f aus 0.39 g (1.26 mMol) Produkt aus 1c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt. C₂₅H₃₂CL₂N₄O₅S (571.52)

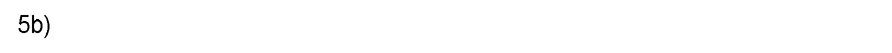

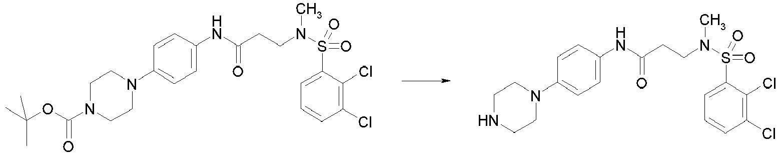

Eine Mischung aus 0.60 g (1.05 mMol) Produkt aus 5a, 3 ml TFA und 3 ml Dichlormethan wird zwei Stunden bei Raumtemperatur gerührt. Man engt die Reaktionsmischung bis zur Trockene ein und reinigt das Rohprodukt durch präparative HPLC.
C₂₀H₂₄Cl₂N₄O₃S (471.40)
[M+H]+ = 471/473/475
HPLC (Methode 2): Retentionszeit = 2.58 min

### Beispiel 6 (Referenzbeispiel)

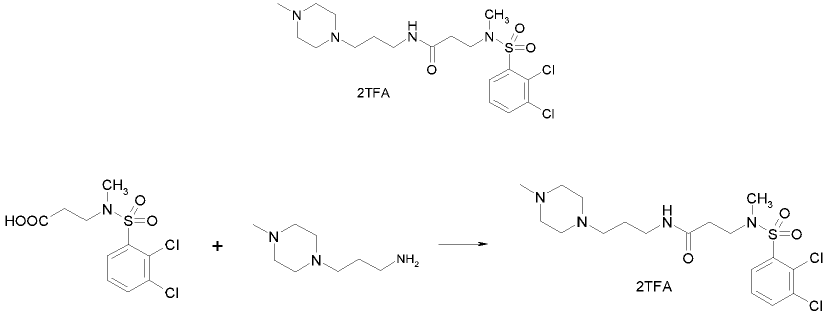

Beispiel 6 wird analog zu 1f aus 0.22 g (0.71 mMol) Produkt aus 1c, 0.12 g (0.78 mMol) 3-(4-Methylpiperazin-1-yl)-propylamin (Bioorg. Med. Chem. Lett. 13, 2003, 2131-2136), 0.30 ml (2.13 mMol) Triethylamin und 0.23 g (0.71 mMol) TBTU in 5.5 ml THF hergestellt.
C₁₈H₂₈Cl₂N₄O₃S x 2C₂HF₃O₂ (679.46)
[M+H]+ = 451/453/455
HPLC (Methode 5): Retentionszeit = 1.37 min

### Beispiel 7 (Referenzbeispiel)

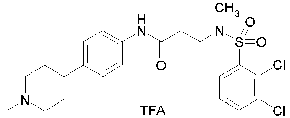

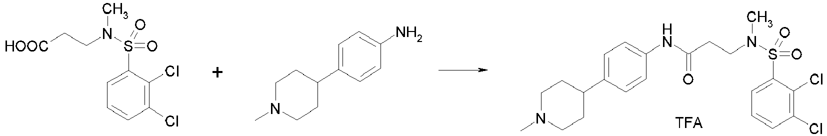

Beispiel 7 wird analog zu 1f aus 0.22 g (0.71 mMol) Produkt aus 1c, 0.14 g (0.71 mMol) 4-(1-Methylpiperidin-4-yl)-anilin (JW Pharmlab), 0.30 ml (2.13 mMol) Triethylamin und 0.23 g (0.71 mMol) TBTU in 5.5 ml THF hergestellt.
C₂₂H₂₇Cl₂N₃O₃S x C₂HF₃O₂ (598.46)
[M+H]+ = 484/486/488
HPLC (Methode 5): Retentionszeit = 1.57 min

### Beispiel 8 (Referenzbeispiel)

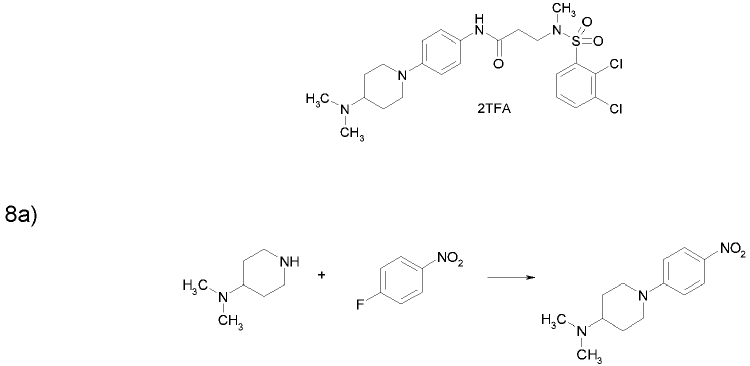

8a wird analog zu 1d aus 0.5 g (3.90 mMol) 4-Dimethylamino-piperidin (Alfa Aesar), 0.44 g (4.18 mMol) 1-Fluor-4-nitrobenzol (Aldrich) und 1.33 ml (76.59 mMol) Triethylamin in 12 ml DMF hergestellt.
C₁₃H₁₉N₃O₂ (249.31)
[M+H]+ = 250

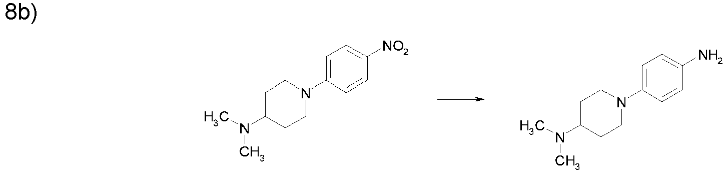

Eine Mischung aus 1.66 g (6.67 mMol) Produkt aus 8a, 0.17 g Palladium auf Kohle (5%) und 132 ml Ethanol wird im Autoklaven bei Raumtemperatur hydriert. Anschließend wird der Katalysator abgesaugt und das Filtrat im Vakuum bis zur Trockene eingeengt.
C₁₃H₂₁N₃ (219.33)
[M+H]+ = 220
DC: Kieselgel, Dichlormethan / Methanol 9:1, Rf-Wert = 0.1

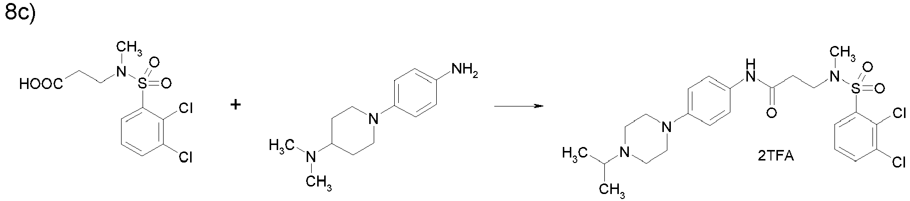

Beispiel 8 wird analog zu 1f aus 0.22 g (0.71 mMol) Produkt aus 1c, 0.16 g (0.71 mMol) Produkt aus 8b, 0.30 ml (2.13 mMol) Triethylamin und 0.23 g (0.71 mMol) TBTU in 5.5 ml THF hergestellt.
C₂₃H₃₀CI₂N₄O₃S x 2C₂HF₃O₂ (741.53)
[M+H]+ = 513/515/517
HPLC (Methode 5): Retentionszeit = 1.46 min

### Beispiel 9 (Referenzbeispiel)

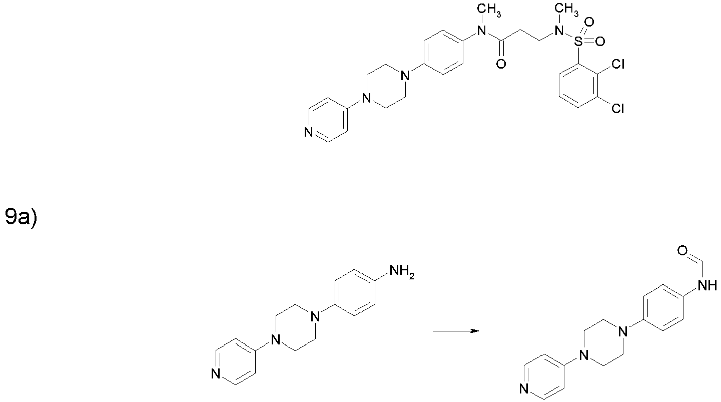

13 ml Essigsäureanhydrid werden vorgelegt und langsam mit 8 ml Ameisensäure versetzt. Die Reaktionsmischung wird 1.5 Stunden auf 50°C erhitzt und dann mit 80 ml Dichlormethan versetzt. Unter Eisbadkühlung werden anschließend 5.0 g (19.66 mMol) hinzugegeben. Es wird eine Stunde bei Raumtemperatur gerührt und danach bis zur Trockene eingeengt. Der Rückstand wird mit halbgesättigter Natriumhydrogencarbonat-Lösung versetzt und mit Dichlormethan zweimal extrahiert. Die organischen Extrakte werden mit gesättigter Natriumchlorid-Lösung gewaschen, über Natriumsulfat getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₁₈N₄O (282.34)
[M+H]+ = 283

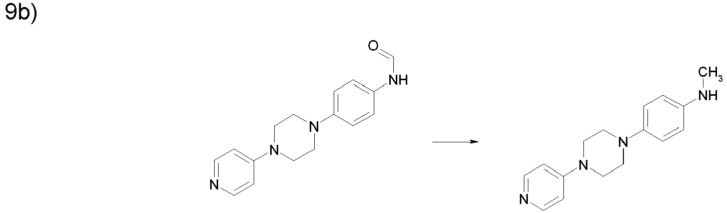

Bei 60°C wird eine Mischung aus 10.63 ml Lithiumaluminiumhydrid 2 M in THF (21.25 mMol) und 50 ml THF langsam mit 3.0 g (10.63 mMol) Produkt aus 9a versetzt. Die Reaktionsmischung wird acht Stunden bei 60°C und vier Stunden bei Raumtemperatur gerührt. Unter Eisbadkühlung werden anschließend 20 ml Wasser hinzugegeben. Es wird über Celite filtriert und mit THF und Dichlormethan nachgewaschen. Das Filtrat wird bis zur Trockene eingeengt. Der Rückstand wird mit Dichlormethan versetzt, mit Wasser und 1 M Natronlauge gewaschen, über Natriumsulfat-Lösung getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₂₀N₄ (268.36)

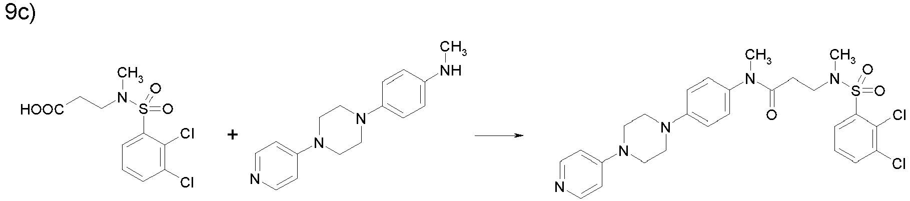

Beispiel 9 wird analog zu 1f aus 0.15 g (0.48 mMol) Produkt aus 1c, 0.14 g (0.51 mMol) Produkt aus 9b, 0.13 ml (0.96 mMol) Triethylamin und 0.19 g (0.51 mMol) HATU in 5 ml DMF hergestellt.
C₂₆H₂₉CI₂N₅O₃S (562.51)
[M+H]+ = 562/564/566
HPLC (Methode 2): Retentionszeit = 2.86 min

### Beispiel 10 (Referenzbeispiel)

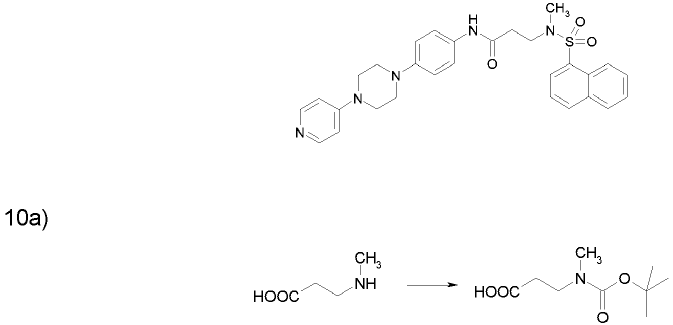

Eine Mischung aus 1.0 g (9.70 mMol) N-Methyl-β-alanin (Convertex), 24 ml Dioxan, 12 ml Wasser und 2.68 g (19.38 mMol) Kaliumcarbonat wasserfrei wird unter Eisbadkühlung mit 2.33 g (10.66 mMol) Boc-Anhydrid versetzt. Die Reaktionsmischung wird drei Tage bei Raumtemperatur gerührt. Danach wird das Dioxan im Vakuum entfernt. Der wässrige Rückstand wird mit Ethylacetat extrahiert (Ethylacetat-Phasen verwerfen), anschließend mit 1 M Salzsäure leicht sauer gestellt und dann mit Dichlormethan extrahiert. Die organischen Dichlormethan-Extrakte werden mit gesättigter Natriumchlorid-Lösung gewaschen, über Natriumsulfat getrocknet und bis zur Trockene eingeengt.
C₉H₁₇NO₄ (203.24)
[M+H]+ = 204

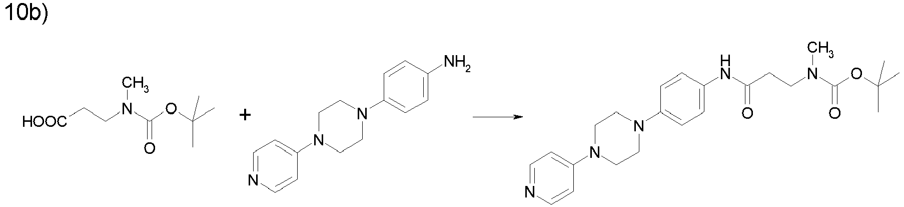

10b wird analog zu 1f aus 1.85 g (9.10 mMol) Produkt aus 10a, 2.32 g (9.10 mMol) Produkt aus 1e, 3.81 ml (27.31 mMol) Triethylamin und 2.92 g (9.10 mMol) TBTU in 80 ml DMF hergestellt.
C₂₄H₃₃N₅O₃ (439.55)
[M+H]+ = 440
DC: Kieselgel, Dichlormethan / Methanol 9:1, Rf-Wert = 0.49

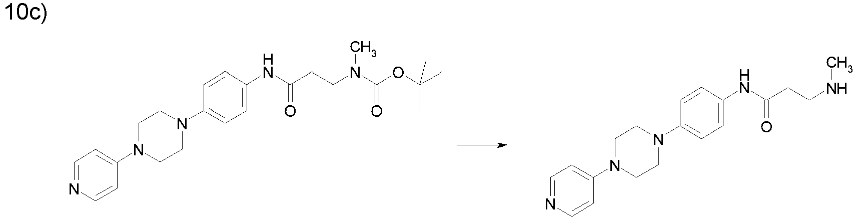

Eine Mischung aus 3.20 g (7.28 mMol) Produkt aus 10b, 20 ml TFA und 60 ml Dichlormethan wird 30 min bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 9:1:0.1 bis 4:1:0.1) gereinigt.
C₁₉H₂₅N₅O (339.43)
[M+H]+ = 340
DC: Kieselgel, Dichlormethan / Methanol / Ammoniak 9:1:0.1, Rf-Wert = 0.25

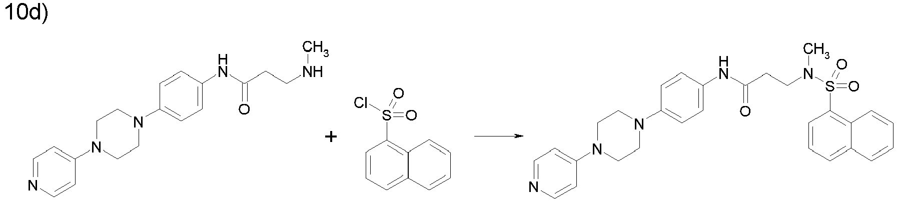

Eine Mischung aus 0.1 g (0.30 mMol) Produkt aus 10c, 0.056 g (0.25 mMol) 1-Naphthylsulfonsäurechlorid, 0.137 ml (0.98 mMol) Triethylamin und 5 ml Dichlormethan wird über Nacht bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 12:1:0.1) gereinigt.
C₂₉H₃₁N₅O₃S (529.65)
[M+H]+ = 530
DC: Kieselgel, Dichlormethan / Methanol / Ammoniak 9:1:0.1, Rf-Wert = 0.44

### Beispiel 11 (Referenzbeispiel)

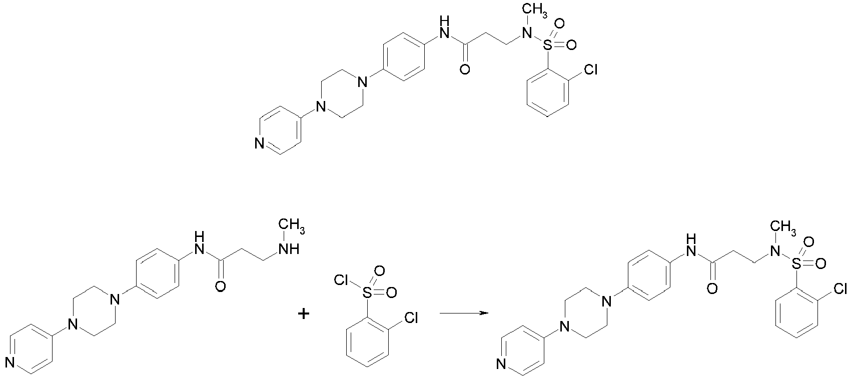

Beispiel 11 wird analog zu 10d aus 0.10 g (0.30 mMol) Produkt aus 10c, 0.052 g (0.25 mMol) 2-Chlorbenzolsulfonsäurechlorid, 0.14 ml (98 mMol) Triethylamin in 5 ml Dichlormethan hergestellt.
C₂₅H₂₈CIN₅O₃S (514.04)
[M+H]+ = 514/516
DC: Kieselgel, Dichlormethan / Methanol / Ammoniak 9:1:0.1, Rf-Wert = 0.47

### Beispiel 12 (Referenzbeispiel)

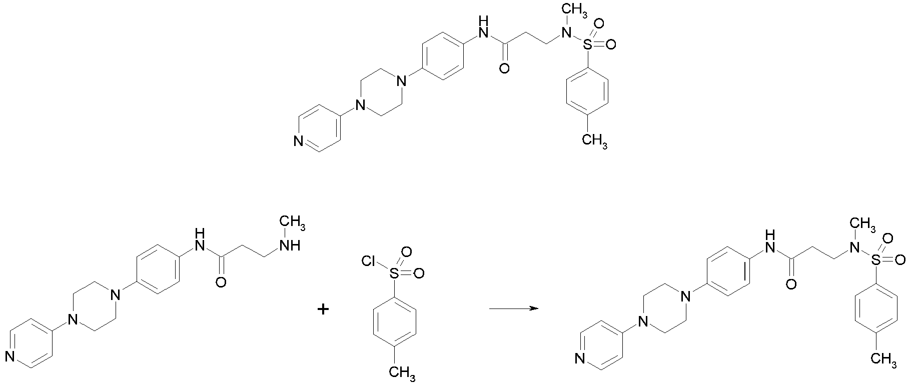

Beispiel 12 wird analog zu 10d aus 0.10 g (0.30 mMol) Produkt aus 10c, 0.047 g (0.25 mMol) p-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.43

### Beispiel 13 (Referenzbeispiel)

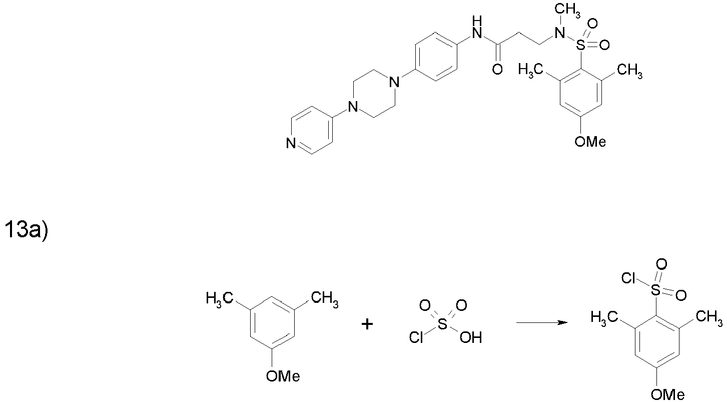

Eine Mischung aus 2.0 g (14.69 mMol) 3,5-Dimethylanisol und 20 ml Dichlormethan wird unter Eisbadkühlung mit 5.85 ml (88.0 mMol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.
C₉H₁₁ClO₃S (234.70)
[M+H]+ = 234/236
DC: Kieselgel, Petrolether / Ethylacetat 9:1, Rf-Wert = 0.46

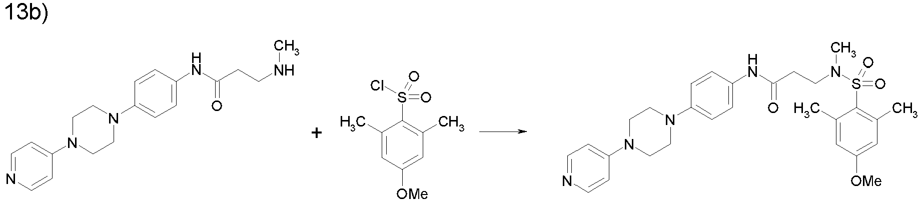

Beispiel 13 wird analog zu 10d aus 0.10 g (0.30 mMol) Produkt aus 10c, 0.058 g (0.25 mMol) Produkt aus 13a, 0.14 ml (98 mMol) Triethylamin in 5 ml Dichlormethan hergestellt.
C₂₈H₃₅N₅O₄S (537.67)
[M+H]+ = 538
DC: Kieselgel, Dichlormethan / Methanol / Amoniak 9:1:0.1, Rf-Wert = 0.62

### Beispiel 14 (Referenzbeispiel)

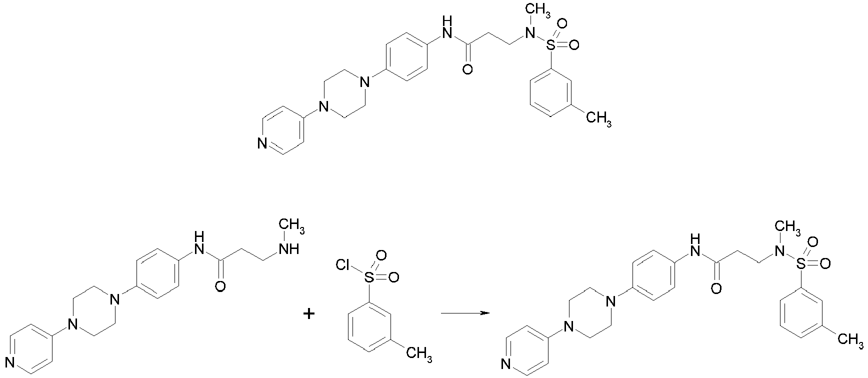

Beispiel 14 wird analog zu 10d aus 0.10 g (0.30 mMol) Produkt aus 10c, 0.047 g (0.25 mMol) m-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.47

### Beispiel 15 (Referenzbeispiel)

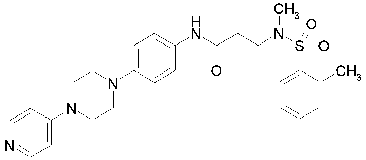

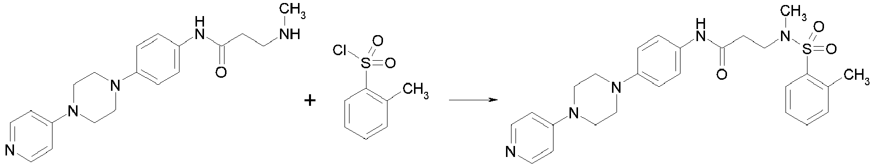

Beispiel 15 wird analog zu 10d aus 0.10 g (0.30 mMol) Produkt aus 10c, 0.047 g (0.25 mMol) o-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
HPLC (Methode 1): Retentionszeit = 2.37 min

### Beispiel 16 (Referenzbeispiel)

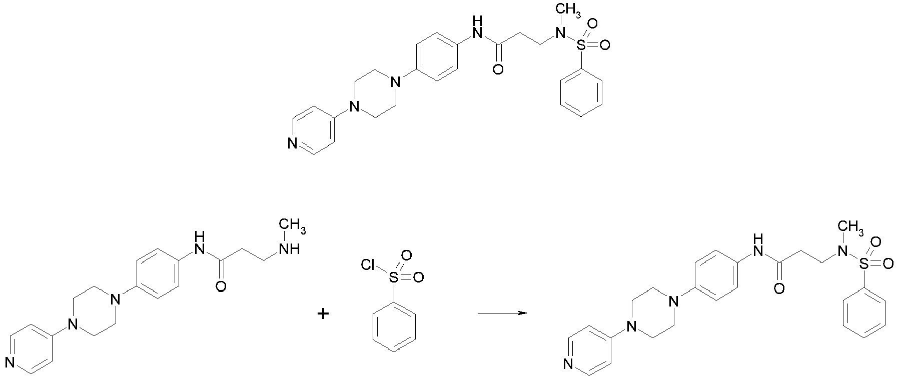

Beispiel 16 wird analog zu 10d aus 0.10 g (0.30 mMol) Produkt aus 10c, 0.043 g (0.25 mMol) Benzolsulfonsäurechlorid, 0.14 ml (98 mMol) Triethylamin in 5 ml Dichlormethan hergestellt.
C₂₅H₂₉N₅O₃S (479.60)
[M+H]+ = 480
HPLC (Methode 1): Retentionszeit = 2.40 min

### Beispiel 17 (Referenzbeispiel)

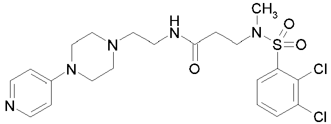

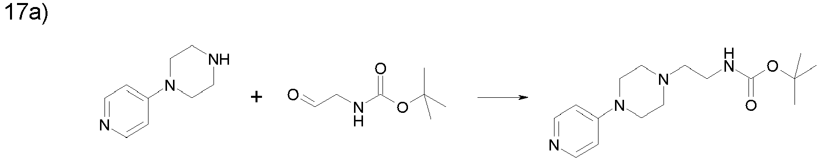

Eine Mischung aus 1.03 g (6.28 mMol) 1-(4-Pyridyl)-piperazin (Girindus) und 50 ml Dichlormethan wird mit 1.0 g (6.28 mMol) tert-Butyl-N-(2-oxoethyl)-carbamat (Aldrich) versetzt. Die Reaktionsmischung wird danach 30 min bei Raumtemperatur gerührt, anschließend unter Eisbadkühlung mit 2.66 g (12.56 mMol) Natrium-triacetoxyborhydrid versetzt und danach über Nacht bei Raumtemperatur gerührt. Man gibt weitere 60 ml Dichlormethan hinzu und wäscht die Reaktionsmischung mit gesättigter Natriumhydrogencarbonat-Lösung und gesättigter Natriumchlorid-Lösung. Die organische Phase wird über Natriumsulfat getrocknet und bis zur Trockene eingeengt. Das so erhaltene Rohprodukt wird durch Säulenchromatographie über Kieselgel (Elutionsmittel: Dichlormethan / Ethanol /Ammoniak 14:1:0.1 bis 10:1:0.1) gereinigt.
C₁₆H₂₆N₄O₂ (306.40)
[M+H]+ = 307

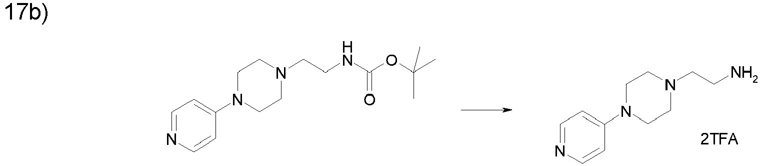

Eine Mischung aus 0.36 g (1.19 mMol) Produkt aus 17a, 1.19 ml (15.50 mMol) TFA und 2 ml Dichlormethan wird zwei Stunden bei Raumtemperatur gerührt. Danach wird die Reaktionsmischung im Vakuum bis zur Trockene eingeengt.
C₁₁H₁₈N₄x 2C₂HF₃O₂ (434.33)
[M+H]+ = 207
HPLC (Methode 2): Retentionszeit = 0.98 min

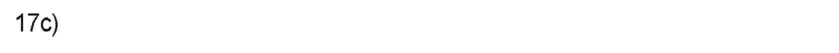

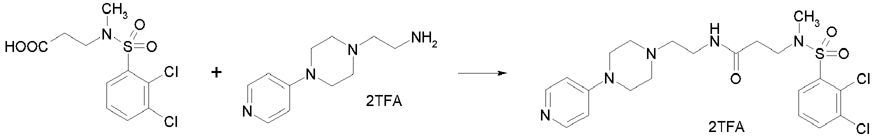

Beispiel 17 wird analog zu 1f aus 0.22 g (0.71 mMol) Produkt aus 1c, 0.34 g (0.78 mMol) Produkt aus 17b, 0.50 ml (3.56 mMol) Triethylamin und 0.23 g (0.71 mMol) TBTU in 5.5 ml THF hergestellt.
C₂₁H₂₇Cl₂N₅O₃S x 2C₂HF₃O₂ (728.49)
[M+H]+ = 500/502/504
HPLC (Methode 2): Retentionszeit = 3.14 min

### Beispiel 18 (Referenzbeispiel)

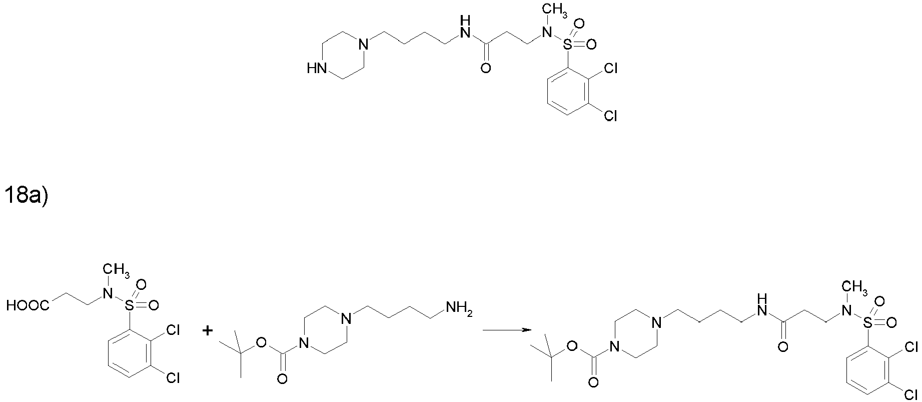

18a wird analog zu 1f aus 0.20 g (0.64 mMol) Produkt aus 1c, 0.17 g (0.64 mMol) 4-(4-Aminobutyl)-piperazin-1-carbonsäure-tert-butylester (J. Med. Chem. 47, 2004, 4300-4315), 0.27 ml (1.92 mMol) Triethylamin und 0.21 g (0.64 mMol) TBTU in 5 ml THF hergestellt.
C₂₃H₃₆Cl₂N₄O₅S (551.53)
M+H]+ = 551/553/555
DC: Kieselgel, Dichlormethan / Methanol 30:1, Rf-Wert = 0.1

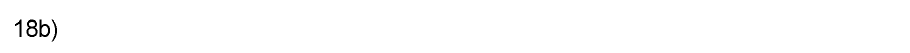

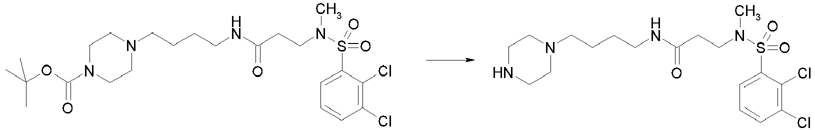

Eine Mischung aus 0.29 g (0.53 mMol) Produkt aus 18a, 0.53 ml TFA und 1 ml Dichlormethan wird zwei Stunden bei Raumtemperatur gerührt. Die Reaktionsmischung wird mit gesättigter Natriumhydrogencarbonat-Lösung gewaschen. Nach der Phasentrennung wird die wässrige Phase noch dreimal mit Dichlormethan extrahiert. Die organischen Extrakte werden über Natriumsulfat getrocknet und bis zur Trockene im Vakuum eingeengt.
C₁₈H₂₈Cl₂N₄O₃S (451.41)
[M+H]+ = 451/453/455
HPLC (Methode 2): Retentionszeit = 2.22 min

### Beispiel 19 (Referenzbeispiel)

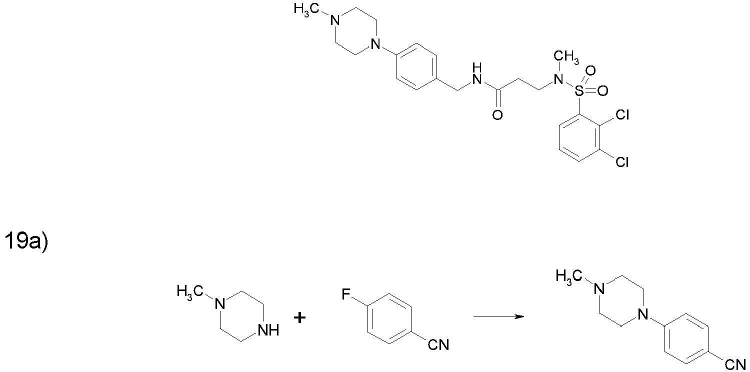

Eine Mischung aus 5.0 ml (45.13 mMol) *N*-Methylpiperazin und 0.73 g (6.00 mMol) 4-Fluorbenzonitril (Aldrich) wird 12 Stunden auf 80°C erhitzt. Danach wird bis zur Trockene eingeengt und der Rückstand mit Wasser versetzt. Es wird dreimal mit Ethylacetat extrahiert. Die organischen Extrakte werden über Natriumsulfat getrocknet und im Vakuum bis zur Trockene eingeengt.
C₁₂H₁₅N₃ (201.27)
M+H]+ = 202
DC: Kieselgel, Dichlormethan / Ethanol 95:5, Rf-Wert = 0.31

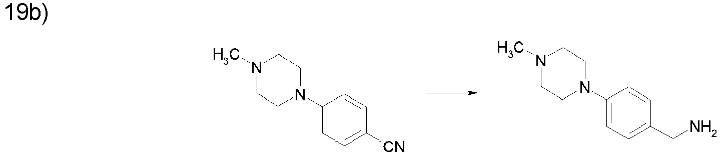

Eine Mischung aus 1.17 g (5.81 mMol) Produkt aus 19a, 0.3 g Raney-Nickel und 50 ml methanolische Ammoniaklösung wird bei 50°C im Autoklaven hydriert. Danach wird der Katalysator abfiltriert und das Filtrat im Vakuum bis zur Trockene eingeengt.
C₁₂H₁₉N₃ (205.30)
M+H]+ = 206

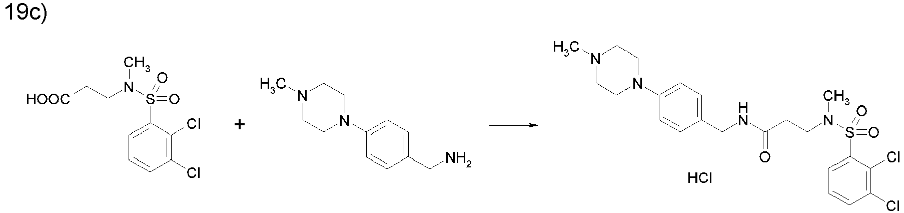

Beispiel 19 wird analog zu 1f aus 0.16 g (0.50 mMol) Produkt aus 1c, 0.10 g (0.50 mMol) Produkt aus 19b, 0.14 ml (1.00 mMol) Triethylamin und 0.16 g (0.50 mMol) TBTU in 3 ml DMF hergestellt.
C₂₂H₂₈Cl₂N₄O₃S x HCl (535.91)
[M+H]+ = 499/501/503
HPLC (Methode 3): Retentionszeit = 3.49 min

### Beispiel 20 (Referenzbeispiel)

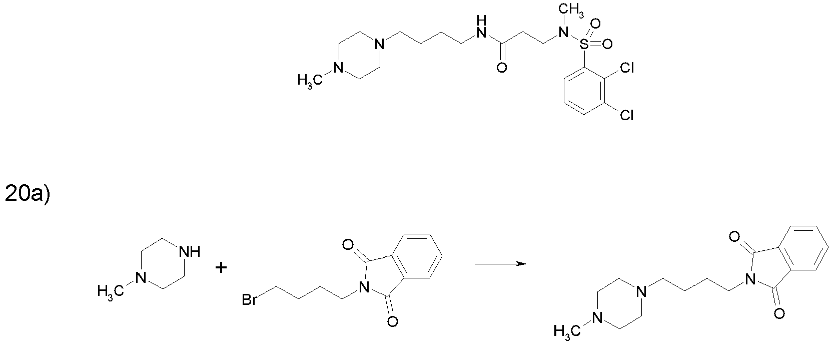

Eine Mischung aus 0.5 g (4.99 mMol) *N*-Methylpiperazin (Aldrich), 1.41 g (4.99 mMol) N-(4-Brombutyl)-phthalimid (Fluka), 0.86 ml (4.99 mMol) DIPEA und 9.3 ml Acetonitril wird 45 min in der Mikrowelle auf 120°C erhitzt. Danach wird bis zur Trockene eingeengt. Das so erhaltene Rohprodukt wird durch Säulenchromatographie über Kieselgel (Elutionsmittel: Dichlormethan / Methanol 98:2) gereinigt.
C₁₇H₂₃N₃O₂ (301.38)
M+H]+ = 302

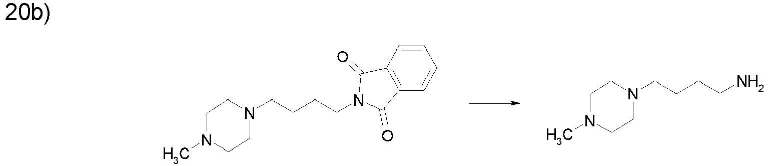

Eine Mischung aus 1.94 g (6.44 mMol) Produkt aus 20a, 1.61 g (25.75 mMol) Hydrazinhydrat Hydrat und 15 ml Ethanol absolut wird 5.5 Stunden im Autoklaven auf 120°C erhitzt. Der entstandene Niederschlag wird abfiltriert. Man engt danach das Filtrat bis zur Trockene ein.
C₉H₂₁N₃ (171.28)

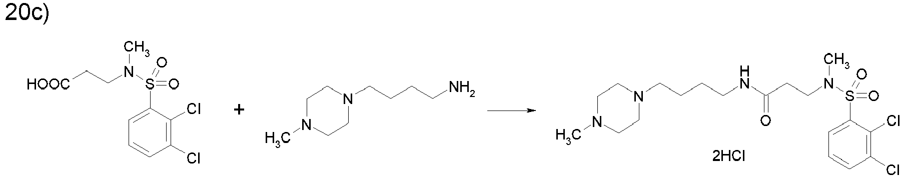

Beispiel 20 wird analog zu 1f aus 0.50 g (1.61 mMol) Produkt aus 1c, 0.55 g (3.22 mMol) Produkt aus 20b, 0.67 ml (4.83 mMol) Triethylamin und 0.52 g (1.61 mMol) TBTU in 30 ml DMF hergestellt.
C₁₉H₃₀Cl₂N₄O₃S x 2HCl (538.36)
[M+H]+ = 465/467/469
HPLC (Methode 1): Retentionszeit = 2.15 min

### Beispiel 21 (Referenzbeispiel)

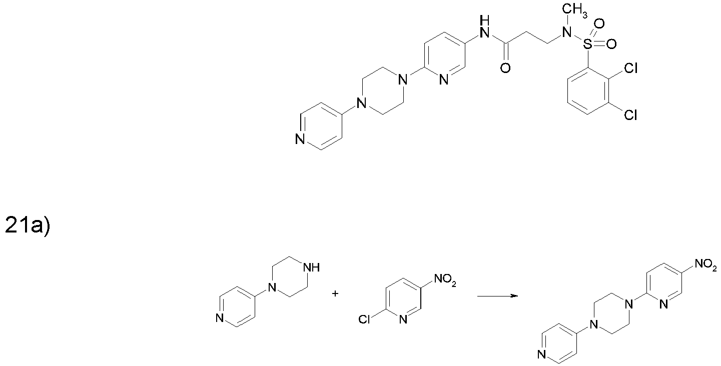

Eine Mischung aus 2.06 g (12.62 mMol) 1-Pyridin-4-yl-piperazin (Girindus), 2.00 g (12.62 mMol) 2-Chlor-5-nitropyridin (Fluka) und 50 ml Dichlormethan wird 15 min bei Raumtemperatur gerührt und anschließend mit 6.31 ml (12.62 mMol) 2 M Natronlauge versetzt. Die Reaktionsmischung wird 20 Stunden bei Raumtemperatur gerührt und danach mit 300 ml Dichlormethan und 100 ml 5%iger Natriumhydrogencarbonat-Lösung versetzt. Nach der Phasentrennung wird die organische Phase über Natriumsulfat getrocknet und im Vakuum bis zur Trockene eingeengt. Das Rohprodukt wird mit 100 ml Diethylether / Ethanol 2:1 verrührt, abfiltriert und getrocknet.
C₁₄H₁₅N₅O₂ (285.30)
[M+H]+ = 286
DC: Kieselgel, Dichlormethan / Ethanol 9:1, Rf-Wert = 0.10

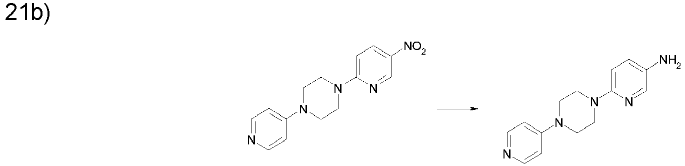

Eine Mischung aus 1.75 g (6.13 mMol) Produkt aus 21a, 0.4 g Palladium auf Kohle (10%), 100 ml Dichlormethan und 50 ml Methanol wird fünf Stunden im Autoklaven bei Raumtemperatur hydriert. Anschließend wird der Katalysator abgesaugt und das Filtrat im Vakuum bis zur Trockene eingeengt. Der Rückstand wird mit 100 ml Diethylether / Ethanol 2:1 verrührt und abgesaugt. Das so erhaltene Rohprodukt wird durch Säulenchromatographie über Kieselgel (Elutionsmittel: Dichlormethan / Methanol / Ammoniak 95:5:0.5) gereinigt.
C₁₄H₁₇N₅ (255.32)
[M+H]+ = 256
DC: Kieselgel, Dichlormethan / Methanol / Ammoniak 9:1:0.1, Rf-Wert = 0.37

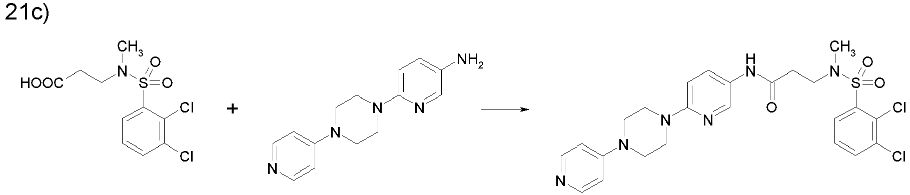

Beispiel 21 wird analog zu 1f aus 0.11 g (0.35 mMol) Produkt aus 1c, 0.089 g (0.35 mMol) Produkt aus 21b, 0.098 ml (0.70 mMol) Triethylamin und 0.13 g (0.42 mMol) TBTU in 15 ml THF hergestellt.
C₂₄H₂₆Cl₂N₆O₃S (549.47)
[M+H]+ = 549/551/553
HPLC (Methode 4): Retentionszeit = 2.7 min

### Beispiel 22 (Referenzbeispiel)

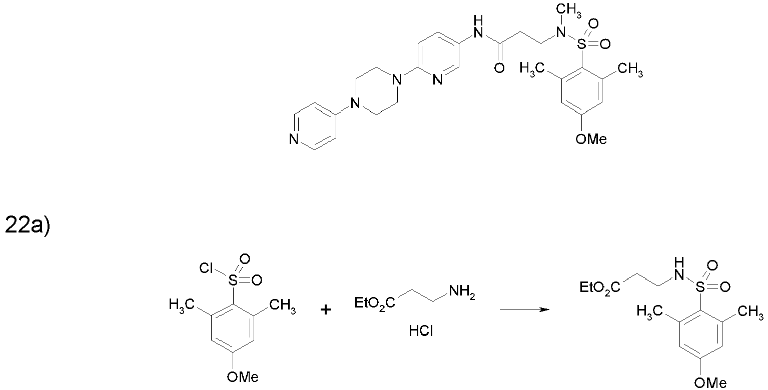

22a wird analog zu 3a aus 3.00 g (12.78 mMol) Produkt aus 13a, 2.16 g (14.06 mMol) ß-Alaninethylester Hydrochlorid, 7.13 ml (51.13 mMol) Triethylamin in 70 ml Dichlormethan hergestellt.
C₁₄H₂₁NO₅S (315.39)
[M+H]+ = 316
DC: Kieselgel, Petrolether / Ethylacetat 2:1, Rf-Wert = 0.23

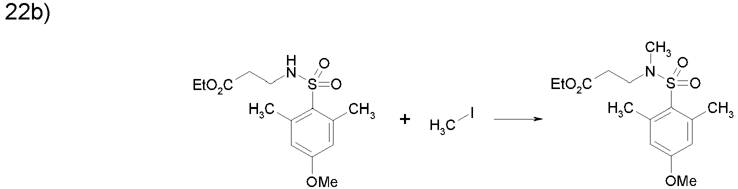

22b wird analog zu 3b aus 4.06 g (12.87 mMol) Produkt aus 22a, 2.40 ml (38.62 mMol) Methyliodid, 3.56 g (25.75 mMol) Kaliumcarbonat wasserfrei in 40 ml DMF hergestellt.
C₁₅H₂₃NO₅S (329.41)
[M+H]+ = 330
DC: Kieselgel, Petrolether / Ethylacetat 2:1, Rf-Wert = 0.36

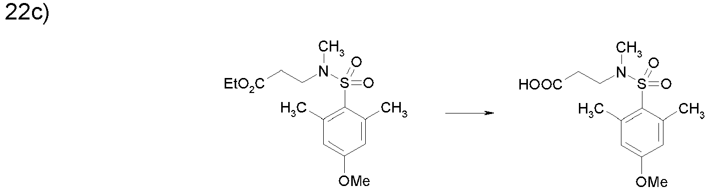

Die Säure wird analog zu 1 c aus 3.83 g (11.63 mMol) Produkt aus 22b, 2.44 g (58.13 mMol) Lithiumhydroxid Monohydrat in 30 ml THF und 30 ml Wasser hergestellt.
C₁₃H₁₉NO₅S (301.36)
[M+H]+ = 302
DC: Kieselgel, Petrolether / Ethylacetat 1:1, Rf-Wert = 0.12

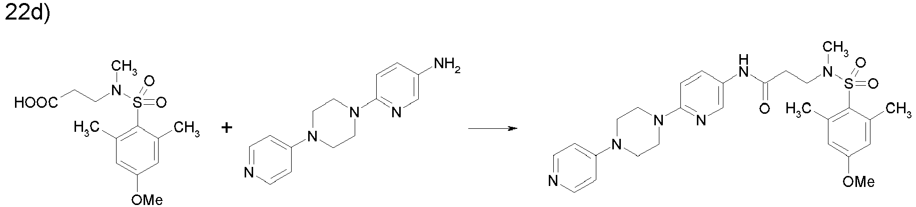

Beispiel 22 wird analog zu 1f aus 0.13 g (0.42 mMol) Produkt aus 22c, 0.089 g (0.35 mMol) Produkt aus 21b, 0.098 ml (0.70 mMol) Triethylamin und 0.13 g (0.42 mMol) TBTU in 15 ml THF hergestellt.
C₂₇H₃₄N₆O₄S (538.66)
[M+H]+ = 539
HPLC (Methode 4): Retentionszeit = 2.6 min

### Beispiel 23 (Referenzbeispiel)

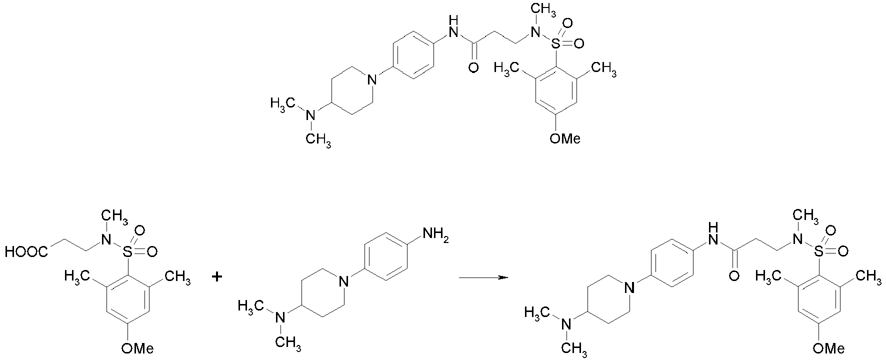

Beispiel 23 wird analog zu 1f aus 0.30 g (1.00 mMol) Produkt aus 22c, 0.22 g (1.00 mMol) Produkt aus 8b, 0.42 ml (2.99 mMol) Triethylamin und 0.32 g (1.00 mMol) TBTU in 15 ml DMF hergestellt.
C₂₆H₃₈N₄O₄S (502.67)
[M+H]+ = 503
HPLC (Methode 1): Retentionszeit = 2.47 min

### Beispiel 24 (Referenzbeispiel)

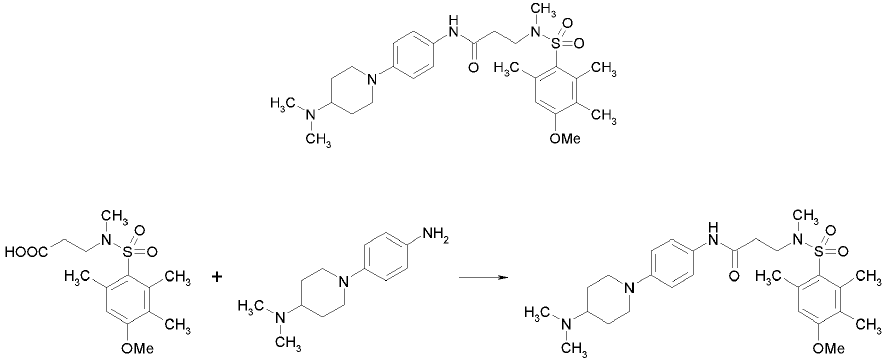

Beispiel 24 wird analog zu 1f aus 0.25 g (0.80 mMol) Produkt aus 3c, 0.18 g (0.80 mMol) Produkt aus 8b, 0.33 ml (2.39 mMol) Triethylamin und 0.26 g (0.80 mMol) TBTU in 10 ml DMF hergestellt.
C₂₇H₄₀N₄O₄S (516.70)
[M+H]+ = 517
HPLC (Methode 1): Retentionszeit = 2.50 min

### Beispiel 25 (Referenzbeispiel)

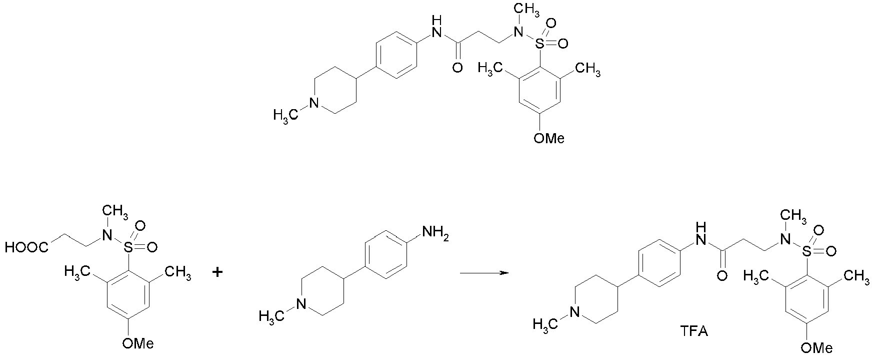

Beispiel 25 wird analog zu 1f aus 0.20 g (0.66 mMol) Produkt aus 22c, 0.14 g (0.73 mMol) 4-(1-Methylpiperidin-4-yl)-anilin (JW Pharmlab), 0.28 ml (1.99 mMol) Triethylamin und 0.21 g (0.66 mMol) TBTU in 50 ml THF hergestellt.
C₂₇H₄₀N₄O₄S x C₂HF₃O₂ (587.65)
[M+H]+ = 474
HPLC (Methode 2): Retentionszeit = 3.03 min

### Beispiel 26 (Referenzbeispiel)

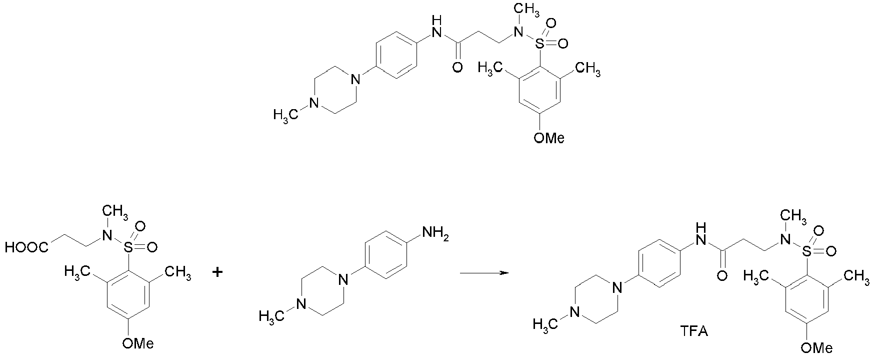

Beispiel 26 wird analog zu 1f aus 0.20 g (0.66 mMol) Produkt aus 22c, 0.14 g (0.73 mMol) 4-(4-Methylpiperazin-1-yl)-anilin (J. Med. Chem. SIR 48, 7, 2005, 2371-2387), 0.28 ml (1.99 mMol) Triethylamin und 0.21 g (0.66 mMol) TBTU in 5 ml THF hergestellt.
C₂₄H₃₄N₄O₄S x C₂HF₃O₂ (588.65)
[M+H]+ = 475
HPLC (Methode 5): Retentionszeit = 1.50 min

### Beispiel 27 (Referenzbeispiel)

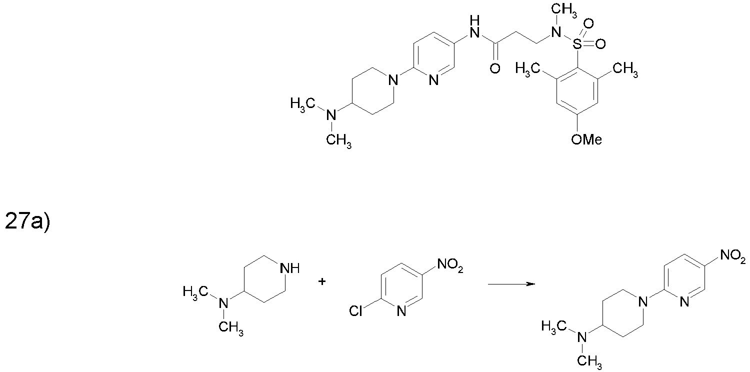

Eine Mischung aus 1.00 g (6.31 mMol) 2-Chlor-5-nitropyridin (Fluka), 1.32 ml (9.46 mMol) Triethylamin und 2 ml Methanol wird vorgelegt und langsam mit 1.13 g (8.83 mMol) 4-Dimethylamino-piperidin (Alfa Aesar) versetzt. Die Reaktionslösung wird anschließend mit halbgesättigter Natriumchlorid gequencht, der entstandene Niederschlag wird abgesaugt und getrocknet.
C₁₂H₁₈N₄O₂ (250.30)

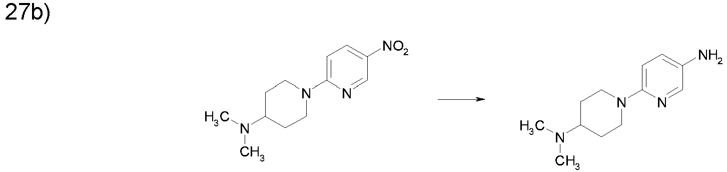

27b wird analog zu 8b aus 1.50 g (5.99 mMol) Produkt aus 27a, 0.20 g Palladium auf Kohle (10%) und 15 ml Methanol hergestellt.
C₁₂H₂₀N₄ (220.31)

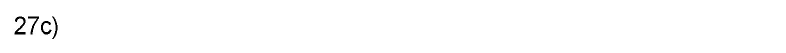

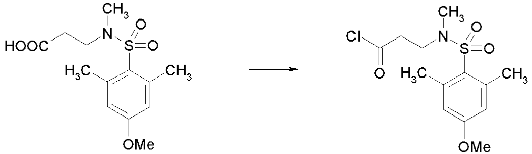

Eine Mischung aus 0.11 g (0.35 mMol) Produkt aus 22c und 2.0 ml Thionylchlorid wird zwei Stunden bei Raumtemperatur gerührt. Die Reaktionsmischung wird danach im Vakuum bis zur Trockene eingeengt.
C₁₃H₁₈ClNO₄S (319.81)

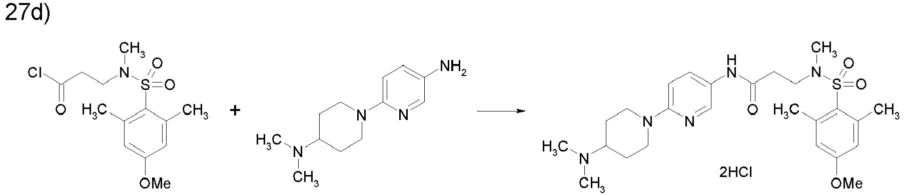

Eine Mischung aus 0.11 g (0.34 mMol) Produkt aus 27c, 0.091 g (0.41 mMol) Produkt aus 27b, 0.18 ml (1.03 mMol) DIPEA und 45 ml THF wird zwei Stunden bei Raumtemperatur gerührt. Die Reaktionsmischung wird anschließend im Vakuum bis zur Trockene eingeengt. Das Rohprodukt wird durch präparative HPLC gereinigt. Danach wird mit Hilfe von 4 M HCl in Dioxan das Hydrochlorid hergestellt.
C₂₅H₃₇N₅O₄S x 2HCl (576.58)
[M+H]+ = 504
HPLC (Methode 2): Retentionszeit = 2.73 min

### Beispiel 28 (Referenzbeispiel)

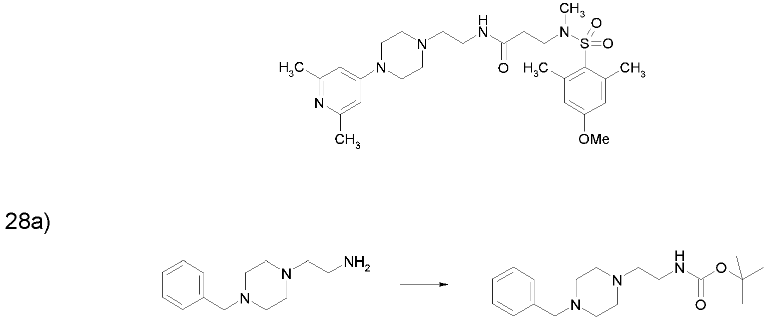

Eine Mischung aus 1.50 g (6.84 mMol) 1-(2-Aminoethyl)-4-benzylpiperazin (Maybridge), 1.64 g (7.52 mMol) Boc-Anhydrid und 30 ml Dichlormethan wird eine Stunde bei Raumtemperatur gerührt. Danach wird die Reaktionslösung mit 100 ml Dichlormethan verdünnt und mit 1 M Natronlauge und Wasser gewaschen. Die organische Phase wird über Natriumsulfat getrocknet und im Vakuum bis zur Trockene eingeengt.
C₁₈H₂₉N₃O₂ (319.44)
HPLC (Methode 4): Retentionszeit = 2.6 min

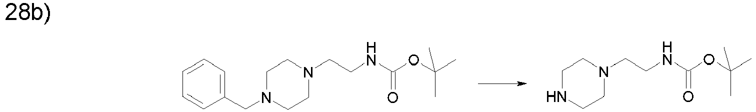

Eine Mischung aus 2.10 g (6.57 mMol) Produkt aus 28a, 0.25 g Palladium auf Kohle (10%) und 30 ml Methanol wird 15 Stunden bei Raumtemperatur im Autoklaven hydriert. Anschließend wird der Katalysator abgesaugt und das Filtrat im Vakuum bis zur Trockene eingeengt.
C₁₁H₂₃N₃O₄ (229.32)
[M+H]+ = 230
DC: Kieselgel, Dichlormethan / Ethanol 9:1, Rf-Wert = 0.08

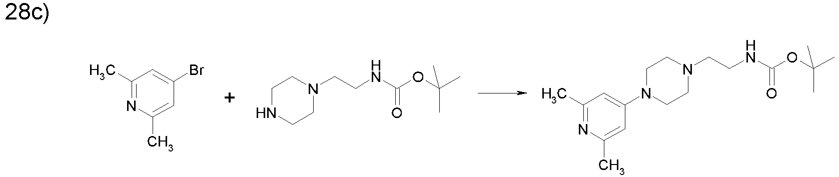

Eine Mischung aus 1.18 g (6.32 mMol) 4-Brom-2,6-dimethylpyridin (Acta Chem. Scand. Ser. B 42, 1988, 373-377), 1.45 g (6.32 mMol) Produkt aus 28b und 2.2 ml DIPEA wird 50 min auf 130°C in der Mikrowelle erhitzt. Man versetzt die Reaktionsmischung mit Ethylacetat und halbgesättigter Kaliumcarbonat-Lösung und trennt danach die Phasen. Die organische Phase wird über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 95:5:0.5) gereinigt. C₁₈H₃₀N₄O₂S (334.46)
[M+H]+ = 335
DC: Kieselgel, Dichlormethan / Methanol / Ammoniak 9:1:0.1, Rf-Wert = 0.37

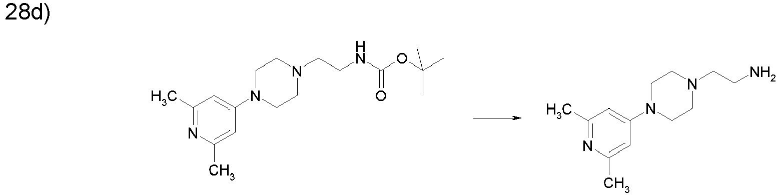

Eine Mischung aus 1.61 g (4.81 mMol) Produkt aus 28c, 3.70 ml TFA und 30 ml Dichlormethan wird sechs Stunden bei Raumtemperatur gerührt. Die Reaktionsmischung wird anschließend mit Dichlormethan verdünnt und mit 5%iger Natriumhydrogencarbonat-Lösung gewaschen. Die wässrige Phase wird noch zweimal mit Ethylacetat extrahiert. Die vereinigten organischen Phasen werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 90:10:1) gereinigt.
C₁₃H₂₂N₄ (234.34)
[M+H]+ = 235
DC: Kieselgel, Dichlormethan / Methanol / Ammoniak 9:1:0.1, Rf-Wert = 0.10

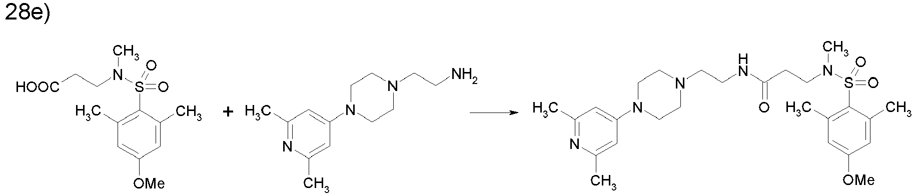

Beispiel 28 wird analog zu 1f aus 0.11 g (0.35 mMol) Produkt aus 22c, 0.082 g (0.35 mMol) Produkt aus 28d, 0.098 ml (0.70 mMol) Triethylamin und 0.13 g (0.42 mMol) TBTU in 7 ml THF und 1 ml DMF hergestellt.
C₂₆H₃₉N₅O₄S (517.69)
[M+H]+ = 518
HPLC (Methode 4): Retentionszeit = 2.4 min

### Beispiel 29 (Referenzbeispiel)

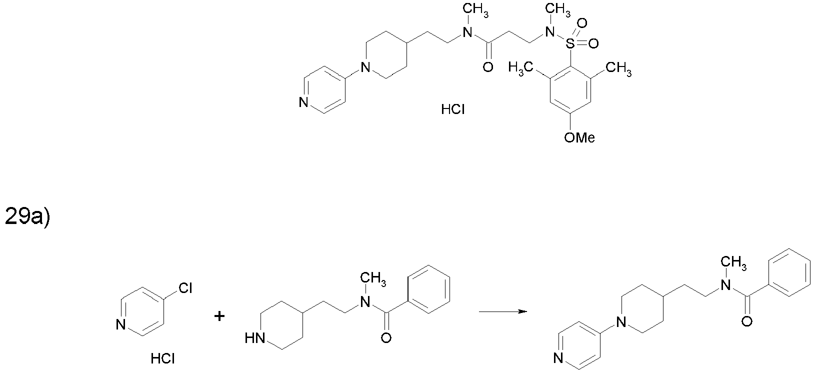

29a wird analog zu 28c aus 0.12 g (0.80 mMol) 4-Chlorpyridin Hydrochlorid (Aldrich), 0.20 g (0.80 mMol) *N*-Methyl-N-(2-piperidin-4-yl-ethyl)-benzamid (J. Med. Chem. 33, 1990, 1880-1887), 0.23 ml (1.68 mMol) Triethylamin in 5 ml Ethanol hergestellt.
C₂₀H₂₅N₃O (323.43)
[M+H]+ = 324

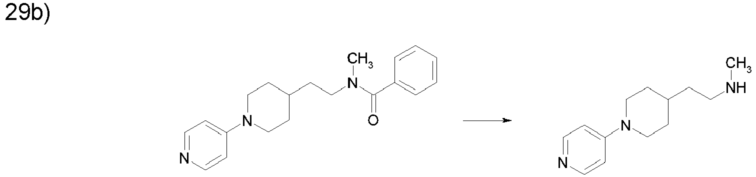

Eine Mischung aus 0.52 g (1.61 mMol) Produkt aus 29a, 10 ml 2 M Kalilauge und 10 ml Ethanol wird 30 Stunden unter Rückfluss erhitzt. Die Reaktionsmischung wird im Vakuum bis auf die Hälfte eingeengt und danach mit Dichlormethan extrahiert. Die organischen Extrakte werden über Natriumsulfat getrocknet und im Vakuum bis zur Trockene eingeengt.
C₁₃H₂₁N₃ (219.33)
[M+H]+ = 220
DC: Kieselgel, Dichlormethan / Methanol / Ammoniak 8:2:0.01, Rf-Wert = 0.46

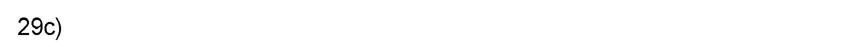

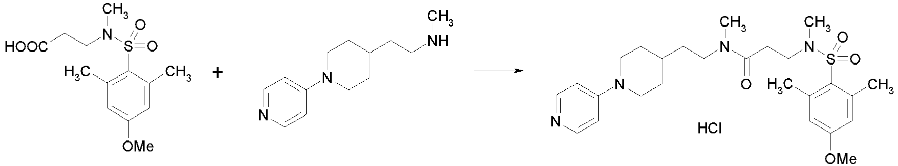

Beispiel 29 wird analog zu 1f aus 0.14 g (0.46 mMol) Produkt aus 22c, 0.10 g (0.46 mMol) Produkt aus 29b, 0.15 ml (1.09 mMol) Triethylamin und 0.16 g (0.50 mMol) TBTU in 30 ml THF und 5 ml DMF hergestellt.
C₂₆H₃₈N₄O₄S x HCl (539.13)
[M+H]+ = 503
DC: Kieselgel, Dichlormethan / Methanol / Ammoniak 8:2:0.01, Rf-Wert = 0.41

### Beispiel 30 (Referenzbeispiel)

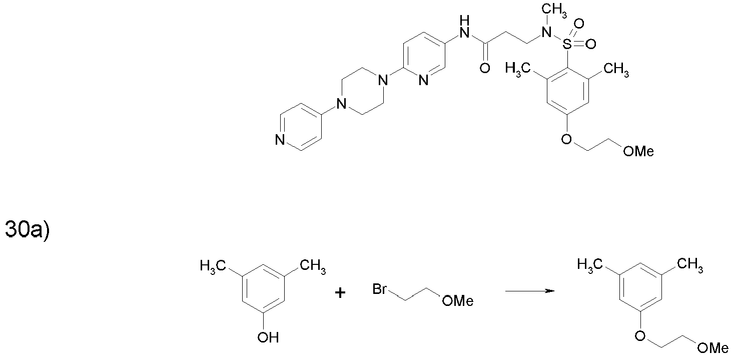

Eine Mischung aus 3.25 g (26.60 mMol) 3,5-Dimethylphenol (Aldrich), 3.20 g (28.52 mMol) Kalium-*tert*-butylat und 40 ml DMSO wird eine Stunde bei Raumtemperatur gerührt. Danach tropft man 3.80 g (27.34 mMol) Bromethylmethylether (Aldrich) hinzu und rührt weitere zwei Stunden bei Raumtemperatur. Die Reaktionsmischung wird auf Wasser gegossen und mit Diethylether extrahiert. Die organischen Extrakte werden mit Wasser und gesättigter Natriumchlorid-Lösung gewaschen, über Natriumsulfat getrocknet und im Vakuum bis zur Trockene eingeengt.
C₁₁H₁₆O₂ (180.24)
[M+H]+ = 181
DC: Kieselgel, Petrolether/Ethylacetat 9:1, Rf-Wert = 0.31

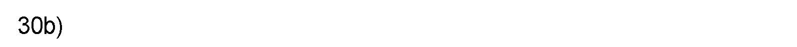

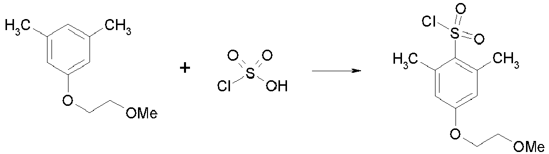

30b wird analog zu 13a aus 4.30 g (23.86 mMol) Produkt aus 30a und 5.60 g (48.06 mMol) Chlorsulfonsäure in 100 ml Dichlormethan hergestellt.
C₁₁H₁₅ClO₄S (278.75)
DC: Kieselgel, Petrolether/Ethylacetat 9:1, Rf-Wert = 0.06

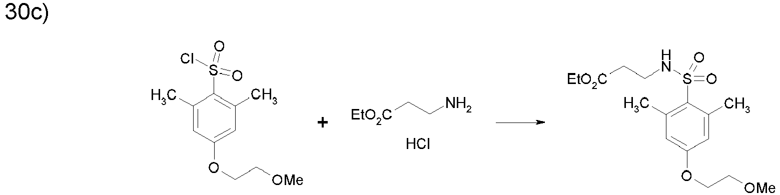

30a wird analog zu 3a aus 1.70 g (6.10 mMol) Produkt aus 30b, 1.20 g (7.81 mMol) ß-Alaninethylester Hydrochlorid, 2.60 ml (18.65 mMol) Triethylamin in 30 ml Dichlormethan hergestellt.
C₁₆H₂₅NO₆S (359.44)
[M+H]+ = 360
DC: Kieselgel, Dichlormethan / Methanol 19:1, Rf-Wert = 0.51

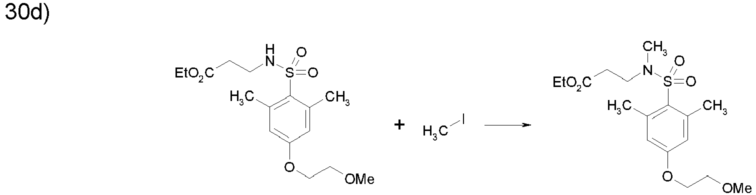

30d wird analog zu 3b aus 1.90 g (5.29 mMol) Produkt aus 30c, 1.10 g (7.75 mMol) Methyliodid, 1.50 g (10.85 mMol) Kaliumcarbonat wasserfrei in 30 ml DMF hergestellt.
C₁₇H₂₇NO₆S (373.47)
[M+H]+ = 374

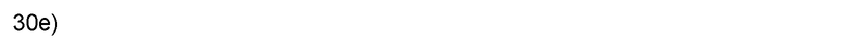

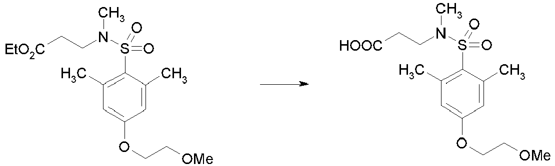

Die Säure wird analog zu 1 c aus 1.70 g (4.55 mMol) Produkt aus 30d, 0.80 g (20.00 mMol) Natriumhydroxid in 30 ml Ethanol und 10 ml Wasser hergestellt.
C₁₅H₂₃NO₆S (345.41)
[M+H]+ = 346

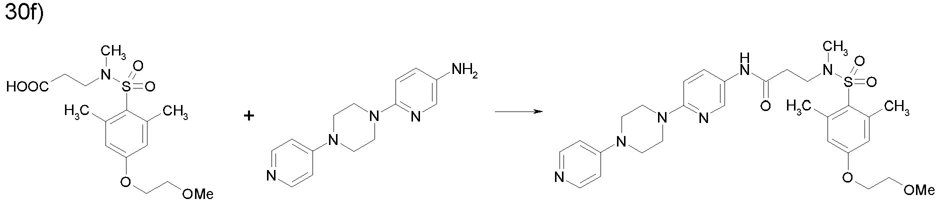

Beispiel 30 wird analog zu 1f aus 0.14 g (0.39 mMol) Produkt aus 30e, 0.10 g (0.39 mMol) Produkt aus 21b, 0.10 ml (0.99 mMol) Triethylamin und 0.14 g (0.44 mMol) TBTU in 30 ml THF und 5 ml DMF hergestellt.
C₂₉H₃₈N₆O₅S (582.72)
[M+H]+ = 583
DC: Kieselgel, Dichlormethan / Methanol / Ammoniak 8:2:0.01, Rf-Wert = 0.44

### Beispiel 31 (Referenzbeispiel)

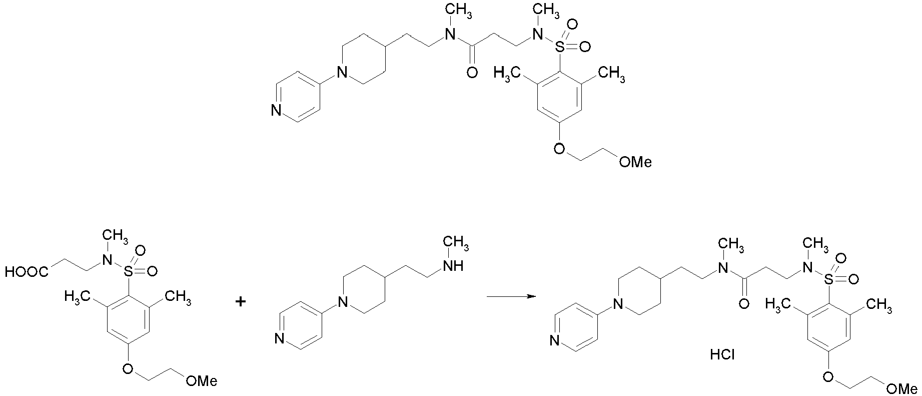

Beispiel 31 wird analog zu 1f aus 0.16 g (0.46 mMol) Produkt aus 30e, 0.10 g (0.46 mMol) Produkt aus 29b, 0.11 ml (1.09 mMol) Triethylamin und 0.16 g (0.50 mMol) TBTU in 30 ml THF und 5 ml DMF hergestellt.
C₂₈H₄₂N₄O₅S x HCl (583.18)
[M+H]+ = 547
DC: Kieselgel, Dichlormethan / Methanol / Ammoniak 8:2:0.01, Rf-Wert = 0.52

### Beispiel 32 (Referenzbeispiel)

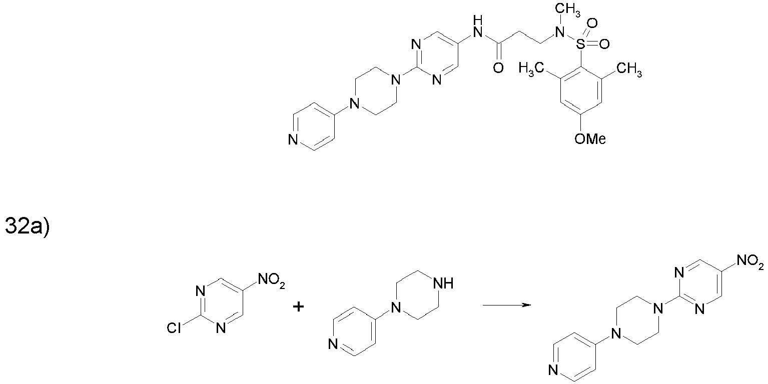

Eine Mischung aus 3.00 g (18.81 mMol) 2-Chlor-5-nitropyrimidin (Apin), 3.07 g (18.81 mMol) 1-(4-Pyridyl)-piperazin (Girindus), 9.40 ml (18.81 mMol) 2 M Natronlauge in 80 ml Dichlormethan wird 2.5 Stunden bei Raumtemperatur gerührt. Danach wird die Reaktionsmischung mit 100 ml Dichlormethan verdünnt und mit 5%iger Natriumhydrogencarbonat-Lösung gewaschen. Die organische Phase wird über Natriumsulfat getrocknet und im Vakuum bis zur Trockene eingeengt. Das Rohprodukt wird mit einer Mischung aus 50 ml Wasser und 30 ml Ethylacetat verrieben, abfiltriert und getrocknet.
C₁₃H₁₄N₆O₂ (286.29)
[M+H]+ = 287
HPLC (Methode 4): Retentionszeit = 2.6 min

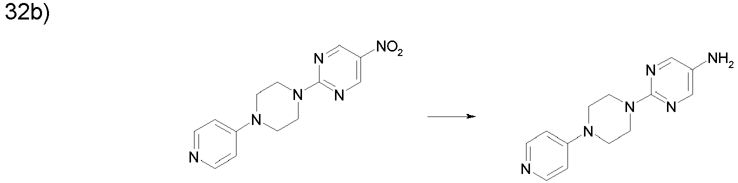

32b wird analog zu 21 b aus 1.93 g (6.74 mMol) Produkt aus 32a und 0.3 g Palladium auf Kohle (10 %) in 60 ml Dichlormethan und 30 ml Methanol hergestellt.
C₁₃H₁₆N₆ (256.31)
[M+H]+ = 257
DC: Kieselgel, Dichlormethan / Methanol 8:2, Rf-Wert = 0.11

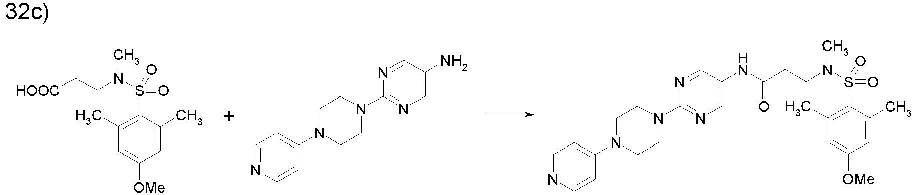

Beispiel 32 wird analog zu 1f aus 0.11 g (0.35 mMol) Produkt aus 22c, 0.090 g (0.35 mMol) Produkt aus 32b, 0.098 ml (0.70 mMol) Triethylamin und 0.13 g (0.42 mMol) TBTU in 7 ml THF und 1 ml DMF hergestellt.
C₂₆H₃₃N₇O₄S (539.65)
[M+H]+ = 540
HPLC (Methode 4): Retentionszeit = 2.9 min

### Beispiel 33 (Referenzbeispiel)

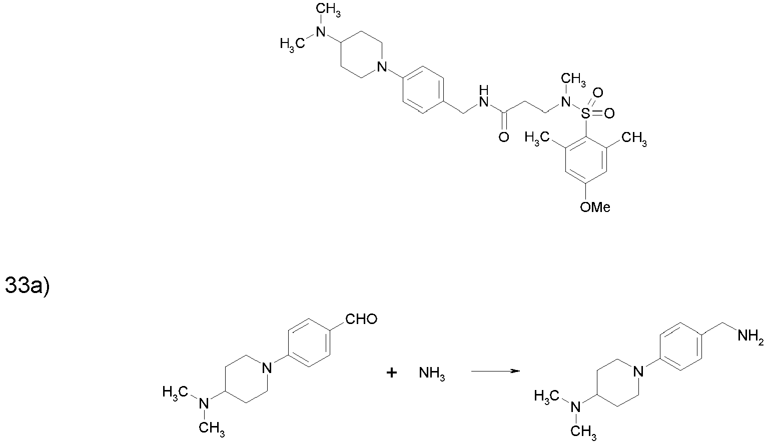

Eine Mischung aus 0.68 g (2.91 mMol) 4-(4-Dimethylamino-piperidin-1-yl)-benzaldehyd (Tetrahedron 57, 2001, 4781-4785), 15 ml 2 M Ammoniak in Ethanol und 0.10 g Raney-Nickel wird bei Raumtemperatur im Autoklaven hydriert. Danach wird der Katalysator abfiltriert und das Filtrat im Vakuum bis zur Trockene eingeengt.
C₁₄H₂₃N₃ (233.35)

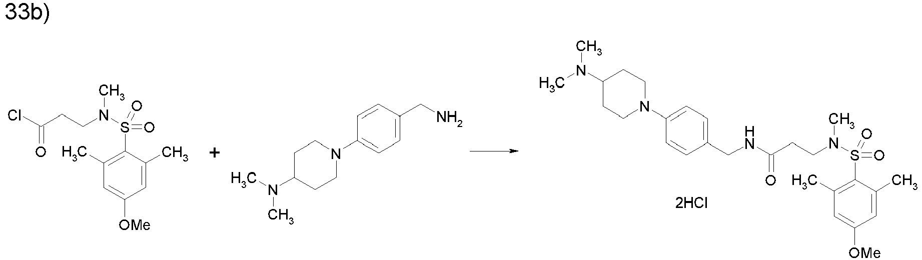

Beispiel 33 wird analog zu 27d aus 0.27 g (0.84 mMol) Produkt aus 27c, 0.63 g (2.68 mMol) Produkt aus 33a, 0.22 ml (1.26 mMol) DIPEA in 3 ml Dichlormethan hergestellt. C₂₇H₄₀N₄O₄S x 2HCl (589.62)
[M+H]+ = 517
HPLC (Methode 5): Retentionszeit = 1.46 min

### Beispiel 34 (Referenzbeispiel)

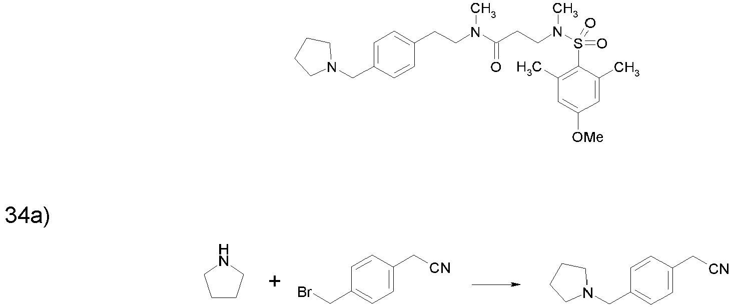

Eine Mischung aus 4.97 ml (59.50 mMol) Pyrrolidin und 100 ml Dichlormethan wird unter Eisbadkühlung langsam mit 5.00 g (23.80 mMol) (4-Brommethyl-phenyl)-acetonitril (Tetrahedron 47, 1991, 3969-3980) versetzt. Danach wird die Reaktionsmischung auf Raumtemperatur erwärmt, mit Wasser gewaschen, über Magnesiumsulfat getrocknet und im Vakuum bis zur Trockene eingeengt.
C₁₃H₁₆N₂ (200.28)
[M+H]+ = 201
DC: Kieselgel, Dichlormethan / Methanol / Ammoniak 9:1:0.1, Rf-Wert = 0.58

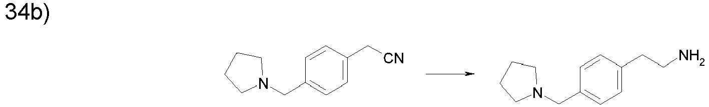

Eine Mischung aus 4.70 g (23.47 mMol) Produkt aus 34a, 0.5 g Raney-Nickel und 50 ml methanolische Ammoniak-Lösung wird im Autoklaven bei 50°C hydriert. Anschließend wird der Katalysator abfiltriert und das Filtrat im Vakuum bis zur Trockene eingeengt.
C₁₃H₂₀N₂ (204.31)
[M+H]+ = 205

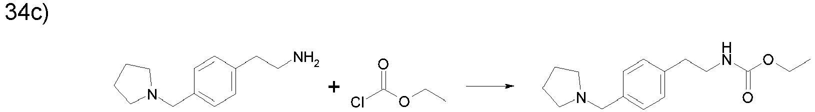

Eine Mischung aus 4.09 g (20.00 mMol) Produkt aus 34b, 5.62 ml (40.00 mMol) Triethylamin und 100 ml Dichlormethan wird unter Eisbadkühlung langsam mit 2.17 ml (22.00 mMol) Chlorameisensäureethylester (Aldrich) versetzt. Danach wird fünf Stunden bei Raumtemperatur gerührt. Die Reaktionsmischung wird anschließend mit Wasser gequencht und mit MTB-Ether extrahiert. Die organischen Extrakte werden zweimal mit Wasser gewaschen, über Magnesiumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₂₄N₂O₂ (276.37)
[M+H]+ = 277

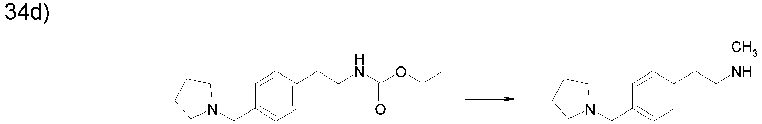

Eine Mischung aus 3.60 g (13.03 mMol) Produkt aus 34c und 25 ml THF wird langsam mit 51.05 ml (51.05 mMol) 1 M Lithiumaluminiumhydrid in THF (Aldrich) versetzt. Danach wird zwei Stunden bei Raumtemperatur und zwei Stunden bei 70°C gerührt. Die Reaktionsmischung wird anschließend mit Wasser und 15%iger Natronlauge gequencht und noch eine Stunde bei Raumtemperatur gerührt. Der entstandene Niederschlag wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₄H₂₂N₂ (218.34)
[M+H]+ = 219

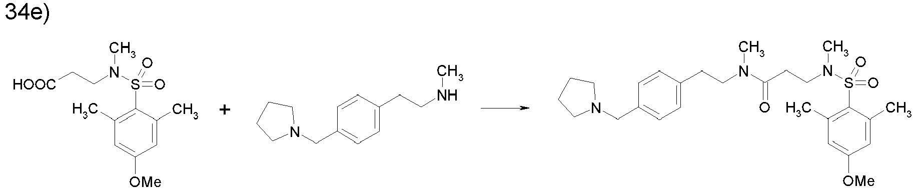

Beispiel 34 wird analog zu 1f aus 0.11 g (0.35 mMol) Produkt aus 22c, 0.076 g (0.35 mMol) Produkt aus 34d, 0.098 ml (0.70 mMol) Triethylamin und 0.13 g (0.42 mMol) TBTU in 7 ml THF und 1 ml DMF hergestellt.
C₂₇H₃₉N₃O₄S (501.68)
[M+H]+ = 502
HPLC (Methode 4): Retentionszeit = 3.1 min

### Beispiel 35 (Referenzbeispiel)

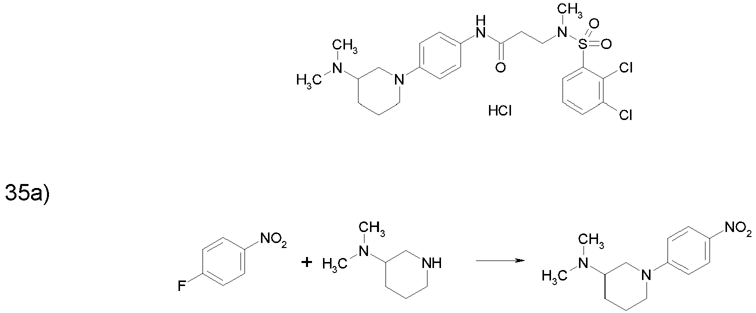

Eine Mischung aus 0.33 g (2.34 mMol) 1-Fluor-4-nitrobenzol (Aldrich), 0.30 g (2.34 mMol) 3-Dimethylamino-piperidin (Chess), 0.46 ml (3.27 mMol) Triethylamin und 4 ml DMF wird sechs Stunden bei Raumtemperatur gerührt. Die Reaktionsmischung wird danach mit Wasser gequencht und mit Dichlormethan extrahiert. Die organischen Extrakte werden mit gesättigter Natriumchlorid-Lösung gewaschen, über Natriumsulfat getrocknet und im Vakuum bis zur Trockene eingeengt.
C₁₃H₁₉N₃O₂ (249.31)
[M+H]+ = 250
DC: Kieselgel, Dichlormethan / Methanol / Ammoniak 9:1:0.1, Rf-Wert = 0.56

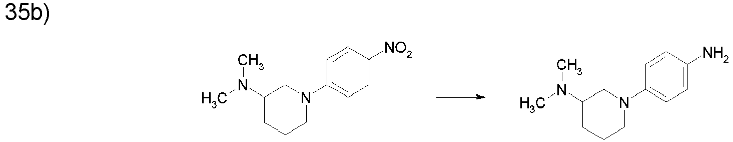

Eine Mischung aus 0.25 g (1.00 mMol) Produkt aus 35a, 30 mg Raney-Nickel und 10 ml Ethylacetat wird im Autoklaven bei Raumtemperatur hydriert. Der Katalysator wird abfiltriert und das Filtrat im Vakuum bis zur Trockene eingeengt.
C₁₃H₂₁N₃ (219.33)
DC: Kieselgel, Dichlormethan / Methanol / Ammoniak 9:1:0.1, Rf-Wert = 0.25

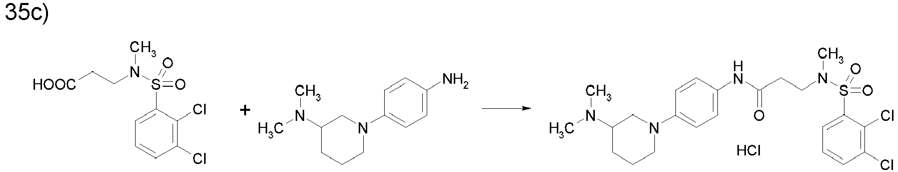

Beispiel 35 wird analog zu 1f aus 0.16 g (0.50 mMol) Produkt aus 1c, 0.11 g (0.50 mMol) Produkt aus 35b, 0.14 ml (1.00 mMol) Triethylamin und 0.16 g (0.50 mMol) TBTU in 3 ml DMF hergestellt.
C₂₃H₃₀Cl₂N₄O₃S x HCl (539.14)
[M+H]+ = 513/515/517
HPLC (Methode 1): Retentionszeit = 2.43 min

### Beispiel 36 (Referenzbeispiel)

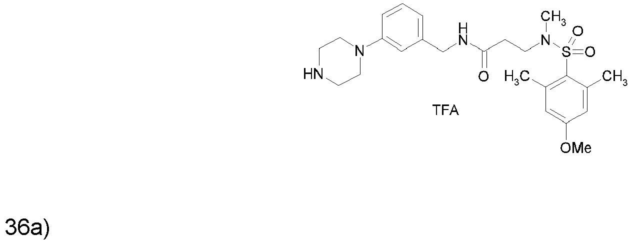

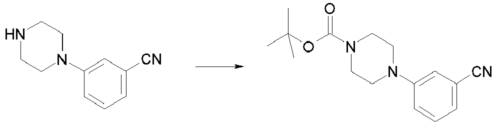

Eine Mischung aus 2.50 g (13.35 mMol) 3-Piperazin-1-yl-benzonitril (Tetrahedron 55, 1999, 13285-13300), 3.00 g (13.75 mMol) Boc-Anhydrid, 2.40 ml (13.78 mMol) DIPEA und 50 ml THF wird vier Stunden bei Raumtemperatur gerührt und danach im Vakuum bis zur Trockene eingeengt. Der Rückstand wird in Wasser aufgenommen und mit Diethylether extrahiert. Die organischen Extrakte werden über Natriumsulfat getrocknet und im Vakuum bis zur Trockene eingeengt.
C₁₆H₂₁N₃O₂ (287.36)
[M+Na]+ = 310

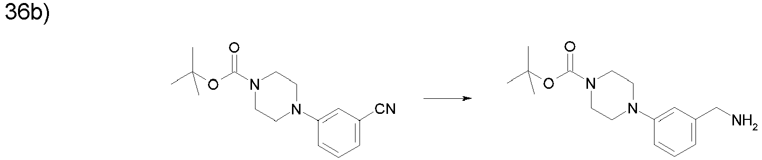

36b wird analog zu 34b aus 4.40 g (15.31 mMol) Produkt aus 36a, 0.7 g Raney-Nickel und 45 ml methanolischer Ammoniak-Lösung hergestellt.
C₁₆H₂₅N₃O₂ (291.39)
[M+H]+ = 292

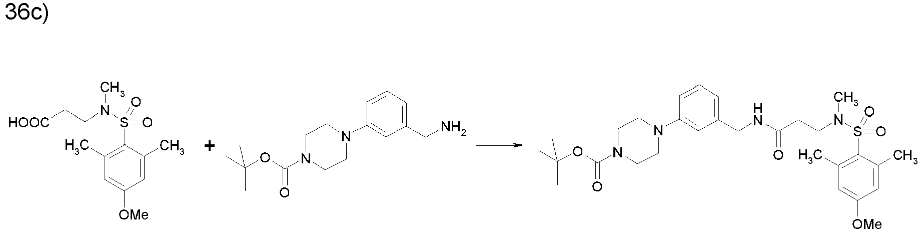

36c wird analog zu 1f aus 0.40 g (1.33 mMol) Produkt aus 22c, 0.43 g (1.46 mMol) Produkt aus 36b, 0.56 ml (3.98 mMol) Triethylamin und 0.43 g (1.33 mMol) TBTU in 10 ml THF hergestellt.
C₂₉H₄₂N₄O₆S (574.73)
M+H]+ = 575
DC: Kieselgel, Dichlormethan / Methanol 9:1, Rf-Wert = 0.53

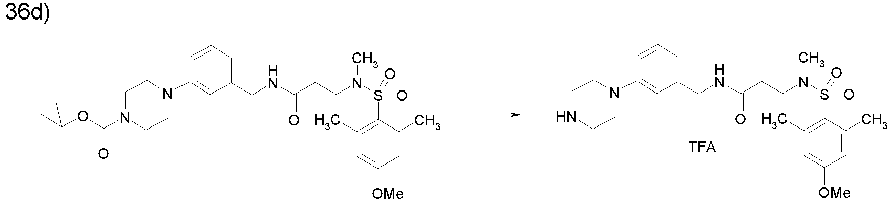

Beispiel 36 wird analog zu 18b aus 0.50 g (0.57 mMol) Produkt aus 36c, 0.44 ml TFA in 5 ml Dichlormethan hergestellt.
C₁₈H₂₈Cl₂N₄O₃S x C₂HF₃O₂ (588.64)
[M+H]+ = 475
HPLC (Methode 2): Retentionszeit = 2.95 min

### Beispiel 37 (Referenzbeispiel)

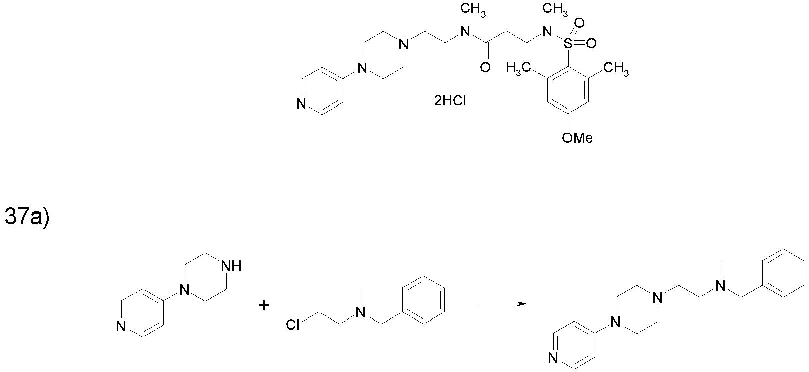

Eine Mischung aus 2.00 g (12.25 mMol) 1-(4-Pyridyl)-piperazin (Girindus), 1.65 g (14.70 mMol) Kalium-tert-butylat und 50 ml DMSO wird 30 min bei Raumtemperatur gerührt und danach mit 2.25 g (12.25 mMol) 1-Benzylmethylamino-2-chlorethan (Chem. Pharm. Bull. 45, 1997, 996-1007) versetzt. Die Reaktionsmischung wird über Nacht bei Raumtemperatur gerührt und anschließend auf Eiswasser gegossen. Man extrahiert viermal mit Dichlormethan. Die organischen Extrakte werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₉H₂₆N₄ (310.44)
[M+H]+ = 311
DC: Kieselgel, Dichlormethan / Methanol / Ammoniak 9:1:0.1, Rf-Wert = 0.22

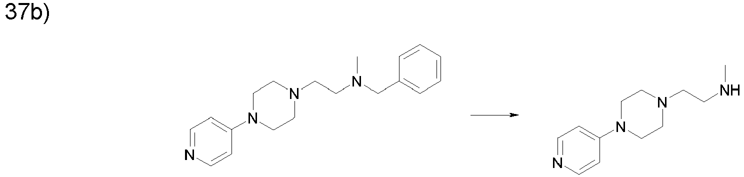

Eine Mischung aus 1.78 g (5.73 mMol) Produkt aus 37a, 0.40 g Palladiumhydroxid und 50 ml Methanol wird bei 40°C im Autoklaven hydriert. Der Katalysator wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₂H₂₀N₄ (220.31)
[M+H]+ = 221
DC: Kieselgel, Dichlormethan / Methanol / Ammoniak 9:1:0.1, Rf-Wert = 0.13

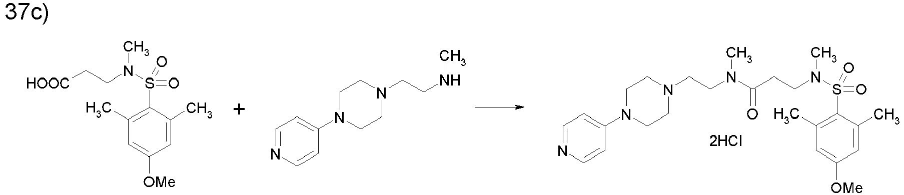

Beispiel 37 wird analog zu 1f aus 0.11 g (0.35 mMol) Produkt aus 22c, 0.072 g (0.35 mMol) Produkt aus 37b, 0.098 ml (0.70 mMol) Triethylamin und 0.13 g (0.42 mMol) TBTU in 7 ml THF hergestellt.
C₂₅H₃₇N₅O₄S x 2HCl (576.58)
[M+H]+ = 504
HPLC (Methode 4): Retentionszeit = 2.5 min

### Beispiel 38 (Referenzbeispiel)

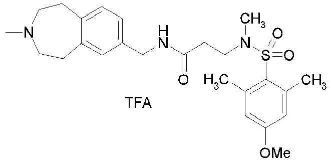

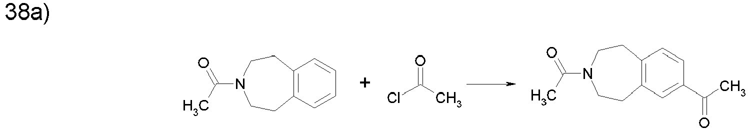

Eine Mischung aus 4.44 g (33.29 mMol) Aluminiumchlorid (Merck) und 16 ml Dichlorethan wird vorgelegt und unter Eisbadkühlung langsam mit 1.24 ml (17.44 mMol) Acetylchlorid (Aldrich) versetzt. Man lässt 30 min bei Raumtemperatur rühren. Anschließend werden 3.00 g (15.85 mMol) 1-(1,2,4,5-Tetrahydrobenzo[d]azepin-3-yl)-ethanon (J. Med.Chem. 46, 2003, 4952-4964) in 7 ml Dichlorethan langsam zur Reaktionsmischung gegeben. Nach zweistündigem Rühren bei Raumtemperatur wird die Reaktionsmischung auf eine Mischung aus 6 M HCl und Eis gegossen. Nach der Phasentrennung wird die wässrige Phase noch dreimal mit Dichlormethan extrahiert. Die vereinigten organischen Phasen werden mit Wasser gewaschen, über Natriumsulfat getrocknet und im Vakuum bis zur Trockene eingeengt. Das so erhaltene Rohprodukt wird mit Diethylether verrieben, abfiltriert und getrocknet.
C₁₄H₁₇NO₂ (231.29)
[M+H]+ = 232

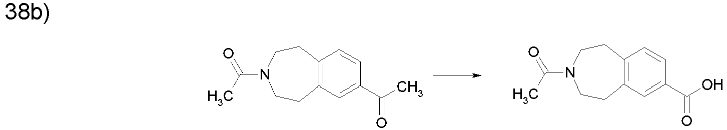

Eine Mischung aus 2.86 g (12.37 mMol) Produkt aus 38a und 79 ml 2.5 M Natronlauge wird bei Raumtemperatur langsam mit 2.48 ml (48.23 mMol) Brom versetzt. Die Reaktionsmischung wird danach eine Stunde bei Raumtemperatur gerührt. Der entstandene Niederschlag wird abfiltriert und das Filtrat mit MTB-Ether extrahiert. Die wässrige Phase wird anschließend unter Eisbadkühlung mit konzentrierter HCl und wenig Natriumdisulfit-Lösung versetzt. Der entstandene Niederschlag wird abfiltriert und im Umlufttrockenschrank bei 30°C getrocknet.
C₁₃H₁₅NO₃ (233.26)
[M+H]+ = 234

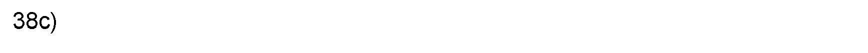

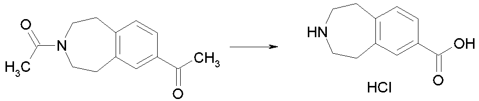

Eine Mischung aus 2.12 g (9.09 mMol) Produkt aus 38b und 20 ml 6 M HCl wird 3.5 Tage auf 100°C erhitzt. Die Reaktionsmischung wird danach mit einer Mischung aus Eis und Ethanol gekühlt. Der entstandene Niederschlag wird abfiltriert, mit wenig gekühltem Aceton und Diethylether gewaschen und im Exsikkator getrocknet.
C₁₁H₁₃NO₂ x HCl (227.69)
[M+H]+ = 192

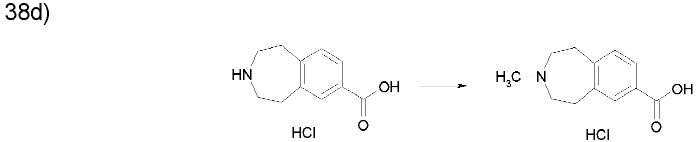

Eine Mischung aus 2.03 g (8.92 mMol) Produkt aus 38c und 3.36 ml (89.16 mMol) Ameisensäure wird erst langsam mit 0.94 ml (17.83 mMol) 50%iger Natronlauge, dann mit 2.66 ml (35.66 mMol) 37%iger Formalin-Lösung versetzt. Die Reaktionsmischung wird zwei Stunden auf 70°C erhitzt und danach im Vakuum bis zur Trockene eingeengt. Der Rückstand wird mit Wasser und konzentrierter HCl versetzt und nochmals bis zur Trockene eingeengt. Das Rohprodukt wird mit wenig eiskaltem Wasser verrieben, abfiltriert und im Umlufttrockenschrank bei 60°C getrocknet.
C₁₂H₁₅NO₂ x HCl (241.71)
[M+H]+ = 206

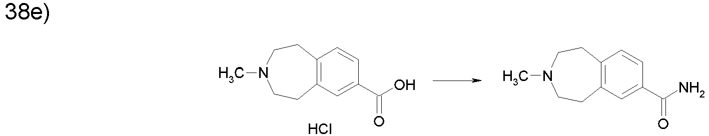

38e wird analog zu 1f aus 2.00 g (8.27 mMol) Produkt aus 38d, 18.20 ml (9.10 mMol) Ammoniak 0.5 M in Dioxan (Aldrich), 3.46 ml (24.82 mMol) Triethylamin und 3.19 g (9.93 mMol) TBTU in 30 ml THF hergestellt.
C₁₂H₁₆N₂O (204.27)
[M+H]+ = 205
HPLC (Methode 2): Retentionszeit = 1.54 min

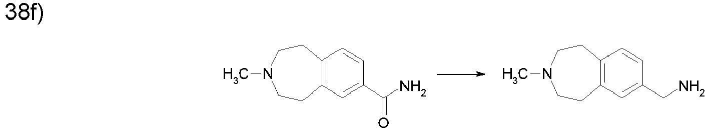

Eine Mischung aus 5.20 ml (5.20 mMol) Lithiumaluminiumhydrid 1 M in THF (Aldrich) und 18 ml THF wird auf 50°C erhitzt und langsam mit 0.84 g (2.64 mMol) Produkt aus 38e versetzt. Die Reaktionsmischung wird danach 30 min bei 50°C gerührt. Man kühlt anschließend auf -20°C ab und quencht die Reaktionsmischung erst mit einer Mischung aus Wasser und THF, dann mit 2 M Natronlauge. Es wird eine Stunde bei Raumtemperatur gerührt. Der entstandene Niederschlag wird abfiltriert, das Filtrat im Vakuum bis zur Trockene eingeengt. Der Rückstand wird in Dichlormethan aufgenommen und mit gesättigter Natriumhydrogensulfat-Lösung gewaschen. Die organische Phase wird über Natriumsulfat getrocknet und im Vakuum bis zur Trockene eingeengt.
C₁₂H₁₈N₂ (190.28)
[M+H]+ = 191
HPLC (Methode 2): Retentionszeit = 2.21 min

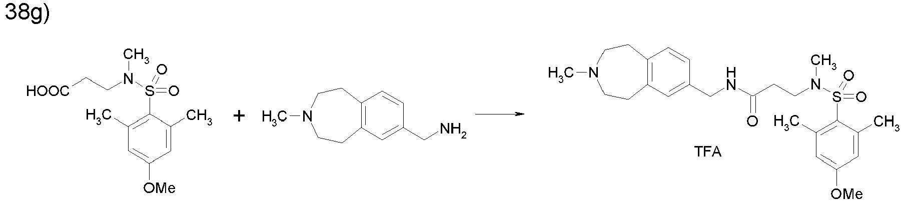

Beispiel 38 wird analog zu 1f aus 0.19 g (0.63 mMol) Produkt aus 22c, 0.12 g (0.63 mMol) Produkt aus 38f, 0.26 ml (1.89 mMol) Triethylamin und 0.20 g (0.63 mMol) TBTU in 5 ml THF hergestellt.
C₂₅H₃₅N₃O₄S x C₂HF₃O₂ (587.65)
[M+H]+ = 474
HPLC (Methode 4): Retentionszeit = 2.98 min

### Beispiel 39 (Referenzbeispiel)

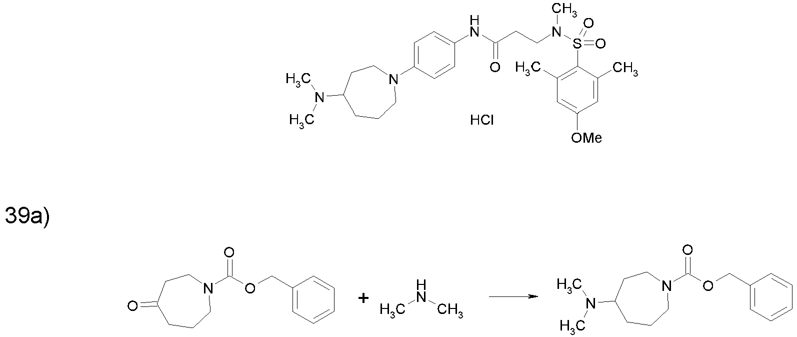

Eine Mischung aus 1.50 g (6.07 mMol) 4-Oxo-azepan-1-carbonsäurebenzylester (Tyger), 20 ml (40.00 mMol) Dimethylamin 2 M in THF (Aldrich) und 0.34 ml (6.07 mMol) Essigsäure wird 20 min bei Raumtemperatur gerührt und danach mit 3.82 g (18.00 mMol) Natriumtriacetoxyborhydrid (Aldrich) versetzt. Man lässt über Nacht bei Raumtemperatur rühren. Die Reaktionsmischung wird anschließend mit gesättigter Natriumhydrogencarbonat-Lösung versetzt und mit Ethylacetat extrahiert. Die organischen Extrakte werden mit gesättigter Natriumchlorid-Lösung gewaschen, über Natriumsulfat getrocknet und im Vakuum bis zur Trockene eingeengt. Das so erhaltene Rohprodukt wird durch Säulenchromatographie über Kieselgel (Elutionsmittel: Dichlormethan / 4-12 % (Methanol + 10% Ammoniak)) gereinigt.
C₁₆H₂₄N₂O₂ (276.37)
[M+H]+ = 277
HPLC (Methode 1): Retentionszeit = 2.12 min

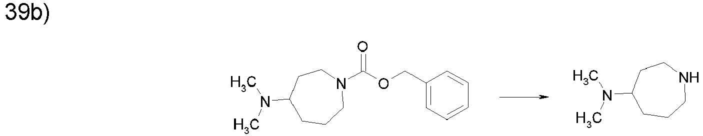

Eine Mischung aus 1.00 g (3.62 mMol) Produkt aus 39a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₈H₁₈N₂ (142.24)
[M+H]+ = 143
DC: Kieselgel, Dichlormethan / Methanol / Ammoniak 9:1:0.1, Rf-Wert = 0.12

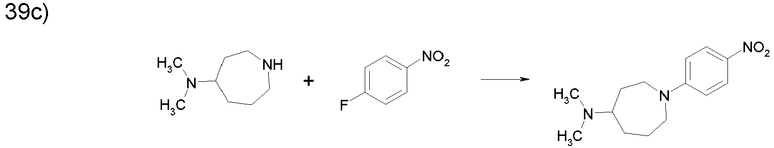

39c wird analog zu 1 d aus 0.56 g (4.37 mMol) Produkt aus 39b, 0.64 g (4.50 mMol) 4-Fluor-nitrobenzol (Aldrich), 0.65 ml (4.60 mMol) Triethylamin in 5 ml DMF hergestellt.
C₁₄H₂₁N₃O₂ (263.34)
[M+H]+ = 264
DC: Kieselgel, Dichlormethan / Methanol / Ammoniak 9:1:0.1, Rf-Wert = 0.60

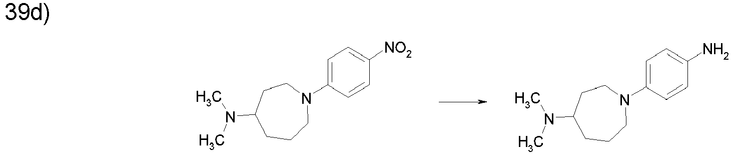

39d wird analog zu 8b aus 0.94 g (3.55 mMol) Produkt aus 39c und 0.10 g Palladium auf Kohle (10%) in 30 ml Methanol hergestellt.
C₁₄H₂₃N₃O₂ (233.35)
DC: Kieselgel, Dichlormethan / Methanol / Ammoniak 9:1:0.1, Rf-Wert = 0.15

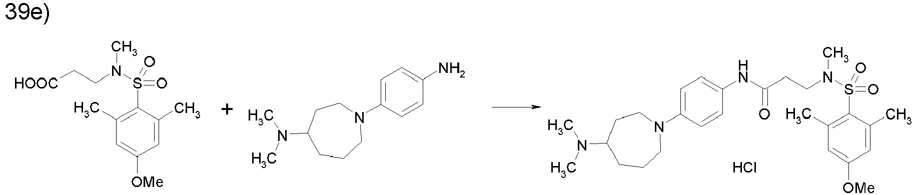

Beispiel 39 wird analog zu 1f aus 0.30 g (1.00 mMol) Produkt aus 22c, 0.23 g (1.00 mMol) Produkt aus 39d, 0.42 ml (3.00 mMol) Triethylamin und 0.32 g (1.00 mMol) TBTU in 15 ml DMF hergestellt.
C₂₇H₄₀N₄O₄S x HCl (553.16)
[M+H]+ = 517
DC: Kieselgel, Dichlormethan / Methanol / Ammoniak 9:1:0.1, Rf-Wert = 0.42
HPLC (Methode 5): Retentionszeit = 1.50 min

### Beispiel 40 (Referenzbeispiel)

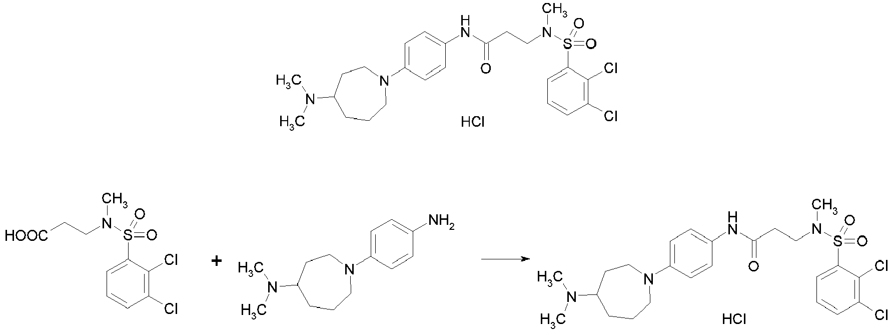

Beispiel 40 wird analog zu 1f aus 0.16 g (0.50 mMol) Produkt aus 1c, 0.12 g (0.50 mMol) Produkt aus 39d, 0.21 ml (1.50 mMol) Triethylamin und 0.16 g (0.50 mMol) TBTU in 5 ml DMF hergestellt.
C₂₄H₃₂Cl₂N₄O₃S x HCl (563,97)
[M+H]+ = 527/529/531
DC: Kieselgel, Dichlormethan / Methanol / Ammoniak 6:1:0.2, Rf-Wert = 0.48
HPLC (Methode 5): Retentionszeit = 1.53 min

### Beispiel 41 (Referenzbeispiel)

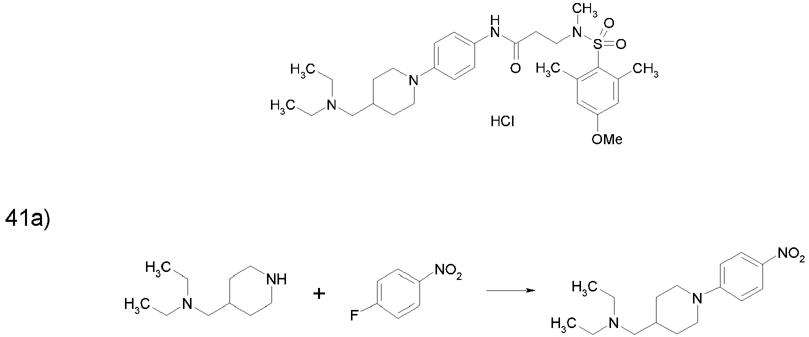

41a wird analog zu 1d aus 1.00 g (5.87 mMol) Diethyl-piperidin-4-ylmethyl-amin (Chem. Pharm. Bull. 42, 1994, 74-84), 0.83 g (5.87 mMol) 4-Fluor-nitrobenzol (Aldrich), 1.14 ml (8.20 mMol) Triethylamin in 12 ml DMF hergestellt.
C₁₆H₂₅N₃O₂ (291.39)
[M+H]+ = 292
DC: Kieselgel, Dichlormethan / Ethanol 9:1, Rf-Wert = 0.5

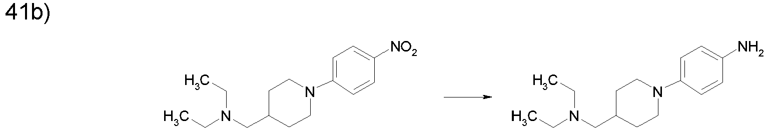

Eine Mischung aus 0.40 g (1.37 mMol) Produkt aus 41a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₆H₂₇N₃ (261.41)
DC: Kieselgel, Dichlormethan / Ethanol 9:1, Rf-Wert = 0.1

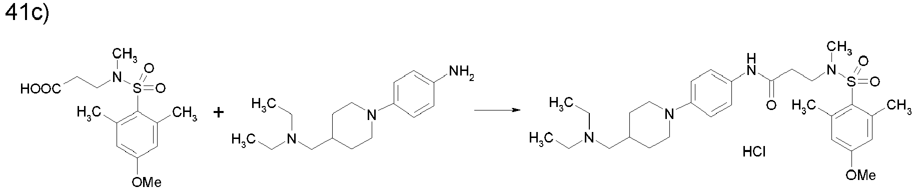

Beispiel 41 wird analog zu 1f aus 0.40 g (1.34 mMol) Produkt aus 22c, 0.35 g (1.34 mMol) Produkt aus 41 b, 0.47 ml (3.35 mMol) Triethylamin und 0.43g (1.34 mMol) TBTU in 7 ml THF und 1 ml DMF hergestellt.
C₂₉H₄₄N₄O₄S x HCl (581.21)
[M+H]+ = 545
HPLC (Methode 5): Retentionszeit = 1.42 min

### Beispiel 42 (Referenzbeispiel)

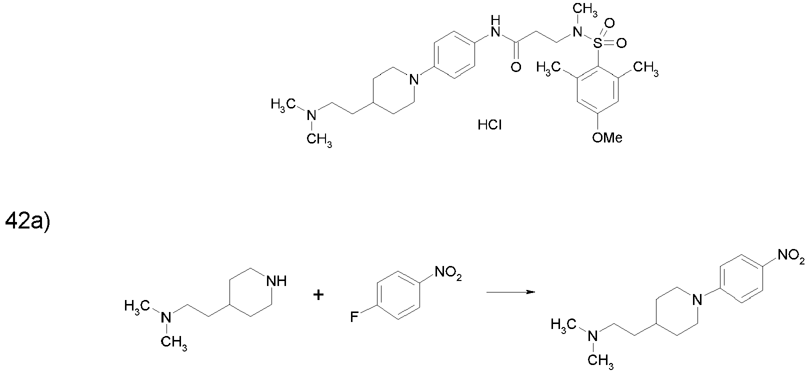

42a wird analog zu 1d aus 1.88 g (12.00 mMol) Dimethyl-(2-piperidin-4-yl-ethyl)-amin (J. Med. Chem. 36, 1993, 162-165), 1.69 g (12.00 mmol) 4-Fluor-nitrobenzol (Aldrich), 2.37 ml (17.00 mMol) Triethylamin in 15 ml DMF hergestellt.
C₁₅H₂₃N₃O₂ (277.36)
[M+H]+ = 278
DC: Kieselgel, Dichlormethan / Ethanol 9:1, Rf-Wert = 0.2

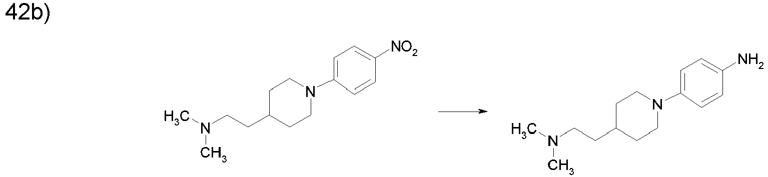

Eine Mischung aus 0.30 g (1.08 mMol) Produkt aus 42a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₅H₂₅N₃ (247.38)

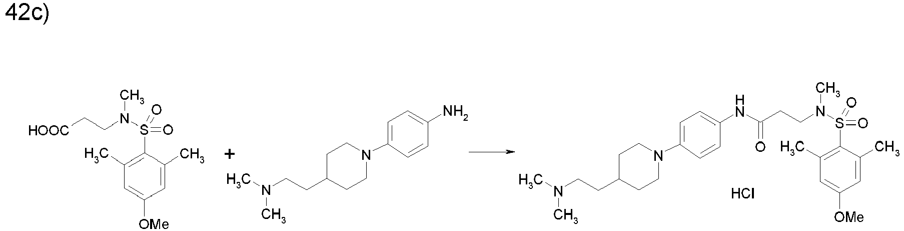

Beispiel 42 wird analog zu 1f aus 0.33 g (1.08 mMol) Produkt aus 22c, 0.27 g (1.08 mMol) Produkt aus 42b, 0.38 ml (2.70 mMol) Triethylamin und 0.35g (1.08 mMol) TBTU in 7 ml THF und 1 ml DMF hergestellt.
C₂₈H₄₂N₄O₄S (530.72)
[M+H]+ = 531
HPLC (Methode 5): Retentionszeit = 1.41 min

### Beispiel 43 (Referenzbeispiel)

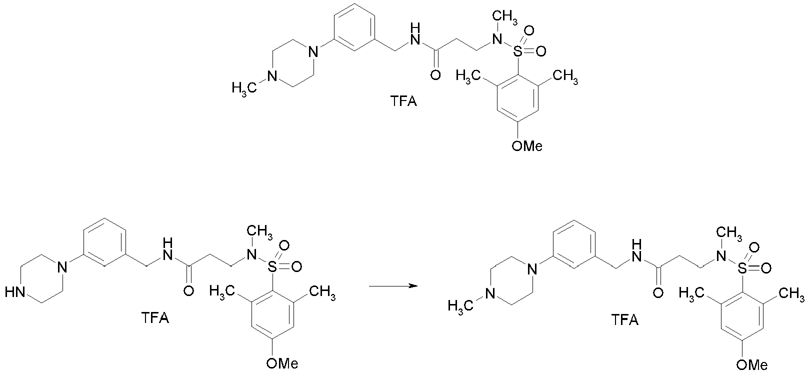

Eine Mischung aus 0.15 g (0.25 mMol) Produkt aus 36d, 0.025 ml (0.40 mMol) Methyliodid (Aldrich), 0.10 ml (0.75 mMol) Kaliumcarbonat und 5 ml Acetonitril wird über Nacht bei Raumtemperatur gerührt. Die Reaktionsmischung wird danach mit 10%iger TFA versetzt, das Produkt durch präparative HPLC abgetrennt.
C₂₅H₃₆N₄O₄S x C₂HF₃O₂ (602.67)
[M+H]+ = 489
HPLC (Methode 2): Retentionszeit = 2.99 min

### Beispiel 44 (Referenzbeispiel)

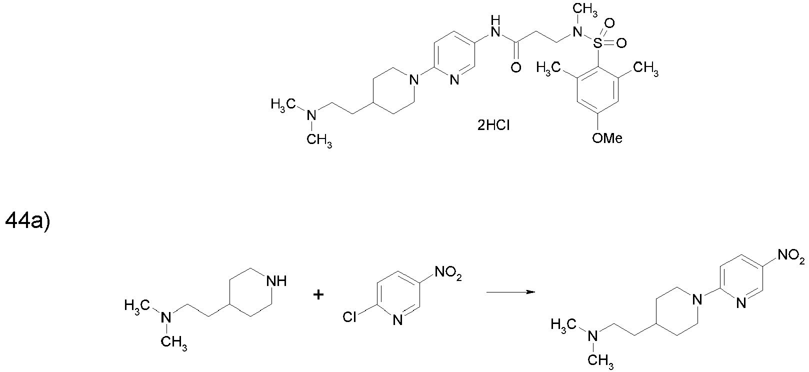

Eine Mischung aus 0.72 g (4.61 mMol) Dimethyl-(2-piperidin-4-yl-ethyl)-amin (J. Med. Chem. 36, 1993, 162-165), 0.73 g (4.61 mMol) 2-Chlor-5-nitropyridin (Fluka), 1.30 g (9.41 mMol) Kaliumcarbonat und 100 ml THF wird über das Wochenende bei Raumtemperatur gerührt. Der Niederschlag wird abfiltriert, das Filtrat wird im Vakuum bis zur Trockene eingeengt. Das so erhaltene Rohprodukt wird durch Säulenchromatographie über Kieselgel (Elutionsmittel: Dichlormethan / Methanol 19:1 bis 4:1) gereinigt.
C₁₄H₂₂N₄O₂ (278.35)
[M+H]+ = 279

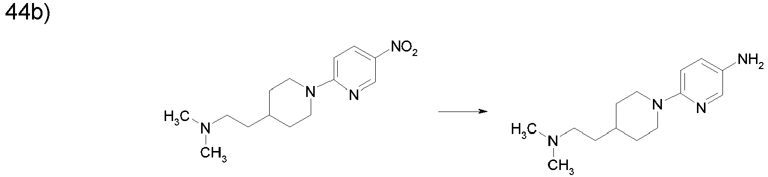

Eine Mischung aus 0.26 g (0.93 mMol) Produkt aus 44a, 0.05 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₄H₂₄N₄ (248.37)
[M+H]+ = 249

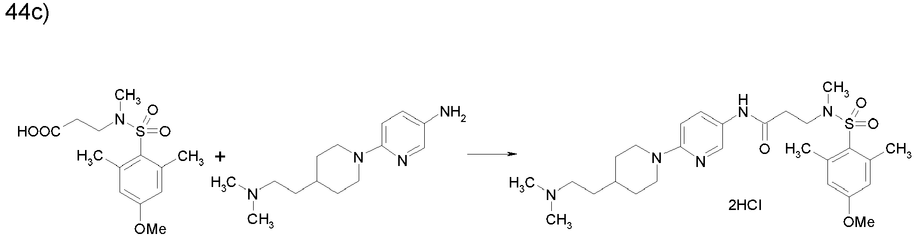

Beispiel 44 wird analog zu 1f aus 0.12 g (0.40 mMol) Produkt aus 22c, 0.10 g (0.40 mMol) Produkt aus 44b, 0.069 ml (0.50 mMol) Triethylamin und 0.14g (0.44 mMol) TBTU in 40 ml THF und 5 ml DMF hergestellt.
C₂₇H₄₁N₅O₄S x 2HCl (604.63)
[M+H]+ = 532
HPLC (Methode 5): Retentionszeit = 1.40 min

### Beispiel 45 (Referenzbeispiel)

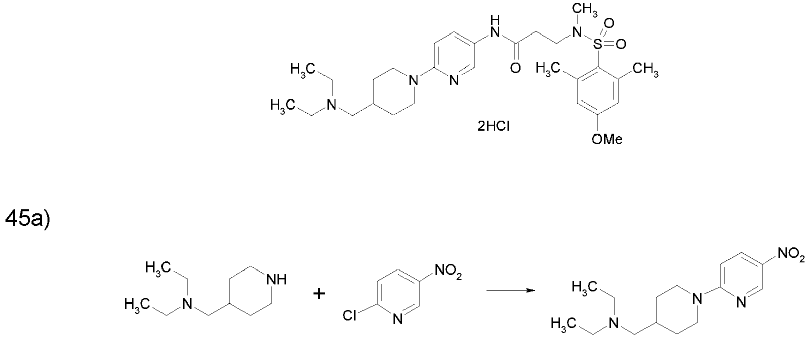

45a wird analog zu 44a aus 1.00 g (5.87 mMol) Diethyl-piperidin-4-ylmethyl-amin (Chem. Pharm. Bull. 42, 1994, 74-84), 0.93 g (5.87 mMol) 2-Chlor-5-nitropyridin (Fluka) und 1.70 g (12.30 mMol) Kaliumcarbonat in 100 ml THF hergestellt.
C₁₅H₂₄N₄O₂ (292.38)
[M+H]+ = 293

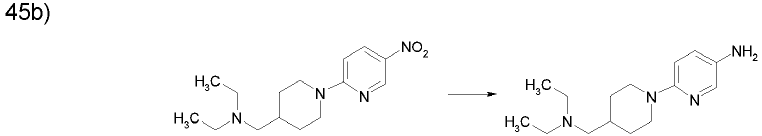

Eine Mischung aus 0.20 g (0.68 mMol) Produkt aus 45a, 0.03 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₅H₂₆N₄ (262.39)
[M+H]+ = 263

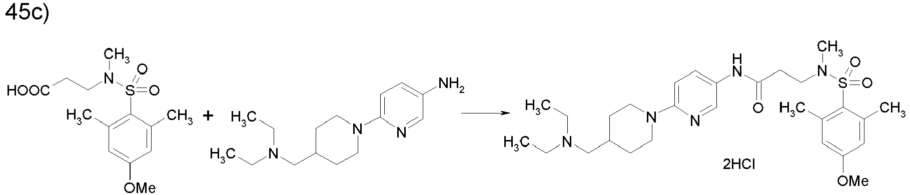

Beispiel 45 wird analog zu 1f aus 0.16 g (0.53 mMol) Produkt aus 22c, 0.14 g (0.53 mMol) Produkt aus 45b, 0.096 ml (0.69 mMol) Triethylamin und 0.19 g (0.58 mMol) TBTU in 40 ml THF und 5 ml DMF hergestellt.
C₂₈H₄₃N₅O₄S x 2HCl (618.66)
[M+H]+ = 546
HPLC (Methode 5): Retentionszeit = 1.40 min

### Beispiel 46 (Referenzbeispiel)

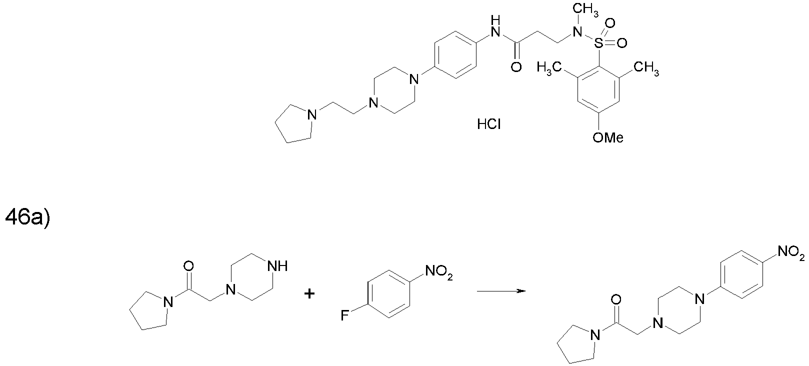

46a wird analog zu 1d aus 3.00 g (15.21 mMol) 2-Piperazin-1-yl-1-pyrrolidin-1-yl-ethanon (Chess), 2.15 g (15.21 mMol) 1-Fluor-4-nitrobenzol (Aldrich) und 3.07 ml (22.00 mMol) Triethylamin in 25 ml DMF hergestellt.
C₁₆H₂₂N₄O₃ (318.37)
[M+H]+ = 319
DC: Kieselgel, Dichlormethan / Ethanol 9:1, Rf-Wert = 0.4

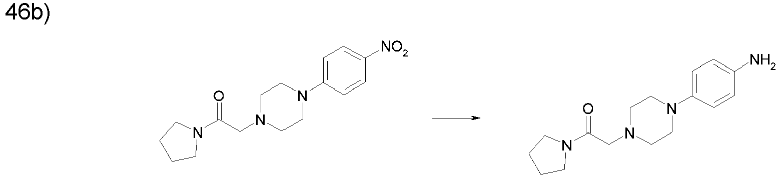

Eine Mischung aus 3.00 g (9.42 mMol) Produkt aus 46a, 0.30 g Palladium auf Kohle (10%) und 200 ml Methanol wird bei Raumtemperatur im Autoklaven hydriert. Der Katalysator wird danach abfiltriert, das Filtrat im Vakuum bis zur Trockene eingeengt.
C₁₆H₂₄N₄O (288.39)
DC: Kieselgel, Dichlormethan / Ethanol 9:1, Rf-Wert = 0.42

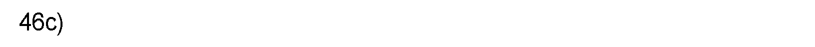

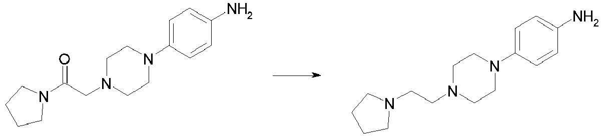

30.00 ml (30.00 mMol) Lithiumaluminiumhydrid 1 M in THF (Aldrich) wird in 50 ml THF vorgelegt und bei Raumtemperatur mit einer Mischung aus 2.70 g (9.36 mMol) Produkt aus 46b und 20 ml THF versetzt. Die Reaktionsmischung wird danach drei Stunden bei Raumtemperatur gerührt und anschließend unter Eisbadkühlung mit 20%iger Natronlauge versetzt. Der entstandene Niederschlag wird abfiltriert, das Filtrat im Vakuum bis zur Trockene eingeengt.
C₁₆H₂₆N₄ (274.40)

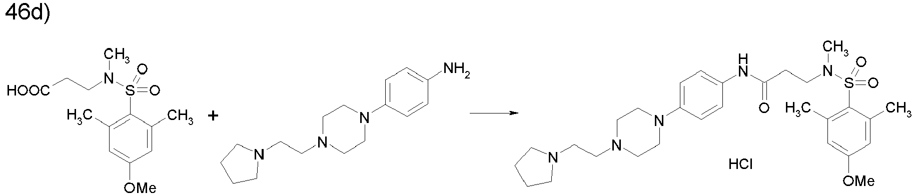

Beispiel 46 wird analog zu 1f aus 0.30 g (1.00 mMol) Produkt aus 22c, 0.27 g (1.00 mMol) Produkt aus 46c, 0.35 ml (2.50 mMol) Triethylamin und 0.32 g (1.00 mMol) TBTU in 7 ml THF und 1 ml DMF hergestellt.
C₂₉H₄₃N₅O₄S x HCl (594.21)
[M+H]+ = 558
HPLC (Methode 5): Retentionszeit = 1.43 min

### Beispiel 47 (Referenzbeispiel)

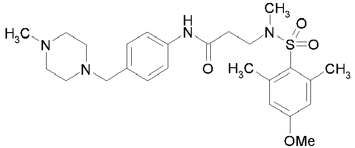

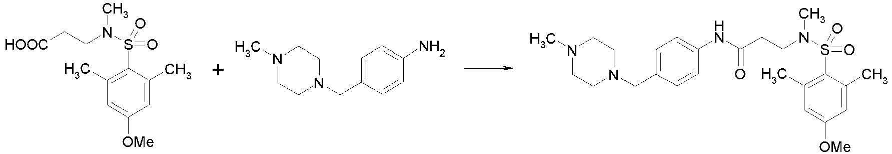

Beispiel 47 wird analog zu 1f aus 0.20 g (0.66 mMol) Produkt aus 22c, 0.14 g (0.66 mMol) 4-(4-Methyl-piperazin-1-ylmethyl)-phenylamin (Med. Chem. Res. 9, 1999, 149-161), 0.28 ml (1.99 mMol) Triethylamin und 0.21 g (0.66 mMol) TBTU in 5 ml THF hergestellt.
C₂₅H₃₆N₄O₄S (488.64)
[M+H]+ = 489
HPLC (Methode 5): Retentionszeit = 1.42 min

### Beispiel 48 (Referenzbeispiel)

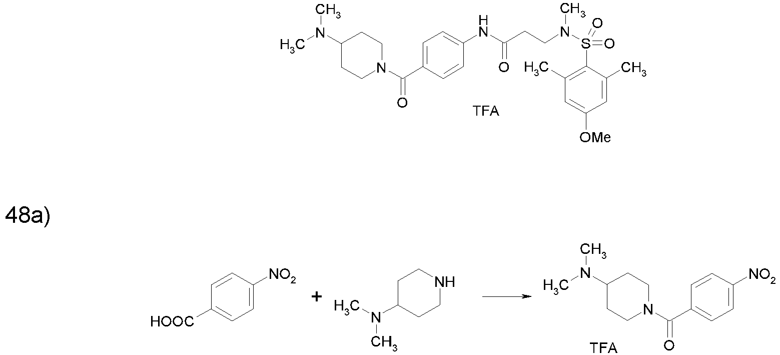

48a wird analog zu 1f aus 0.24 g (1.45 mMol) 4-Nitrobenzoesäure (Aldrich), 0.19 g (1.45 mMol) 4-Dimethylamino-piperidin (Alfa Aesar), 0.21 ml (1.52 mMol) Triethylamin und 0.49 g (1.52 mMol) TBTU in 8 ml DMF hergestellt.
C₁₄H₁₉N₃O₃ x C₂HF₃O₂ (391.34)
[M+H]+ = 278
HPLC (Methode 2): Retentionszeit = 2.29 min

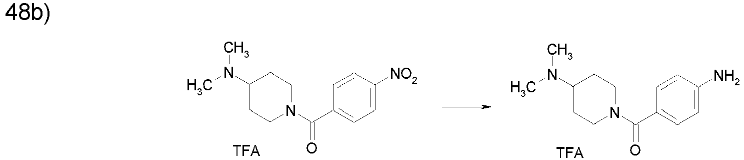

Eine Mischung aus 0.36 g (0.92 mMol) Produkt aus 48a, 0.092 g Palladium auf Kohle (10%) und 5 ml Methanol wird bei Raumtemperatur im Autoklaven hydriert. Der Katalysator wird danach abfiltriert, das Filtrat im Vakuum bis zur Trockene eingeengt.
C₁₄H₂₁N₃O x C₂HF₃O₂ (361.36)
[M+H]+ = 248
HPLC (Methode 2): Retentionszeit = 0.66 min

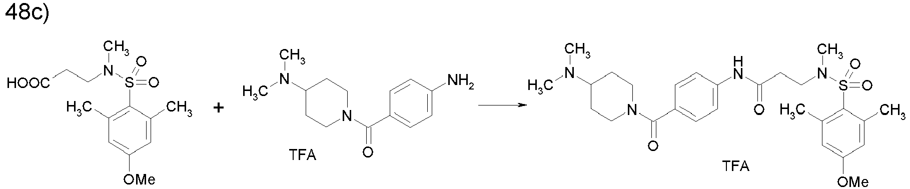

Beispiel 48 wird analog zu 1f aus 0.15 g (0.50 mMol) Produkt aus 22c, 0.18 g (0.50 mMol) Produkt aus 48b, 0.21 ml (1.49 mMol) Triethylamin und 0.16 g (0.50 mMol) TBTU in 3 ml THF hergestellt.
C₂₇H₃₈N₄O₅S x C₂HF₃O₂ (644.70)
[M+H]+ = 531
HPLC (Methode 5): Retentionszeit = 1.48 min

### Beispiel 49 (Referenzbeispiel)

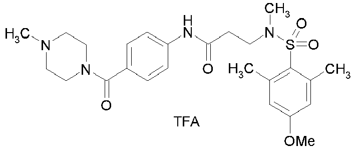

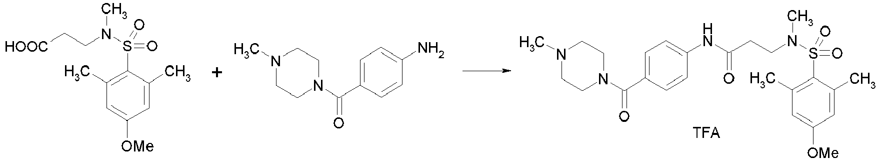

Beispiel 49 wird analog zu 1f aus 0.15 g (0.50 mMol) Produkt aus 22c, 0.11 g (0.50 mMol) (4-Aminophenyl)-(4-methylpiperazin-1-yl)-methanon (J. Org. Chem. 24, 1959, 459-463), 0.21 ml (1.49 mMol) Triethylamin und 0.16 g (0.50 mMol) TBTU in 3 ml THF hergestellt.
C₂₅H₃₄N₄O₅S x C₂HF₃O₂ (616.65)
[M+H]+ = 503
HPLC (Methode 5): Retentionszeit = 1.47 min

### Beispiel 50 (Referenzbeispiel)

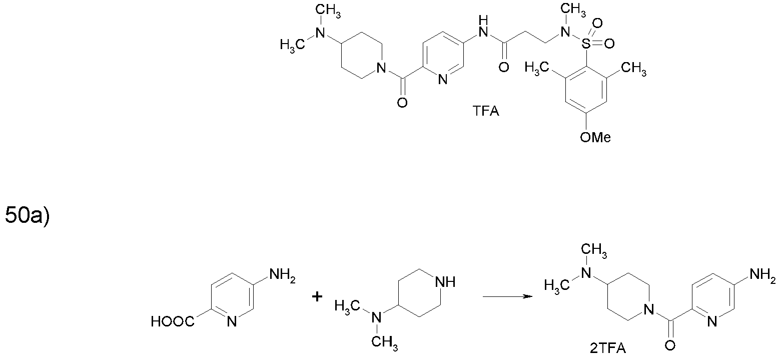

50a wird analog zu 1f aus 0.60 g (4.34 mMol) 5-Amino-pyridin-2-carbonsäure (Pharm. Acta Helv. 44, 1969, 637-643), 0.56 g (4.34 mmol) 4-Dimethylamino-piperidin (Alfa Aesar), 0.64 ml (4.56 mMol) Triethylamin und 1.46 g (4.56 mMol) TBTU in 24 ml DMF hergestellt.
C₁₃H₂₀N₄O x 2C₂HF₃O₂ (476.37)
HPLC (Methode 2): Retentionszeit = 0.65 min

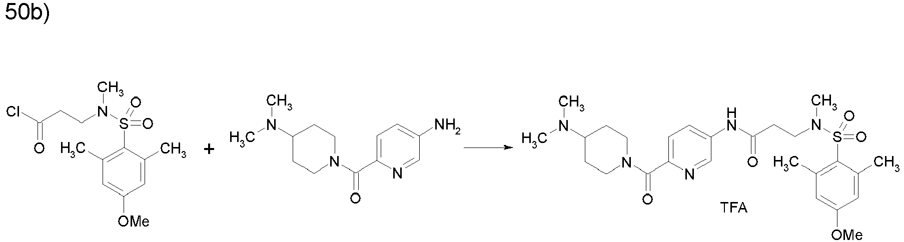

Eine Mischung aus 0.64 g (1.99 mMol) Produkt aus 27c, 1.90 g (2.39 mMol) Produkt aus 50a, 0.08 g (0.33 mMol) DMAP und 16 ml Chlorbenzol wird 39 Stunden auf 15°C erhitzt. Die Reaktionsmischung wird danach im Vakuum bis zur Trockene eingeengt. Das Produkt wird durch präparative HPLC erhalten.
C₂₆H₃₇N₅O₅S x C₂HF₃O₂ (645.69)
[M+H]+ = 532
HPLC (Methode 5): Retentionszeit = 1.44 min

Beispiel 51 (Referenzbeispiel)

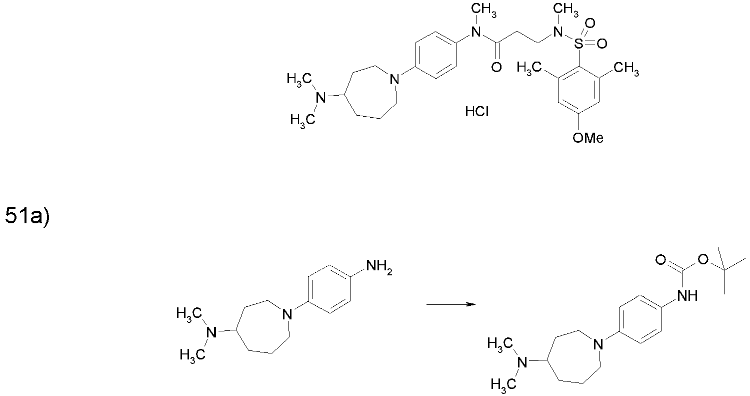

Eine Mischung aus 0.26 g (1.11 mMol) Produkt aus 39d, 0.27 g (1.22 mMol) Boc-Anhydrid, 0.17 ml (1.22 mMol) Triethylamin und 15 ml Dichlormethan wird über Nacht bei Raumtemperatur gerührt. Danach wird die Reaktionsmischung mit Dichlormethan verdünnt, mit gesättigter Natriumhydrogencarbonat-Lösung und gesättigter Natriumchlorid-Lösung gewaschen, über Natriumsulfat getrocknet und im Vakuum bis zur Trockene eingeengt.
C₁₉H₃₁N₃O₂ (333.47)
[M+H]+ = 334
HPLC (Methode 1): Retentionszeit = 2.40 min

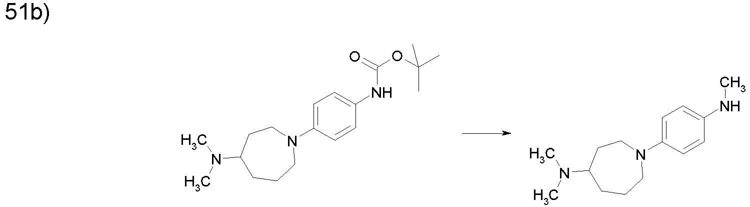

0.13 g (3.40 mMol) Lithiumaluminiumhydrid werden in 5 ml THF vorgelegt, auf 60°C erhitzt und mit 0.38 g (1.14 mMol) Produkt aus 51a in 5 ml THF versetzt. Die Reaktionsmischung wird danach vier Stunden unter Rückfluß erhitzt und über Nacht bei Raumtemperatur gerührt. Anschließend wird die Reaktionsmischung mit Wasser und 1 M Natronlauge gequencht. Der entstandene Niederschlag wird über Celite abfiltriert, das Filtrat im Vakuum bis zur Trockene eingeengt.
C₁₅H₂₅N₃ (247.38)
DC: Kieselgel, Dichlormethan / Methanol / Ammoniak 4:1:0.2, Rf-Wert = 0.68

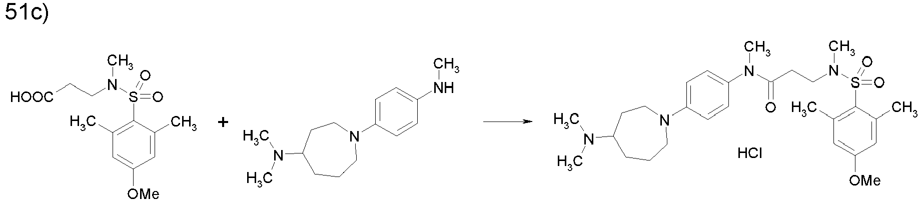

Beispiel 51 wird analog zu 1f aus 0.15 g (0.50 mMol) Produkt aus 22c, 0.12 g (0.50 mMol) Produkt aus 51b, 0.21 ml (1.50 mMol) Triethylamin und 0.18 g (0.55 mMol) TBTU in 8 ml DMF hergestellt.
C₂₈H₄₂N₄O₄S x HCl (567.18)
[M+H]+ = 531
HPLC (Methode 1): Retentionszeit = 2.5 min

### Beispiel 52 (Referenzbeispiel)

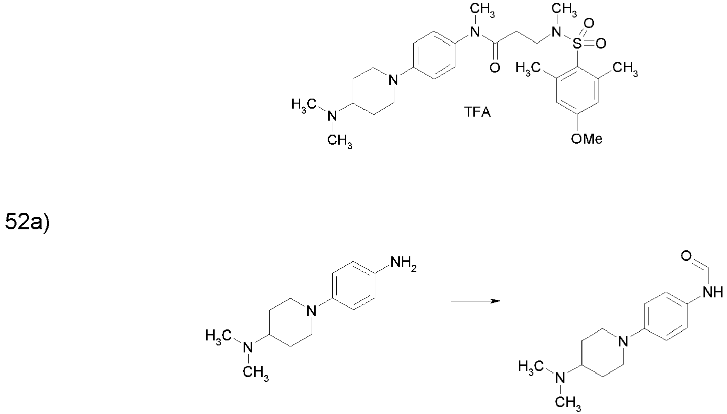

0.70 ml (7.36 mMol) Essigsäureanhydrid werden unter Stickstoff-Atmosphäre vorgelegt und unter Eisbadkühlung langsam mit 0.42 ml (9.06 mMol) Ameisensäure versetzt. Die Reaktionsmischung wird zwei Stunden auf 50-60°C erhitzt und anschließend unter Eisbadkühlung mit 0.50 g (2.28 mMol) Produkt aus 8b in 7 ml Dichlormethan versetzt. Nach 20-minütigem Rühren bei Raumtemperatur wird die Reaktionsmischung im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₄H₂₁N₃O (247.34)
[M+H]+ = 248
HPLC (Methode 5): Retentionszeit = 0.50 min

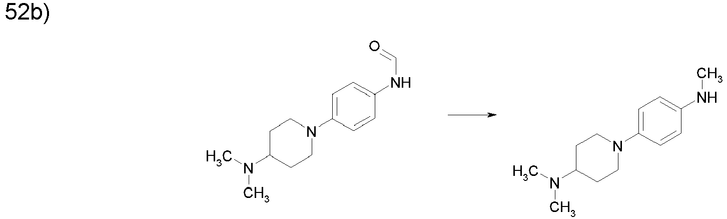

52b wird analog zu 51 b aus 0.17 g (4.51 mMol) Lithiumaluminiumhydrid und 0.58 g (2.34 mMol) Produkt aus 52a in 10 ml THF hergestellt.
C₁₄H₂₃N₃ (233.35)
DC: Kieselgel, Dichlormethan / Methanol / Ammoniak 9:1:0.1, Rf-Wert = 0.5

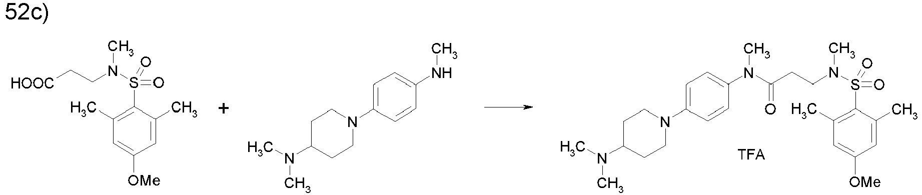

Beispiel 52 wird analog zu 1f aus 0.21 g (0.68 mMol) Produkt aus 22c, 0.24 g (0.68 mMol) Produkt aus 52b, 0.28 ml (2.04 mMol) Triethylamin und 0.22 g (0.68 mMol) TBTU in 4 ml THF hergestellt.
C₂₇H₄₀N₄O₄S x C₂HF₃O₂ (630.72)
[M+H]+ = 517
HPLC (Methode 5): Retentionszeit = 1.50 min

### Beispiel 53 (Referenzbeispiel)

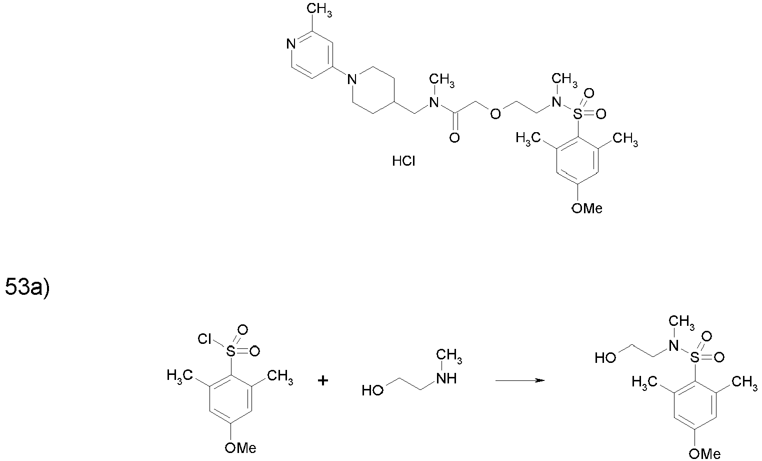

53a wird analog zu 3a aus 4.50 g (19.17 mMol) Produkt aus 13a, 1.69 g (21.10 mMol) N-Methylaminoethanol (BASF), 6.68 ml (47.90 mMol) Triethylamin in 150 ml Dichlormethan hergestellt.
C₁₂H₁₉NO₄S (273.35)
[M+H]+ = 274
DC: Kieselgel, Dichlormethan / Ethanol 19:1, Rf-Wert = 0.43

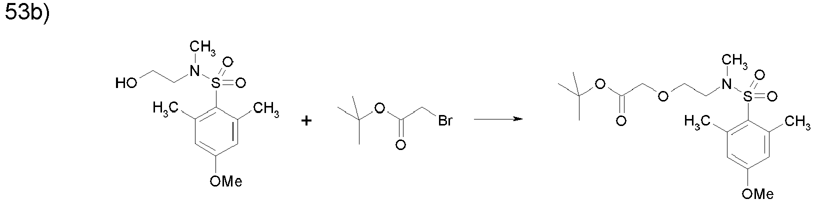

Eine Mischung aus 5.15 g (18.84 mMol) Produkt aus 53a, 1.75 g (6.60 mMol) Tetrabutylammoniumchlorid (Fluka) und 80 ml Toluol wird bei 0°C erst mit 100 ml 35%iger Natronlauge, dann mit 4.18 ml (28.26 mMol) Bromessigsäure-tert-butylester in 20 ml Toluol versetzt. Die Reaktionsmischung wird anschließend 1.5 Stunden bei Raumtemperatur gerührt, dann mit Diethylether verdünnt. Nach der Phasentrennung wird die organische Phase viermal mit Wasser neutral gewaschen, über Natriumsulfat getrocknet und im Vakuum bis zur Trockene eingeengt. Das so erhaltene Rohprodukt wird durch Säulenchromatographie über Kieselgel (Elutionsmittel: Petrolether/ Ethylacetat 4:1) gereinigt.
C₁₈H₂₉NO₆S (387.49)
[M+H]+ = 388
DC: Kieselgel, Petrolether / Ethylacetat 7:3, Rf-Wert = 0.59

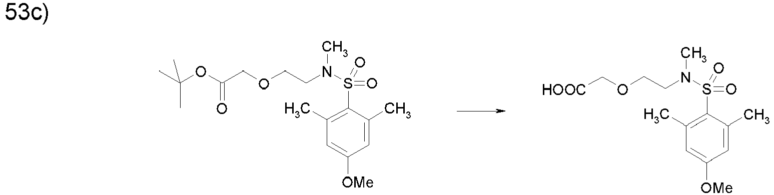

Eine Mischung aus 6.80 g (17.55 mMol) Produkt aus 53b, 8 ml TFA und 100 ml Dichlormethan wird 2.5 Stunden bei Raumtemperatur gerührt. Die Reaktionsmischung wird danach im Vakuum bis zur Trockene eingeengt. Man versetzt den Rückstand mit 1 M Natronlauge und extrahiert zweimal mit Ethylacetat (organische Extrakte verwerfen). Die wässrige Phase wird mit 2 M HCl angesäuert, dann nochmals mit Ethylacetat extrahiert. Die organischen Extrakte werden mit Wasser gewaschen, über Natriumsulfat getrocknet und im Vakuum bis zur Trockene eingeengt.
C₁₄H₂₁NO₆S (331.29)
[M+H]+ = 332
HPLC (Methode 4): Retentionszeit = 3.4 min

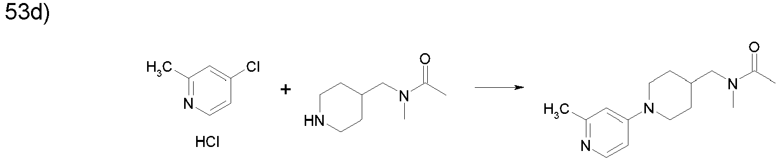

53d wird analog zu 28c aus 1.00 g (6.10 mMol) 4-Chlor-2-methylpyridin Hydrochlorid (Alfa Aesar) und 2.08 g (12.20 mMol) N-Methyl-N-piperidin-4-ylmethyl-acetamid (DE 1100635, Rhône-Poulenc, 1961) hergestellt.
C₁₅H₂₃N₃O (261.36)
[M+H]+ = 262
HPLC (Methode 4): Retentionszeit = 1.9 min

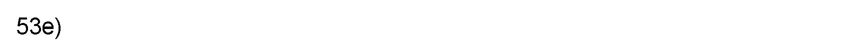

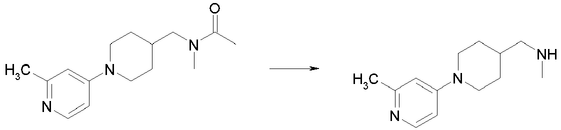

Eine Mischung aus 1.37 g (5.24 mMol) Produkt aus 53d und 15 ml halbkonzentrierte HCl wird vier Tage unter Rückfluss erhitzt. Die Reaktionsmischung wird mit 20%iger Natronlauge alkalisch gestellt und mit Dichlormethan extrahiert. Die organischen Extrakte werden über Natriumsulfat getrocknet und im Vakuum bis zur Trockene eingeengt.
C₁₃H₂₁N₃ (219.33)
[M+H]+ = 220
DC: Kieselgel, Dichlormethan / Methanol / Ammoniak 8:2:0.2, Rf-Wert = 0.48

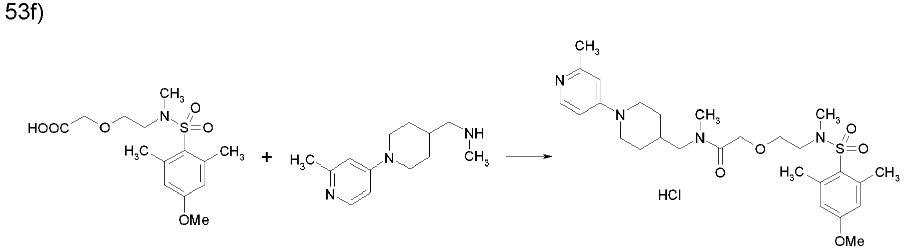

Beispiel 53 wird analog zu 1f aus 0.099 g (0.30 mMol) Produkt aus 53c, 0.066 g (0.30 mMol) Produkt aus 53e, 0.10 ml (0.75 mMol) Triethylamin und 0.12 g (0.36 mMol) TBTU in 8 ml THF und 1 ml DMF hergestellt.
C₂₇H₄₀N₄O₅S x HCl (569.16)
[M+H]+ = 533
HPLC (Methode 4): Retentionszeit = 3.1 min

### Beispiel 54 (Referenzbeispiel)

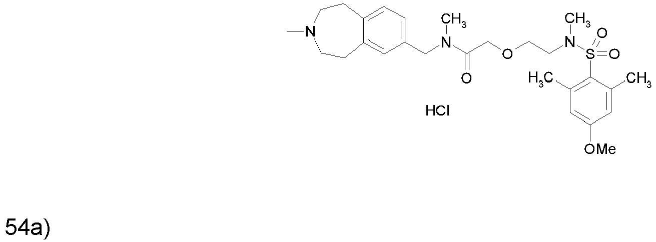

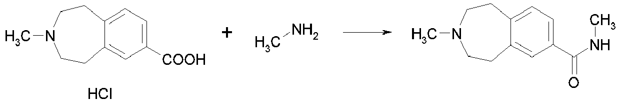

54a wird analog zu 1f aus 2.00 g (8.27 mMol) Produkt aus 38d, 8.28 ml (16.55 mMol) Methylamin 2 M in THF (Aldrich), 3.46 ml (24.82 mMol) Triethylamin und 3.19 g (9.93 mMol) TBTU in 30 ml THF hergestellt.
C₁₃H₁₈N₂O (218.29)
[M+H]+ = 219
DC: Kieselgel, Dichlormethan / Methanol 8:2, Rf-Wert = 0.14

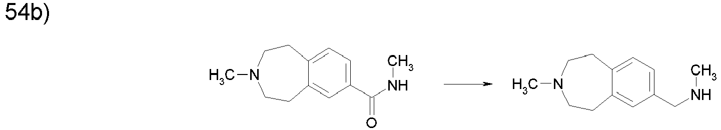

54b wird analog zu 38f aus 1.00 g (4.58 mMol) Produkt aus 54a und 9.00 ml (9.00 mMol) Lithiumaluminiumhydrid 1 M in THF (Aldrich) in 30 ml THF hergestellt.
C₁₃H₂₀N₂ (204.31)
[M+H]+ = 205
DC: Kieselgel, Dichlormethan / Methanol 8:2, Rf-Wert = 0.07

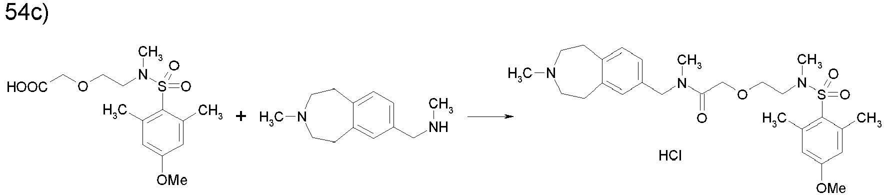

Beispiel 54 wird analog zu 1f aus 0.099 g (0.30 mMol) Produkt aus 53c, 0.088 g (0.30 mMol) Produkt aus 54b, 0.10 ml (0.75 mMol) Triethylamin und 0.12 g (0.36 mMol) TBTU in 7 ml THF und 1 ml DMF hergestellt.
C₂₇H₃₉N₃O₅S x HCl (554.14)
[M+H]+ = 518
HPLC (Methode 4): Retentionszeit = 3.1 min

### Beispiel 55 (Referenzbeispiel)

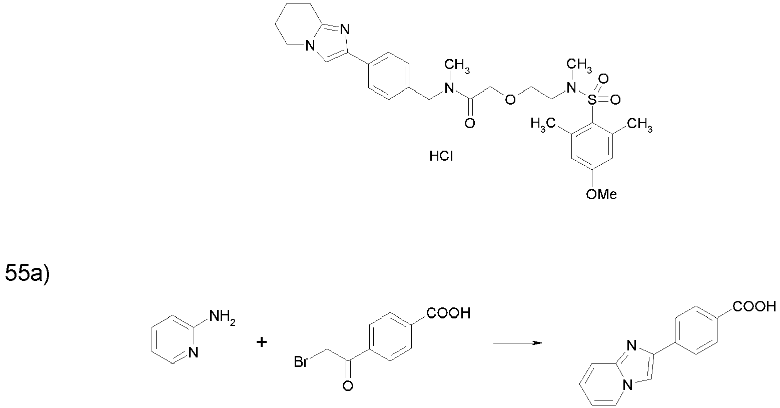

Eine Mischung aus 3.20 g (34.00 mMol) 2-Aminopyridin (Aldrich), 2.75 g (11.31 mMol) 4-Bromacetylbenzoesäure (Fluorochem) und 100 ml Ethanol wird sechs Stunden unter Rückfluß erhitzt und über Nacht bei Raumtemperatur gerührt. Der entstandene Niederschlag wird abfiltriert und getrocknet.
C₁₄H₁₀N₂O₂ (238.24)
[M+H]+ = 239

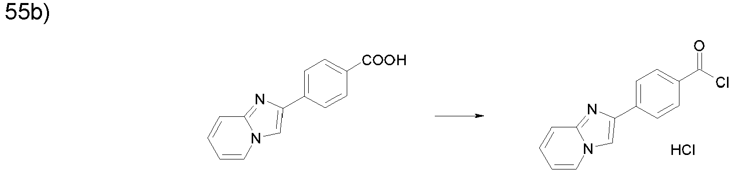

55b wird analog zu 27c aus 1.7 g (7.14 mMol) Produkt aus 55a und 30 ml Thionylchlorid hergestellt.
C₁₄H₉ClN₂O x HCl (293.15)

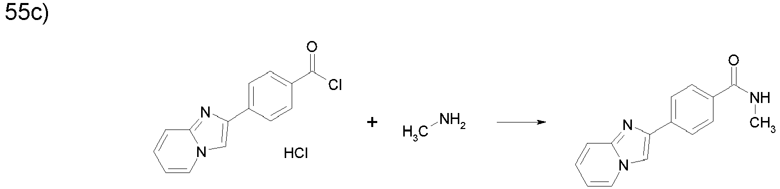

2.10 g (7.14 mMol) Produkt aus 55b in 100 ml Dichlormethan werden unter Eisbadkühlung mit 25 ml (50.00 mMol) Methylamin 2 M in THF (Aldrich) versetzt. Die Reaktionsmischung wird danach zwei Stunden bei Raumtemperatur gerührt und anschließend im Vakuum bis zur Trockene eingeengt. Der Rückstand wird mit Wasser verrieben, abfiltriert und getrocknet. Das so erhaltene Rohprodukt wird durch Säulenchromatographie über Kieselgel (Elutionsmittel: Dichlormethan / Methanol 97:3) gereinigt.
C₁₅H₁₃N₃O (251.28)
[M+H]+ = 252

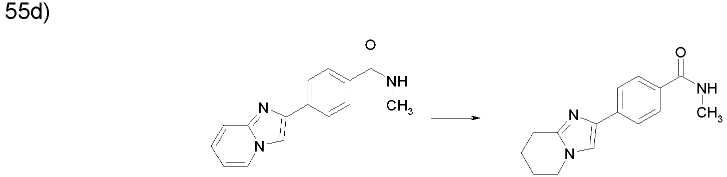

Eine Mischung aus 0.70 g (2.79 mMol) Produkt aus 55c, 0.15 g Palladium auf Kohle (20%), 100 ml Methanol und 30 ml Dichlormethan wird bei Raumtemperatur im Autoklaven hydriert. Der Katalysator wird abfiltriert, das Filtrat im Vakuum bis zur Trockene eingeengt.
C₁₅H₁₇N₃O (255.32)
[M+H]+ = 256

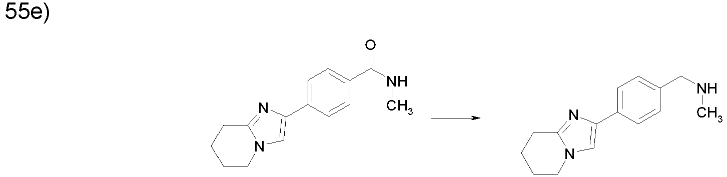

55e wird analog zu 38f aus 0.80 g (3.13 mMol) Produkt aus 55d und 20.00 ml (20.00 mMol) Lithiumaluminiumhydrid 1 M in THF (Aldrich) in 50 ml Pyridin hergestellt.
C₁₅H₁₉N₃ (241.33)
[M+H]+ = 242

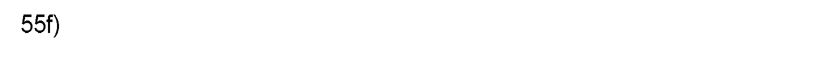

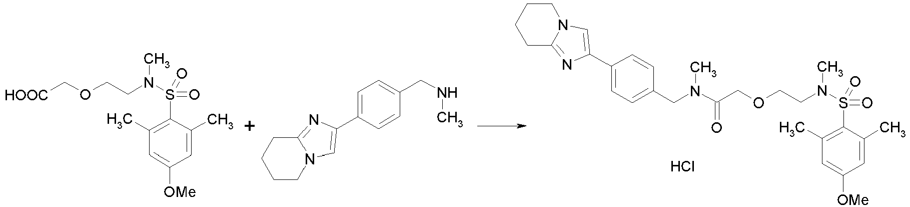

Beispiel 55 wird analog zu 1f aus 0.14 g (0.42 mMol) Produkt aus 53c, 0.10 g (0.41 mMol) Produkt aus 55e, 0.14 ml (0.99 mMol) Triethylamin und 0.15 g (0.46 mMol) TBTU in 30 ml THF und 5 ml DMF hergestellt.
C₂₉H₃₈N₄O₅S x HCl (591.16)
[M+H]+ = 555
DC: Kieselgel, Dichlormethan / Methanol 9:1, Rf-Wert = 0.26

### Beispiel 56 (Referenzbeispiel)

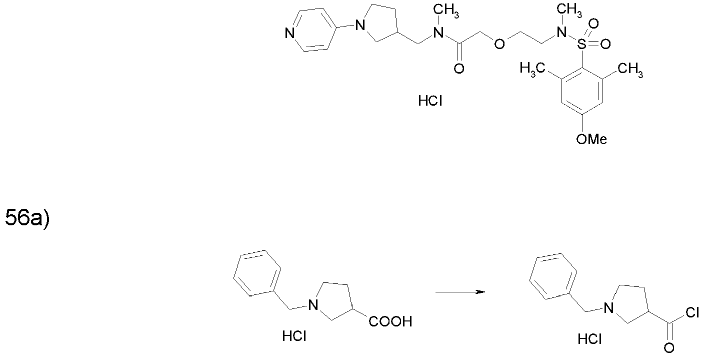

56a wird analog zu 27c aus 1.35 g (5.59 mMol) 1-Benzyl-pyrrolidine-3-carbonsäure (J. Org. Chem. 33, 1968, 3637-3639) und 10 ml Thionylchlorid hergestellt.
C₁₂H₁₄ClNO x HCl (260.16)

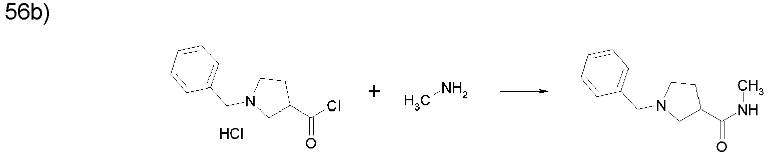

56b wird analog zu 55c aus 1.45 g (5.57 mMol) Produkt aus 56a, 10 ml (50.00 mMol) Methylamin 2 M in THF (Aldrich) in 50 ml THF hergestellt.
C₁₃H₁₈N₂O (218.29)
[M+H]+ = 219

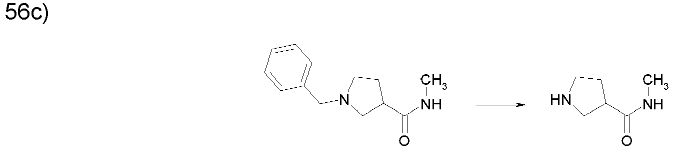

Eine Mischung aus 1.10 g (5.04 mMol) Produkt aus 56b, 0.20 g Palladiumhydroxid und 40 ml Methanol wird bei 50°C im Autoklaven hydriert. Der Katalysator wird abfiltriert, das Filtrat im Vakuum bis zur Trockene eingeengt.
C₆H₁₂N₂O (128.17)
[M+H]+ = 129

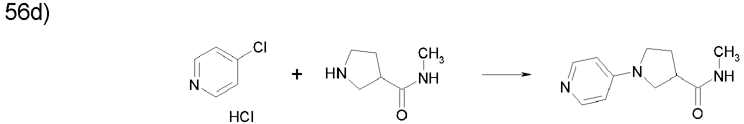

56d wird analog zu 28c aus 0.76 g (5.08 mMol) 4-Chlorpyridin Hydrochlorid (Aldrich), 0.65 g (5.07 mMol) Produkt aus 56c und 1.52 ml (10.88 mMol) Triethylamin in 10 ml Ethanol hergestellt.
C₁₁H₁₅N₃O (205.26)
[M+H]+ = 206

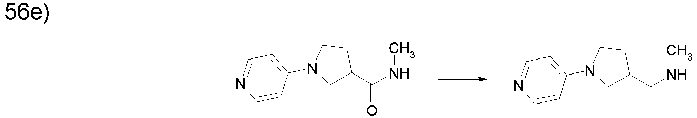

56e wird analog zu 38f aus 0.45 g (2.19 mMol) Produkt aus 56d und 7.00 ml (7.00 mMol) Lithiumaluminiumhydrid 1 M in THF (Aldrich) in 30 ml THF hergestellt.
C₁₁H₁₇N₃ (191.27)
[M+H]+ = 192

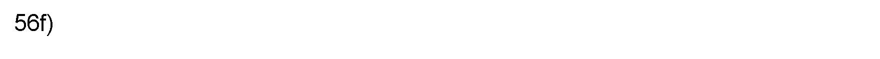

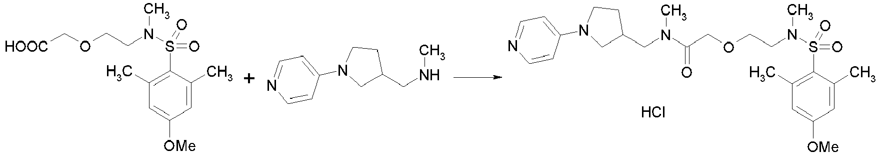

Beispiel 56 wird analog zu 1f aus 0.14 g (0.42 mMol) Produkt aus 53c, 0.10 g (0.42 mMol) Produkt aus 56e, 0.18 ml (1.29 mMol) Triethylamin und 0.18 g (0.56 mMol) TBTU in 30 ml THF und 5 ml DMF hergestellt.
C₂₅H₃₆N₄O₅S x HCl (541.10)
[M+H]+ = 505
HPLC (Methode 5): Retentionszeit = 1.51 min

### Beispiel 57 (Referenzbeispiel)

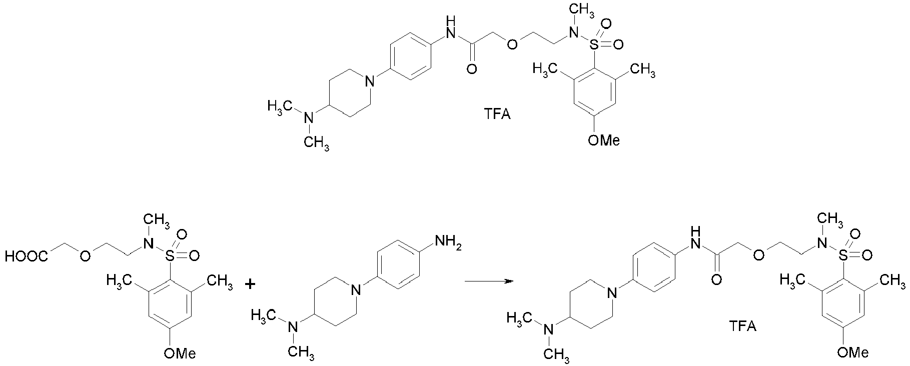

Beispiel 57 wird analog zu 1f aus 0.10 g (0.30 mMol) Produkt aus 53c, 0.066 g (0.30 mMol) Produkt aus 8b, 0.13 ml (0.91 mMol) Triethylamin und 0.097 g (0.30 mMol) TBTU in 5 ml DMF hergestellt.
C₂₇H₄₀N₄O₅S x C₂HF₃O₂ (646.72)
[M+H]+ = 533
HPLC (Methode 5): Retentionszeit = 1.51 min

### Beispiel 58 (Referenzbeispiel)

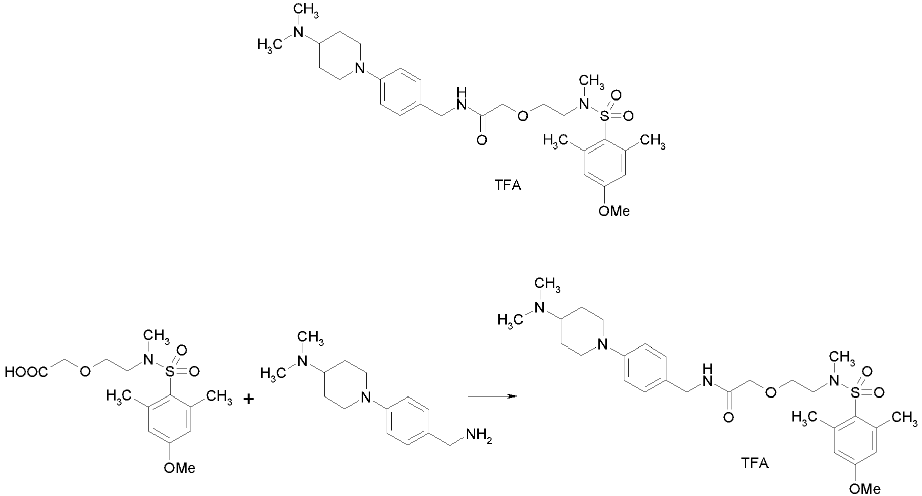

Beispiel 58 wird analog zu 1f aus 0.10 g (0.30 mMol) Produkt aus 53c, 0.07 g (0.30 mMol) Produkt aus 33a, 0.13 ml (0.91 mMol) Triethylamin und 0.097 g (0.30 mMol) TBTU in 5 ml DMF hergestellt.
C₂₈H₄₂N₄O₅S x C₂HF₃O₂ (660.75)
[M+H]+ = 547
HPLC (Methode 5): Retentionszeit = 1.48 min

### Beispiel 59 (Referenzbeispiel)

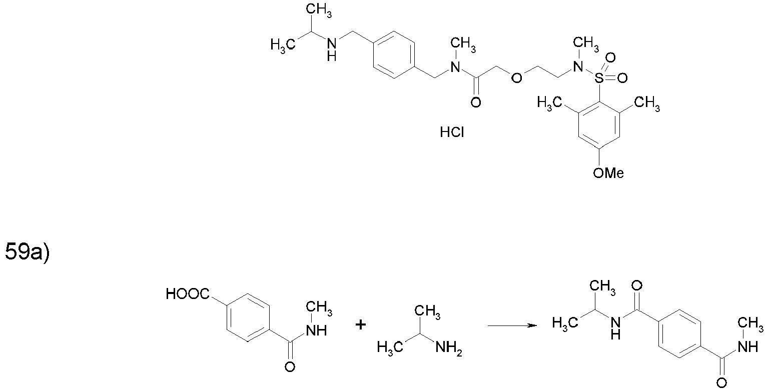

59a wird analog zu 1f aus 2.00 g (11.16 mMol) Terephthalsäuremonomethylamid (EMKA), 1.90 ml (22.32 mMol) Isopropylamin (Aldrich), 3.11 ml (22.32 mMol) Triethylamin und 4.30 g (13.40 mMol) TBTU in 60 ml THF hergestellt.
C₁₂H₁₆N₂O₂ (220.27)
[M+H]+ = 221
HPLC (Methode 4): Retentionszeit = 2.3 min

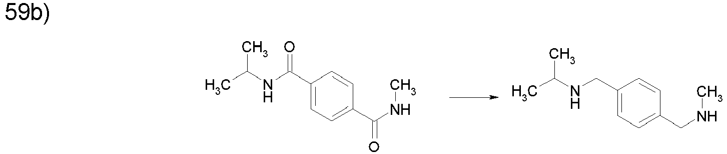

59b wird analog zu 38f aus 1.34 g (6.08 mMol) Produkt aus 59a und 25.00 ml (25.00 mMol) Lithiumaluminiumhydrid 1 M in THF (Aldrich) in 150 ml THF hergestellt.
C₁₂H₂₀N₂ (192.30)
[M+H]+ = 193
DC: Kieselgel, Dichlormethan / Methanol / Ammoniak 9:1:0.1, Rf-Wert = 0.17

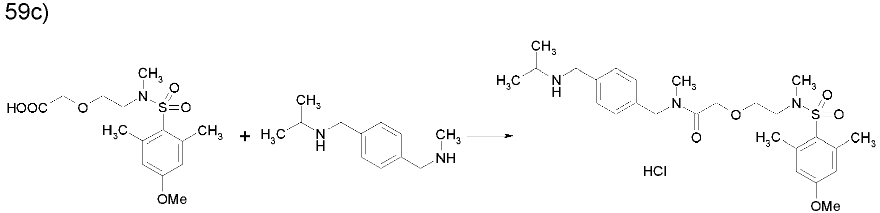

Beispiel 59 wird analog zu 1f aus 0.099 g (0.30 mMol) Produkt aus 53c, 0.058 g (0.30 mMol) Produkt aus 59b, 0.10 ml (0.75 mMol) Triethylamin und 0.12 g (0.36 mMol) TBTU in 8 ml THF hergestellt.
C₂₆H₃₉N₃O₅S x HCl (542.13)
[M+H]+ = 506
HPLC (Methode 4): Retentionszeit = 3.1 min

### Beispiel 60 (Referenzbeispiel)

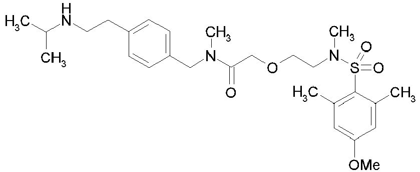

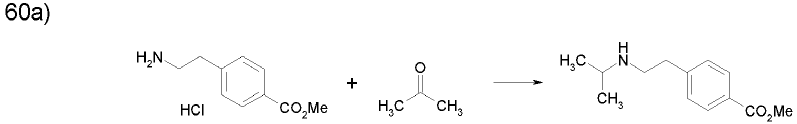

Eine Mischung aus 3.00 g (13.91 mMol) 4-Aminoethylbenzoesäuremethylester (EMKA), 1.94 ml (13.91 mMol) Triethylamin und 50 ml THF wird 10 min bei Raumtemperatur gerührt und danach mit 1.13 ml (15.30 mMol) Aceton versetzt. Die Reaktionsmischung wird weitere 30 min bei Raumtemperatur gerührt, dann werden 3.24 g (15.30 mMol) Natriumtriacetoxyborhydrid und 1.19 ml (20.86 mMol) Essigsäure hinzugefügt. Man lässt 16 Stunden bei Raumtemperatur rühren. Die Reaktionsmischung wird im Vakuum bis zur Trockene eingeengt, der Rückstand wird in 1 M HCl aufgenommen und mit Ethylacetat extrahiert (organische Phase verwerfen). Die wässrige Phase wird mit gesättigter Kaliumcarbonat-Lösung alkalisch gestellt und mit Ethylacetat extrahiert. Die organischen Extrakte werden über Natriumsulfat getrocknet und im Vakuum bis zur Trockene eingeengt.
C₁₃H₁₉NO₂ (221.30)
[M+H]+ = 222
HPLC (Methode 4): Retentionszeit = 2.2 min

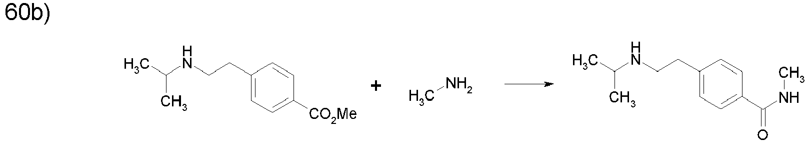

Eine Mischung aus 2.52 g (11.39 mMol) Produkt aus 60a, 11.40 ml (22.80 mMol) Methylamin 2 M in THF (Aldrich), 0.54 g (5.70 mMol) Magnesiumchlorid (Aldrich) und 100 ml THF wird 17 Stunden bei 120°C im Autoklaven gerührt. Die Reaktionsmischung wird filtriert, das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₃H₂₀N₂O (220.31)
[M+H]+ = 221
DC: Kieselgel, Dichlormethan / Methanol / Ammoniak 9:1:0.1, Rf-Wert = 0.21

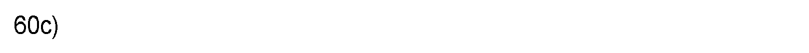

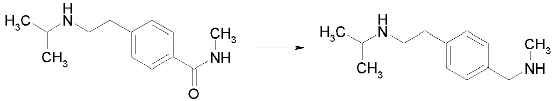

60c wird analog zu 38f aus 1.49 g (6.76 mMol) Produkt aus 60b und 10.00 ml (10.00 mMol) Lithiumaluminiumhydrid 1 M in THF (Aldrich) in 60 ml THF hergestellt.
C₁₃H₂₂N₂ (206.33)
[M+H]+ = 207
DC: Kieselgel, Dichlormethan / Methanol / Ammoniak 9:1:0.1, Rf-Wert = 0.10

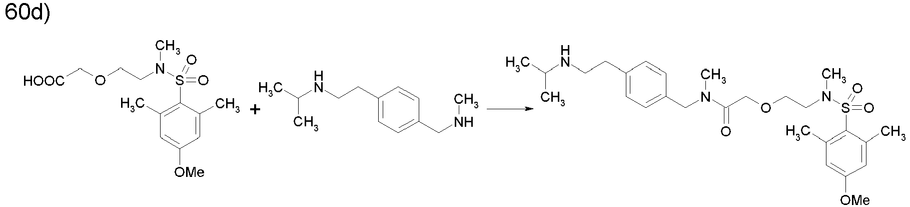

Beispiel 60 wird analog zu 1f aus 0.099 g (0.30 mMol) Produkt aus 53c, 0.062 g (0.30 mMol) Produkt aus 60c, 0.083 ml (0.60 mMol) Triethylamin und 0.12 g (0.36 mMol) TBTU in 8 ml THF hergestellt.
C₂₇H₄₁N₃O₅S (519.70)
[M+H]+ = 520
HPLC (Methode 4): Retentionszeit = 3.2 min

### Beispiel 61 (Referenzbeispiel)

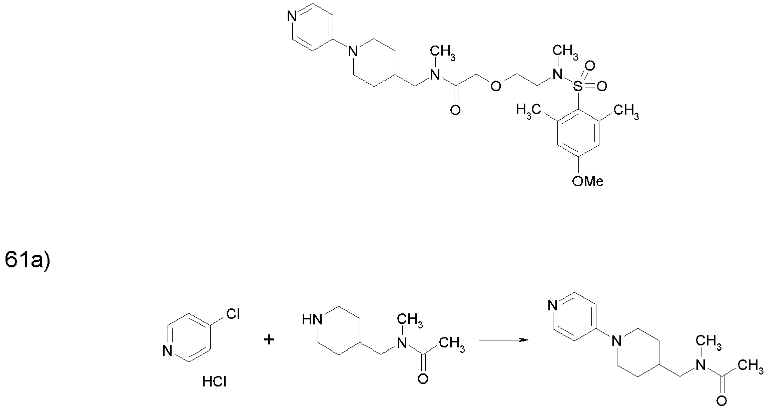

61a wird analog zu 28c aus 1.00 g (6.67 mMol) 4-Chlorpyridin Hydrochlorid (Aldrich) und 2.55 g (15.00 mMol) N-Methyl-N-piperidin-4-ylmethyl-acetamid (DE 110635, Rhöne-Poulenc, 1961) in 1 ml Wasser hergestellt.
C₁₄H₂₁N₃O (247.34)
[M+H]+ = 248

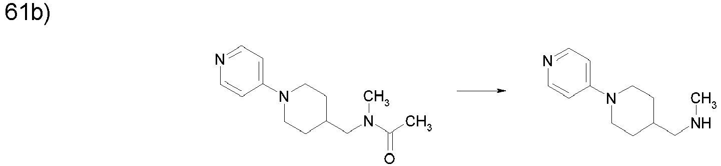

Eine Mischung aus 1.00 g (4.04 mMol) Produkt aus 61a und 10 ml halbkonzentrierte HCl wird drei Tage unter Rückfluss erhitzt. Die Reaktionsmischung wird danach mit Wasser verdünnt, mit 20%iger Natronlauge alkalisch gestellt und mit Ethylacetat extrahiert. Die organischen Extrakte werden über Natriumsulfat getrocknet und im Vakuum bis zur Trockene eingeengt.
C₁₂H₁₉N₃ (205.30)
DC: Kieselgel, Dichlormethan / Ethanol 9:1, Rf-Wert = 0.2

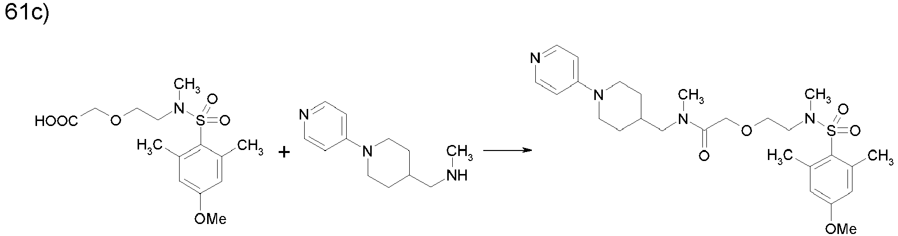

Beispiel 61 wird analog zu 1f aus 0.099 g (0.30 mMol) Produkt aus 53c, 0.062 g (0.30 mMol) Produkt aus 61 b, 0.083 ml (0.60 mMol) Triethylamin und 0.12 g (0.36 mMol) TBTU in 8 ml THF und 1 ml DMF hergestellt.
C₂₆H₃₈N₄O₅S (518.67)
[M+H]+ = 519
HPLC (Methode 4): Retentionszeit = 3.2 min

### Beispiel 62 (Referenzbeispiel)

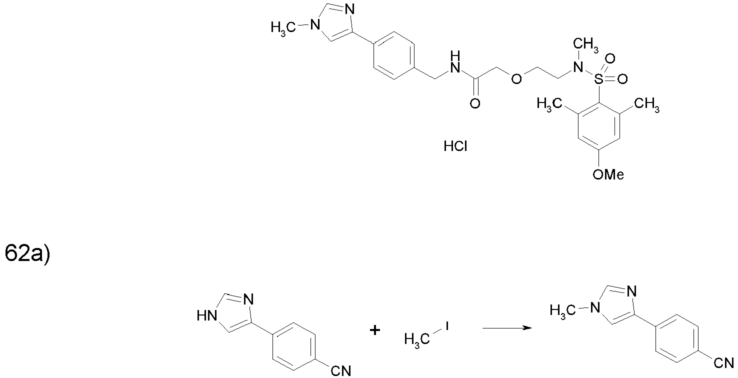

Eine Mischung aus 1.69 g (10.00 mMol) 4-(1H-Imidazol-4-yl)-benzonitril (J. Am. Chem. Soc. 93, 1971, 4256-4263), 1.12 g (10.00 mMol) Kalium-tert-butylat und 25 ml DMSO wird erst 30 min bei Raumtemperatur gerührt, dann mit 0.62 ml (10.00 mMol) Methyliodid langsam versetzt und anschließend noch weitere 2.5 Stunden bei Raumtemperatur gerührt. Die Reaktionsmischung wird danach in Wasser gegeben, der entstandene Niederschlag wird abfiltriert und im Vakuum getrocknet. Das so erhaltene Rohprodukt wird durch Säulenchromatographie über Kieselgel (Elutionsmittel: Dichlormethan / Ethanol 19:1) gereinigt.
C₁₁H₉N₃ (183.21)
[M+H]+ = 184
HPLC (Methode 4): Retentionszeit = 1.9 min

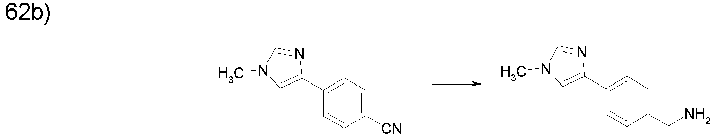

62b wird analog zu 34b aus 1.02 g (5.57 mMol) Produkt aus 62a, 0.20 g Raney-Nickel und 30 ml methanolischer Ammoniak-Lösung hergestellt.
C₁₁H₁₃N₃ (187.24)
DC: Kieselgel, Dichlormethan / Methanol / Ammoniak 9:1:0.1, Rf-Wert = 0.27

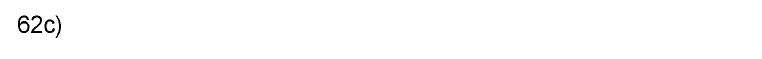

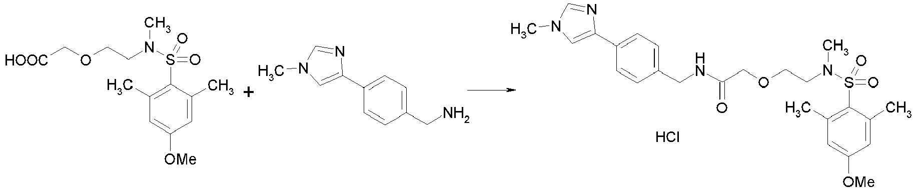

Beispiel 62 wird analog zu 1f aus 0.099 g (0.30 mMol) Produkt aus 53c, 0.056 g (0.30 mMol) Produkt aus 62b, 0.083 ml (0.60 mMol) Triethylamin und 0.12 g (0.36 mMol) TBTU in 7 ml THF und 1 ml DMF hergestellt.
C₂₅H₃₂N₄O₅S x HCl (537.07)
[M+H]+ = 501
HPLC (Methode 4): Retentionszeit = 3.2 min

### Beispiel 63 (Referenzbeispiel)

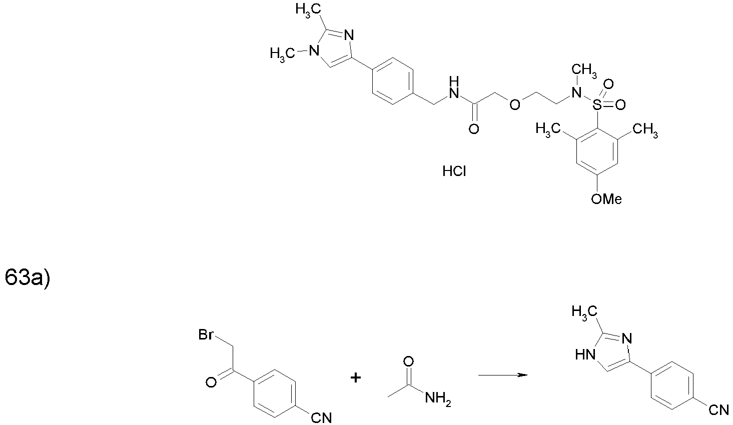

5.00 g (22.32 mMol) 2-Brom-4'-cyano-acetophenon (Aldrich) und 10.00 g (169.29 mMol) Acetamid (Merck) werden zusammen unter Rühren zwei Stunden auf 210°C erhitzt. Die Reaktionsmischung wird nach dem Abkühlen in Wasser eingerührt und mit 2 M HCl angesäuert. Der Niederschlag wird abfiltriert und verworfen. Das Filtrat wird mit konzentrierter Ammoniak-Lösung alkalisch gestellt, der Niederschlag abfiltriert und im Vakuum getrocknet. Das so erhaltene Rohprodukt wird durch Säulenchromatographie über Kieselgel (Elutionsmittel: Dichlormethan / Ethanol 9:1) gereinigt.
C₁₁H₉N₃ (183.21)
[M-H]- = 182
HPLC (Methode 4): Retentionszeit = 1.9 min

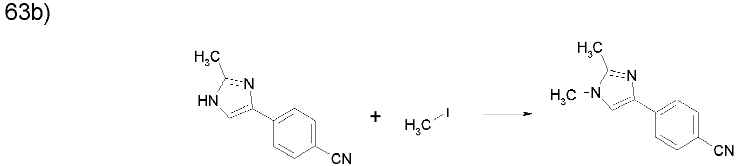

63b wird analog zu 62a aus 2.39 g (13.05 mMol) Produkt aus 63a, 1.46 g (13.05 mMol) Kalium-tert-butylat und 0.81 ml (13.05 mMol) Methyliodid in 50 ml DMSO hergestellt.
C₁₂H₁₁N₃ (197.24)
[M+H]+ = 198
HPLC (Methode 4): Retentionszeit = 1.9 min

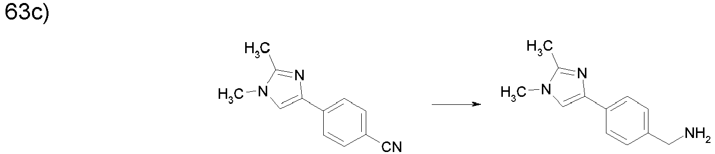

63c wird analog zu 34b aus 2.04 g (10.34 mMol) Produkt aus 63b, 0.40 g Raney-Nickel und 50 ml methanolischer Ammoniak-Lösung hergestellt.
C₁₂H₁₅N₃ (201.27)
DC: Kieselgel, Dichlormethan / Methanol / Ammoniak 9:1:0.1, Rf-Wert = 0.19

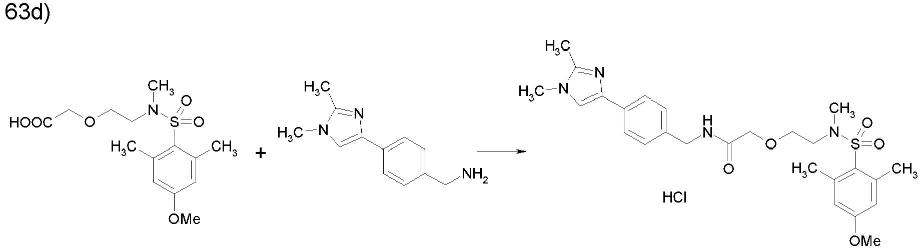

Beispiel 63 wird analog zu 1f aus 0.099 g (0.30 mMol) Produkt aus 53c, 0.060 g (0.30 mMol) Produkt aus 63c, 0.083 ml (0.60 mMol) Triethylamin und 0.12 g (0.36 mMol) TBTU in 7 ml THF und 1 ml DMF hergestellt.
C₂₆H₃₄N₄O₅S x HCl (551.10)
[M+H]+ = 515
HPLC (Methode 4): Retentionszeit = 3.2 min

### Beispiel 64 (Referenzbeispiel)

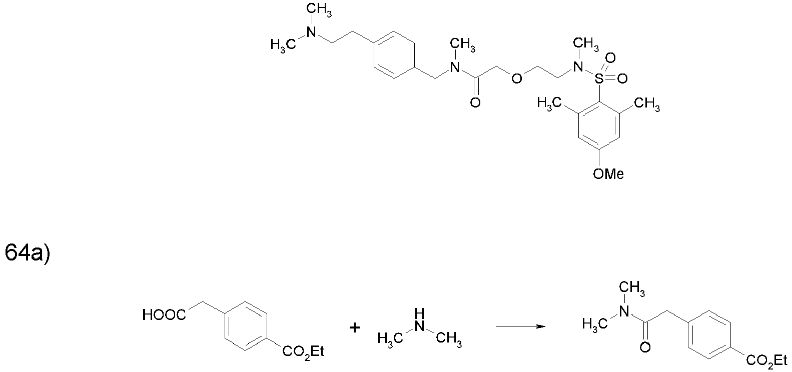

64a wird analog zu 1f aus 4.00 g (19.21 mMol) 4-Carboxymethyl-benzoesäureethylester (J. Med. Chem. 41, 1998, 5219-5246), 19.21 ml (38.42 mmol) Dimethylamin 2 M in THF (Aldrich), 5.36 ml (38.42 mMol) Triethylamin und 7.39 g (23.00 mMol) TBTU in 100 ml THF hergestellt.
C₁₃H₁₇NO₃ (235.28)
[M+H]+ = 236
HPLC (Methode 4): Retentionszeit = 3.3 min

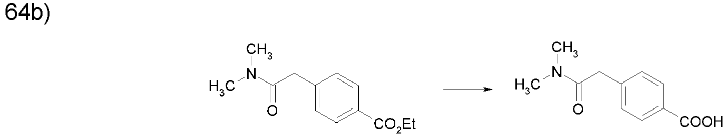

Eine Mischung aus 3.07 g (13.05 mMol) Produkt aus 64a, 9.75 ml (39.00 mMol) 4 M Natronlauge, 9.75 ml Wasser und 50 ml Ethanol wird über Nacht bei Raumtemperatur gerührt. Danach wird das Ethanol im Vakuum entfernt. Der wässrige Rückstand wird mit 4 M HCl sauer gestellt und mit Ethylacetat extrahiert. Die organischen Extrakte werden über Natriumsulfat getrocknet und im Vakuum bis zur Trockene eingeengt. Das so erhaltene Produkt wird mit Diethylether verrieben und getrocknet.
C₁₁H₁₃NO₃ (207.23)
[M+H]+ = 208
HPLC (Methode 4): Retentionszeit = 2.3 min

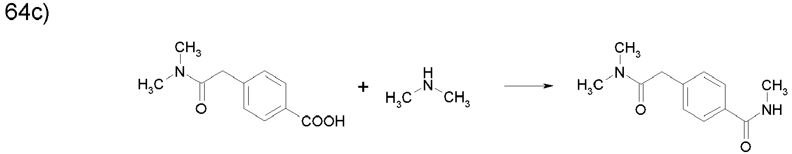

64c wird analog zu 1f aus 2.30 g (11.10 mMol) Produkt aus 64b, 11.10 ml (22.20 mMol) Dimethylamin 2 M in THF (Aldrich), 3.09 ml (22.20 mMol) Triethylamin und 4.28 g (13.32 mMol) TBTU in 70 ml THF hergestellt.
C₁₂H₁₆N₂O₂ (220.27)
[M+H]+ = 221
DC: Kieselgel, Dichlormethan / Methanol / Ammoniak 9:1:0.1, Rf-Wert = 0.44

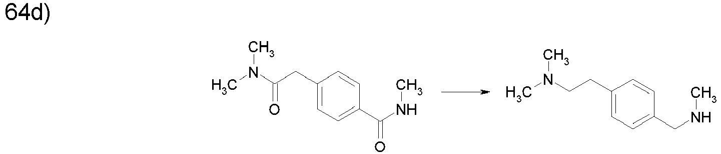

64d wird analog zu 38f aus 1.92 g (8.72 mMol) Produkt aus 64c und 40.00 ml (40.00 mMol) Lithiumaluminiumhydrid 1 M in THF (Aldrich) in 150 ml THF hergestellt.
C₁₂H₂₀N₂ (192.30)
[M+H]+ = 193
DC: Kieselgel, Dichlormethan / Methanol / Ammoniak 9:1:0.1, Rf-Wert = 0.10

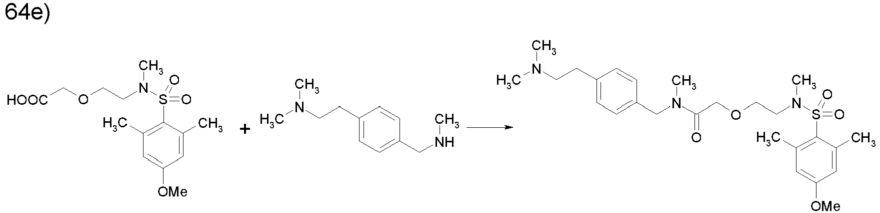

Beispiel 64 wird analog zu 1f aus 0.099 g (0.30 mMol) Produkt aus 53c, 0.058 g (0.30 mMol) Produkt aus 64d, 0.083 ml (0.60 mMol) Triethylamin und 0.12 g (0.36 mMol) TBTU in 7 ml THF und 1 ml DMF hergestellt.
C₂₆H₃₉N₃O₅S (505.67)
[M+H]+ = 508
HPLC (Methode 4): Retentionszeit = 3.0 min

### Beispiel 65 (Referenzbeispiel)

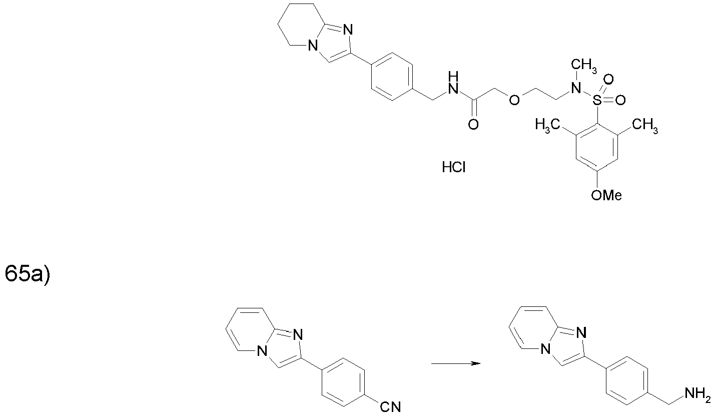

65a wird analog zu 34b aus 0.40 g (1.82 mMol) 4-Imidazo[1,2-a]pyridin-2-yl-benzonitril (J. Med. Chem. 41, 1998, 4317-4328), 0.10 g Raney-Nickel und 40 ml methanolischer Ammoniak-Lösung hergestellt.
C₁₄H₁₃N₃ (223.27)
[M+H]+ = 224
DC: Kieselgel, Dichlormethan / Methanol 9:1, Rf-Wert = 0.06

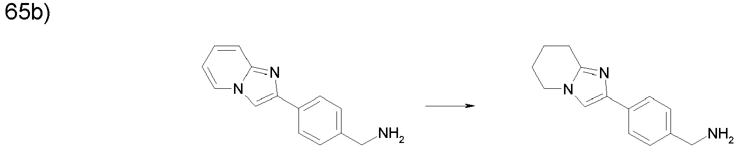

Eine Mischung aus 0.40 g (1.79 mMol) Produkt aus 65a, 0.05 g Platinoxid und 40 ml Methanol wird bei 50°C im Autoklaven hydriert. Der Katalysator wird abfiltriert, das Filtrat im Vakuum bis zur Trockene eingeengt.
C₁₄H₁₇N₃ (227.30)
[M+H]+ = 228

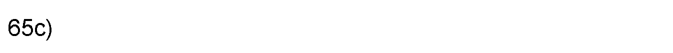

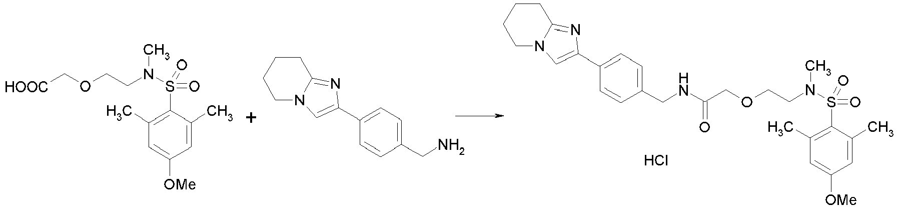

Beispiel 65 wird analog zu 1f aus 0.15 g (0.44 mMol) Produkt aus 53c, 0.10 g (0.44 mMol) Produkt aus 65b, 0.15 ml (1.09 mMol) Triethylamin und 0.16 g (0.48 mMol) TBTU in 30 ml THF und 10 ml DMF hergestellt.
C₂₈H₃₆N₄O₅S x HCl (577.14)
[M+H]+ = 541
HPLC (Methode 5): Retentionszeit = 1.57 min

### Beispiel 66 (Referenzbeispiel)

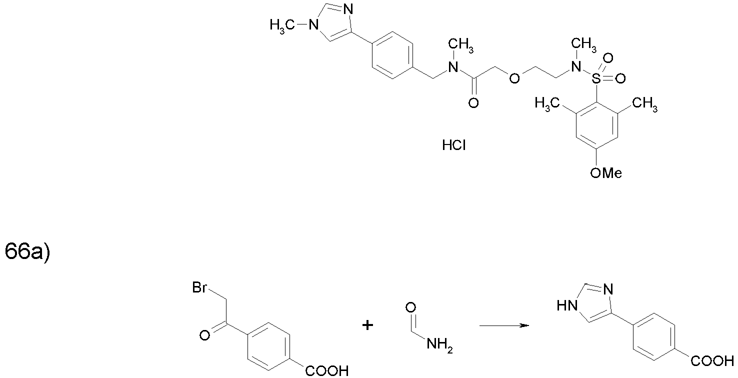

Eine Mischung aus 0.50 g (2.06 mMol) 4-Bromacetylbenzoesäure (Fluorochem) und 5 ml Formamid wird eine Stunde bei 150°C in der Mikrowelle gerührt. Nach dem Abkühlen wird das ausgefallene Produkt abfiltriert, mit Diethylether gewaschen und getrocknet.
C₁₀H₈N₂O₂ (188.18)
[M+H]+ = 189

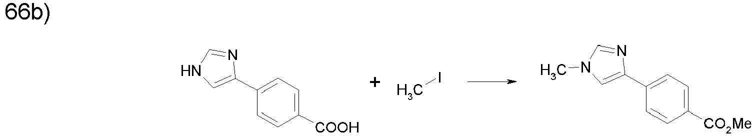

Eine Mischung aus 1.60 g (8.50 mMol) Produkt aus 66a, 5.40 g (38.04 mMol) Methyliodid, 7.00 g (25.33 mMol) Kaliumcarbonat und 30 ml DMF wird über Nacht bei Raumtemperatur gerührt. Die Reaktionsmischung wird filtriert, das Filtrat wird im Vakuum bis zur Trockene eingeengt. Das so erhaltene Rohprodukt wird durch Säulenchromatographie über Kieselgel (Elutionsmittel: Dichlormethan / Methanol 100:1 bis 75:1) gereinigt.
C₁₂H₁₂N₂O₂ (216.24)
[M+H]+ = 217

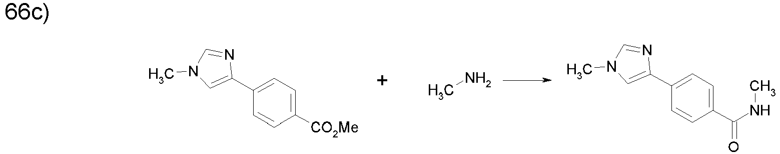

Eine Mischung aus 0.75 g (3.47 mMol) Produkt aus 66b und 20 ml Methylamin 33%ig in Ethanol (Fluka) über Nacht im Autoklaven auf 160°C erhitzt. Die Reaktionsmischung wird im Vakuum bis zur Trockene eingeengt. Das so erhaltene Rohprodukt wird durch Säulenchromatographie über Kieselgel (Elutionsmittel: Dichlormethan / Methanol 50:1 bis 25:1) gereinigt.
C₁₂H₁₃N₃O (215.25)
[M+H]+ = 216

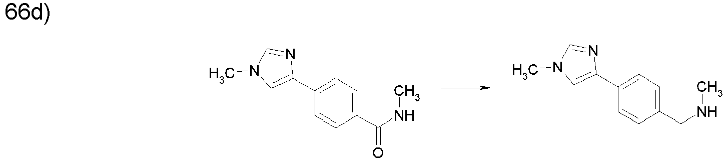

66d wird analog zu 38f aus 0.41 g (1.91 mMol) Produkt aus 66c und 3.00 ml (3.00 mMol) Lithiumaluminiumhydrid 1 M in THF (Aldrich) in 70 ml THF hergestellt.
C₁₂H₁₅N₃ (201.27)
[M+H]+ = 202
DC: Kieselgel, Dichlormethan / Methanol / Ammoniak 8:2:0.01, Rf-Wert = 0.16

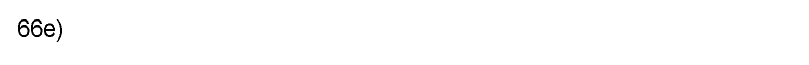

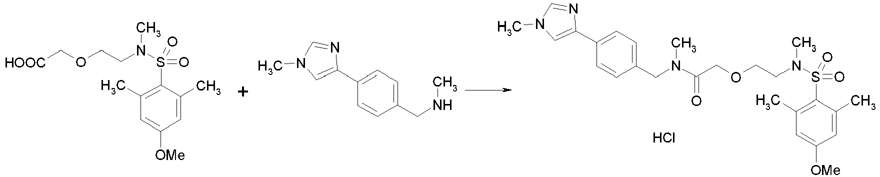

Beispiel 66 wird analog zu 1f aus 0.15 g (0.45 mMol) Produkt aus 53c, 0.09g (0.45 mMol) Produkt aus 66d, 0.11 ml (1.09 mMol) Triethylamin und 0.16 g (0.50 mMol) TBTU in 30 ml THF und 5 ml DMF hergestellt.
C₂₆H₃₄N₄O₅S x HCl (551.10)
[M+H]+ = 515
DC: Kieselgel, Dichlormethan / Methanol 9:1, Rf-Wert = 0.52

### Beispiel 67 (Referenzbeispiel)

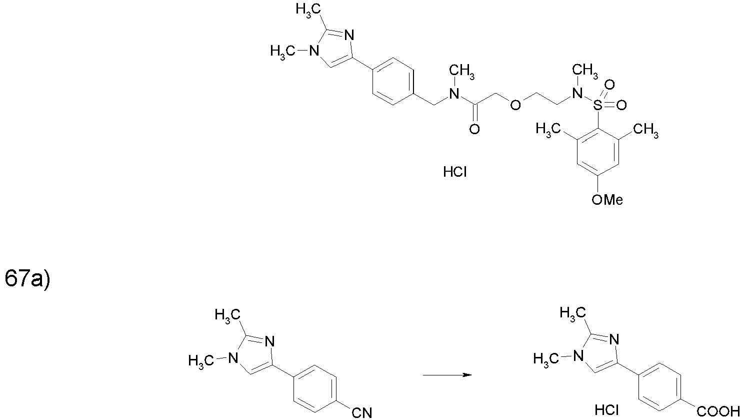

Eine Mischung aus 1.21 g (6.14 mMol) Produkt aus 63b, 20 ml 20%iger Natronlauge und 40 ml Ethanol wird über Nacht unter Rückfluss gerührt. Das Ethanol wird im Vakuum entfernt. Der Rückstand wird mit Wasser verdünnt und mit 4 M HCl sauer gestellt. Das ausgefallene Produkt wird abfiltriert und im Umlufttrockenschrank bei 50°C getrocknet.
C₁₂H₁₂N₂O₂ (252.70)
[M+H]+ = 217
HPLC (Methode 4): Retentionszeit = 1.7 min

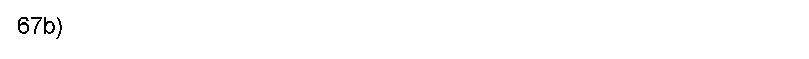

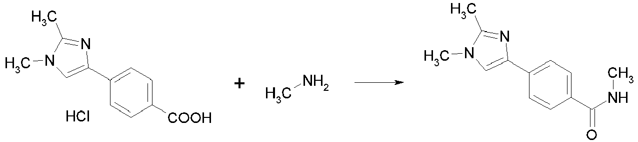

67b wird analog zu 1f aus 1.55 g (6.13 mMol) Produkt aus 67a, 4.60 ml (9.20 mMol) Methylamin 2 M in THF (Aldrich), 1.71 ml (12.30 mMol) Triethylamin und 2.38 g (7.40 mMol) TBTU in 50 ml THF hergestellt.
C₁₃H₁₅N₃O (229.28)
[M+H]+ = 230
DC: Kieselgel, Dichlormethan / Methanol 9:1, Rf-Wert = 0.47

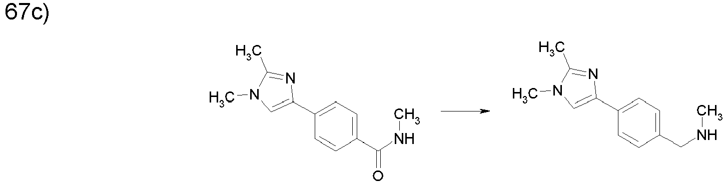

67c wird analog zu 38f aus 1.33 g (5.80 mMol) Produkt aus 67b und 15.00 ml (15.00 mMol) Lithiumaluminiumhydrid 1 M in THF (Aldrich) in 80 ml THF hergestellt.
C₁₃H₁₇N₃ (215.29)
[M+H]+ = 216
DC: Kieselgel, Dichlormethan / Methanol / Ammoniak 9:1:0.1, Rf-Wert = 0.29

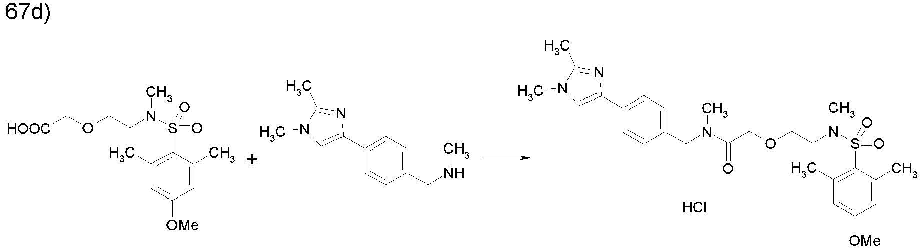

Beispiel 67 wird analog zu 1f aus 0.099 g (0.30 mMol) Produkt aus 53c, 0.065 g (0.30 mMol) Produkt aus 67c, 0.083 ml (0.60 mMol) Triethylamin und 0.12 g (0.36 mMol) TBTU in 7 ml THF und 1 ml DMF hergestellt.
C₂₇H₃₆N₄O₅S x HCl (565.13)
[M+H]+ = 529
HPLC (Methode 4): Retentionszeit = 3.1 min

### Beispiel 68 (Referenzbeispiel)

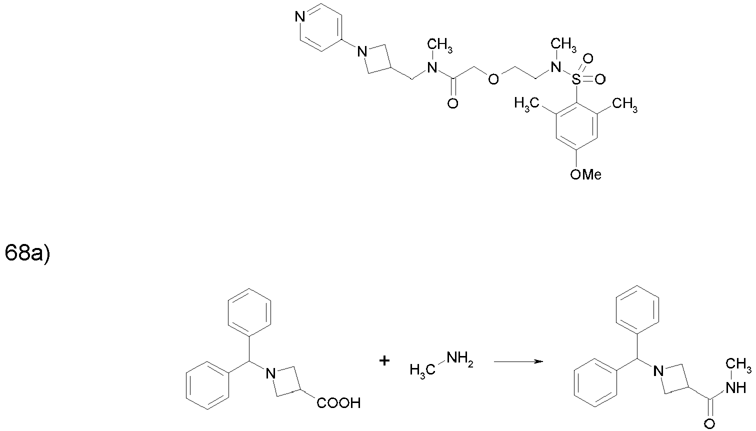

68a wird analog zu 1f aus 5.39 g (20.16 mMol) 1-Benzhydryl-azetidine-3-carbonsäure (Acros), 15 ml (30.00 mMol) Methylamin 2 M in THF (Aldrich), 5.58 ml (40.00 mMol) Triethylamin und 7.71 g (24.00 mMol) TBTU in 150 ml THF hergestellt.
C₁₈H₂₀N₂O (280.36)
HPLC (Methode 4): Retentionszeit = 2.5 min

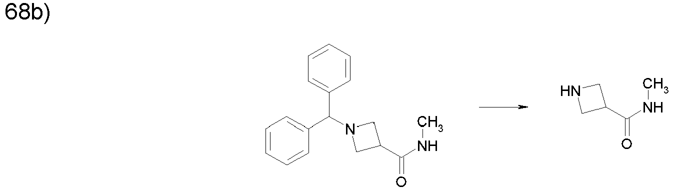

Eine Mischung aus 5.32 g (18.98 mMol) Produkt aus 68a, 0.50 g Palladium auf Kohle (10%) und 100 ml Methanol wird 24 Stunden bei Raumtemperatur im Autoklaven hydriert. Der Katalysator wird abfiltriert, das Filtrat wird im Vakuum bis zur Trockene eingeengt. Das so erhaltene Rohprodukt wird durch Säulenchromatographie über Kieselgel (Elutionsmittel: Dichlormethan / Methanol / Ammoniak 70:30:3) gereinigt.
C₅H₁₀N₂O (114.15)
DC: Kieselgel, Dichlormethan / Methanol / Ammoniak 7:3:0.3, Rf-Wert = 0.17

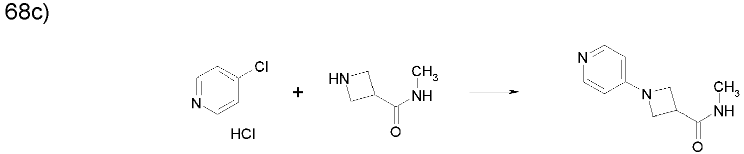

68c wird analog zu 28c aus 1.31 g (8.76 mMol) 4-Chlorpyridin Hydrochlorid (Aldrich), 1.00 g (8.76 mMol) Produkt aus 68b und 2.40 ml (17.22 mMol) Triethylamin in 5 ml Ethanol hergestellt.
C₁₀H₁₃N₃O (191.23)
[M+H]+ = 192
DC: Kieselgel, Dichlormethan / Methanol / Ammoniak 9:1:0.1, Rf-Wert = 0.25

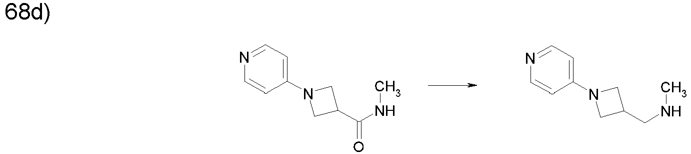

68d wird analog zu 38f aus 0.46 g (2.41 mMol) Produkt aus 68c und 5.00 ml (5.00 mMol) Lithiumaluminiumhydrid 1 M in THF (Aldrich) in 20 ml THF hergestellt.
C₁₀H₁₅N₃ (177.25)
[M+H]+ = 178
DC: Kieselgel, Dichlormethan / Methanol / Ammoniak 8:2:0.2, Rf-Wert = 0.40

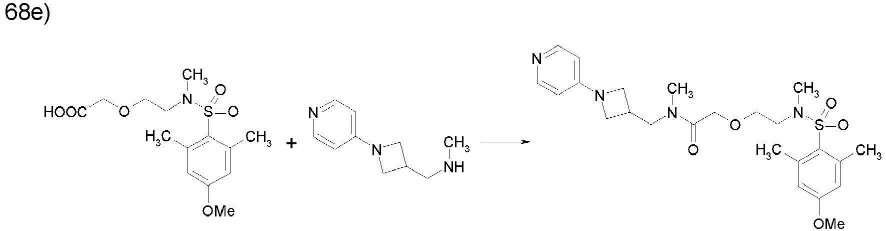

Beispiel 68 wird analog zu 1f aus 0.083 g (0.25 mMol) Produkt aus 53c, 0.044 g (0.25 mMol) Produkt aus 68d, 0.070 ml (0.50 mMol) Triethylamin und 0.096 g (0.30 mMol) TBTU in 7 ml THF und 1 ml DMF hergestellt.
C₂₄H₃₄N₄O₅S (490.62)
[M+H]+ = 491
HPLC (Methode 4): Retentionszeit = 2.9 min

### Beispiel 69 (Referenzbeispiel)

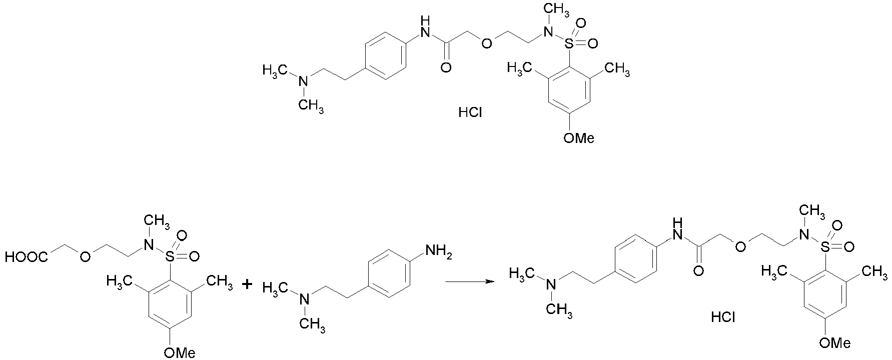

Beispiel 69 wird analog zu 1f aus 0.083 g (0.25 mMol) Produkt aus 53c, 0.041 g (0.25 mMol) 4-(2-Dimethylamino-ethyl)-phenylamin (JW Pharmlab), 0.070 ml (0.50 mMol) Triethylamin und 0.096 g (0.30 mMol) TBTU in 7 ml THF und 1 ml DMF hergestellt.
C₂₄H₃₅N₃O₅S x HCl (514.08)
[M+H]+ = 478
HPLC (Methode 4): Retentionszeit = 3.1 min

### Beispiel 70 (Referenzbeispiel)

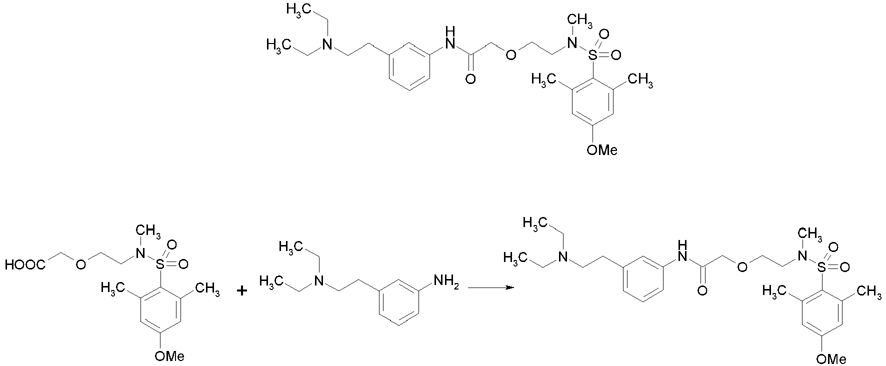

Beispiel 70 wird analog zu 1f aus 0.083 g (0.25 mMol) Produkt aus 53c, 0.048 g (0.25 mMol) 3-(2-Diethylamino-ethyl)-phenylamin (analog J. Med. Chem. 28, 1985, 1533-1536), 0.070 ml (0.50 mMol) Triethylamin und 0.096 g (0.30 mMol) TBTU in 7 ml THF und 1 ml DMF hergestellt.
C₂₆H₃₉N₃O₅S (505.67)
[M+H]+ = 506
HPLC (Methode 4): Retentionszeit = 3.4 min

### Beispiel 71 (Referenzbeispiel)

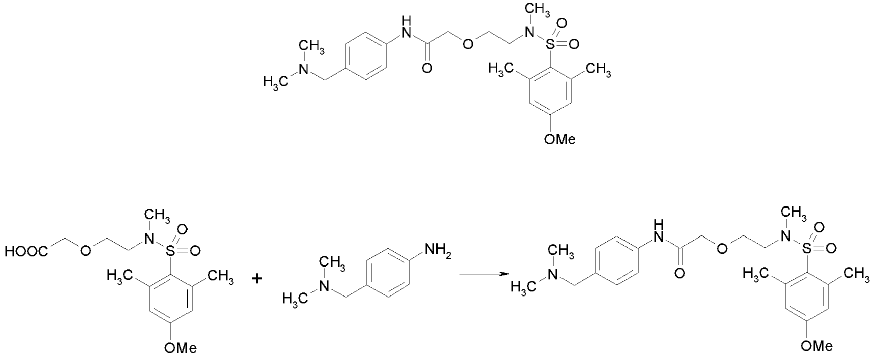

Beispiel 71 wird analog zu 1f aus 0.083 g (0.25 mMol) Produkt aus 53c, 0.038 g (0.25 mMol) 4-Dimethylaminomethyl-phenylamin (J. Chem. Soc. 1935, 871), 0.070 ml (0.50 mMol) Triethylamin und 0.096 g (0.30 mMol) TBTU in 7 ml THF und 1 ml DMF hergestellt.
C₂₃H₃₃N₃O₅S (463.59)
[M+H]+ = 464
HPLC (Methode 4): Retentionszeit = 3.1 min

### Beispiel 72 (Referenzbeispiel)

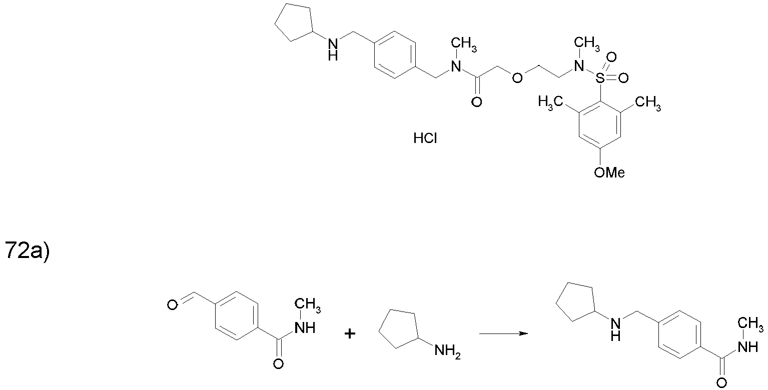

72a wird analog zu 60a aus 0.82 g (5.00 mMol) 4-Formyl-benzoesäuremethylamid (EMKA), 0.50 ml (5.00 mMol) Cyclopentylamin (Aldrich), 0.37 ml (6.50 mMol) Essigsäure und 1.59 g (7.50 mMol) Natriumtriacetoxyborhydrid in 30 ml THF hergestellt.
C₁₄H₂₀N₂O (232.32)
DC: Kieselgel, Dichlormethan / Ethanol 9:1, Rf-Wert = 0.15

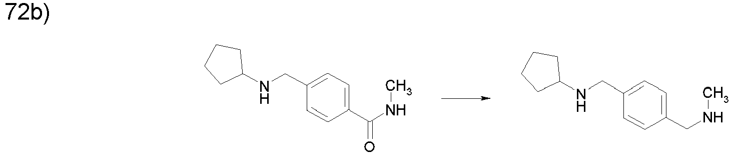

72b wird analog zu 38f aus 1.00 g (4.30 mMol) Produkt aus 72a und 9.00 ml (9.00 mMol) Lithiumaluminiumhydrid 1 M in THF (Aldrich) in 40 ml THF hergestellt.
C₁₄H₂₂N₂ (218.34)
[M+H]+ = 219
DC: Kieselgel, Dichlormethan / Ethanol 9:1, Rf-Wert = 0.2

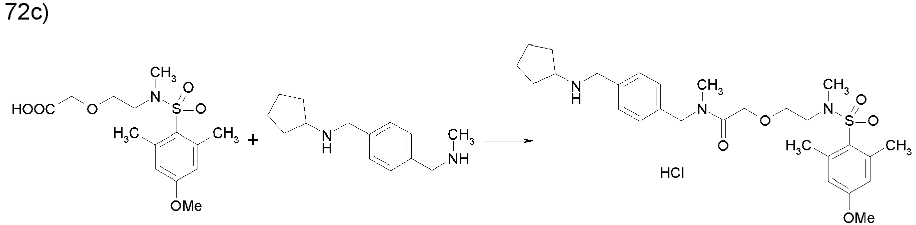

Beispiel 72 wird analog zu 1f aus 0.20 g (0.60 mMol) Produkt aus 53c, 0.13 g (0.60 mMol) Produkt aus 72b, 0.21 ml (1.50 mMol) Triethylamin und 0.23 g (0.72 mMol) TBTU in 10 ml THF hergestellt.
C₂₈H₄₁N₃O₅S x HCl (568.17)
[M+H]+ = 532
HPLC (Methode 5): Retentionszeit = 1.60 min

### Beispiel 73 (Referenzbeispiel)

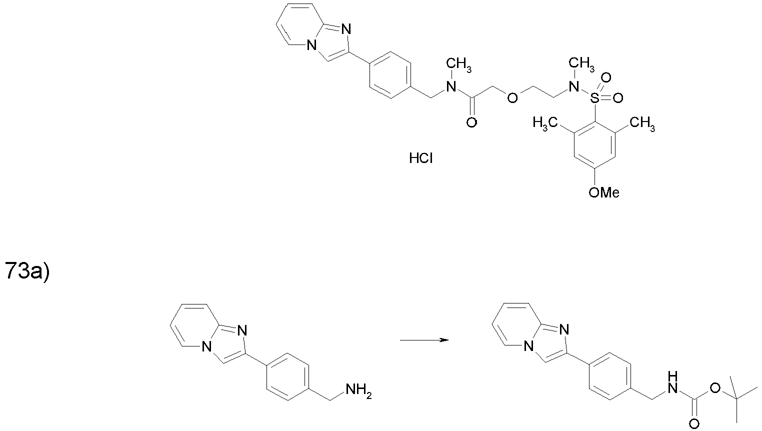

Eine Mischung aus 2.60 g (11.65 mMol) Produkt aus 65a, 2.55 g (11.68 mMol) Boc-Anhydrid und 100 ml DMF wird zwei Stunden bei Raumtemperatur gerührt. Danach gibt man langsam 100 ml Wasser hinzu. Das ausgefallene Produkt wird abfiltriert, mit Wasser und Petrolether gewaschen und getrocknet.
C₁₉H₂₁N₃O₂ (323.39)
[M+H]+ = 324
DC: Kieselgel, Dichlormethan / Methanol 9:1, Rf-Wert = 0.78

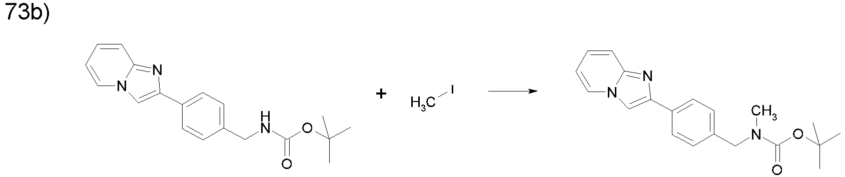

73b wird analog zu 62a aus 1.00 g (3.09 mMol) Produkt aus 73a, 0.70 g (6.24 mMol) Kalium-tert-butylat und 0.98 ml (6.90 mMol) Methyliodid in 30 ml DMSO hergestellt.
C₁₇H₁₇N₃O₂ (295.34)
[M+H]+ = 296

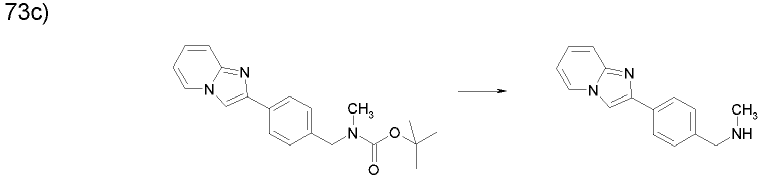

Eine Mischung aus 0.59 g (1.75 mMol) Produkt aus 73b, 2 ml TFA und 30 ml Dichlormethan wird drei Stunden bei Raumtemperatur gerührt. Die Reaktionsmischung wird danach mit Wasser gewaschen, über Natriumsulfat getrocknet und im Vakuum bis zur Trockene eingeengt.
C₁₅H₁₅N₃ (237.30)
[M+H]+ = 238

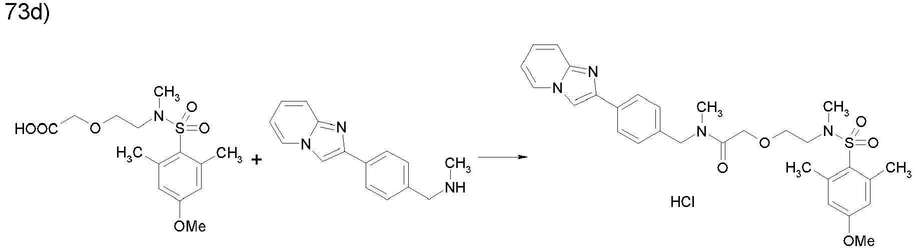

Beispiel 73 wird analog zu 1f aus 0.14 g (0.42 mMol) Produkt aus 53c, 0.10 g (0.42 mMol) Produkt aus 73c, 0.10 ml (0.99 mMol) Triethylamin und 0.15 g (0.46 mMol) TBTU in 30 ml THF und 5 ml DMF hergestellt.
C₂₉H₃₄N₄O₅S x HCl (587.13)
[M+H]+ = 551
HPLC (Methode 5): Retentionszeit = 1.58 min

### Beispiel 74 (Referenzbeispiel)

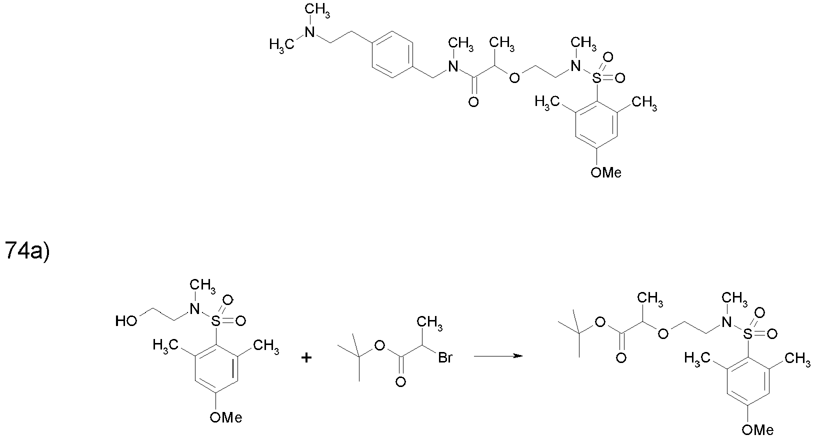

74a wird analog zu 53b aus 4.08 g (14.93 mMol) Produkt aus 53a, 4.68 g (22.39 mMol) 2-Brompropionsäure-tert-butylester (TCl), 1.38 g (4.98 mMol) Tetrabutylammoniumchlorid (Fluka) und 70 ml 35%iger Natronlauge in 70 ml Toluol hergestellt.
C₁₉H₃₁NO₆S (401.52)
[M+H]+ = 402
DC: Kieselgel, Petrolether / Ethylacetat 7:3, Rf-Wert = 0.69

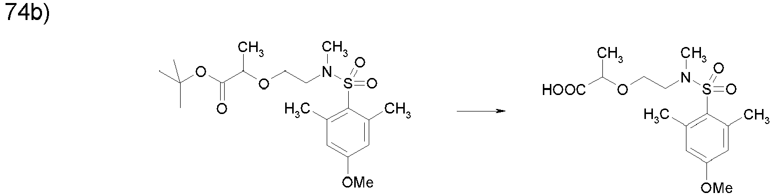

74b wird analog zu 53c aus 5.12 g (12.75 mMol) Produkt aus 74a und 5.89 ml TFA in 80 ml Dichlormethan hergestellt.
C₁₅H₂₃NO₆S (345.41)
[M+H]+ = 346
DC: Kieselgel, Dichlormethan / Ethanol 19:1, Rf-Wert = 0.25

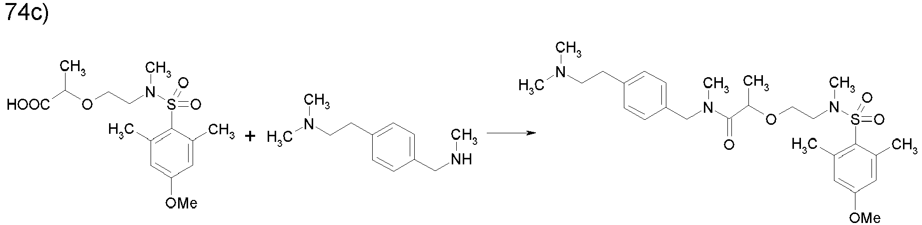

Beispiel 74 wird analog zu 1f aus 0.10 g (0.30 mMol) Produkt aus 74b, 0.058 g (0.30 mMol) Produkt aus 64d, 0.084 ml (0.60 mMol) Triethylamin und 0.12 g (0.36 mMol) TBTU in 7 ml THF und 1 ml DMF hergestellt.
C₂₇H₄₁N₃O₅S (519.70)
[M+H]+ = 520
HPLC (Methode 4): Retentionszeit = 3.1 min

### Beispiel 75 (Referenzbeispiel)

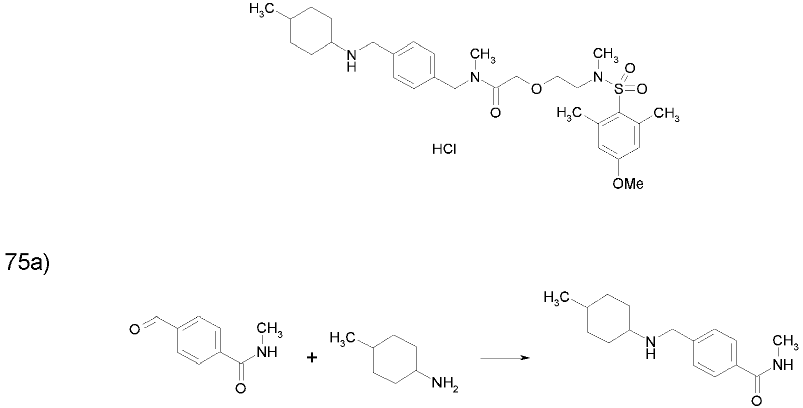

75a wird analog zu 60a aus 0.82 g (5.00 mMol) 4-Formyl-benzoesäuremethylamid (EMKA), 0.67 ml (5.00 mMol) 4-Methylcyclohexylamin cis/trans-Gemisch (Acros), 0.37 ml (6.50 mMol) Essigsäure und 1.59 g (7.50 mMol) Natriumtriacetoxyborhydrid in 30 ml THF hergestellt.
C₁₆H₂₄N₂O (260.37)
DC: Kieselgel, Dichlormethan / Ethanol 9:1, Rf-Wert = 0.25

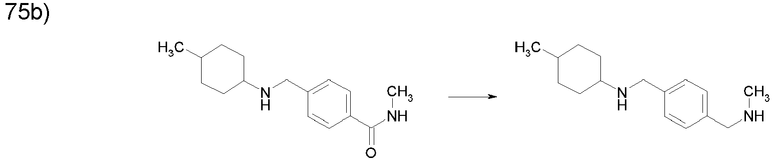

75b wird analog zu 38f aus 1.30 g (4.99 mMol) Produkt aus 75a und 10.00 ml (10.00 mMol) Lithiumaluminiumhydrid 1 M in THF (Aldrich) in 50 ml THF hergestellt.
C₁₆H₂₆N₂ (246.39)
DC: Kieselgel, Dichlormethan / Ethanol 9:1, Rf-Wert = 0.2

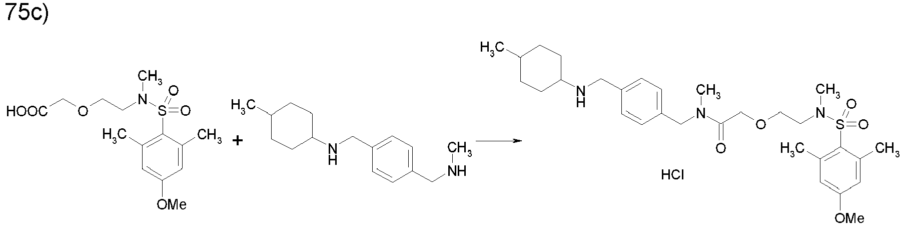

Beispiel 75 wird analog zu 1f aus 0.20 g (0.60 mMol) Produkt aus 53c, 0.15 g (0.60 mMol) Produkt aus 75b, 0.21 ml (1.50 mMol) Triethylamin und 0.23 g (0.72 mMol) TBTU in 10 ml THF hergestellt.
C₃₀H₄₅N₃O₅S x HCl (596.22)
[M+H]+ = 560
HPLC (Methode 5): Retentionszeit = 1.65 min

### Beispiel 76 (Referenzbeispiel)

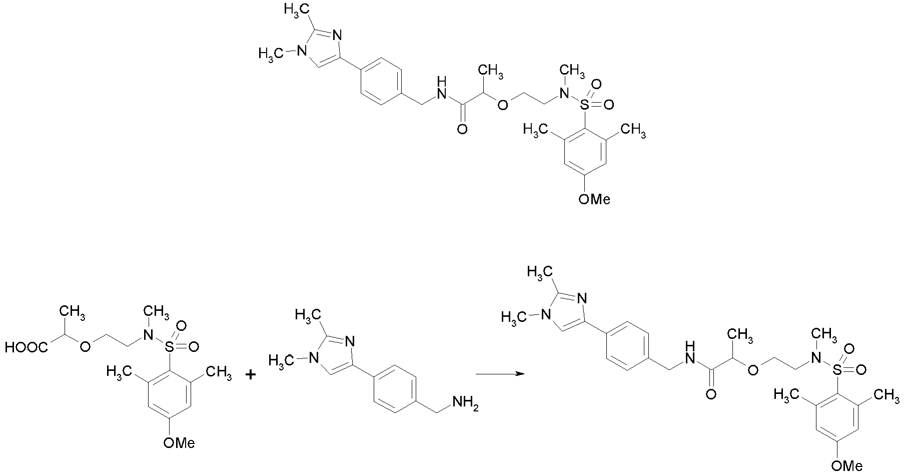

Beispiel 76 wird analog zu 1f aus 0.10 g (0.30 mMol) Produkt aus 74b, 0.060 g (0.30 mMol) Produkt aus 63c, 0.084 ml (0.60 mMol) Triethylamin und 0.12 g (0.36 mMol) TBTU in 7 ml THF und 1 ml DMF hergestellt.
C₂₇H₃₆N₄O₅S (528.66)
[M+H]+ = 529
HPLC (Methode 4): Retentionszeit = 3.2 min

### Beispiel 77 (Referenzbeispiel)

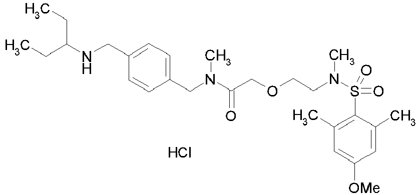

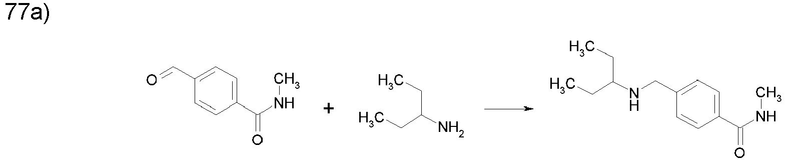

77a wird analog zu 60a aus 0.82 g (5.00 mMol) 4-Formyl-benzoesäuremethylamid (EMKA), 0.58 ml (5.00 mMol) 3-Pentylamin (Aldrich), 0.37 ml (6.50 mMol) Essigsäure und 1.59 g (7.50 mMol) Natriumtriacetoxyborhydrid in 30 ml THF hergestellt.
C₁₄H₂₂N₂O (234.34)
[M+H]+ = 235
DC: Kieselgel, Dichlormethan / Ethanol 9:1, Rf-Wert = 0.4

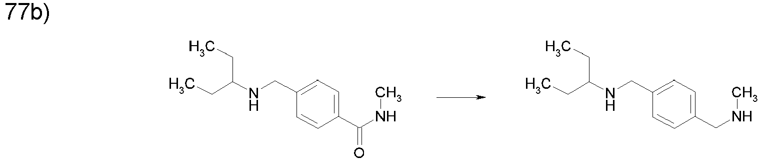

77b wird analog zu 38f aus 1.10 g (4.69 mMol) Produkt aus 77a und 9.40 ml (9.40 mMol) Lithiumaluminiumhydrid 1 M in THF (Aldrich) in 40 ml THF hergestellt.
C₁₄H₂₄N₂ (220.35)
DC: Kieselgel, Dichlormethan / Ethanol 9:1, Rf-Wert = 0.1

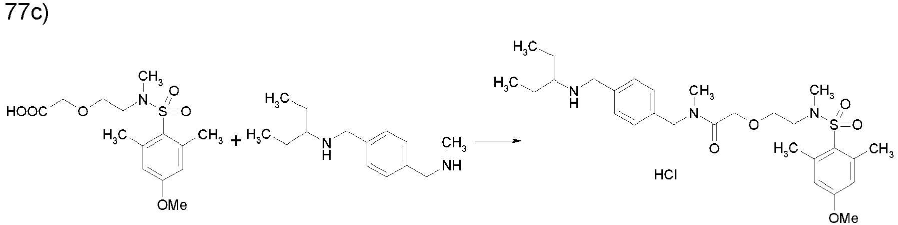

Beispiel 77 wird analog zu 1f aus 0.20 g (0.60 mMol) Produkt aus 53c, 0.13 g (0.60 mMol) Produkt aus 77b, 0.21 ml (1.50 mMol) Triethylamin und 0.23 g (0.72 mMol) TBTU in 10 ml THF hergestellt.
C₂₈H₄₃N₃O₅S x HCl (570.18)
[M+H]+ = 534
DC: Kieselgel, Dichlormethan / Ethanol 9:1, Rf-Wert = 0.45

### Beispiel 78 (Referenzbeispiel)

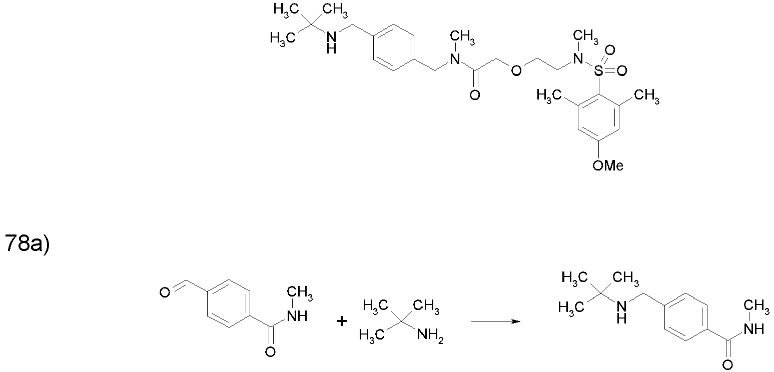

78a wird analog zu 60a aus 0.82 g (5.00 mMol) 4-Formyl-benzoesäuremethylamid (EMKA), 0.53 ml (5.00 mMol) tert-Butylamin (Fluka), 0.37 ml (6.50 mMol) Essigsäure und 1.59 g (7.50 mMol) Natriumtriacetoxyborhydrid in 30 ml THF hergestellt.
C₁₃H₂₀N₂O (220.31)
[M+H]+ = 221
DC: Kieselgel, Dichlormethan / Ethanol 9:1, Rf-Wert = 0.15

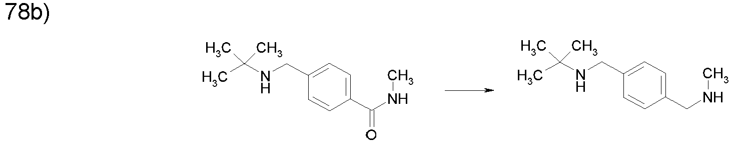

78b wird analog zu 38f aus 1.00 g (4.54 mMol) Produkt aus 78a und 9.10 ml (9.10 mMol) Lithiumaluminiumhydrid 1 M in THF (Aldrich) in 40 ml THF hergestellt.
C₁₃H₂₂N₂ (206.33)

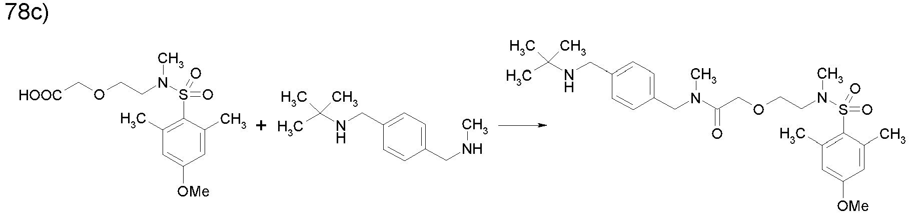

Beispiel 78 wird analog zu 1f aus 0.20 g (0.60 mMol) Produkt aus 53c, 0.12 g (0.60 mMol) Produkt aus 78b, 0.21 ml (1.50 mMol) Triethylamin und 0.23 g (0.72 mMol) TBTU in 10 ml THF hergestellt.
C₂₇H₄₁N₃O₅S (519.70)
[M+H]+ = 520
HPLC (Methode 5): Retentionszeit = 1.58 min

### Beispiel 79 (Referenzbeispiel)

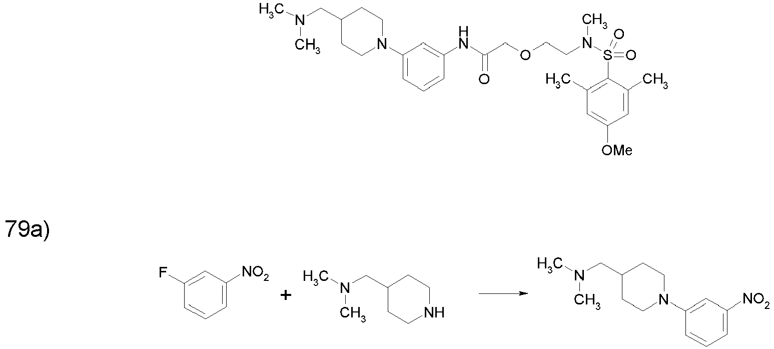

Eine Mischung aus 0.50 ml (4.70 mMol) 1-Fluor-3-nitrobenzol (Fluka), 1.34 g (9.39 mMol) 4-(N,N-Dimethylaminomethyl)-piperidin (Eur. J. Med.Chem. Chim. Ther. 37, 2002, 487-502), 0.65 g (4.70 mMol) Kaliumcarbonat und 6.6 ml DMSO wird fünf Tage bei 110°C gerührt. Die Reaktionsmischung wird danach auf Eis gegossen, der entstandene Niederschlag wird abfiltriert. Das so erhaltene Produkt wird über Nacht im Vakuum-Exsikkator getrocknet.
C₁₄H₂₁N₃O₂ (263.34)
[M+H]+ = 264
DC: Kieselgel, Dichlormethan / Methanol 9:1, Rf-Wert = 0.20

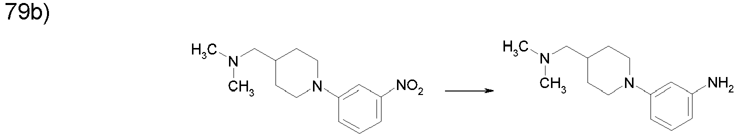

Eine Mischung aus 0.95 ml (3.60 mMol) Produkt aus 79a, 0.095 g Palladium auf Kohle (5%) und 72 ml Ethanol wird bei Raumtemperatur im Autoklaven hydriert. Der Katalysator wird abfiltriert, das Filtrat wird im Vakuum bis zur Trockene eingeengt.
C₁₄H₂₃N₃ (233.35)
[M+H]+ = 234

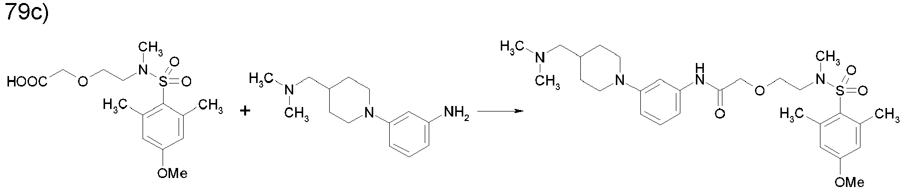

Beispiel 79 wird analog zu 1f aus 0.15 g (0.46 mMol) Produkt aus 53c, 0.11 g (0.46 mMol) Produkt aus 79b, 0.084 ml (0.60 mMol) Triethylamin und 0.18 g (0.56 mMol) TBTU in 3 ml DMF hergestellt.
C₂₈H₄₂N₄O₅S (546.72)
[M+H]+ = 547
HPLC (Methode 5): Retentionszeit = 1.49 min

### Beispiel 80 (Referenzbeispiel)

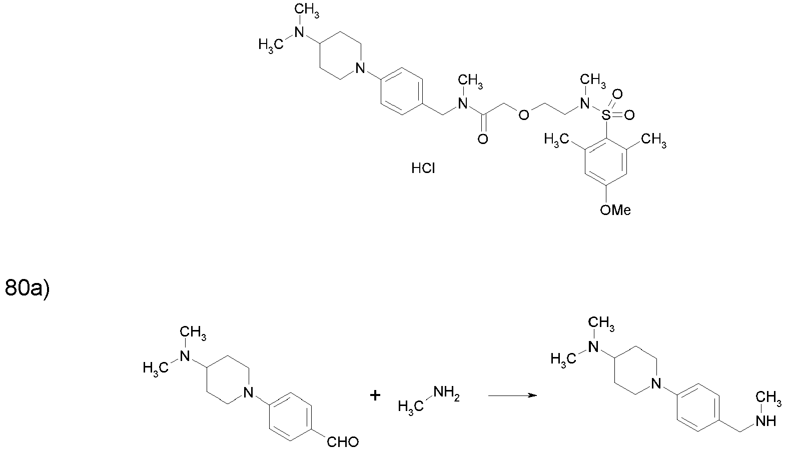

80a wird analog zu 33a aus 0.70 g (3.01 mMol) 4-(4-Dimethylamino-piperidin-1-yl)-benzaldehyd (Tetrahedron 57, 2001, 4781-4785), 3.00 ml (31.88 mmol) Methylamin 33% in Ethanol (Aldrich), 0.20 g Raney-Nickel in 25 ml Ethanol hergestellt.
C₁₅H₂₅N₃ (247.38)
[M+H]+ = 248
DC: Kieselgel, Dichlormethan / Methanol / Ammoniak 9:1:0.1, Rf-Wert = 0.16

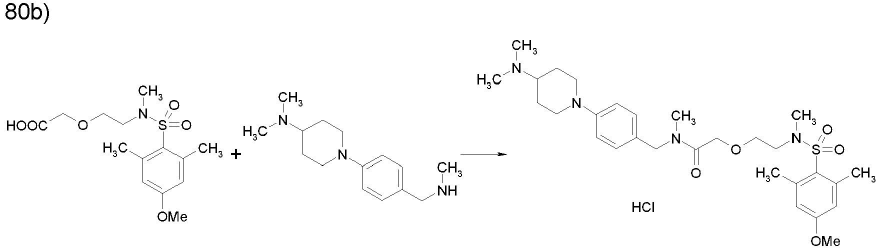

Beispiel 80 wird analog zu 1f aus 0.15 g (0.45 mMol) Produkt aus 53c, 0.11 g (0.45 mMol) Produkt aus 80a, 0.13 ml (0.90 mMol) Triethylamin und 0.17 g (0.54 mMol) TBTU in 5 ml DMF hergestellt.
C₂₉H₄₄N₄O₅S x HCl (597.21)
[M+H]+ = 561
HPLC (Methode 1): Retentionszeit = 2.33 min

### Beispiel 81 (Referenzbeispiel)

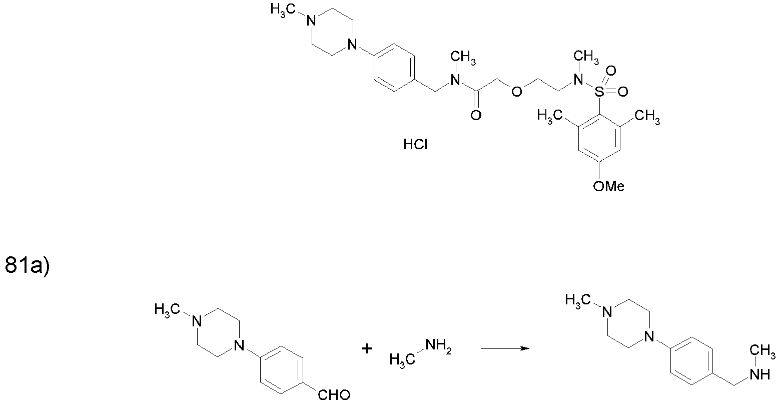

81a wird analog zu 33a aus 1.00 g (4.90 mMol) 4-(4-Methyl-piperazin-1-yl)-benzaldehyd (Chem. Pharm Bull. 45, 1997, 1458-1469), 4.00 ml (42.50 mMol) Methylamin 33% in Ethanol (Aldrich), 0.20 g Raney-Nickel in 30 ml Ethanol hergestellt.
C₁₃H₂₁N₃ (219.33)
[M+H]+ = 220
DC: Kieselgel, Dichlormethan / Methanol / Ammoniak 9:1:0.1, Rf-Wert = 0.15

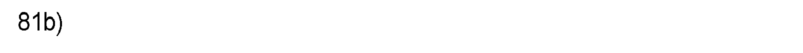

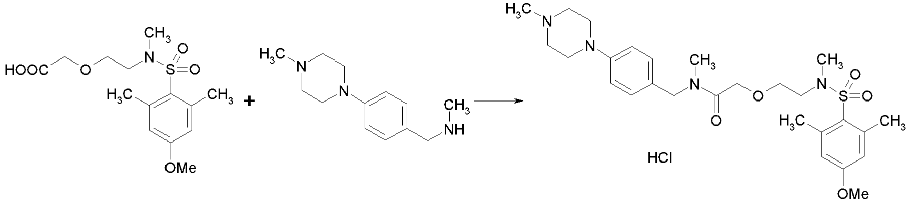

Beispiel 81 wird analog zu 1f aus 0.15 g (0.45 mMol) Produkt aus 53c, 0.099 g (0.45 mMol) Produkt aus 81a, 0.13 ml (0.90 mMol) Triethylamin und 0.17 g (0.54 mMol) TBTU in 5 ml DMF hergestellt.
C₂₇H₄₀N₄O₅S x HCl (569.16)
[M+H]+ = 533
HPLC (Methode 1): Retentionszeit = 2.28 min

### Beispiel 82 (Referenzbeispiel)

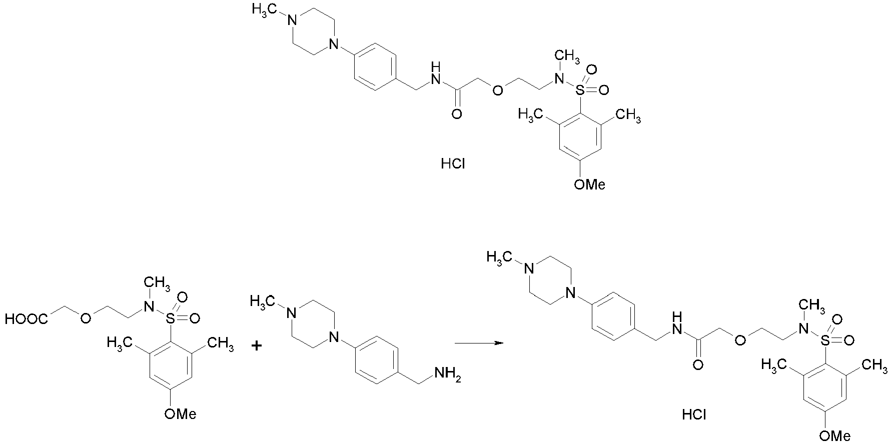

Beispiel 82 wird analog zu 1f aus 0.15 g (0.45 mMol) Produkt aus 53c, 0.092 g (0.45 mMol) Produkt aus 19b, 0.13 ml (0.90 mMol) Triethylamin und 0.17 g (0.54 mMol) TBTU in 5 ml DMF hergestellt.
C₂₆H₃₈N₄O₅S x HCl (555.13)
[M+H]+ = 519
HPLC (Methode 1): Retentionszeit = 2.29 min

### Beispiel 83 (Referenzbeispiel)

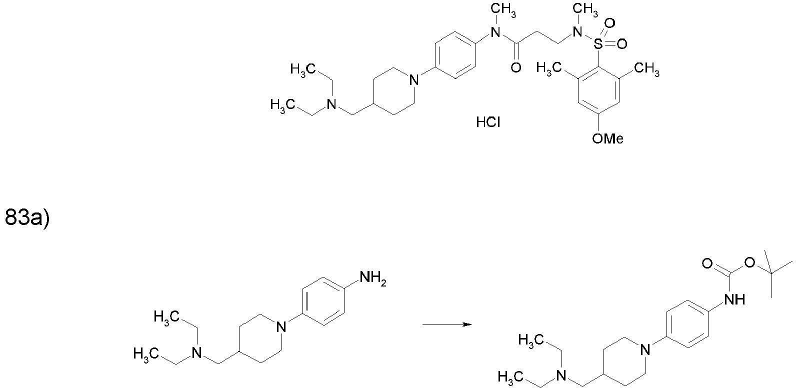

83a wird analog zu 51 a aus 1.35 g (5.16 mMol) Produkt aus 41 b, 1.24 g (5.68 mMol) Boc-Anhydrid und 0.80 ml (5.68 mMol) Triethylamin in 50 ml Dichlormethan hergestellt.
C₂₁H₃₅N₃O₂ (361.52)
[M+H]+ = 362
DC: Kieselgel, Dichlormethan / Methanol / Ammoniak 9:1:0.1, Rf-Wert = 0.42

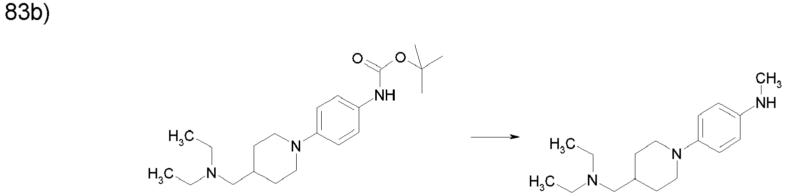

83b wird analog zu 51 b aus 1.80 g (4.98 mMol) Produkt aus 83a und 0.57 g (15.00 mMol) Lithiumaluminiumhydrid (Aldrich) in 25 ml THF hergestellt.
C₁₇H₂₉N₃ (275.43)
[M+H]+ = 276
HPLC (Methode 1): Retentionszeit = 1.77 min

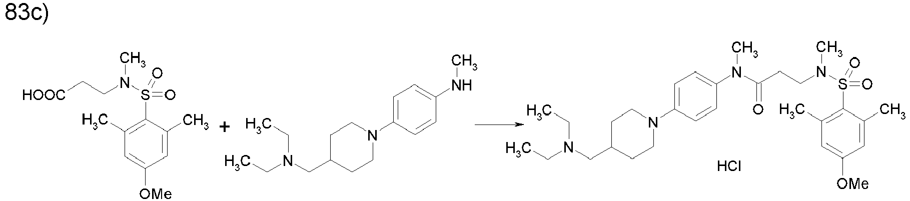

Beispiel 83 wird analog zu 1f aus 0.14 g (0.50 mMol) Produkt aus 22c, 0.15 g (0.50 mMol) Produkt aus 83b, 0.14 ml (1.00 mMol) Triethylamin und 0.18 g (0.55 mMol) TBTU in 6 ml DMF hergestellt.
C₃₀H₄₆N₄O₄S x HCl (595.24)
[M+H]+ = 559
HPLC (Methode 1): Retentionszeit = 2.46 min

### Beispiel 84 (Referenzbeispiel)

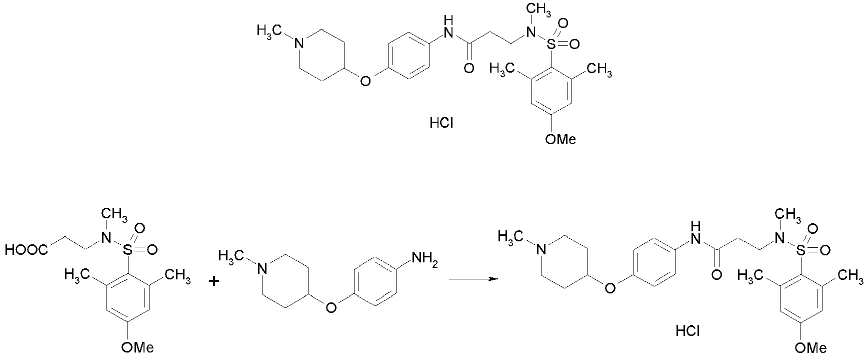

Beispiel 84 wird analog zu 1f aus 0.15 g (0.50 mMol) Produkt aus 22c, 0.10 g (0.50 mMol) 4-(1-Methylpiperidin-4-yloxy)-phenylamin (ART-CHEM), 0.14 ml (1.00 mMol) Triethylamin und 0.18 g (0.55 mMol) TBTU in 6 ml DMF hergestellt.
C₂₅H₃₅N₃O₅S x HCl (526.09)
[M+H]+ = 490
HPLC (Methode 1): Retentionszeit = 2.40 min

### Beispiel 85 (Referenzbeispiel)

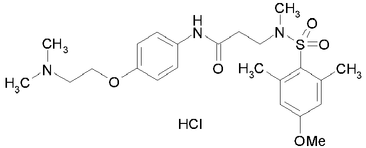

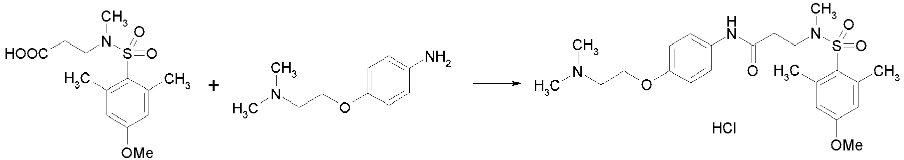

Beispiel 85 wird analog zu 1f aus 0.15 g (0.50 mMol) Produkt aus 22c, 0.09 g (0.50 mMol) 4-(2-Dimethylamino-ethoxy)-phenylamin (Collect. Czech. Chem. Commun. 55, 1990, 282-295), 0.14 ml (1.00 mMol) Triethylamin und 0.18 g (0.55 mMol) TBTU in 6 ml DMF hergestellt.
C₂₃H₃₃N₃O₅S x HCl (500.05)
[M+H]+ = 464
HPLC (Methode 1): Retentionszeit = 2.35 min

### Beispiel 86 (Referenzbeispiel)

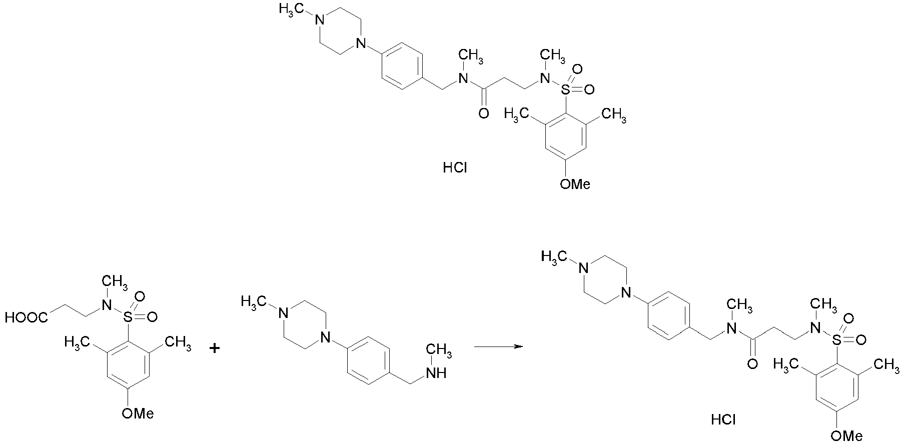

Beispiel 86 wird analog zu 1f aus 0.14 g (0.45 mMol) Produkt aus 22c, 0.099 g (0.45 mMol) Produkt aus 81a, 0.13 ml (0.90 mMol) Triethylamin und 0.17 g (0.54 mMol) TBTU in 5 ml DMF hergestellt.
C₂₆H₃₈N₄O₅S x HCl (539.13)
[M+H]+ = 503
HPLC (Methode 1): Retentionszeit = 2.43 min

### Beispiel 87 (Referenzbeispiel)

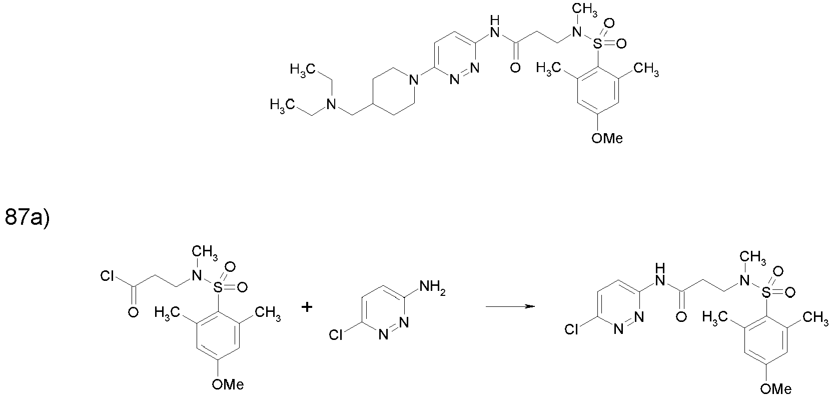

Eine Mischung aus 0.11 g (0.33 mMol) Produkt aus 27c, 0.043 g (0.33 mMol) 3-Amino-6-chlorpyridazin (Acros), 0.12 ml (0.66 mMol) DIPEA und 10 ml Dichlormethan wird drei Tage unter Rückfluss gerührt. Der Niederschlag wird danach abfiltriert. Das Filtrat wird im Vakuum bis zur Trockene eingeengt und das Rohpodukt durch präparative HPLC gereinigt.
C₁₇H₂₁C1N₄O₄S (412.89)
[M+H]+ = 413/415

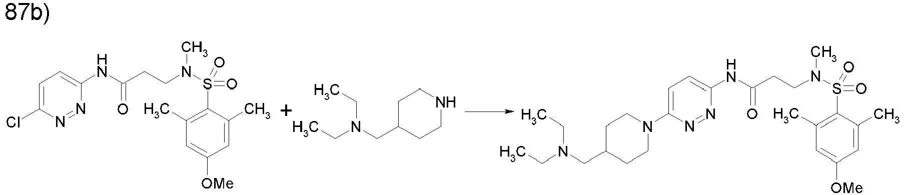

Eine Mischung aus 0.03 g (0.073 mMol) Produkt aus 87a und 0.013 g (0.073 mMol) Diethyl-piperidin-4-ylmethyl-amin (Chem. Pharm. Bull. 42, 1994, 74-84) wird bei 173°C geschmolzen. Das Produkt wird danach durch präparative HPLC aus der Reaktionsmischung gewonnen.
C₂₇H₄₂N₆O₄S (546.73)
[M+H]+ = 547
HPLC (Methode 6): Retentionszeit = 2.12 min

### Beispiel 88 (Referenzbeispiel)

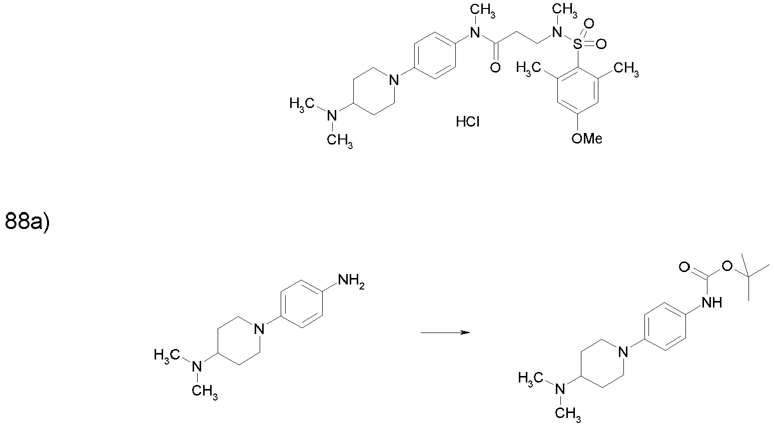

88a wird analog zu 51 a aus 0.70 g (3.18 mMol) Produkt aus 27b, 0.80 g (3.65 mMol) Boc-Anhydrid und 5.50 ml (11.00 mMol) 2 M Natronlauge in 40 ml Dioxan und 20 ml Wasser hergestellt.
C₁₇H₂₈N₄O₂ (320.43)
[M+H]+ = 321
DC: Kieselgel, Dichlormethan / Methanol / Ammoniak 4:1:0.2, Rf-Wert = 0.83

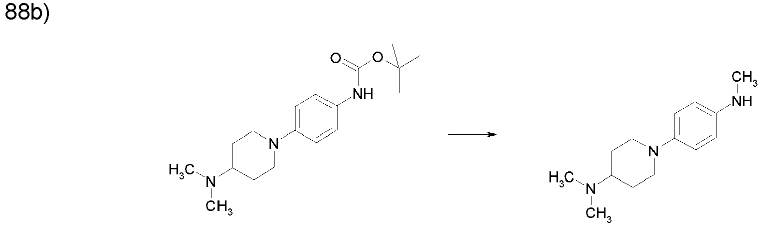

88b wird analog zu 51 b aus 0.60 g (1.87 mMol) Produkt aus 88a und 0.21 g (5.60 mMol) Lithiumaluminiumhydrid (Aldrich) in 20 ml THF hergestellt.
C₁₃H₂₂N₄ (234.34)
[M+H]+ = 235
DC: Kieselgel, Dichlormethan / Methanol / Ammoniak 4:1:0.2, Rf-Wert = 0.62

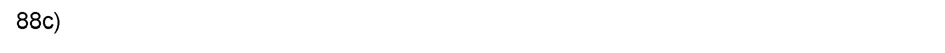

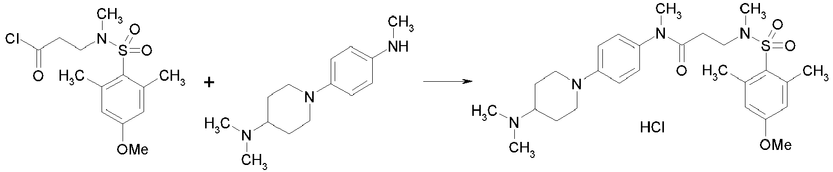

Beispiel 88 wird analog zu 27d aus 0.16 g (0.50 mMol) Produkt aus 27c, 0.12 g (0.50 mMol) Produkt aus 88b und 0.17 ml (1.00 mMol) DIPEA in 5 ml THF hergestellt.
C₂₆H₃₉N₅O₄S x HCl (554.15)
[M+H]+ = 518
DC: Kieselgel, Dichlormethan / Methanol / Ammoniak 9:1:0.1, Rf-Wert = 0.61

### Beispiel 89 (Referenzbeispiel)

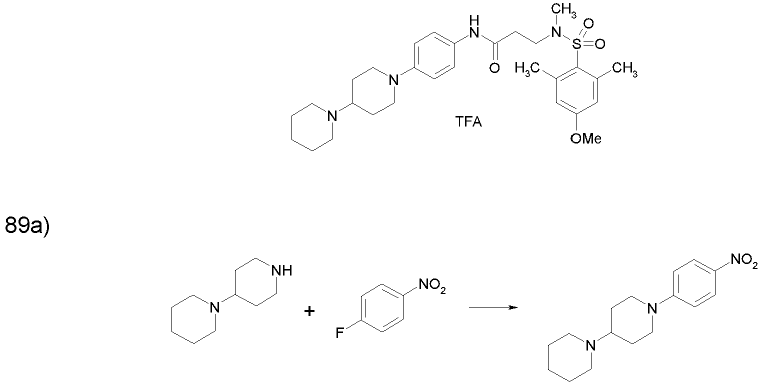

89a wird analog zu 8a aus 0.70 g (4.18 mMol) 4-Piperidinopiperidin (Aldrich), 0.44 ml (4.18 mMol) 1-Fluor-4-nitrobenzol (Acros) und 1.33 ml (9.61 mMol) Triethylamin in 12 ml DMF hergestellt.
C₁₆H₂₃N₃O₂ (289.37)
[M+H]+ = 290
DC: Kieselgel, Dichlormethan / Methanol / Ammoniak 9:1:0.1, Rf-Wert = 0.3

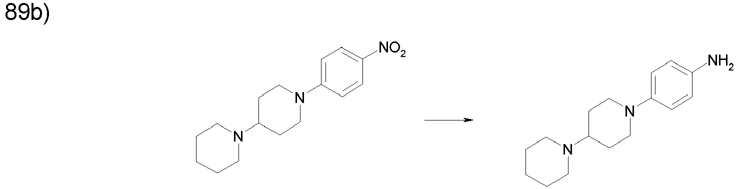

89b wird analog zu 8b aus 0.96 g (3.32 mMol) Produkt aus 89a und 0.093 g Palladium auf Kohle (5%) in 45 ml Ethanol hergestellt.
C₁₆H₂₅N₃ (259.39)
[M+H]+ = 260
DC: Kieselgel, Dichlormethan / Methanol / Ammoniak 9:1:0.1, Rf-Wert = 0.2

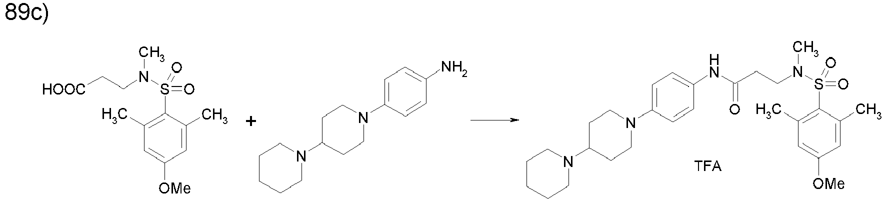

Beispiel 89 wird analog zu 1f aus 0.10 g (0.33 mMol) Produkt aus 22c, 0.086 g (0.33 mMol) Produkt aus 89b, 0.14 ml (1.00 mMol) Triethylamin und 0.11 g (0.33 mMol) TBTU in 2 ml THF hergestellt.
C₂₉H₄₂N₄O₄S x C₂HF₃O₂ (656.76)
[M+H]+ = 543
HPLC (Methode 5): Retentionszeit = 1.47 min

### Beispiel 90 (Referenzbeispiel)

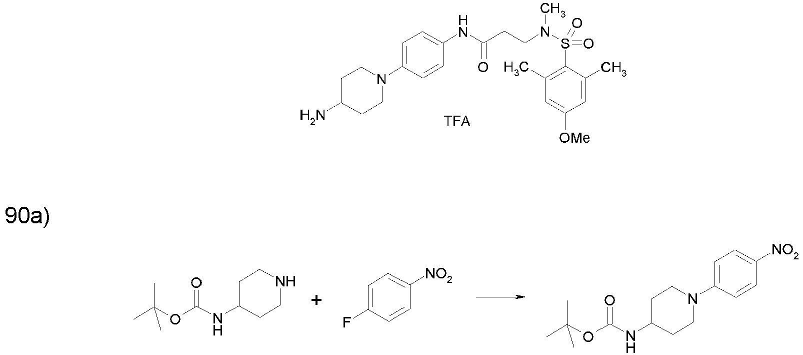

90a wird analog zu 8a aus 0.84 g (4.18 mMol) 4-N-Boc-Aminopiperidin (Acros), 0.44 ml (4.18 mMol) 1-Fluor-4-nitrobenzol (Acros) und 1.33 ml (9.61 mMol) Triethylamin in 12 ml DMF hergestellt.
C₁₆H₂₃N₃O₄ (321.37)
[M+H]+ = 322

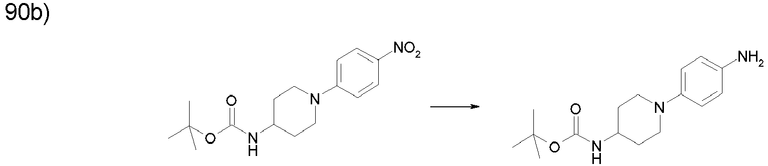

90b wird analog zu 8b aus 1.01 g (3.43 mMol) Produkt aus 90a und 0.11 g Palladium auf Kohle (5%) in 45 ml Ethanol hergestellt.
C₁₆H₂₅N₃O₂ (291.39)
[M+H]+ = 292

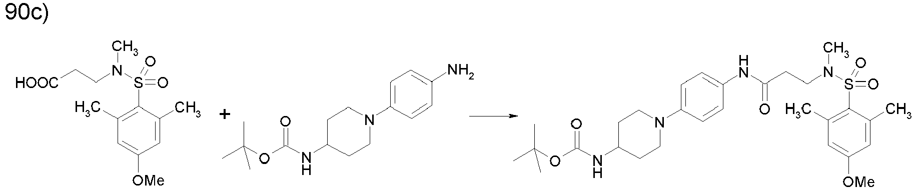

90c wird analog zu 1f aus 0.10 g (0.33 mMol) Produkt aus 22c, 0.097 g (0.33 mMol) Produkt aus 90b, 0.14 ml (1.00 mMol) Triethylamin und 0.11 g (0.33 mMol) TBTU in 2 ml THF hergestellt.
C₂₉H₄₂N₄O₆ (574.73)
[M+H]+ = 575
HPLC (Methode 5): Retentionszeit = 1.62 min

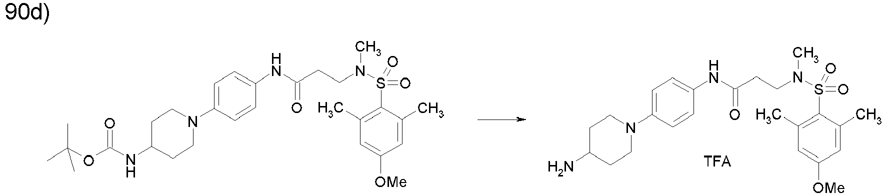

Beispiel 90 wird analog zu 18b aus 0.19 g (0.33 mMol) Produkt aus 90c und 0.33 ml TFA in 0.5 ml Dichlormethan hergestellt.
C₂₄H₃₄N₄O₄S x C₂HF₃O₂ (588.64)
[M+H]+ = 475
HPLC (Methode 5): Retentionszeit = 1.41 min

### Beispiel 91 (Referenzbeispiel)

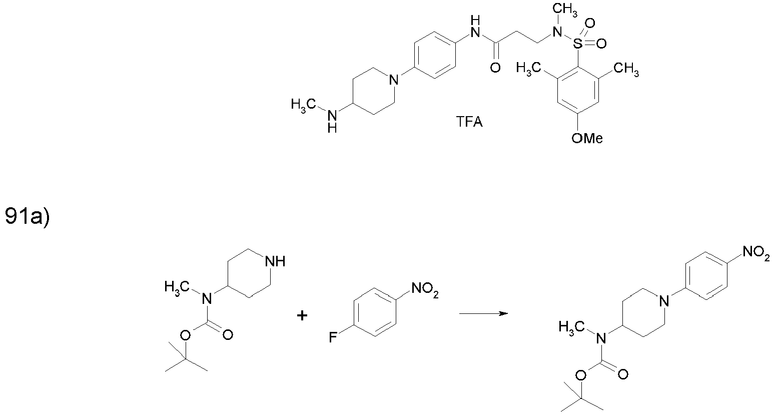

91a wird analog zu 8a aus 0.90 g (4.18 mMol) Methyl-piperidin-4-yl-carbamatsäure-tert-butylester (Fluorochem), 0.44 ml (4.18 mMol) 1-Fluor-4-nitrobenzol (Acros) und 1.33 ml (9.61 mMol) Triethylamin in 12 ml DMF hergestellt.
C₁₇H₂₅N₃O₄ (335.40)
[M+H]+ = 336
DC: Kieselgel, Dichlormethan / Methanol 30:1, Rf-Wert = 0.6

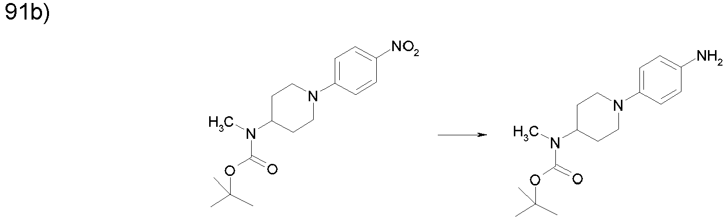

91b wird analog zu 8b aus 1.08 g (3.22 mMol) Produkt aus 91a und 0.11 g Palladium auf Kohle (5%) in 45 ml Ethanol hergestellt.
C₁₇H₂₇N₃O₂ (305.42)
[M+H]+ = 306
DC: Kieselgel, Dichlormethan / Methanol 30:1, Rf-Wert = 0.4

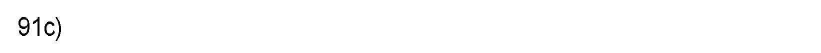

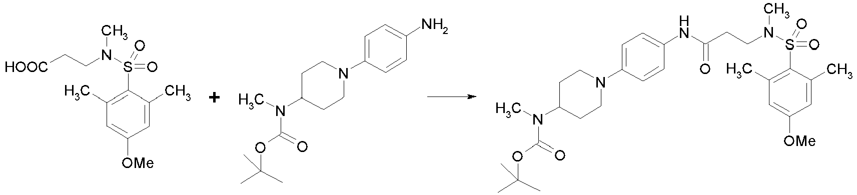

91 c wird analog zu 1f aus 0.10 g (0.33 mMol) Produkt aus 22c, 0.10 g (0.33 mMol) Produkt aus 91b, 0.14 ml (1.00 mMol) Triethylamin und 0.11 g (0.33 mMol) TBTU in 2 ml THF hergestellt.
C₃₀H₄₄N₄O₆S (588.76) [M+H]+ = 589

HPLC (Methode 5): Retentionszeit = 1.69 min

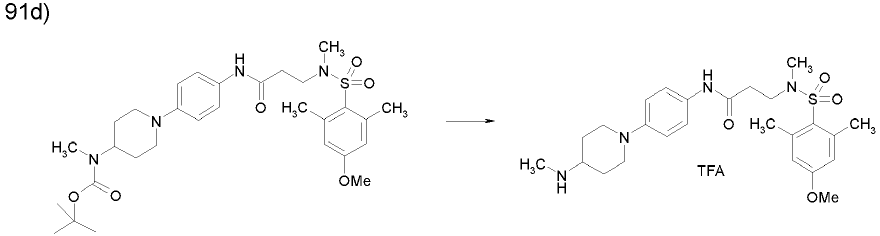

Beispiel 91 wird analog zu 18b aus 0.21 g (0.36 mMol) Produkt aus 91c und 0.36 ml TFA in 0.6 ml Dichlormethan hergestellt.
C₂₅H₃₆N₄O₄S x C₂HF₃O₂ (602.67)
[M+H]+ = 489
HPLC (Methode 5): Retentionszeit = 1.42 min

### Beispiel 92 (Referenzbeispiel)

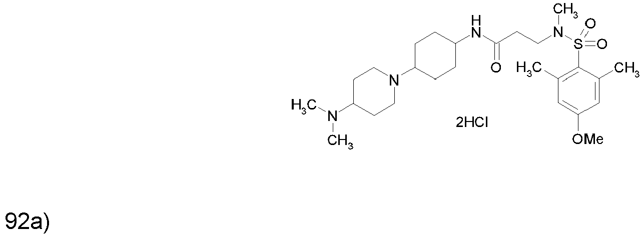

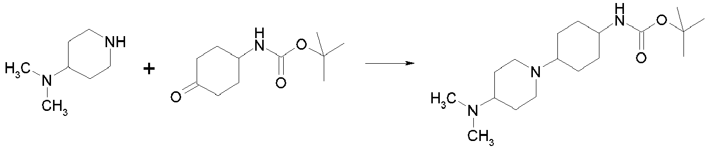

Eine Mischung aus 0.66 g (5.16 mMol) 4-Dimethylamino-piperidin (Alfa Aesar), 1.00 g (4.69 mMol) (4-Oxocyclohexyl)-carbamatsäure-tert-butylester (Fluorochem), 1.19 g (5.16 mMol) Natriumtriacetoxyborhydrid und 20 ml Dichlormethan wird unter Stickstoff vier Stunden bei Raumtemperatur gerührt. Die Reaktionsmischung wird danach mit Dichlormethan verdünnt, mit gesättigter Natriumhydrogencarbonat-Lösung gewaschen, über Natriumsulfat getrocknet und im Vakuum bis zur Trockene eingeengt.
C₁₈H₃₅N₃O₂ (325.49)

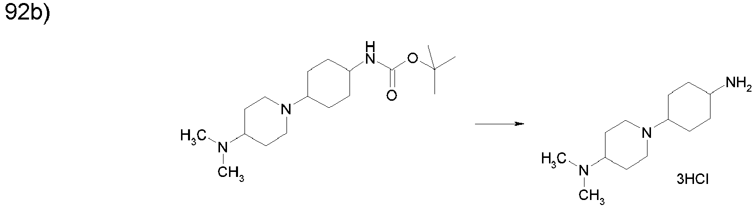

Eine Mischung aus 0.80 g (2.46 mMol) Produkt aus 92a, 4 ml 6 M HCl, 3 ml 37%ige HCl und 3 ml Methanol wird zwei Stunden bei 50°C gerührt. Die Reaktionsmischung wird danach im Vakuum bis zur Trockene eingeengt.
C₁₃H₂₇N₃ x 3HCl (334.76)

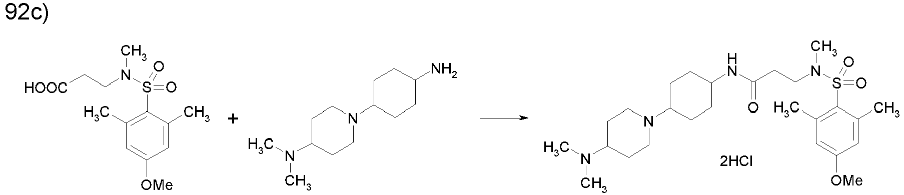

Beispiel 92 wird analog zu 1f aus 0.10 g (0.33 mMol) Produkt aus 22c, 0.13 g (0.40 mMol) Produkt aus 92b, 0.23 ml (1.66 mMol) Triethylamin und 0.13 g (0.40 mMol) TBTU in 8 ml DMF hergestellt.
C₂₆H₄₄N₄O₄S x 2HCl (581.64)
[M+H]+ = 509
HPLC (Methode 5): Retentionszeit = 1.36 min

### Beispiel 93 (Referenzbeispiel)

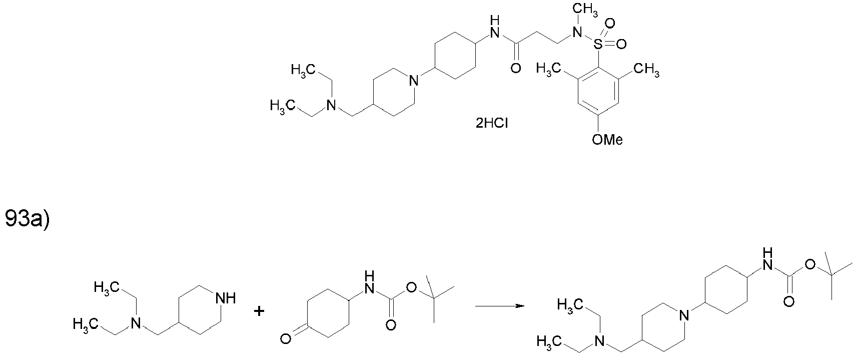

93a wird analog zu 92a aus 0.63 g (3.70 mMol) Diethyl-piperidin-4-ylmethyl-amin (Chem. Pharm. Bull. 42, 1994, 74-84), 0.72 g (3.37 mMol) (4-Oxocyclohexyl)-carbamatsäure-tert-butylester (Fluorochem) und 0.86 g (4.04 mMol) Natriumtriacetoxyborhydrid in 20 ml Dichlormethan durchgeführt.
C₂₁H₄₁N₃O₂ (367.57)

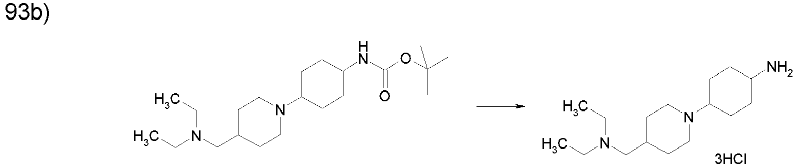

93b wird analog zu 92b aus 0.90 g (2.45 mMol) Produkt aus 93a, 4 ml 6 M HCl und 3 ml 37%ige HCl in 3 ml Methanol hergestellt.
C₁₆H₃₃N₃ x 3HCl (376.84)

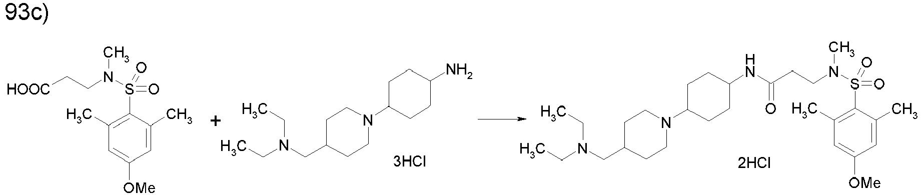

Beispiel 93 wird analog zu 1f aus 0.10 g (0.33 mMol) Produkt aus 22c, 0.14 g (0.37 mMol) Produkt aus 93b, 0.23 ml (1.66 mMol) Triethylamin und 0.13 g (0.40 mMol) TBTU in 8 ml DMF hergestellt.
C₂₉H₅₀N₄O₄S x 2HCl (623.72)
[M+H]+ = 551
HPLC (Methode 5): Retentionszeit = 1.39 min

### Beispiel 94 (Referenzbeispiel)

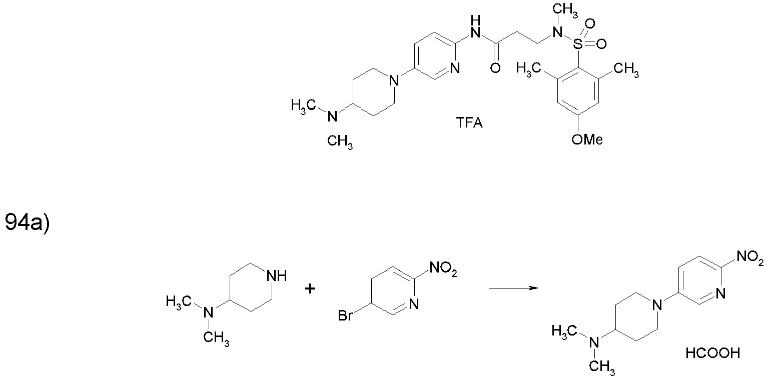

Eine Mischung aus 0.88 g (6.88 mMol) 4-Dimethylamino-piperidin (Alfa Aesar), 1.00 g (4.93 mMol) 3-Brom-6-nitropyriridin (Aldrich), 0.18 g (0.49 mMol) Tetrabutyl-ammoniumiodid, 0.74 g (5.33 mMol) Kaliumcarbonat und 5 ml DMSO wird zwei Stunden bei 80°C gerührt. Danach wird die Reaktionsmischung auf Wasser gegossen und mit Dichlormethan extrahiert. Die organischen Extrakte werden mit gesättigter Natriumhydrogencarbonat-Lösung und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt.
C₁₂H₁₈N₄O₂ x CH₂O₂ (296.32)
HPLC (Methode 1): Retentionszeit = 1.49 min

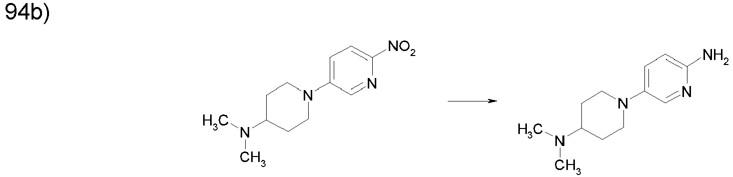

94b wird analog zu 8b aus 0.50 g (2.00 mMol) Produkt aus 94a und 0.08 g Palladium auf Kohle (10%) in 40 ml Methanol hergestellt.
C₁₂H₂₀N₄ (220.31)

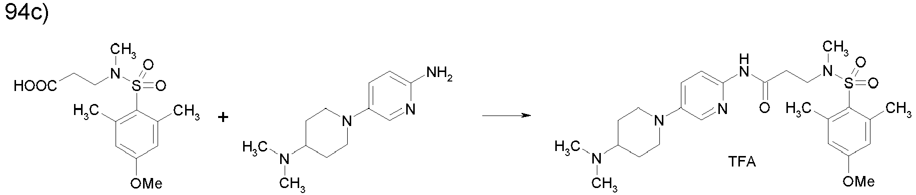

Eine Mischung aus 0.63 g (2.10 mMol) Produkt aus 22c, 0.91 g (9.00 mMol) N-Methylmorpholin, 0.45 g (2.04 mMol) Produkt aus 94b und 50 ml THF wird 10 Minuten bei Raumtemperatur gerührt und danach mit 5.22 ml (9.00 mMol) Propylphosphonsäureanhydrid 50% in Ethylacetat (Fluka) versetzt. Man lässt die Reaktionsmischung über Nacht bei Raumtemperatur rühren und engt sie anschließend im Vakuum bis zur Trockene ein. Der Rückstand wird mit 2 N Kaliumcarbonat-Lösung versetzt und mit Dichlormethan extrahiert. Die organischen Extrakte werden mit Wasser und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt. C₂₅H₃₇N₅O₄S x C₂HF₃O₂ (617.68)
[M+H]+ = 504
HPLC (Methode 5): Retentionszeit = 1.38 min

### Beispiel 95 (Referenzbeispiel)

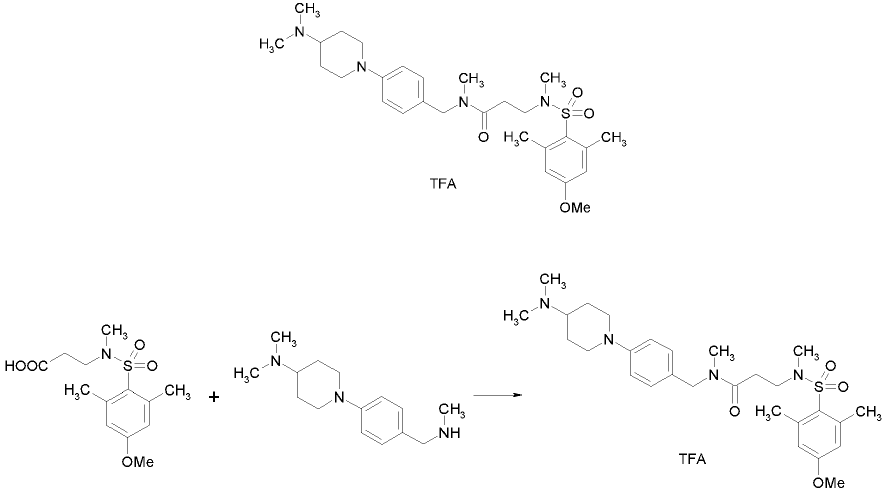

Beispiel 95 wird analog zu 1f aus 0.08 g (0.27 mMol) Produkt aus 22c, 0.066 g (0.27 mMol) Produkt aus 80a, 0.11 ml (0.80 mMol) Triethylamin und 0.085 g (0.27 mMol) TBTU in 2 ml THF hergestellt.
C₂₈H₄₂N₄O₄S x C₂HF₃O₂ (644.75)
[M+H]+ = 531
HPLC (Methode 5): Retentionszeit = 1.50 min

### Beispiel 96 (Referenzbeispiel)

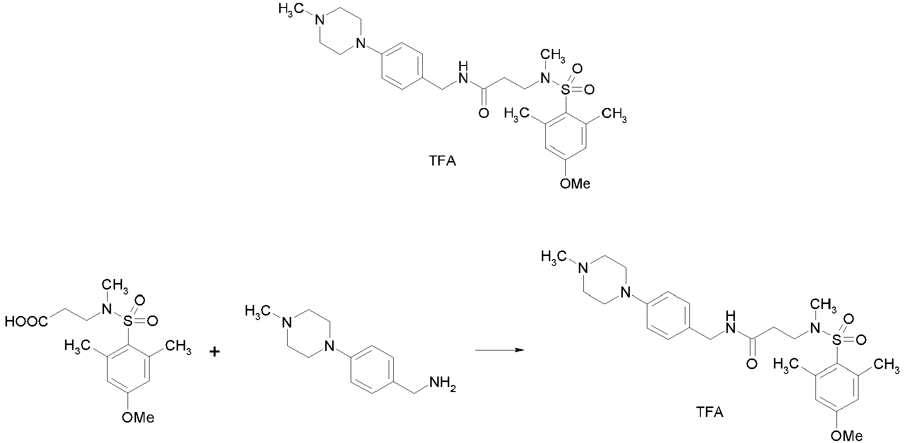

Beispiel 96 wird analog zu 1f aus 0.08 g (0.27 mMol) Produkt aus 22c, 0.054 g (0.27 mMol) Produkt aus 19b, 0.11 ml (0.80 mMol) Triethylamin und 0.085 g (0.27 mMol) TBTU in 2 ml THF hergestellt.
C₂₅H₃₆N₄O₄S x C₂HF₃O₂ (602.67)
[M+H]+ = 489
HPLC (Methode 5): Retentionszeit = 1.49 min

### Beispiel 97 (Referenzbeispiel)

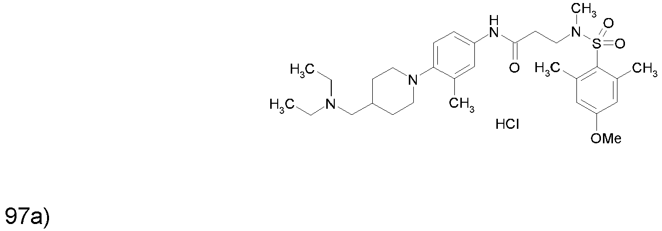

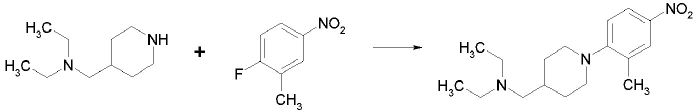

97a wird analog zu 8a aus 1.00 g (5.87 mMol) Diethyl-piperidin-4-ylmethyl-amin (Chem. Pharm. Bull. 42, 1994, 74-84), 0.91 g (5.87 mMol) 1-Fluor-2-methyl-4-nitrobenzol (ABCR) und 1.14 ml (8.20 mMol) Triethylamin in 12 ml DMF hergestellt.
C₁₇H₂₇N₃O₂ (305.42)
[M+H]+ = 306

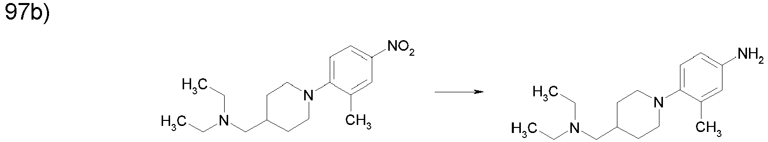

97b wird analog zu 8b aus 0.91 g (2.98 mMol) Produkt aus 97a und 0.20 g Palladium auf Kohle (10%) in 50 ml Methanol hergestellt.
C₁₇H₂₉N₃ (275.43)

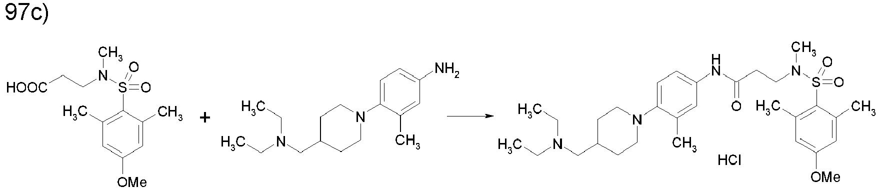

Beispiel 97 wird analog zu 1f aus 0.15 g (0.50 mMol) Produkt aus 22c, 0.14 g (0.50 mMol) Produkt aus 97b, 0.21 ml (1.50 mMol) Triethylamin und 0.16 g (0.50 mMol) TBTU in 7 ml THF und 1 ml DMF hergestellt.
C₃₀H₄₆N₄O₄S x HCl (595.24)
[M+H]+ = 559
HPLC (Methode 5): Retentionszeit = 1.44 min

### Beispiel 98 (Referenzbeispiel)

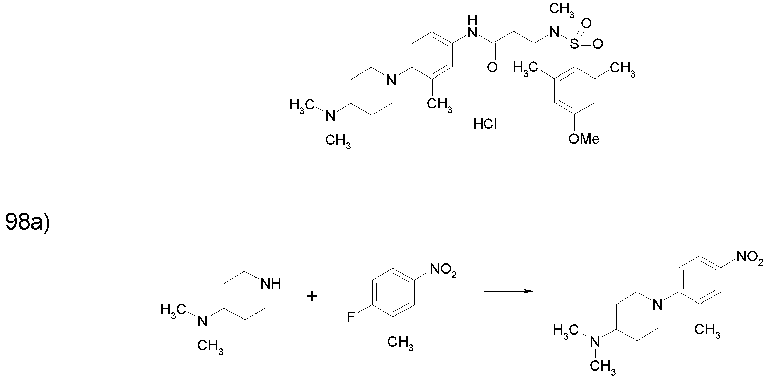

98a wird analog zu 8a aus 0.75 g (5.87 mMol) 4-Dimethylamino-piperidin (Alfar Aesar), 0.91 g (5.87 mMol) 1-Fluor-2-methyl-4-nitrobenzol (ABCR) und 1.14 ml (8.20 mMol) Triethylamin in 12 ml DMF hergestellt.
C₁₄H₂₁N₃O₂ (263.34)

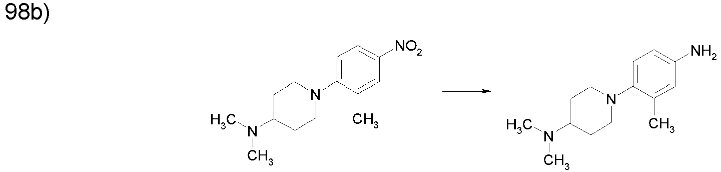

98b wird analog zu 8b aus 0.30 g (1.14 mMol) Produkt aus 98a und 0.10 g Palladium auf Kohle (10%) in 25 ml Methanol hergestellt.
C₁₄H₂₃N₃ (233.35)

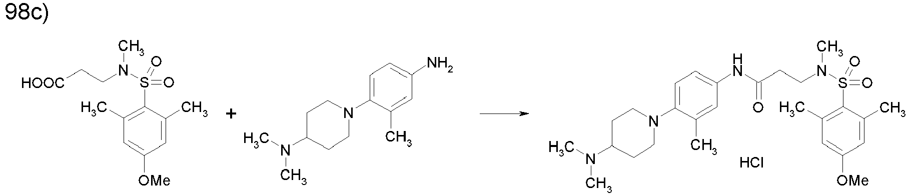

Beispiel 98 wird analog zu 1f aus 0.32 g (1.07 mMol) Produkt aus 22c, 0.25 g (1.07 mMol) Produkt aus 98b, 0.42 ml (3.00 mMol) Triethylamin und 0.34 g (1.07 mMol) TBTU in 7 ml THF und 1 ml DMF hergestellt.
C₂₇H₄₀N₄O₄S x HCl (553.16)
[M+H]+ = 517
HPLC (Methode 5): Retentionszeit = 1.52 min

### Beispiel 99 (Referenzbeispiel)

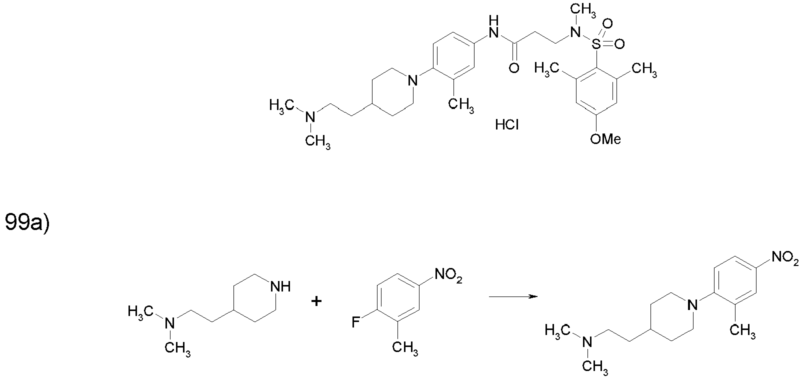

99a wird analog zu 8a aus 0.92 g (5.87 mMol) Dimethyl-(2-piperidin-4-yl-ethyl)-amin (J. Med. Chem. 36, 1993, 162-165), 0.91 g (5.87 mMol) 1-Fluor-2-methyl-4-nitrobenzol (ABCR) und 2.49 g (18.00 mMol) Kaliumcarbonat in 12 ml DMF hergestellt.
C₁₆H₂₅N₃O₂ (291.39)
[M+H]+ = 292

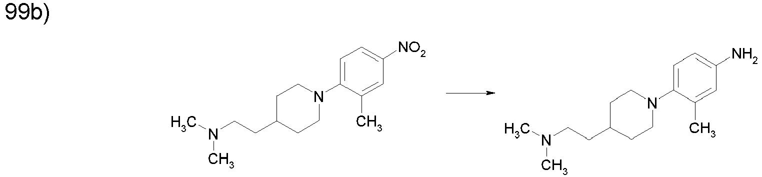

99b wird analog zu 8b aus 0.60 g (1.14 mMol) Produkt aus 99a und 0.20 g Palladium auf Kohle (10%) in 50 ml Methanol hergestellt.
C₁₆H₂₇N₃ (261.41)

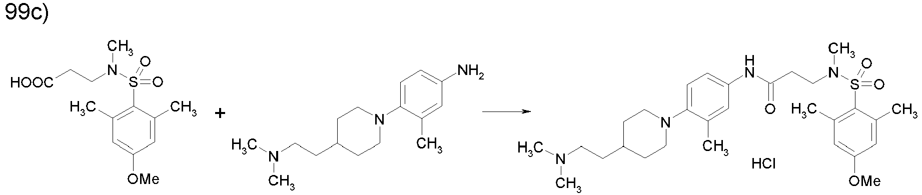

Beispiel 99 wird analog zu 1f aus 0.15 g (0.50 mMol) Produkt aus 22c, 0.13 g (0.50 mMol) Produkt aus 99b, 0.21 ml (1.50 mMol) Triethylamin und 0.16 g (0.50 mMol) TBTU in 7 ml THF und 1 ml DMF hergestellt.
C₂₉H₄₄N₄O₄S x HCl (581.21)
[M+H]+ = 545
HPLC (Methode 5): Retentionszeit = 1.42 min

### Beispiel 100 (Referenzbeispiel)

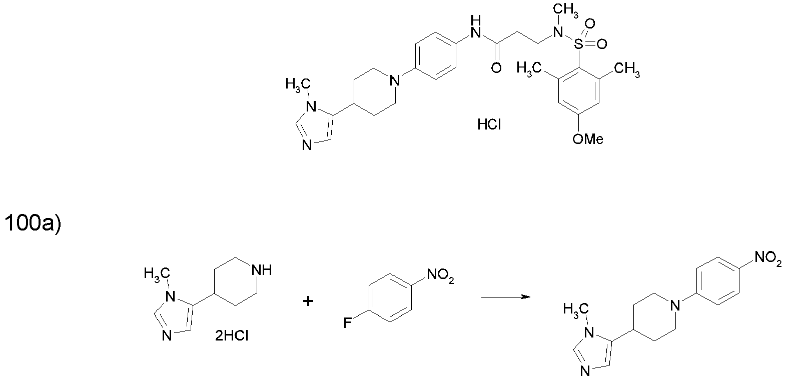

100a wird analog zu 8a aus 1.00 g (4.47 mMol) 4-(3-Methyl-3H-imidazol-4-yl)-piperidin (J. Med. Chem. 46, 2003, 5445-5457), 0.63 g (4.47 mMol) 4-Fluor-nitrobenzol (ABCR) und 2.10 g (15.20 mMol) Kaliumcarbonat in 50 ml DMF hergestellt.
C₁₄H₁₆N₄O₂ (272.30)
[M+H]+ = 273

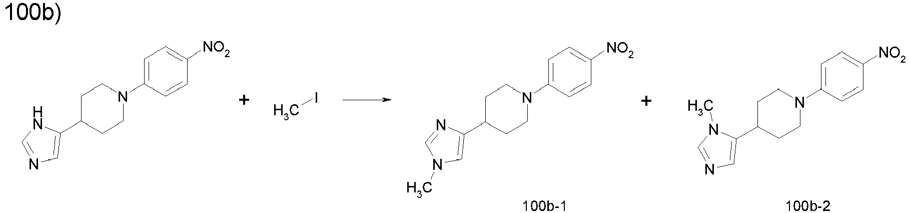

100b-1 und 100b-2 werden analog zu 62a aus 1.10 g (4.04 mMol) Produkt aus 100a, 0.60 g (4.23 mMol) Methyliodid und 0.46 g (4.10 mMol) Kalium-tert-butylat in 50 ml DMSO hergestellt. Das so entstandene Isomerengemisch wird durch Säulen-chromatographie über Kieselgel (Elutionsmittel: Dichlormethan / Methanol 100:1 bis 30:1) aufgetrennt.

| | |
|---|---|
| 100b-1: | C₁₅H₁₈N₄O₂ (286.33) |
| | [M+H]+ = 287 |
| | DC: Kieselgel, Dichlormethan / Methanol 9:1, Rf-Wert = 0.50 |
| 100b-2: | C₁₅H₁₈N₄O₂ (286.33) |
| | [M+H]+ = 287 |
| | DC: Kieselgel, Dichlormethan / Methanol 9:1, Rf-Wert = 0.38 |

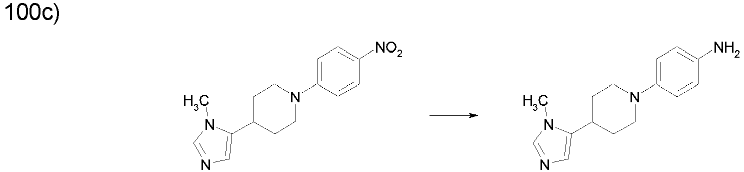

100c wird analog zu 8b aus 0.10 g (0.35 mMol) 100b-2 und 0.20 g Palladium auf Kohle (10%) in 30 ml Methanol hergestellt.
C₁₅H₂₀N₄ (256.35)
DC: Kieselgel, Dichlormethan / Methanol 9:1, Rf-Wert = 0.10

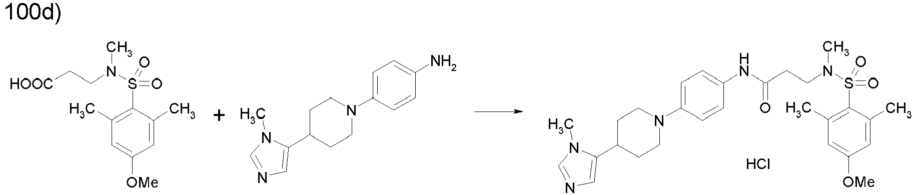

Beispiel 100 wird analog zu 1f aus 0.085 g (0.28 mMol) Produkt aus 22c, 0.070 g (0.27 mMol) Produkt aus 100c, 0.048 ml (0.35 mMol) Triethylamin und 0.095 g (0.30 mMol) TBTU in 20 ml THF und 3 ml DMF hergestellt.
C₂₈H₃₇N₅O₄S x HCl (576.15)
[M+H]+ = 540
HPLC (Methode 5): Retentionszeit = 1.41 min

### Beispiel 101 (Referenzbeispiel)

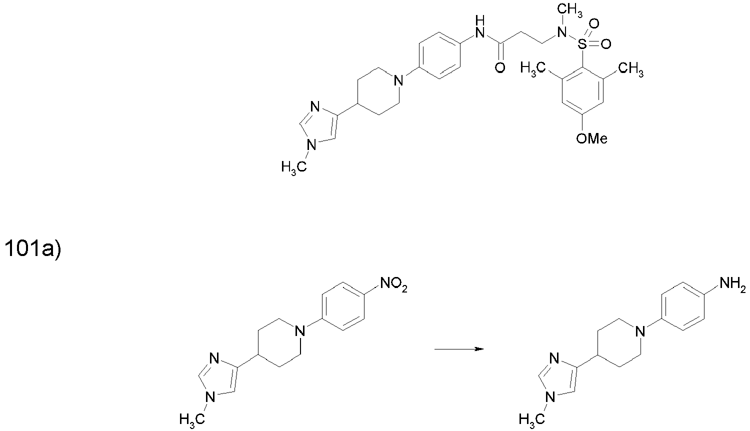

101 a wird analog zu 8b aus 0.35 g (1.22 mMol) 100b-1 und 0.50 g Palladium auf Kohle (10%) in 50 ml Methanol hergestellt.
C₁₅H₂₀N₄ (256.35)
DC: Kieselgel, Dichlormethan / Methanol 9:1, Rf-Wert = 0.14

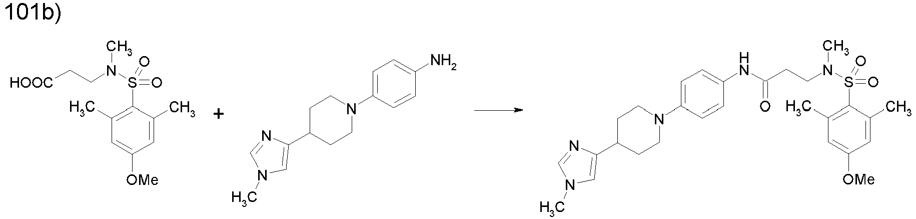

Beispiel 101 wird analog zu 1f aus 0.12 g (0.40 mMol) Produkt aus 22c, 0.10 g (0.39 mMol) Produkt aus 101a, 0.05 ml (0.50 mMol) Triethylamin und 0.14 g (0.42 mMol) TBTU in 30 ml THF und 5 ml DMF hergestellt.
C₂₈H₃₇N₅O₄S (539.69)
[M+H]+ = 540
DC: Kieselgel, Dichlormethan / Methanol 9:1, Rf-Wert = 0.47

### Beispiel 102 (Referenzbeispiel)

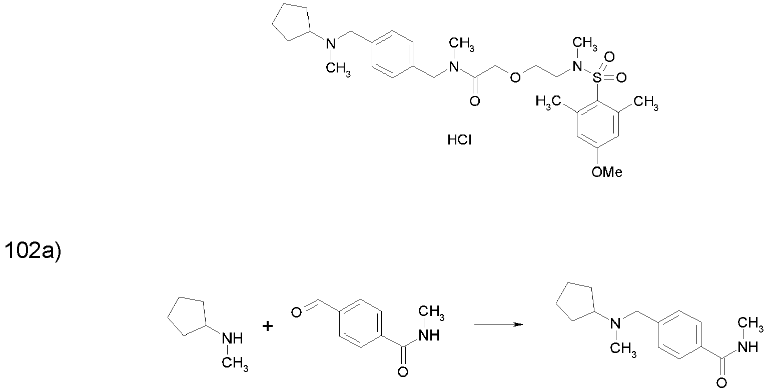

102a wird analog zu 60a aus 0.50 g (5.00 mMol) N-Methylcyclohexylamin (CHESS), 0.82 g (5.00 mMol) 4-Formyl-benzoesäuremethylamid (EMKA), 1.59 g (7.50 mMol) Natriumtriacetoxyborhydrid und 0.37 ml (6.50 mMol) Essigsäure in 30 ml THF hergestellt.
C₁₅H₂₂N₂ (246.35)

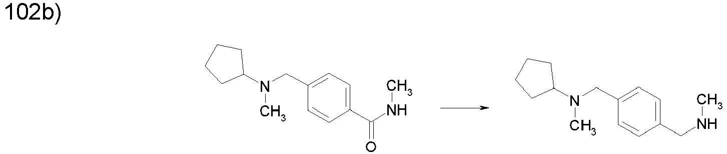

102b wird analog zu 38f aus 1.06 g (4.30 mMol) Produkt aus 102a und 8.60 ml (8.60 mMol) Lithiumaluminiumhydrid 1 M in THF (Aldrich) in 40 ml THF hergestellt.
C₁₅H₂₄N₂ (232.36)

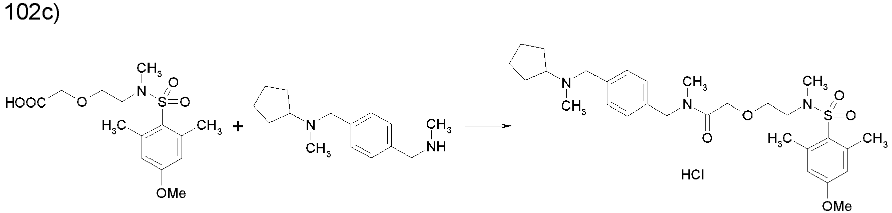

Beispiel 102 wird analog zu 1f aus 0.20 g (0.60 mMol) Produkt aus 53c, 0.14 g (0.60 mMol) Produkt aus 102b, 0.21 ml (1.50 mMol) Triethylamin und 0.23 g (0.72 mMol) TBTU in 10 ml THF hergestellt.
C₂₉H₄₃N₃O₅S x HCl (582.20)
[M+H]+ = 546
HPLC (Methode 5): Retentionszeit = 1.58 min

### Beispiel 103 (Referenzbeispiel)

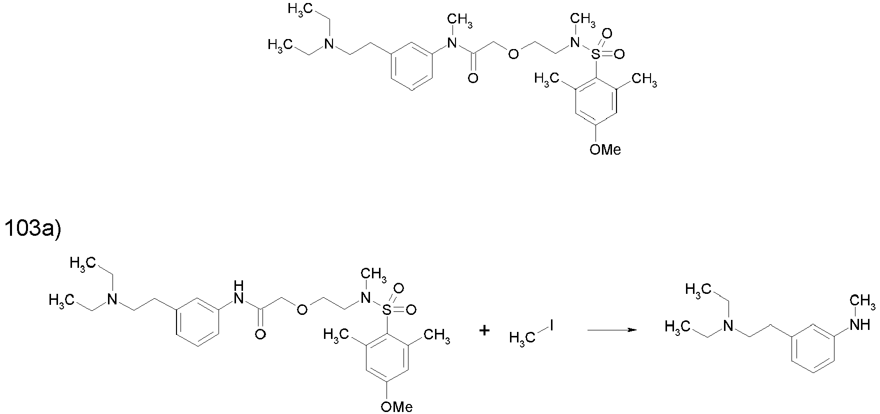

Eine Mischung aus 0.04 g (0.079 mMol) 70, 4.8 mg (0.12 mMol) Natriumhydrid 60%ig, 1 ml THF und 0.5 ml DMF wird 30 Minuten bei Raumtemperatur gerührt. Danach gibt man 4.9 µl (0.079 mMol) Methyliodid hinzu und lässt weitere zwei Stunden bei Raumtemperatur rühren. Die Reaktionsmischung wird anschließend im Vakuum bis zur Trockene eingeengt, der Rückstand mit Wasser versetzt und mit Ethylacetat extrahiert. Die organischen Extrakte werden über Natriumsulfat getrocknet und im Vakuum bis zur Trockene eingeengt. Das so erhaltene Rohprodukt wird durch Säulenchromatographie (Elutionsmittel: Dichlormethan / Methanol / Ammoniak 95:5:0.5) gereinigt.
C₁₃H₂₂N₂ (206.33)
[M+H]+ = 207

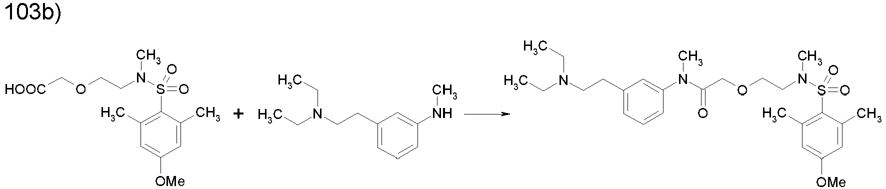

Beispiel 103 wird analog zu 1f aus 0.08 g (0.24 mMol) Produkt aus 53c, 0.05 g (0.24 mMol) Produkt aus 103a, 0.067 ml (0.48 mMol) Triethylamin und 0.093 g (0.29 mMol) TBTU in 7 ml THF und 1 ml DMF hergestellt.
C₂₇H₄₁N₃O₅S (519.70)
[M+H]+ = 520
HPLC (Methode 4): Retentionszeit = 3.2 min

### Beispiel 104 (Referenzbeispiel)

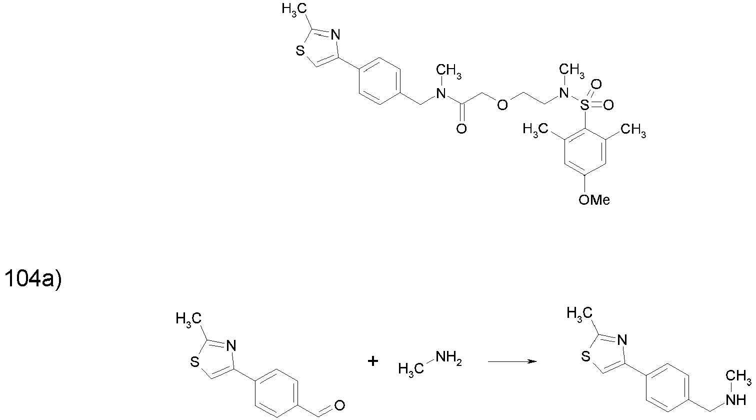

104a wird analog zu 60a aus 0.35 g (1.72 mMol) 4-(2-Methyl-1,3-thiazol-4-yl)-benzaldehyd (Maybridge), 1.50 ml (3.00 mMol) Methylamin 2 M in THF (Acros), 0.70 g (3.30 mMol) Natriumtriacetoxyborhydrid und 0.23 ml (4.00 mMol) Essigsäure in 20 ml THF hergestellt.
C₁₂H₁₄N₂S (218.32)
[M+H]+ = 219

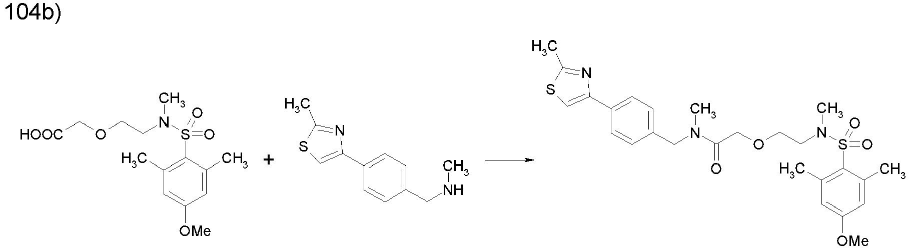

Beispiel 104 wird analog zu 1f aus 0.16 g (0.47 mMol) Produkt aus 53c, 0.10 g (0.46 mMol) Produkt aus 104a, 0.11 ml (1.09 mMol) Triethylamin und 0.16 g (0.48 mMol) TBTU in 30 ml THF und 5 ml DMF hergestellt.
C₂₆H₃₃N₃O₅S₂ (531.69)
[M+H]+ = 532
DC: Kieselgel, Dichlormethan / Methanol 9:1, Rf-Wert = 0.66

### Beispiel 105 (Referenzbeispiel)

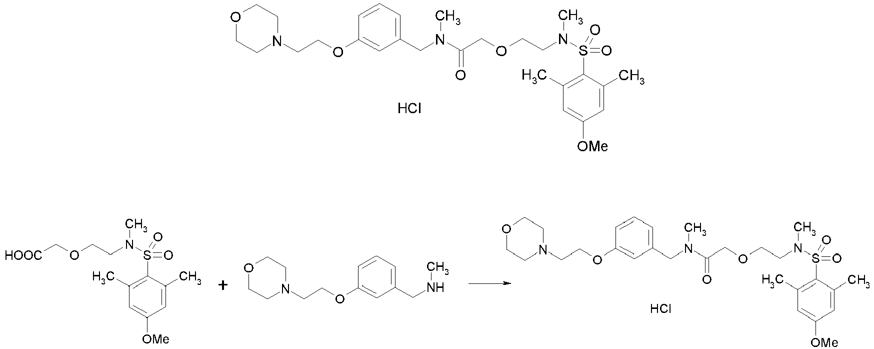

Beispiel 105 wird analog zu 1f aus 0.14 g (0.41 mMol) Produkt aus 53c, 0.10 g (0.40 mMol) Methyl-[3-(2-morpholin-4-yl-ethoxy)-benzyl]-amin (Maybridge), 0.14 ml (0.99 mMol) Triethylamin und 0.14 g (0.44 mMol) TBTU in 30 ml THF und 5 ml DMF hergestellt. C₂₈H₄₁N₃O₇S x HCl (600.17)
[M+H]+ = 564
DC: Kieselgel, Dichlormethan / Methanol 9:1, Rf-Wert = 0.59

### Beispiel 106 (Referenzbeispiel)

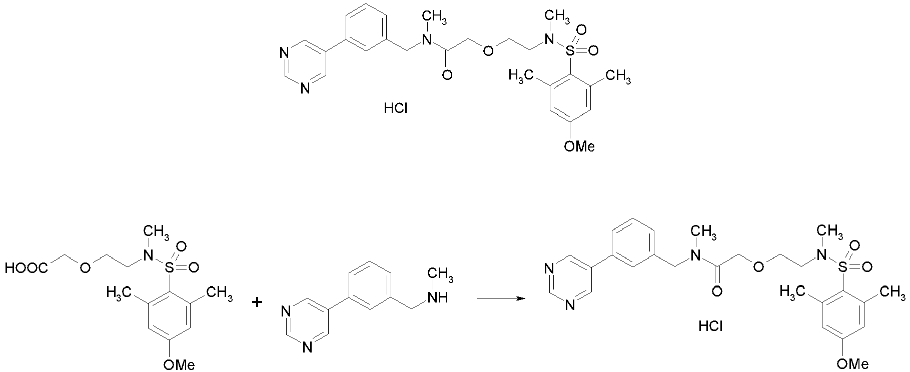

Beispiel 106 wird analog zu 1f aus 0.17 g (0.51 mMol) Produkt aus 53c, 0.10 g (0.50 mMol) Methyl-(3-pyrimidin-5-yl-benzyl)-amin (Maybridge), 0.17 ml (1.19 mMol) Triethylamin und 0.17 g (0.53 mMol) TBTU in 30 ml THF und 5 ml DMF hergestellt.
C₂₆H₃₂N₄O₅S x HCl (549.08)
[M+H]+ = 513
HPLC (Methode 5): Retentionszeit = 1.78 min

### Beispiel 107 (Referenzbeispiel)

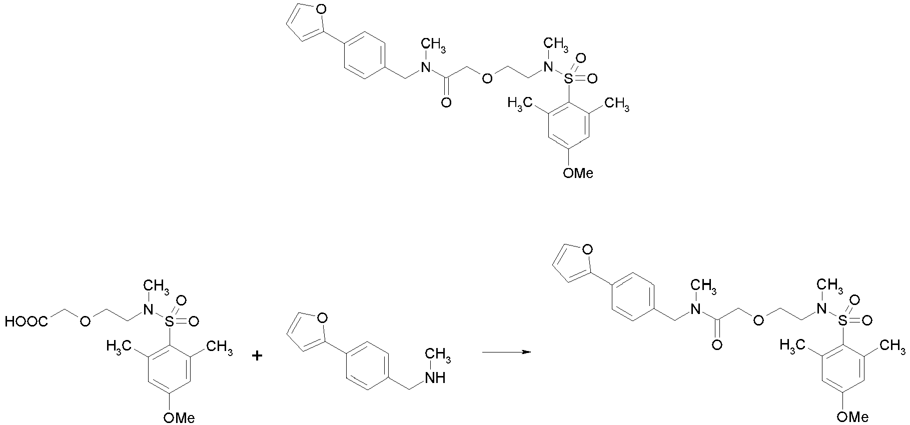

Beispiel 107 wird analog zu 1f aus 0.18 g (0.54 mMol) Produkt aus 53c, 0.10 g (0.53 mMol) (4-Furan-2-yl-benzyl)-methyl-amin (Maybridge), 0.18 ml (1.29 mMol) Triethylamin und 0.18 g (0.56 mMol) TBTU in 30 ml THF und 5 ml DMF hergestellt.
C₂₆H₃₂N₂O₆S (500.61)
[M+H]+ = 501
HPLC (Methode 5): Retentionszeit = 2.09 min

### Beispiel 108 (Referenzbeispiel)

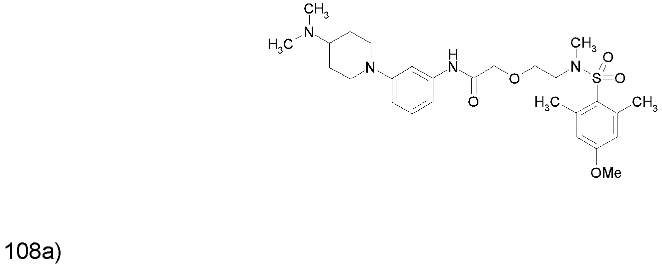

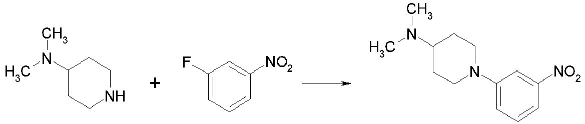

107a wird analog zu 79a aus 2.41 g (18.78 mMol) 4-Dimethylamino-piperidin (Alfa Aesar), 1.00 ml (9.39 mMol) 1-Fluor-3-nitrobenzol (Fluka) und 1.30 g (9.39 mMol) Kaliumcarbonat in 15 ml DMSO hergestellt.
C₁₃H₁₉N₃O₂ (249.31)
[M+H]+ = 250

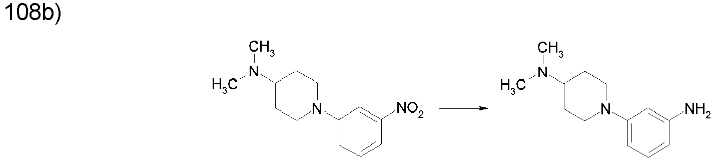

Eine Mischung aus 1.74 g (6.98 mMol) Produkt aus 108a, 12.00 g (68.92 mMol) Natriumdithionit, 10.00 g (72.35 mMol) Kaliumcarbonat, 60 ml THF und 30 ml Wasser sechs Stunden auf 80°C erhitzt. Nach dem Abkühlen wird die organische Phase abgetrennt, mit gesättigter Natriumchlorid-Lösung gewaschen, über Natriumsulfat getrocknet und im Vakuum bis zur Trockene eingeengt.
C₁₃H₂₁N₃ (219.33)

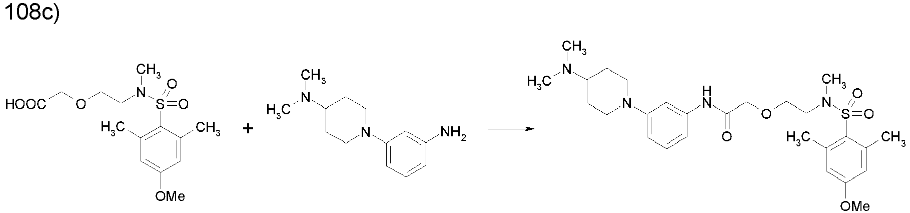

Beispiel 108 wird analog zu 1f aus 0.13 g (0.40 mMol) Produkt aus 53c, 0.10 g (0.47 mMol) Produkt aus 108b, 0.067 ml (0.48 mMol) Triethylamin und 0.15 g (0.48 mMol) TBTU in 5 ml DMF hergestellt.
C₂₇H₄₀N₄O₅S (532.70)
[M+H]+ = 533
HPLC (Methode 5): Retentionszeit = 1.56 min

### Beispiel 109 (Referenzbeispiel)

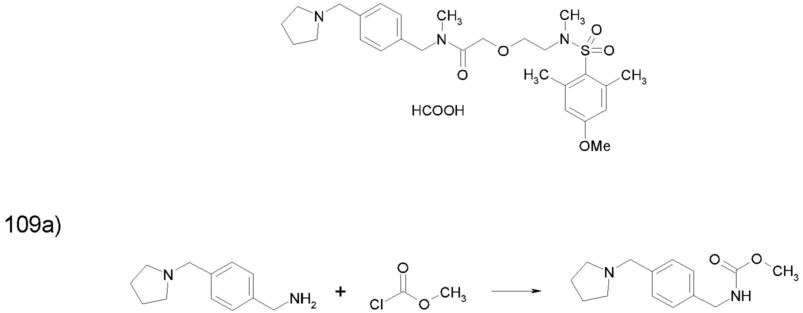

Eine Mischung aus 0.59 g (3.12 mMol) 4-Pyrrolidin-1-ylmethyl-benzylamin (Enamine-BB), 0.29 ml (3.75 mMol) Chlorameisensäuremethylester (Fluka), 0.52 ml (3.75 mMol) Triethylamin und 10 ml Dichlormethan wird zwei Stunden bei Raumtemperatur gerührt. Die Reaktionsmischung wird mit 10 ml Dichlormethan verdünnt, zweimal mit Wasser gewaschen, über Natriumsulfat getrocknet und im Vakuum bis zur Trockene eingeengt.
C₁₄H₂₀N₂O₂ (248.32)

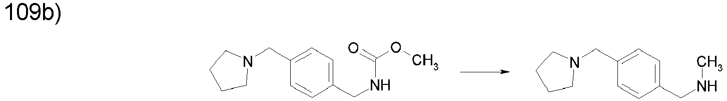

109b wird analog zu 51b aus 0.61 g (2.44 mMol) Produkt aus 109a und 5.00 ml (5.00 mMol) Lithiumaluminiumhydrid 1 M in THF (Aldrich) in 5 ml THF hergestellt.
C₁₃H₂₀N₂ (204.31)

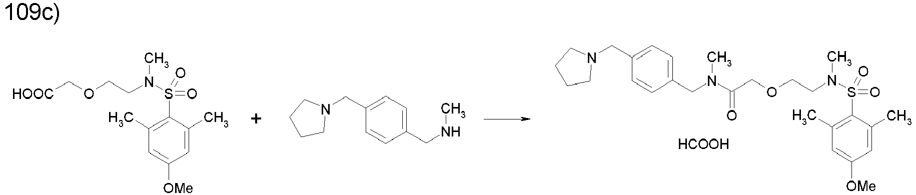

Beispiel 109 wird analog zu 1f aus 0.18 g (0.54 mMol) Produkt aus 53c, 0.17 g (0.81 mMol) Produkt aus 109b, 0.15 ml (1.07 mMol) Triethylamin und 0.21 g (0.65 mMol) TBTU in 4 ml DMF hergestellt.
C₂₇H₃₉N₃O₅S x CH₂O₂ (631.71)
[M+H]+ = 518
HPLC (Methode 5): Retentionszeit = 1.56 min

### Beispiel 110 (Referenzbeispiel)

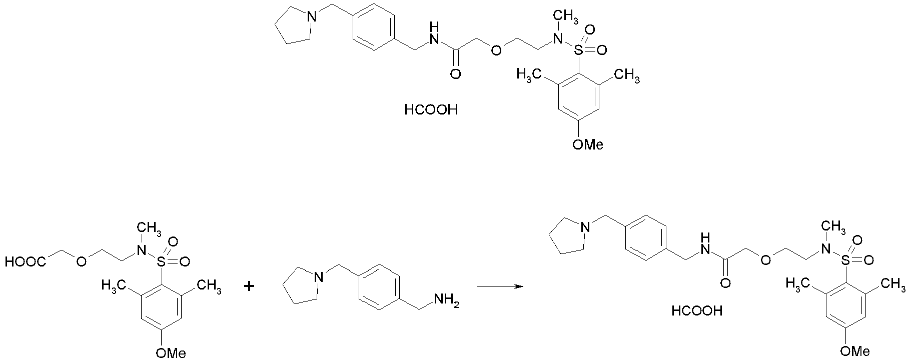

Beispiel 110 wird analog zu 1f aus 0.12 g (0.36 mMol) Produkt aus 53c, 0.096 g (0.51 mMol) 4-Pyrrolidin-1-ylmethyl-benzylamin (Enamine-BB), 0.10 ml (0.72 mMol) Triethylamin und 0.14 g (0.44 mMol) TBTU in 4 ml DMF hergestellt.
C₂₆H₃₇N₃O₅S x CH₂O₂ (617.68)
[M+H]+ = 504
HPLC (Methode 5): Retentionszeit = 1.56 min

### Beispiel 111 (Referenzbeispiel)

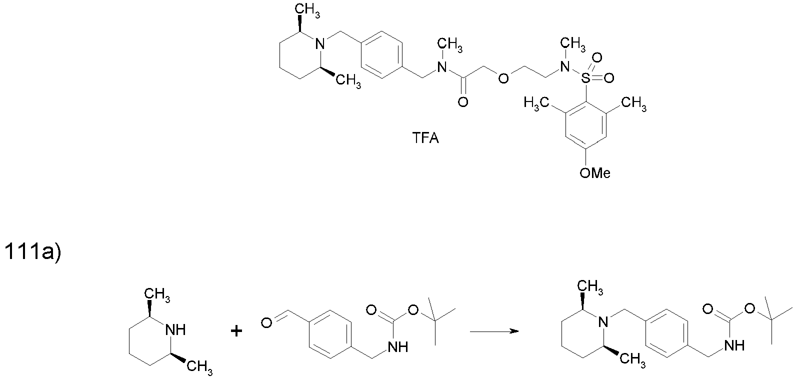

Eine Mischung aus 1.00 g (4.25 mMol) (4-Formylbenzyl)-carbamatsäure-tert-butylester (Acros) und 10 ml Dichlormethan wird unter Eisbadkühlung nacheinander mit 1.15 ml (8.50 mMol) cis-2,6-Dimethylpiperidin (Aldrich) und 1.80 g (8.50 mMol) Natriumtriacetoxyborhydrid versetzt. Die Reaktionsmischung wird drei Tage bei Raumtemperatur gerührt, danach langsam mit gesättigter Natriumhydrogencarbonat-Lösung gequencht und mit Dichlormethan extrahiert. Die organischen Extrakte werden mit Wasser und gesättigter Natriumchlorid-Lösung gewaschen, über Natriumsulfat getrocknet und im Vakuum bis zur Trockene eingeengt. Das so erhaltene Rohprodukt wird durch Säulenchromatographie (Elutionsmittel: Dichlormethan / Methanol 93:7) gereinigt.
C₂₀H₃₂N₂O₂ (332.48)
[M+H]+ = 333
HPLC (Methode 5): Retentionszeit = 1.47 min

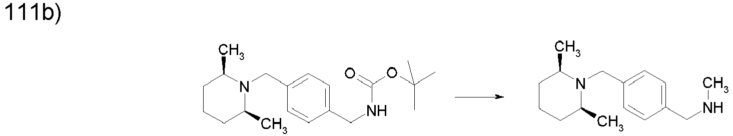

111 b wird analog zu 51 b aus 0.92 g (2.76 mMol) Produkt aus 111 a und 8.27 ml (8.27 mMol) Lithiumaluminiumhydrid 1 M in THF (Aldrich) in 30 ml THF hergestellt.
C₁₆H₂₆N₂ (246.39)
[M+H]+ = 247
HPLC (Methode 5): Retentionszeit = 1.03 min

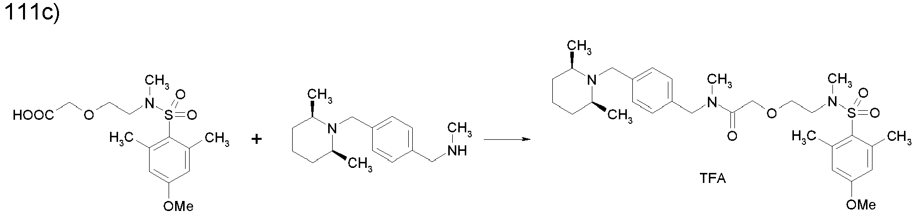

Beispiel 111 wird analog zu 1f aus 0.08 g (0.24 mMol) Produkt aus 53c, 0.059 g (0.24 mMol) Produkt aus 111 b, 0.10 ml (0.72 mMol) Triethylamin und 0.078 g (0.24 mMol) TBTU in 2 ml THF hergestellt.
C₃₀H₄₅N₃O₅S x C₂HF₃O₂ (673.78)
[M+H]+ = 560
HPLC (Methode 5): Retentionszeit = 1.59 min

### Beispiel 112 (Referenzbeispiel)

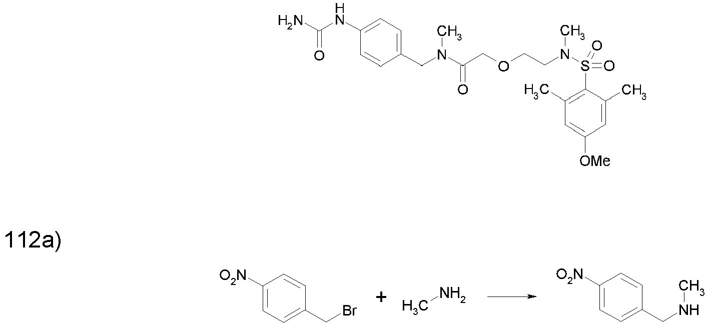

250.00 ml (500.00 mMol) Methylamin 2 M in Methanol (Fluka) werden unter Eisbadkühlung langsam mit 54.00 g (250.00 mMol) 4-Nitrobenzylbromid (Fluka) versetzt. Die Reaktionsmischung wird eine Stunde unter Eisbadkühlung und 30 Minuten bei Raumtemperatur gerührt und danach im Vakuum bis zur Trockene eingeengt. Der Rückstand wird mit Diethylether verrührt und abfiltriert. Das Filtrat wird im Vakuum bis zur Trockene eingeengt, mit Natriumcarbonat-Lösung versetzt und mit Diethylether extrahiert. Die organischen Extrakte werden über Natriumsulfat getrocknet und im Vakuum bis zur Trockene eingeengt. Das so erhaltene Rohprodukt wird durch Säulenchroamtographie (Elutionsmittel: Dichlormethan / Methanol 5:0 bis 5:1) gereinigt.
C₈H₁₀N₂O₂ (166.18)
[M+H]+ = 167

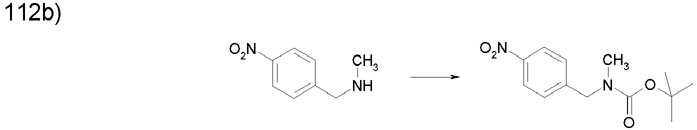

Eine Mischung aus 13.40 g (80.64 mMol) Produkt aus 112a und 25 ml Ethylacetat wird unter Eisbadkühlung langsam mit 17.68 g (81.00 mMol) Boc-Anhydrid versetzt. Die Reaktionsmischung wird drei Stunden bei Raumtemperatur gerührt, danach mit Wasser gewaschen, über Natriumsulfat getrocknet und im Vakuum bis zur Trockene eingeengt.
C₁₃H₁₈N₂O₄ (266.29)

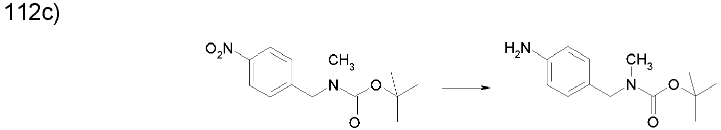

Eine Mischung aus 23.00 g (86.37 mMol) Produkt aus 112b, 2.30 g Raney-Nickel, 230 ml Ethanol und 230 ml Ethylacetat wird im Autoklaven bei Raumtemperatur hydriert. Der Katalysator wird abfiltriert, das Filtrat im Vakuum bis zur Trockene eingeengt. Das so erhaltene Rohprodukt wird durch Säulenchromatographie (Elutionsmittel: Petrolether / Ethylacetat 1:1) gereinigt.
C₁₃H₂₀N₂O₂ (236.31)
[M+H]+ = 237
DC: Kieselgel, Petrolether / Ethylacetat 1:1, Rf-Wert = 0.55

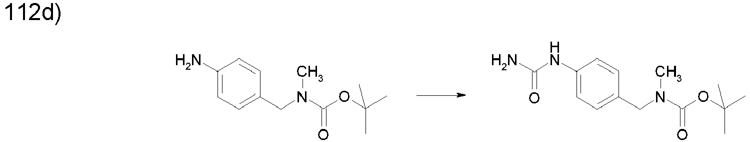

Eine Mischung aus 0.50 g (2.12 mMol) Produkt aus 112c, 0.48 g (3.56 mMol) Trimethylsilylisocyanat (Fluka) und 15 ml THF wird über das Wochenende unter Rückfluss gerührt. Anschließend wird die Reaktionsmischung im Vakuum bis zur Trockene eingeengt. C₁₄H₂₁N₃O₃ (279.33)
[2M+H]+ = 559

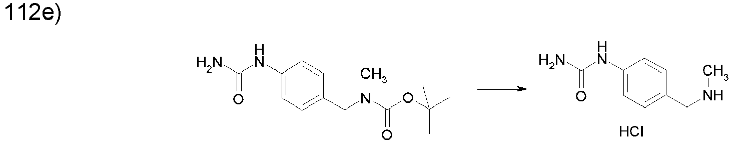

Eine Mischung aus 0.64 g (2.29 mMol) Produkt aus 112d und 10 ml methanolische HCl wird drei Stunden bei Raumtemperatur und zwei Stunden bei 50°C gerührt. Anschließend wird die Reaktionsmischung im Vakuum bis zur Trockene eingeengt. Der Rückstand wird im Vakuum über Nacht getrocknet.
C₉H₁₃N₃ x HCl (215.68)
[M+H]+ = 180

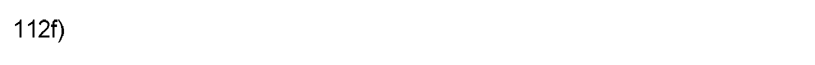

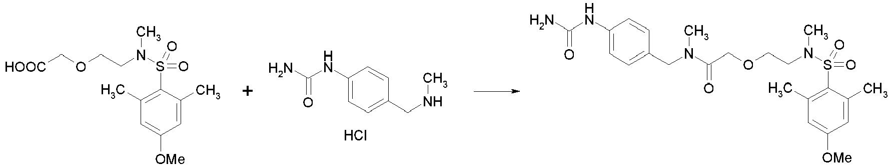

Beispiel 112 wird analog zu 1f aus 0.20 g (0.60 mMol) Produkt aus 53c, 0.13 g (0.60 mMol) Produkt aus 112e, 0.42 ml (3.02 mMol) Triethylamin und 0.22 g (0.66 mMol) TBTU in 4 ml DMF hergestellt.
C₂₃H₃₂N₄O₆S (492.59)
[M+H]+ = 493
HPLC (Methode 6): Retentionszeit = 2.93 min

### Beispiel 113 (Referenzbeispiel)

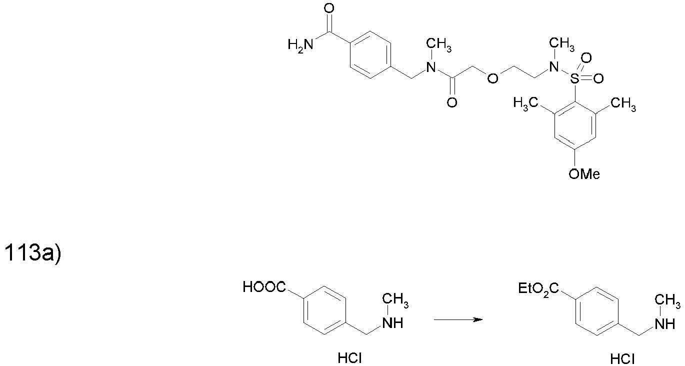

Eine Mischung aus 2.00 g (9.92 mMol) 4-Methylaminomethyl-benzoesäure Hydrochlorid (J. Med. Chem. 26, 1983, 309-312) und 25 ml ethanolischer HCl wird 1.5 Stunden unter Rückfluß gerührt. Danach wird die Reaktionsmischung im Vakuum bis zur Trockene eingeengt.
C₁₁H₁₅NO₂ x HCl (229.70)
[M+H]+ = 194
HPLC (Methode 6): Retentionszeit = 1.39 min

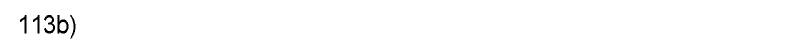

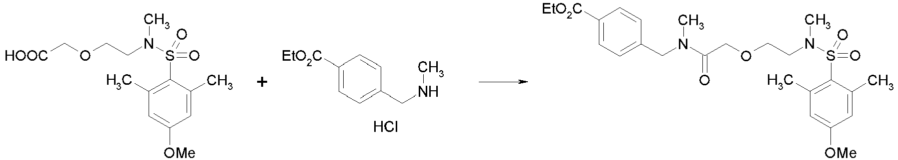

113b wird analog zu 1f aus 0.70 g (2.11 mMol) Produkt aus 53c, 0.49 g (2.11 mMol) Produkt aus 113a, 0.88 ml (6.34 mMol) Triethylamin und 0.78 g (2.32 mMol) TBTU in 12 ml DMF hergestellt.
C₂₅H₃₄N₂O₇S (506.61)
[M+H]+ = 507
HPLC (Methode 6): Retentionszeit = 3.95 min

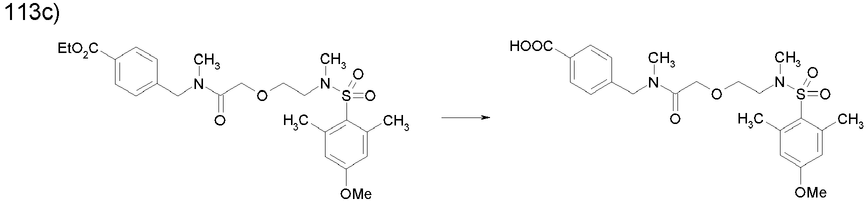

Eine Mischung aus 1.06 g (2.09 mMol) Produkt aus 133b, 7.00 ml (7.00 mMol) 1 M Natronlauge, 15 ml THF und 1.5 ml Ethanol wird vier Stunden bei 50°C gerührt. Die Reaktionsmischung wird danach mit 7 ml 1 M HCl versetzt und im Vakuum bis zur Trockene eingeengt. Der Rückstand wird in Aceton aufgenommen, über Magnesiumsulfat getrocknet und im Vakuum bis zur Trockene eingeengt.
C₂₃H₃₀N₂O₇S (478.56)
[M+H]+ = 479
HPLC (Methode 6): Retentionszeit = 3.21 min

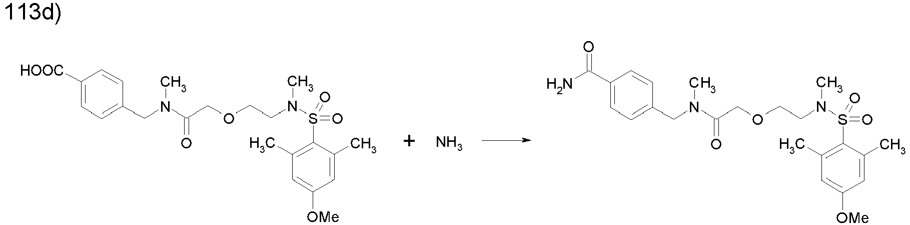

Beispiel 113 wird analog zu 1f aus 0.50 g (1.05 mMol) Produkt aus 113c, 4.00 ml (2.00 mMol) Ammoniak 0.5 M in Dioxan, 0.44 ml (3.14 mMol) Triethylamin und 0.38 g (1.15 mMol) TBTU in 4 ml DMF hergestellt.
C₂₃H₃₁N₃O₆S (477.57)
[M+H]+ = 478
HPLC (Methode 6): Retentionszeit = 2.92 min

### Beispiel 114 (Referenzbeispiel)

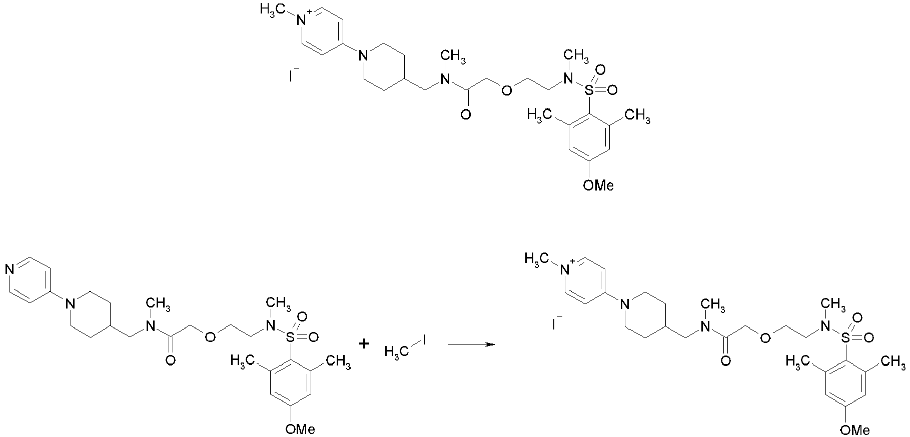

Eine Mischung aus 0.075 g (0.15 mMol) 61, 0.1 g (0.71 mMol) Methyliodid und 5 ml Dichlormethan wird über Nacht bei Raumtemperatur gerührt. Die Reaktionsmischung wird danach im Vakuum bis zur Trockene eingeengt.
C₂₇H₄₁N₄O₅S x I (660.61)
[M+H]+ = 533
HPLC (Methode 5): Retentionszeit = 1.55 min

### Beispiel 115 (Referenzbeispiel)

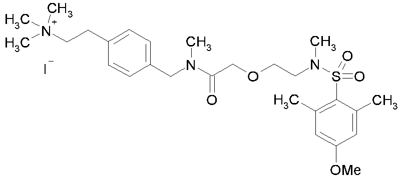

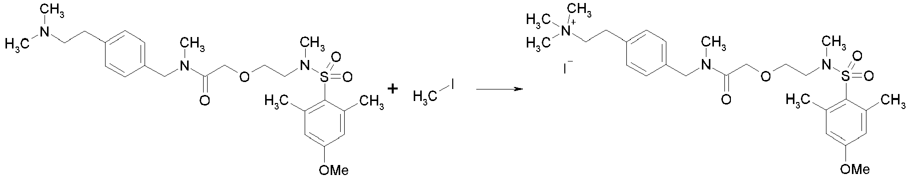

Beispiel 115 wird analog zu 114 aus 0.03 g (0.059 mMol) 64 und 0.05 g (0.35 mMol) Methyliodid in 5 ml Dichlormethan hergestellt.
C₂₇H₄₂N₃O₅S x I (647.61)
[M+H]+ = 520
HPLC (Methode 5): Retentionszeit = 1.55 min

### Beispiel 116 (Referenzbeispiel)

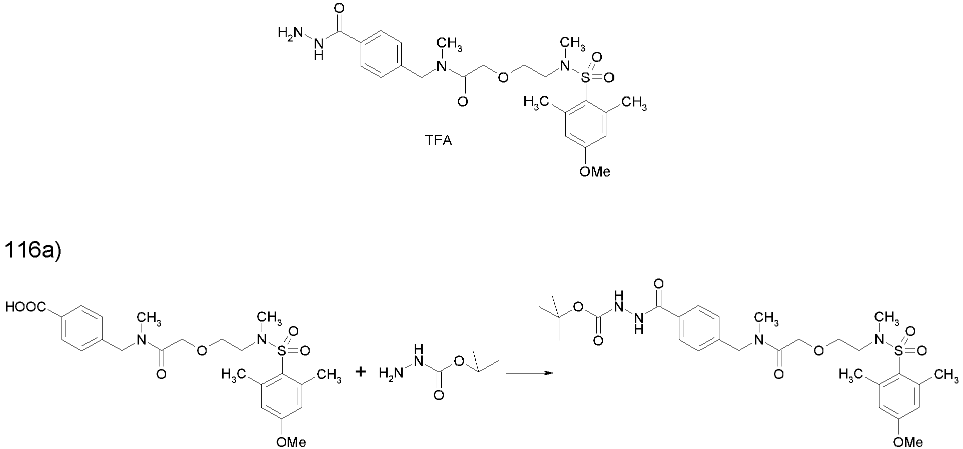

116a wird analog zu 1f aus 0.50 g (1.05 mMol) Produkt aus 113c, 0.14 g (1.05 mMol) Hydrazincarbonsäure-tert-butylester (Aldrich), 0.58 ml (4.18 mMol) Triethylamin und 0.38 g (1.15 mMol) TBTU in 6 ml DMF hergestellt.
C₂₈H₄₀N₄O₈S (592.71)
[M+H]+ = 593
HPLC (Methode 6): Retentionszeit = 3.46 min

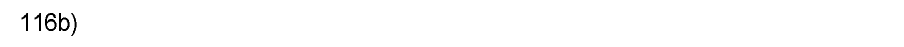

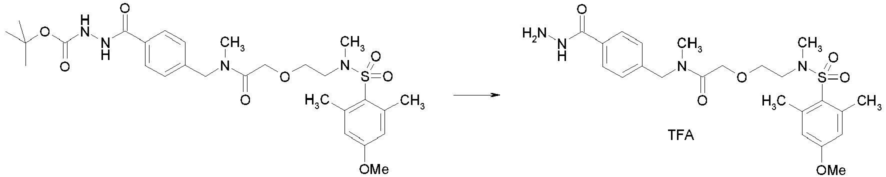

Beispiel 116 wird analog zu 112e aus 0.68 g (1.15 mMol) Produkt aus 116a und 10 ml methanolischer HCl hergestellt.
C₂₃H₃₂N₄O₆S x C₂HF₃O₂ (606.61)
[M+H]+ = 493 HPLC (Methode 6): Retentionszeit = 3.46 min

### Beispiel 117 (Referenzbeispiel)

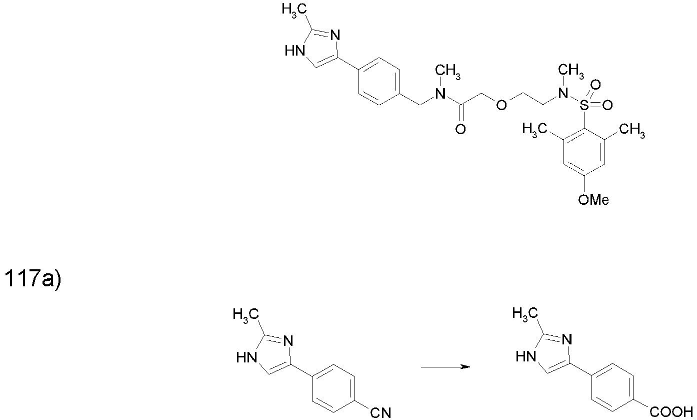

Eine Mischung aus 3.20 g (17.47 mMol) Produkt aus 63a, 50 ml 20%iger Natronlauge und 50 ml Ethanol wird über Nacht unter Rückfluss gerührt. Danach wird das Ethanol im Vakuum entfernt und der wässrige Rückstand mit konzentrierter HCl neutralisiert. Der entstandene Niederschlag wird abfiltriert und getrocknet.
C₁₁H₁₀N₂O₂ (202.21)
[M+H]+ = 203

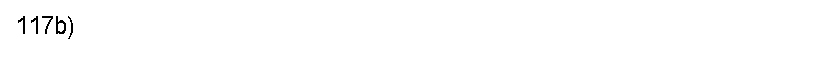

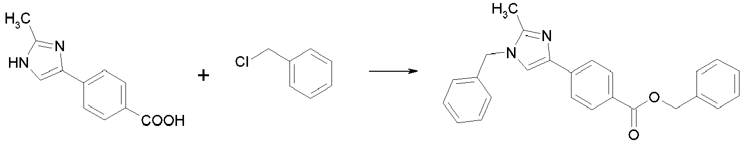

Eine Mischung aus 2.70 g (13.35 mMol) Produkt aus 117a, 8.00 g (57.89 mMol) Kaliumcarbonat und 100 ml DMF wird eine Stunde bei 60°C gerührt. Nach dem Abkühlen wird die Reaktionsmischung mit 3.50 g (27.65 mMol) Benzylchlorid (Aldrich) bei Raumtemperatur versetzt und anschließend über das Wochenende bei 60°C gerührt. Die Reaktionsmischung wird auf Wasser gegossen und eine Stunde bei Raumtemperatur gerührt. Der entstandene Niederschlag wird abfiltriert und getrocknet.
C₂₅H₂₂N₂O₂ (382.45)
[M+H]+ = 383
DC: Kieselgel, Dichlormethan / Methanol 9:1, Rf-Wert = 0.64

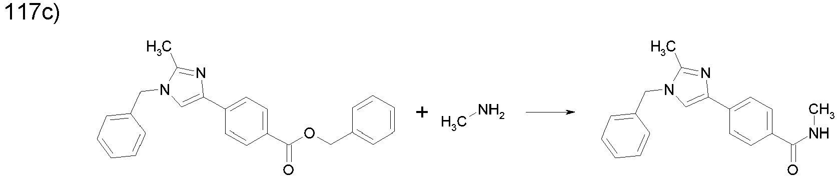

Eine Mischung aus 1.80 g (4.71 mMol) Produkt aus 117b und 100 ml Methylamin 33% in Ethanol (Aldrich) wird sechs Stunden bei 180°C und über Nacht bei 160°C im Autoklaven gerührt. Die Reaktionsmischung wird danach im Vakuum bis zur Trockene eingeengt. Das so erhaltene Rohprodukt wird durch Säulen-chromatographie (Elutionsmittel: Dichlormethan / Methanol 19:1) gereinigt.
C₁₉H₁₉N₃O (305.37)
[M+H]+ = 306

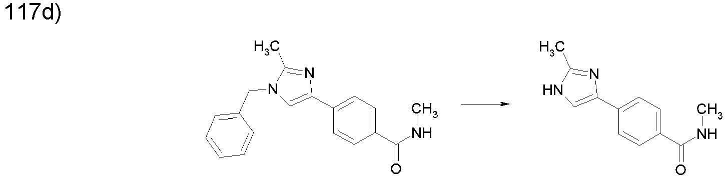

Eine Mischung aus 1.20 g (3.93 mMol) Produkt aus 117c, 0.20 g Palladium auf Kohle (20%) und 50 ml Methanol wird bei 50°C im Autoklaven hydriert. Der Katalysator wird abfiltriert, das Filtrat im Vakuum bis zur Trockene eingeengt.
C₁₂H₁₃N₃O (215.25)
[M+H]+ = 216

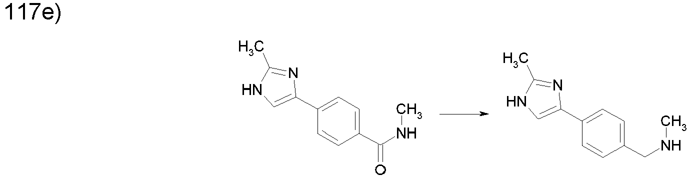

117e wird analog zu 38f aus 0.70 g (3.25 mMol) Produkt aus 117d und 10.00 ml (10.00 mMol) Lithiumaluminiumhydrid 1 M in THF (Aldrich) in 200 ml THF hergestellt. C₁₂H₁₅N₃ (201.27)
[M+H]+ = 202

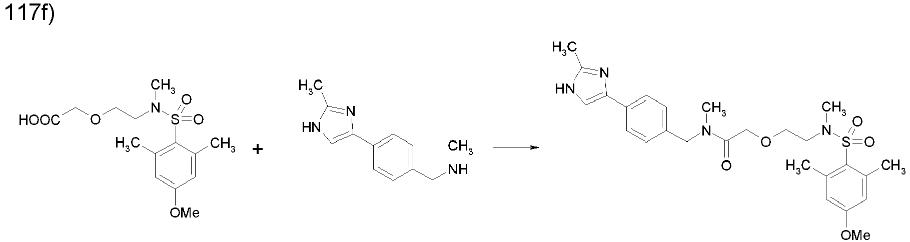

Beispiel 117 wird analog zu 1f aus 0.17 g (0.51 mMol) Produkt aus 53c, 0.10 g (0.50 mMol) Produkt aus 117e, 0.17 ml (1.19 mMol) Triethylamin und 0.17 g (0.53 mMol) TBTU in 30 ml THF und 5 ml DMF hergestellt.
C₂₆H₃₄N₄O₅S (514.64)
[M+H]+ = 515

DC: Kieselgel, Dichlormethan / Methanol 9:1, Rf-Wert = 0.28

### Beispiel 118 (Referenzbeispiel)

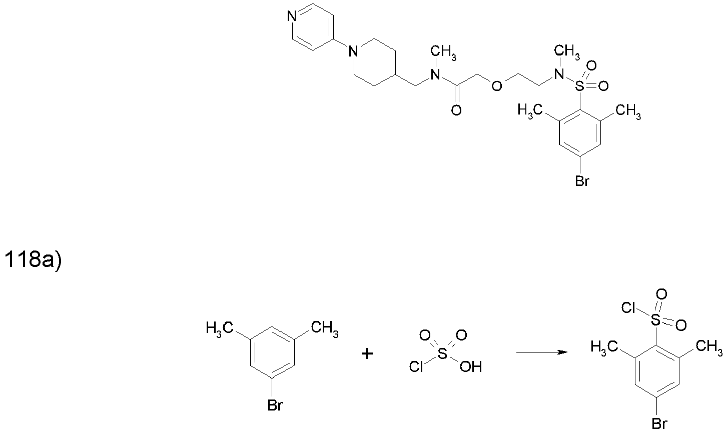

118a wird analog zu 13a aus 0.75 g (4.05 mMol) 5-Brom-m-xylol (Aldrich), 0.55 ml (8.30 mMol) Chlorsulfonsäure (Aldrich) und 10 ml Dichlormethan hergestellt.
C₈H₈BrClO₂S (283.57)
HPLC (Methode 6): Retentionszeit = 4.76 min

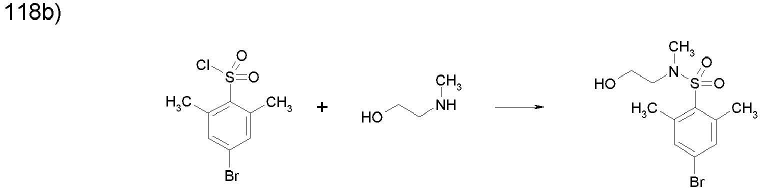

118b wird analog zu 3a aus 0.65 g (2.29 mMol) Produkt aus 118a und 0.28 ml (3.44 mMol) N-Methylaminoethanol (BASF) in 5 ml THF hergestellt.
C₁₁H₁₆BrNO₃S (322.22)
HPLC (Methode 6): Retentionszeit = 3.38 min

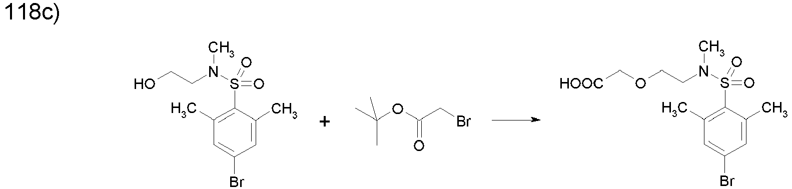

118c wird zuerst analog zu 53b aus 0.74 g (2.29 mMol) Produkt aus 118b, 0.75 g (5.06 mMol) 2-Brompropionsäure-tert-butylester (Fluka), 0.42 g (1.14 mMol) Tetrabutylammoniumiodid (Aldrich) und 8.67 g (75.90 mMol) 35%iger Natronlauge in 40 ml Toluol hergestellt. Der tert-Butylester wird anschließend zusammen mit 2 ml HCl 4 M in Dioxan (Aldrich) in 4 ml Dioxan über Nacht bei Raumtemperatur gerührt. Das Produkt wird danach durch Einengen der Reaktionsmischung im Vakuum erhalten.
C₁₃H₁₈BrNO₅S (380.26)
HPLC (Methode 6): Retentionszeit = 3.48 min

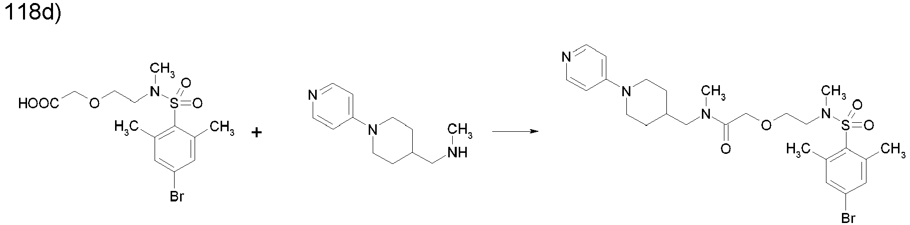

Beispiel 118 wird analog zu 1f aus 0.10 g (0.26 mMol) Produkt aus 118c, 0.054 g (0.26 mMol) Produkt aus 61 b, 0.11 ml (0.79 mMol) Triethylamin und 0.084 g (0.26 mMol) TBTU in 10 ml THF und 3 ml DMF hergestellt.
C₂₅H₃₅BrN₄O₄S (567.54)
[M+H]+ = 568/569/571
HPLC (Methode 6): Retentionszeit = 2.77 min

### Beispiel 119 (Referenzbeispiel)

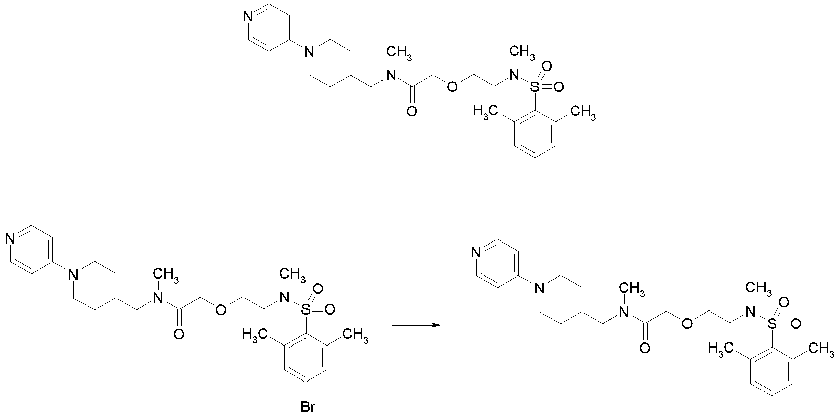

Eine Mischung aus 0.03 g (0.053 mMol) 118 und 0.03 g Palladium auf Kohle in 5 ml Methanol wird im Autoklaven bei Raumtemperatur hydriert. Der Katalysator wird abfiltriert, das Filtrat im Vakuum bis zur Trockene eingeengt. Das so erhaltene Rohprodukt wird durch präparative HPLC gereinigt.
C₂₅H₃₆N₄O₄S (488.64)
[M+H]+ = 489
HPLC (Methode 6): Retentionszeit = 2.45 min

### Beispiel 120 (Referenzbeispiel)

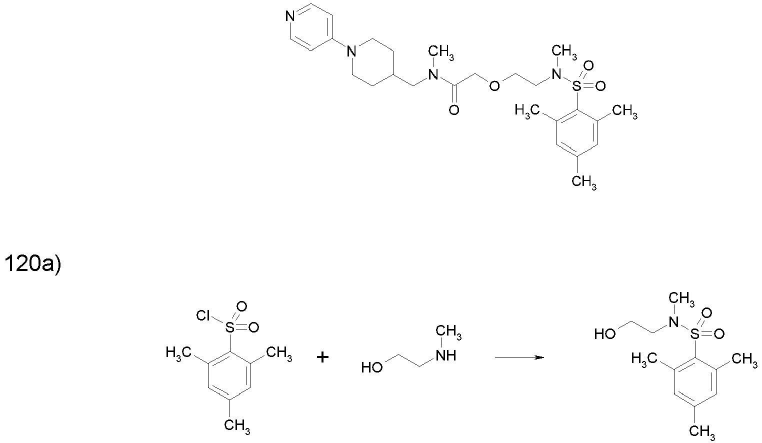

120a wird analog zu 3a aus 0.50 g (2.29 mMol) 2,4,6-Trimethylbenzol-sulfonsäurechlorid (Fluka) und 0.19 g (2.52 mMol) N-Methylaminoethanol (BASF) in 5 ml THF hergestellt.
C₁₂H₁₉NO₃S (257.35)
[M+H]+ = 258

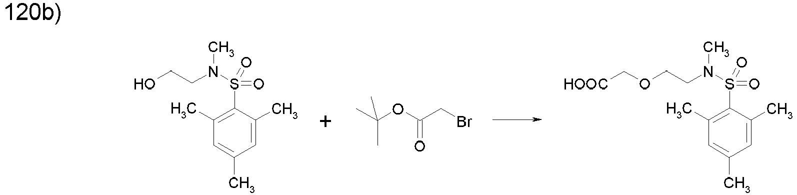

120b wird analog zu 118c aus 0.56 g (2.18 mMol) Produkt aus 120a, 0.48 ml (3.26 mMol) 2-Brompropionsäure-tert-butylester (Fluka), 0.18 g (0.65 mMol) Tetrabutylammoniumchlorid (Fluka) und 7.46 g (65.28 mMol) 35%iger Natronlauge in 20 ml Toluol und anschließendes Rühren in 2 ml HCl 4 M in Dioxan hergestellt.
C₁₄H₂₁NO₅S (315.39)

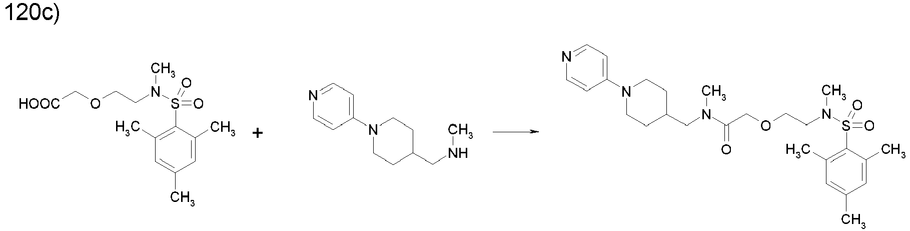

Beispiel 120 wird analog zu 1f aus 0.10 g (0.32 mMol) Produkt aus 120b, 0.065 g (0.32 mMol) Produkt aus 61b, 0.13 ml (0.95 mMol) Triethylamin und 0.20 g (0.63 mMol) TBTU in 10 ml THF hergestellt.
C₂₆H₃₈N₄O₄S (502.67)
[M+H]+ = 503
HPLC (Methode 5): Retentionszeit = 1.57 min

### Beispiel 121 (Referenzbeispiel)

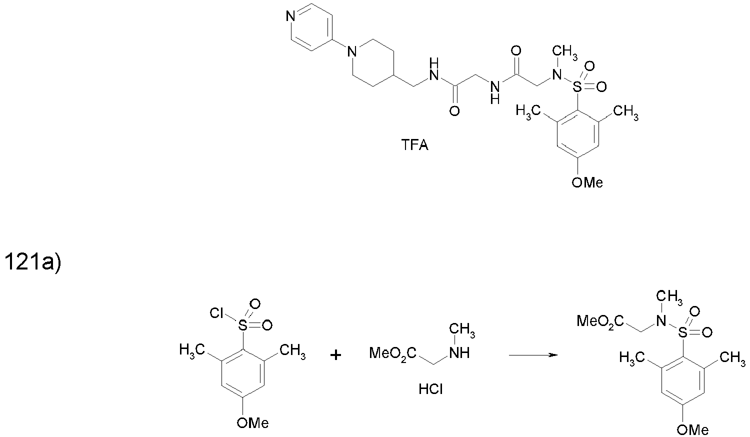

Eine Mischung aus 0.98 g (7.05 mMol) Sarcosin Methylester Hydrochlorid (Fluka), 1.65 g (7.05 mMol) Produkt aus 13a und 50 ml Pyridin wird eine Stunde bei Raumtemperatur gerührt. Die Reaktionsmischung wird danach im Vakuum bis zur Trockene eingeengt. Der Rückstand wird danach in 1 M HCl aufgenommen und mit Ethylacetat extrahiert. Die organischen Extrakte werden über Natriumsulfat getrocknet und im Vakuum bis zur Trockene eingeengt.
C₁₃H₁₉NO₅S (301.36)
[M+H]+ = 302

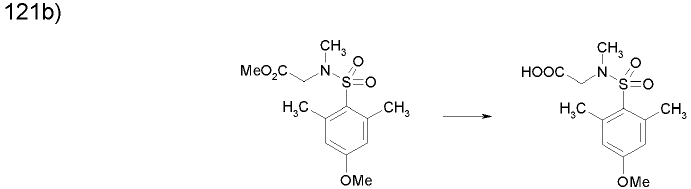

Eine Mischung aus 1.90 g (6.29 mMol) Produkt aus 121a, 6.45 ml (12.90 mMol) 2 M Natronlauge und 9 ml Methanol wird drei Tage bei Raumtemperatur gerührt. Das Methanol wird im Vakuum entfernt, der wässrige Rückstand wird auf 1 M HCl gegossen. Der entstandene Niederschlag wird abfiltriert und im Vakuumexsikkator über Nacht getrocknet.
C₁₂H₁₇NO₅S (287.33)
[M+H]+ = 288

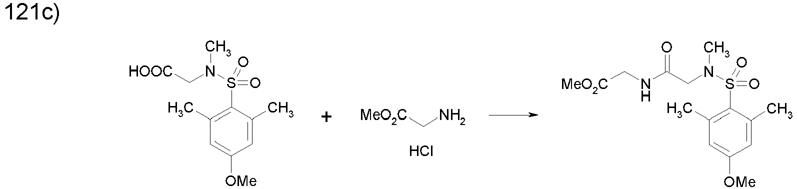

121c wird analog zu 1f aus 0.20 g (0.70 mMol) Produkt aus 121b, 0.087 g (0.70 mMol) Glycin Methylester Hydrochlorid (Aldrich), 0.29 ml (2.09 mMol) Triethylamin und 0.22 g (0.70 mMol) TBTU in 5 ml THF hergestellt.
C₁₅H₂₂N₂O₆S (358.41)
[M+H]+ = 359
HPLC (Methode 5): Retentionszeit = 1.72 min

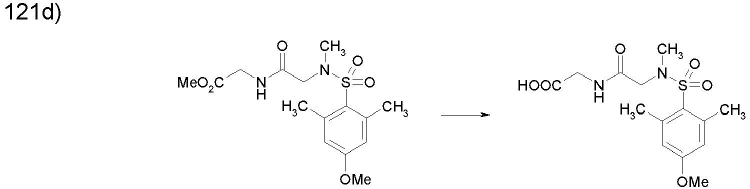

121d wird analog zu 121 b aus 0.23 g (0.63 mMol) Produkt aus 121 c und 0.64ml (1.29 mMol) 2 M Natronlauge in 1 ml Methanol hergestellt.
C₁₄H₂₀N₂O₆S (344.38)
[M+H]+ = 345

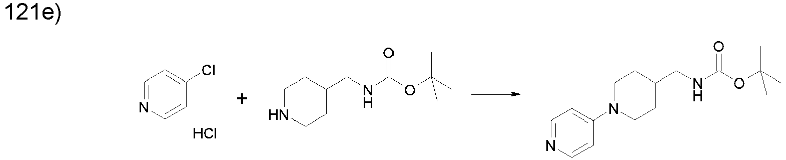

121 e wird analog zu 28c aus 3.00 g (14.00 mMol) Piperidin-4-ylmethyl-carbamatsäure-tert-butylester (EMKA), 2.10 g (14.00 mMol) 4-Chlorpyridin Hydrochlorid (Aldrich) und 7.80 ml (56.32 mMol) Triethlamin in 15 ml Isopropanol hergestellt.
C₁₆H₂₅N₃O₂ (291.39)
[M+H]+ = 292
HPLC (Methode 5): Retentionszeit = 1.40 min

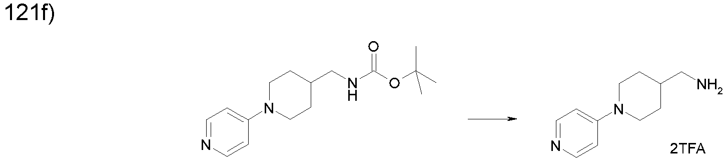

121f wird analog zu 18b aus 1.44 g (4.95 mMol) Produkt aus 121e und 4.95 ml TFA in 8 ml Dichlormethan hergestellt.
C₁₁H₁₇N₃ x 2C₂HF₃O₂ (419.32)
[M+H]+ = 192
HPLC (Methode 5): Retentionszeit = 0.36 min

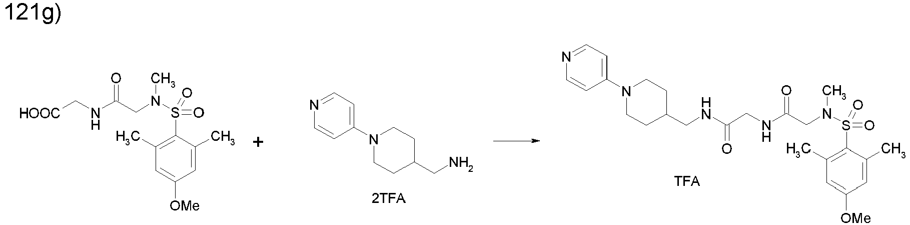

Beispiel 121 wird analog zu 1f aus 0.09 g (0.26 mMol) Produkt aus 121d, 0.11 g (0.26 mMol) Produkt aus 121f, 0.15 ml (1.05 mMol) Triethylamin und 0.084 g (0.26 mMol) TBTU in 1.9 ml THF hergestellt.
C₂₅H₃₅N₅O₅S x C₂HF₃O₂ (631.67)
[M+H]+ = 518
HPLC (Methode 5): Retentionszeit = 1.46 min

### Beispiel 122 (Referenzbeispiel)

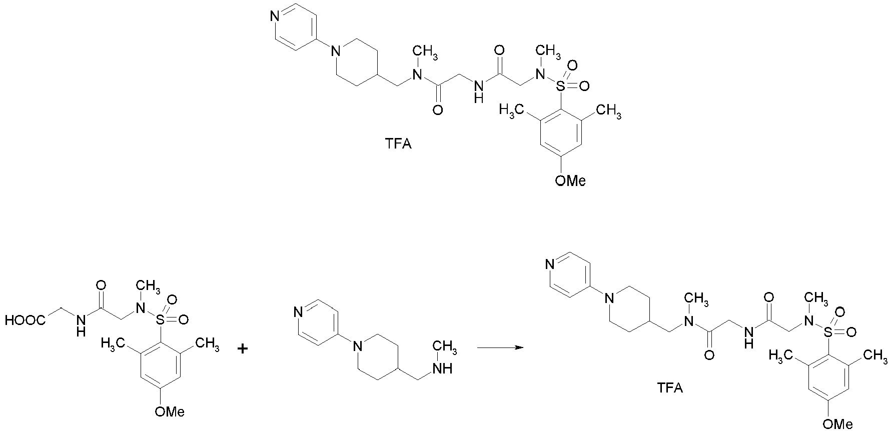

Beispiel 122 wird analog zu 1f aus 0.09 g (0.26 mMol) Produkt aus 121d, 0.054 g (0.26 mMol) Produkt aus 61 b, 0.11 ml (0.78 mMol) Triethylamin und 0.084 g (0.26 mMol) TBTU in 1.9 ml THF hergestellt.
C₂₆H₃₇N₅O₅S x C₂HF₃O₂ (645.69)
[M+H]+ = 532
HPLC (Methode 5): Retentionszeit = 1.50 min

### Beispiel 123 (Referenzbeispiel)

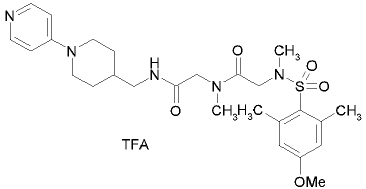

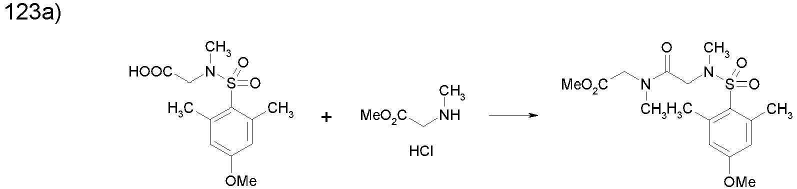

123a wird analog zu 1f aus 0.20 g (0.70 mMol) Produkt aus 121b, 0.097 g (0.70 mMol) Sarcosin Methylester Hydrochlorid (Fluka), 0.29 ml (2.09 mMol) Triethylamin und 0.22 g (0.70 mMol) TBTU in 5 ml THF hergestellt.
C₁₆H₂₄N₂O₆S (372.44)
[M+H]+ = 373
HPLC (Methode 5): Retentionszeit = 1.78 min

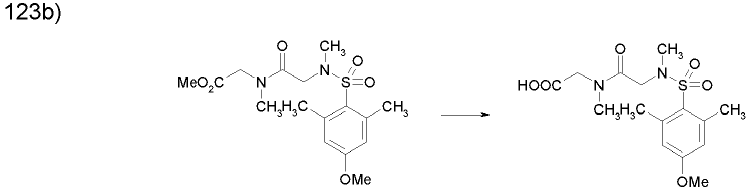

123b wird analog zu 121 b aus 0.23 g (0.60 mMol) Produkt aus 123a und 0.62 ml (1.24 mMol) 2 M Natronlauge in 1 ml Methanol hergestellt.
C₁₅H₂₂N₂O₆S (258.41)
[M+H]+ = 359

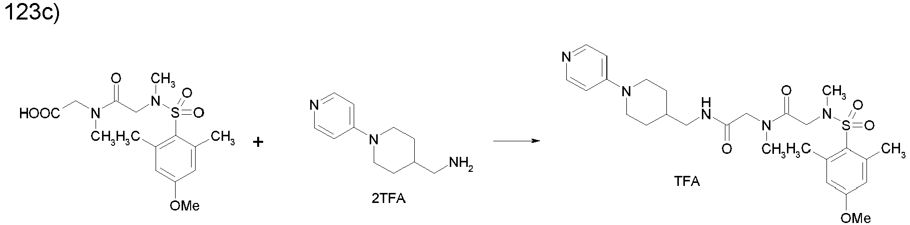

Beispiel 123 wird analog zu 1f aus 0.094 g (0.26 mMol) Produkt aus 123b, 0.11 g (0.26 mMol) Produkt aus 121f, 0.15 ml (1.05 mMol) Triethylamin und 0.084 g (0.26 mMol) TBTU in 1.9 ml THF hergestellt.
C₂₆H₃₇N₅O₅S x C₂HF₃O₂ (645.69)
[M+H]+ = 532
HPLC (Methode 5): Retentionszeit = 1.47 min

### Beispiel 124 (Referenzbeispiel)

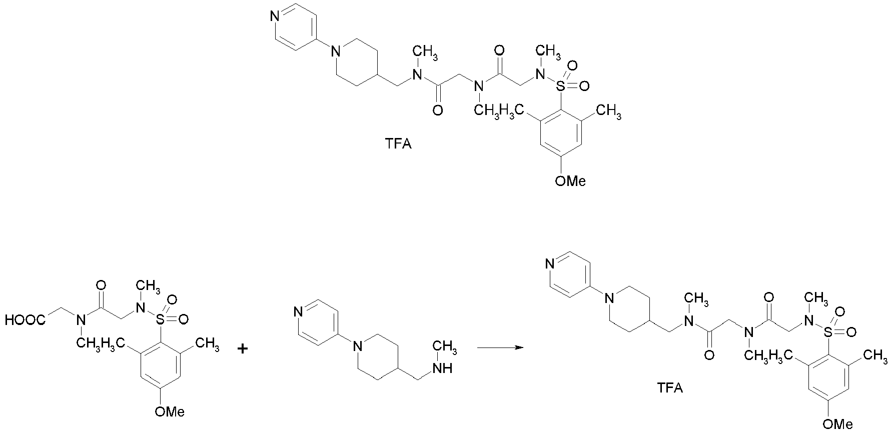

Beispiel 124 wird analog zu 1f aus 0.094 g (0.26 mMol) Produkt aus 123b, 0.054 g (0.26 mMol) Produkt aus 61 b, 0.11 ml (0.78 mMol) Triethylamin und 0.084 g (0.26 mMol) TBTU in 1.9 ml THF hergestellt.
C₂₇H₃₉N₅O₅S x C₂HF₃O₂ (659.72)
[M+H]+ = 548
HPLC (Methode 5): Retentionszeit = 1.49 min

### Beispiel 125 (Referenzbeispiel)

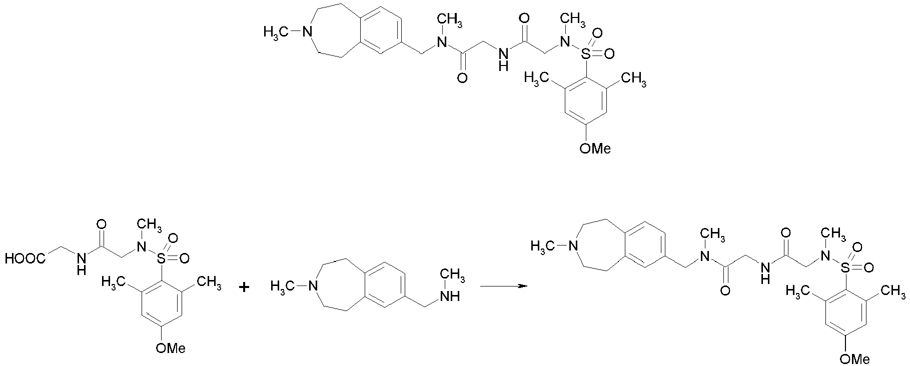

Beispiel 125 wird analog zu 1f aus 0.09 g (0.26 mMol) Produkt aus 121d, 0.076 g (0.26 mMol) Produkt aus 54b, 0.11 ml (0.78 mMol) Triethylamin und 0.084 g (0.26 mMol) TBTU in 1.9 ml THF hergestellt.
C₂₇H₃₈N₄O₅S (530.68)
[M+H]+ = 531
HPLC (Methode 1): Retentionszeit = 2.43 min

### Beispiel 126 (Referenzbeispiel)

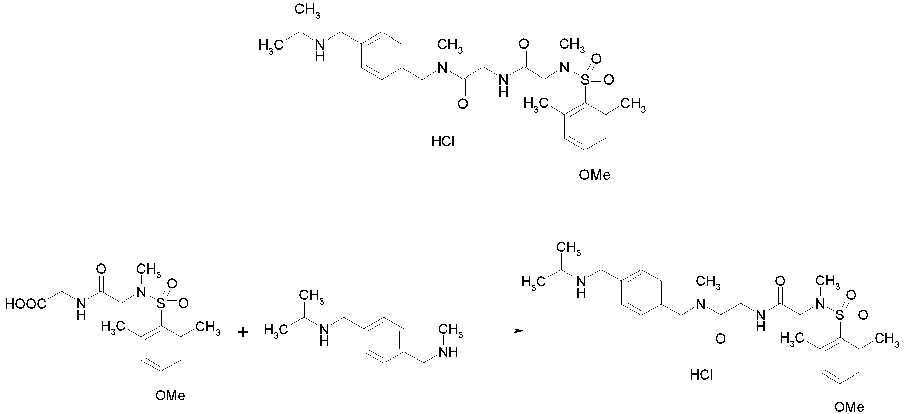

Beispiel 126 wird analog zu 1f aus 0.09 g (0.26 mMol) Produkt aus 121d, 0.05 g (0.26 mMol) Produkt aus 59b, 0.11 ml (0.78 mMol) Triethylamin und 0.084 g (0.26 mMol) TBTU in 1.9 ml THF hergestellt.
C₂₆H₃₈N₄O₅S x HCl (555.13)
[M+H]+ = 519
HPLC (Methode 1): Retentionszeit = 2.42 min

### Beispiel 127 (Referenzbeispiel)

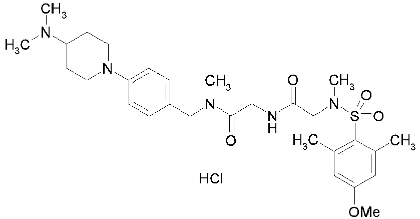

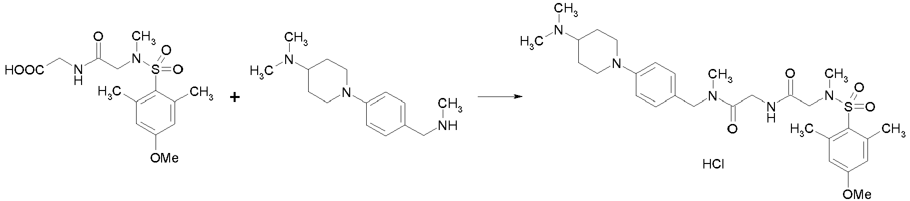

Beispiel 127 wird analog zu 1f aus 0.09 g (0.26 mMol) Produkt aus 121d, 0.065 g (0.26 mMol) Produkt aus 80a, 0.11 ml (0.78 mMol) Triethylamin und 0.084 g (0.26 mMol) TBTU in 1.9 ml THF hergestellt.
C₂₉H₄₃N₅O₅S x HCl (610.21)
[M+H]+ = 574
HPLC (Methode 1): Retentionszeit = 2.38 min

### Beispiel 128 (Referenzbeispiel)

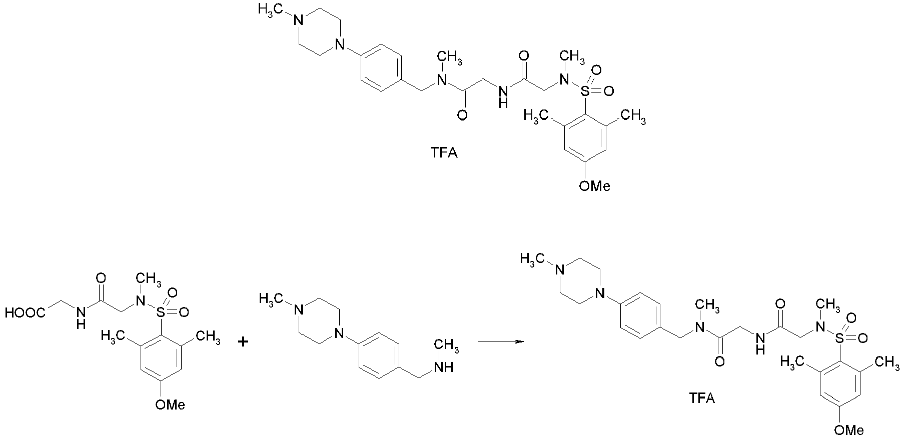

Beispiel 128 wird analog zu 1f aus 0.09 g (0.26 mMol) Produkt aus 121d, 0.057 g (0.26 mMol) Produkt aus 81 a, 0.11 ml (0.78 mMol) Triethylamin und 0.084 g (0.26 mMol) TBTU in 1.9 ml THF hergestellt.
C₂₇H₃₉N₅O₅S x C₂HF₃O₂ (610.21)
[M+H]+ = 546
HPLC (Methode 5): Retentionszeit = 1.51 min

### Beispiel 129 (Referenzbeispiel)

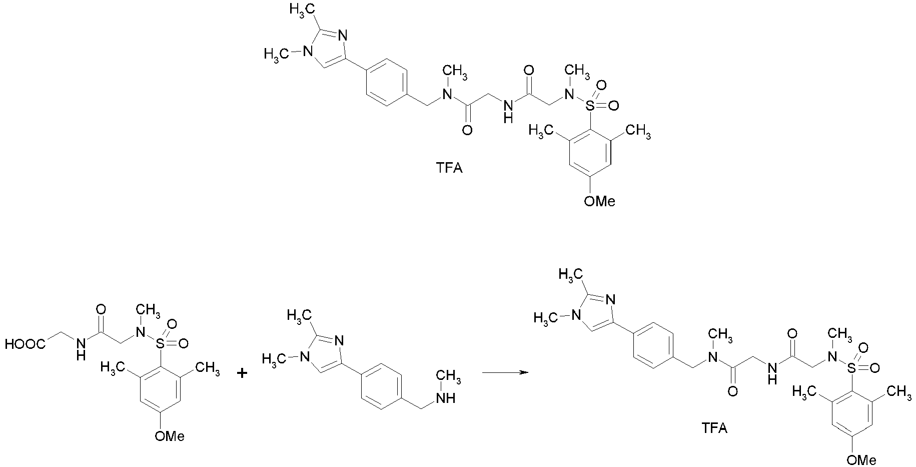

Beispiel 129 wird analog zu 1f aus 0.09 g (0.26 mMol) Produkt aus 121d, 0.056 g (0.26 mMol) Produkt aus 67c, 0.11 ml (0.78 mMol) Triethylamin und 0.084 g (0.26 mMol) TBTU in 1.9 ml THF hergestellt.
C₂₇H₃₅N₅O₅S x C₂HF₃O₂ (655.69)
[M+H]+ = 542
HPLC (Methode 5): Retentionszeit = 1.52 min

### Beispiel 130 (Referenzbeispiel)

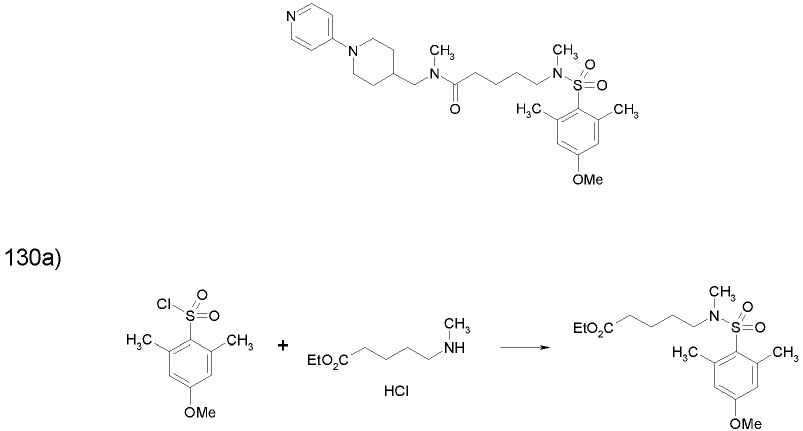

130a wird analog zu 10d aus 0.50 g (2.13 mMol) Produkt aus 13a, 0.42 g (2.13 mMol) 5-Methylaminovaleriansäureethylester (J. Am. Chem. Soc. 55, 1933, 1233-1241) und 1.18 ml (8.52 mMol) Triethylamin in 15 ml THF hergestellt.
C₁₇H₂₇NO₅S (357.47)
[M+H]+ = 358
HPLC (Methode 6): Retentionszeit = 4.10 min

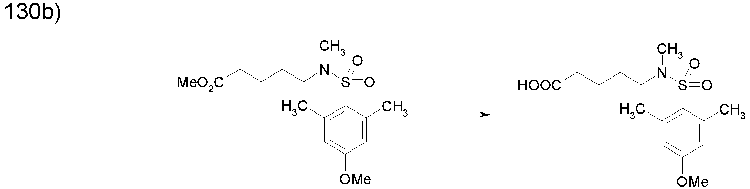

130b wird analog zu 121b aus 0.62 g (1.73 mMol) Produkt aus 130a und 7.00 ml (7.00 mMol) 1 M Natronlauge in 1.5 ml Methanol und 15 ml THF hergestellt.
C₁₅H₂₃NO₅S (329.41)
[M+H]+ = 330
HPLC (Methode 6): Retentionszeit = 3.24 min

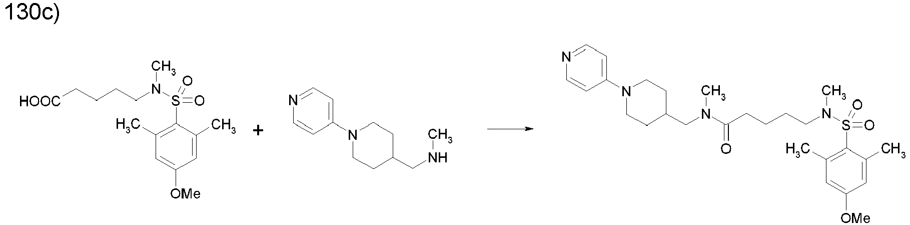

Beispiel 130 wird analog zu 1f aus 0.18 g (0.54 mMol) Produkt aus 130b, 0.10 g (0.49 mMol) Produkt aus 61 b, 0.14 ml (0.97 mMol) Triethylamin und 0.18 g (0.54 mMol) TBTU in 3 ml DMF hergestellt.
C₂₇H₄₀N₄O₄S (516.70)
[M+H]+ = 517
HPLC (Methode 6): Retentionszeit = 2.65 min

### Beispiel 131 (Referenzbeispiel)

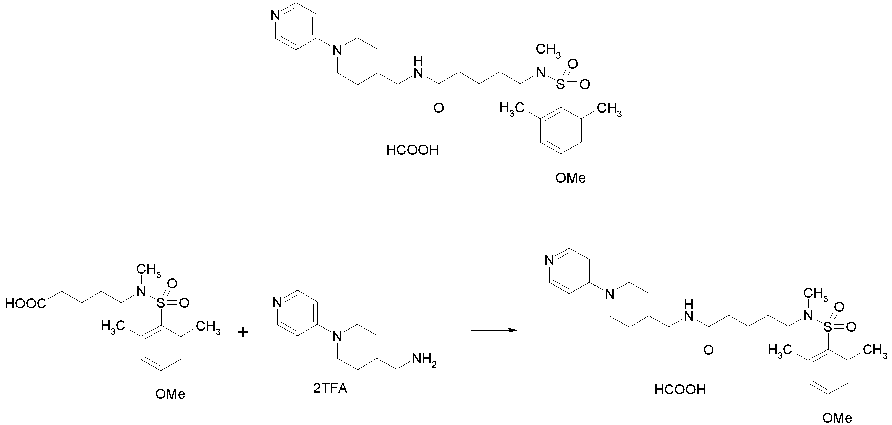

Beispiel 131 wird analog zu 1f aus 0.069 g (0.21 mMol) Produkt aus 130b, 0.088 g (0.21 mMol) Produkt aus 121f, 0.087 ml (0.63 mMol) Triethylamin und 0.077 g (0.23 mMol) TBTU in 1 ml DMF hergestellt.
C₂₆H₃₈N₄O₄S x CH₂O₂ (548.71)
[M+H]+ = 503
HPLC (Methode 6): Retentionszeit = 2.52 min

### Beispiel 132 (Referenzbeispiel)

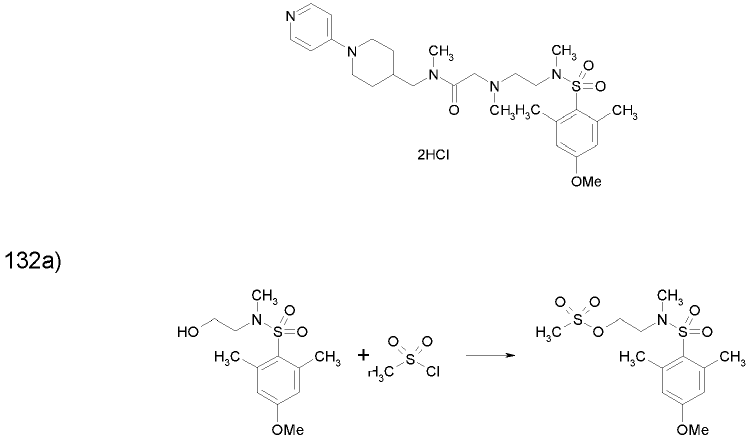

Eine Mischung aus 2.50 g (9.15 mMol) Produkt aus 53a, 1.39 ml (10.00 mMol) Triethylamin und 50 ml THF wird bei Raumtemperatur mit 0.77 ml (10.00 mMol) Methansulfonsäurechlorid (Aldrich) versetzt. Die Reaktionsmischung wird danach über Nacht bei Raumtemperatur gerührt. Der entstandene Niederschlag wird abfiltriert. Das Filtrat wird im Vakuum bis zur Trockene eingeengt. Der Rückstand wird in Ethylacetat aufgenommen und mit Wasser und gesättigter Natriumchlorid-Lösung gewaschen, über Natriumsulfat getrocknet und im Vakuum bis zur Trockene eingeengt.
C₁₃H₂₁NO₆S₂ (351.44)
[M+H]+ = 352
DC: Kieselgel, Dichlormethan / Methanol 9.5:0.5, Rf-Wert = 0.95

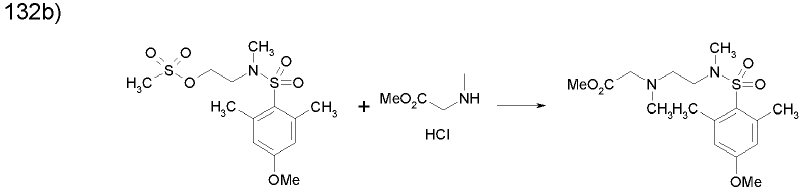

Eine Mischung aus 0.50 g (1.42 mMol) Produkt aus 132a, 0.20 g (1.42 mMol) Sarcosin Methylester Hydrochlorid (Aldrich), 0.52 ml (3.00 mMol) DIPEA und 5 ml DMF wird 24 Stunden bei 80°C gerührt. Die Reaktionsmischung wird im Vakuum eingeengt. Der Rückstand wird in Dichlormethan aufgenommen und mit Wasser und gesättigter Natriumhydrogencarbonat-Lösung gewaschen, über Natriumsulfat getrocknet und im Vakuum bis zur Trockene eingeengt. Das so erhaltene Rohprodukt wird durch Säulenchromatographie (Elutionsmittel: Dichlormethan / 0-3 % Methanol) gereinigt.
C₁₆H₂₆N₂O₅S (358.45)
[M+H]+ = 359

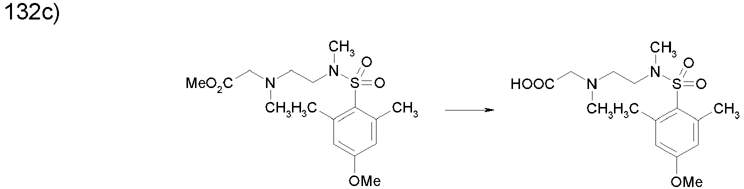

132c wird analog zu 1 c aus 0.29 g (0.81 mMol) Produkt aus 132b und 0.17 g (4.00 mMol) Lithiumhydroxid Monohydrat (Aldrich) in 5 ml THF und 4 ml Wasser hergestellt.
C₁₅H₂₄N₂O₅S (344.43)
HPLC (Methode 1): Retentionszeit = 2.32 min

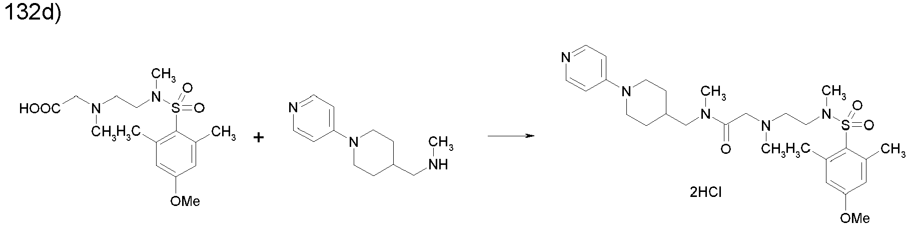

Beispiel 132 wird analog zu 1f aus 0.10 g (0.29 mMol) Produkt aus 132c, 0.06 g (0.29 mMol) Produkt aus 61 b, 0.084 ml (0.60 mMol) Triethylamin und 0.096 g (0.30 mMol) TBTU in 10 ml DMF hergestellt.
C₂₇H₄₁N₅O₄S x 2HCl (604.63)
[M+H]+ = 532
HPLC (Methode 5): Retentionszeit = 1.39 min

### Beispiel 133 (Referenzbeispiel)

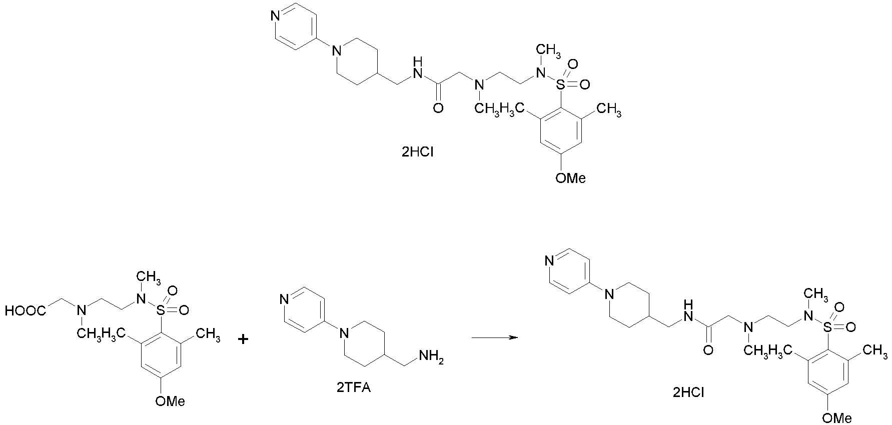

Beispiel 133 wird analog zu 1f aus 0.10 g (0.29 mMol) Produkt aus 132c, 0.12 g (0.29 mMol) Produkt aus 121f, 0.17 ml (1.20 mMol) Triethylamin und 0.096 g (0.30 mMol) TBTU in 60 ml DMF hergestellt.
C₂₆H₃₉N₅O₄S x 2HCl (590.61)
[M+H]+ = 518
HPLC (Methode 5): Retentionszeit = 1.37 min

### Beispiel 134 (Referenzbeispiel)

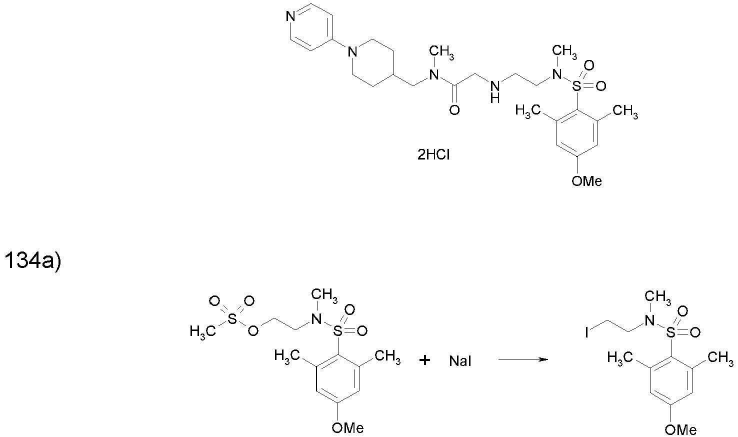

Eine Mischung aus 1.60 g (4.55 mMol) Produkt aus 132a, 2.10 g (14.00 mMol) Natriumiodid und 30 ml Aceton wird acht Stunden unter Rückfluß gerührt. Die Reaktionsmischung wird danach über Kieselgel filtriert. Das Filtrat wird im Vakuum bis zur Trockene eingeengt. Der Rückstand wird in Ethylacetat aufgenommen, mit Wasser gewaschen, über Natriumsulfat getrocknet und im Vakuum bis zur Trockene eingeengt.
C₁₂H₁₈INO₃S (383.25)
[M+H]+ = 384
HPLC (Methode 1): Retentionszeit = 3.75 min

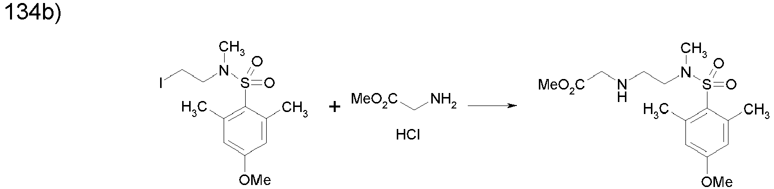

134b wird analog zu 132b aus 1.30 g (3.39 mMol) Produkt aus 134a, 1.28 g (10.20 mMol) Glycin Methylester Hydrochlorid (Aldrich) und 3.48 ml (20.00 mMol) DIPEA in 15 ml Acetonitril hergestellt.
C₁₅H₂₄N₂O₅S (344.43)
[M+H]+ = 345
DC: Kieselgel, Dichlormethan / Methanol 9.5:0.5, Rf-Wert = 0.38

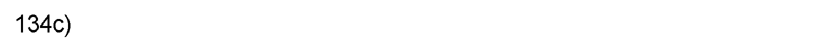

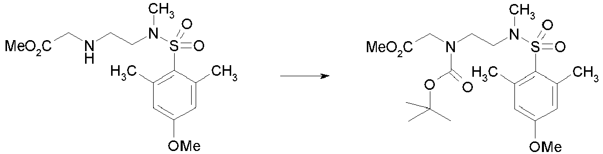

Eine Mischung aus 0.46 g (1.34 mMol) Produkt aus 134b, 0.33 g (1.50 mMol) Boc-Anhydrid, 0.21 ml (1.50 mMol) Triethylamin und 30 ml Dichlormethan wird über Nacht bei Raumtemperatur gerührt. Die Reaktionsmischung wird anschließend mit Dichlormethan verdünnt und mit gesättigter Natriumhydrogencarbonat-Lösung und gesättigter Natriumchlorid-Lösung gewaschen, über Natriumsulfat getrocknet und im Vakuum bis zur Trockene eingeengt.
C₂₀H₃₂N₂O₇S (444.54)
[M+H]+ = 445
DC: Kieselgel, Dichlormethan / Methanol 9.5:0.5, Rf-Wert = 0.45

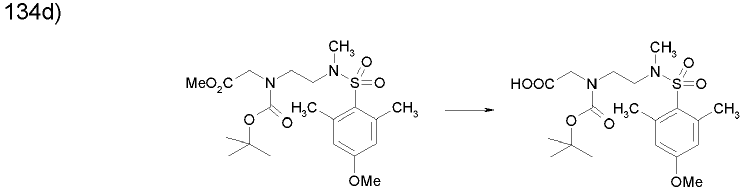

134d wird analog zu 1c aus 0.59 g (1.33 mMol) Produkt aus 134c und 0.28 g (6.60 mMol) Lithiumhydroxid Monohydrat (Aldrich) in 7 ml THF und 6.6 ml Wasser hergestellt.
C₁₉H₃₀N₂O₇S (430.52)
[M+H]+ = 431

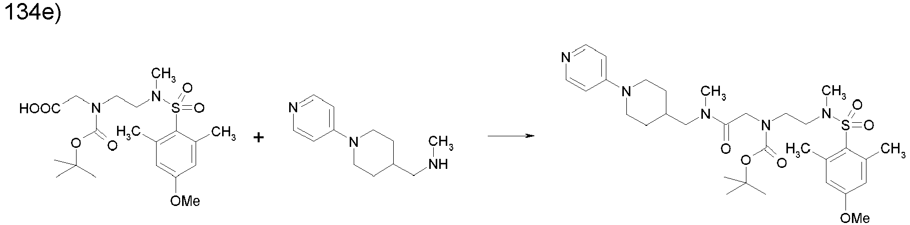

134e wird analog zu 1f aus 0.15 g (0.35 mMol) Produkt aus 134d, 0.072 g (0.35 mMol) Produkt aus 61 b, 0.098 ml (0.70 mMol) Triethylamin und 0.11 g (0.35 mMol) TBTU in 7 ml DMF hergestellt.
C₃₁H₄₇N₅O₆S (617.80)
[M+H]+ = 618
HPLC (Methode 1): Retentionszeit = 2.62 min

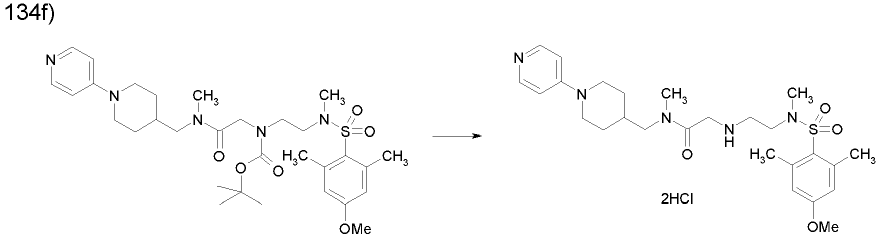

Beispiel 134 wird analog zu 18b aus 0.16 g (0.26 mMol) Produkt aus 134e und 3 ml TFA in 3 ml Dichlormethan hergestellt.
C₂₆H₃₉N₅O₄S x 2HCl (590.61)
[M+H]+ = 518
HPLC (Methode 5): Retentionszeit = 1.40 min

### Beispiel 135 (Referenzbeispiel)

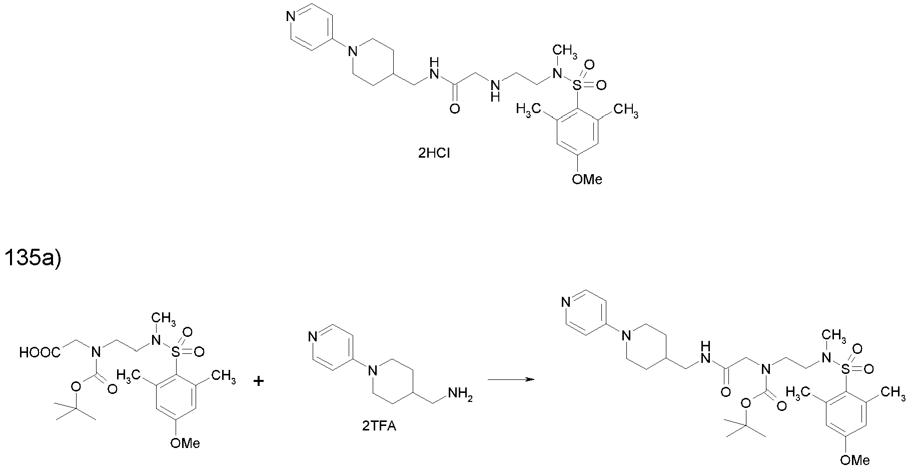

135a wird analog zu 1f aus 0.15 g (0.35 mMol) Produkt aus 134d, 0.15 g (0.35 mMol) Produkt aus 121f, 0.20 ml (1.40 mMol) Triethylamin und 0.11 g (0.35 mMol) TBTU in 7 ml DMF hergestellt.
C₃₀H₄₅N₅O₆S (603.77)
[M+H]+ = 604
DC: Kieselgel, Dichlormethan / Methanol / Ammoniak 9:1:0.1, Rf-Wert = 0.48

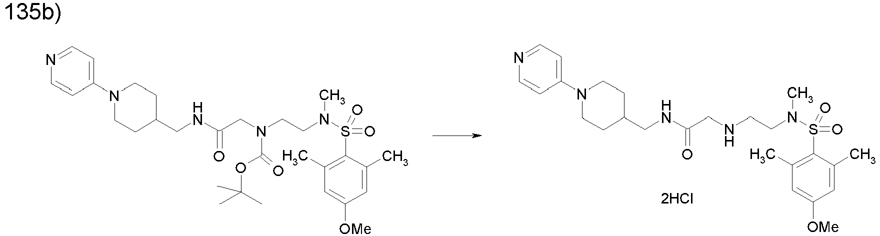

Beispiel 135 wird analog zu 18b aus 0.16 g (0.27 mMol) Produkt aus 135a und 5 ml TFA in 5 ml Dichlormethan hergestellt.
C₂₅H₃₇N₅O₄S x 2HCl (576.58)
[M+H]+ = 504
HPLC (Methode 1): Retentionszeit = 2.17 min

### Beispiel 136 (Referenzbeispiel)

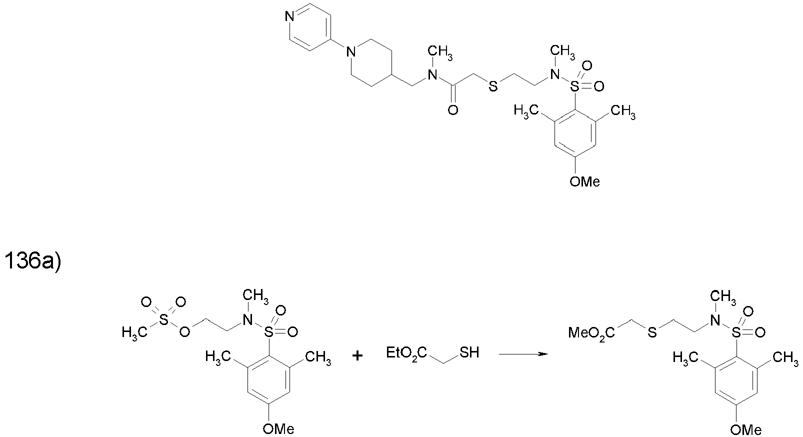

Eine Mischung aus 0.21 g (0.60 mMol) Produkt aus 132a, 0.065 ml (0.60 mMol) Mercaptoessigsäureethylester (Aldrich), 0.17 g (1.20 mMol) Kaliumcarbonat und 10 ml Acetonitril wird 24 Stunden bei Raumtemperatur gerührt. Der Niederschlag wird abfiltriert, das Filtrat wird im Vakuum eingeengt. Das so erhaltene Rohprodukt wird durch präparative HPLC gereinigt.
C₁₆H₂₅NO₅S₂ (375.51)
[M+H]+ = 376

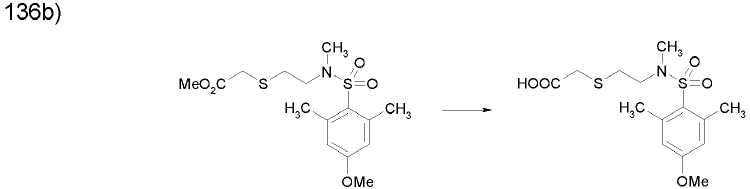

136b wird analog zu 121 b aus 0.070 g (0.19 mMol) Produkt aus 136a und 2.00 ml (2.00 mMol) 1 M Natronlauge in 5 ml THF hergestellt.
C₁₄H₂₁NO₅S₂ (347.45)
[M+H]+ = 348

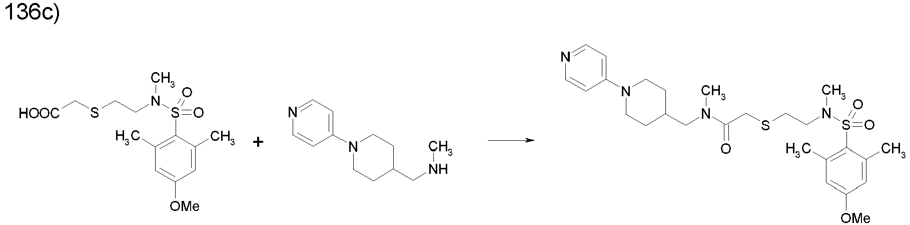

Beispiel 136 wird analog zu 1f aus 0.064 g (0.18 mMol) Produkt aus 136b, 0.038 g (0.18 mMol) Produkt aus 61b, 0.079 ml (0.46 mMol) DIPEA und 0.059 g (0.18 mMol) TBTU in 5 ml DMF hergestellt.
C₂₆H₃₈N₄O₄S₂ (534.74)
[M+H]+ = 535
HPLC (Methode 6): Retentionszeit = 2.68 min

### Beispiel 137 (Referenzbeispiel)

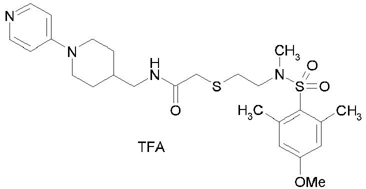

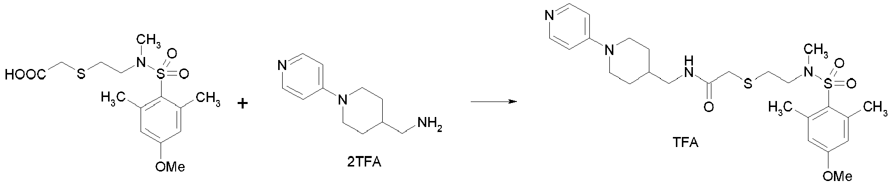

Beispiel 137 wird analog zu 1f aus 0.073 g (0.21 mMol) Produkt aus 136b, 0.088 g (0.21 mMol) Produkt aus 121f, 0.11 ml (0.63 mMol) DIPEA und 0.087 g (0.27 mMol) TBTU in 5 ml DMF hergestellt.
C₂₅H₃₆N₄O₄S₂ x C₂HF₃O₂ (634.73)
[M+H]+ = 521
HPLC (Methode 6): Retentionszeit = 2.57 min

### Beispiel 138 (Referenzbeispiel)

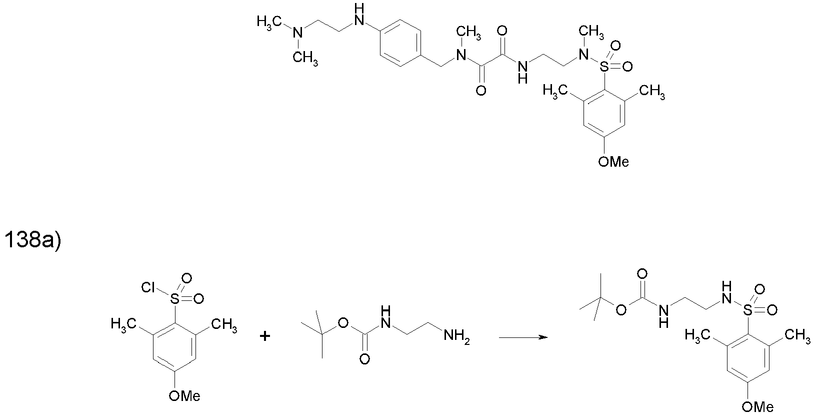

138a wird analog zu 3a aus 2.0 g (8.52 mMol) Produkt aus 13a, 1.37 g (8.55 mMol) N-Boc-Ethylendiamin (Fluka) und 1.0 g (9.89 mMol) Triethylamin in 50 ml THF hergestellt.
C₁₆H₂₆N₂O₅S (358.45)
[M-H]- = 357
HPLC (Methode 6): Retentionszeit = 3.63 min

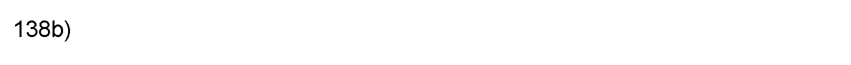

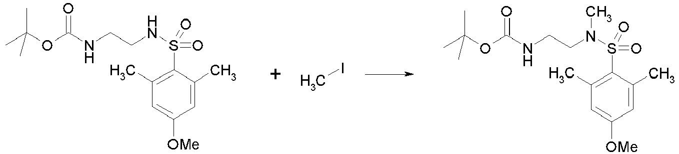

138b wird analog zu 3b aus 3.38 g (9.43 mMol) Produkt aus 138a, 0.53 ml (8.55 mMol) Methyliodid, 1.77 g (12.83 mMol) Kaliumcarbonat wasserfrei in 30 ml DMSO hergestellt.
C₁₇H₂₈N₂O₅S (372.48)
[M+H]+ = 373
HPLC (Methode 6): Retentionszeit = 3.89 min

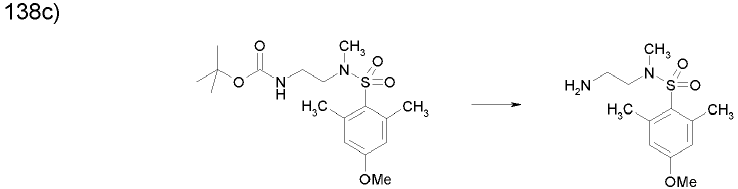

138b wird analog zu 28d aus 3.61 g (9.69 mMol) Produkt aus 138b und 10 ml TFA in 50 ml Dichlormethan hergestellt.
C₁₂₇H₂₀N₂O₃S (272.36)
[M+H]+ = 273
HPLC (Methode 6): Retentionszeit = 1.95 min

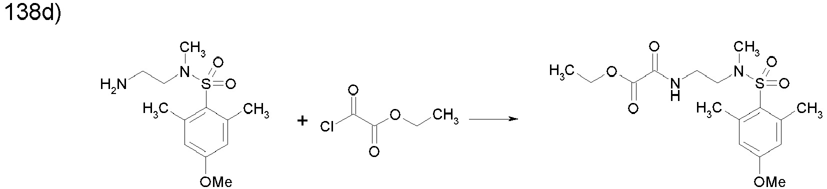

Eine Mischung aus 0.50 g (1.84 mMol) Produkt aus 138c, 0.56 ml (5.51 mMol) Triethylamin und 25 ml Dichlormethan wird unter Eisbadkühlung mit 0.38 ml (2.75 mMol) Oxalsäure-monoethylesterchlorid (Fluka) in 5 ml Dichlormethan versetzt und zwei Stunden bei Raumtemperatur gerührt. Die Reaktionsmischung wird anschließend mit Dichlormethan verdünnt, mit 10%iger wässriger Zitronensäure-Lösung, gesättigter Natriumsulfat-Lösung und mit Wasser gewaschen, über Natriumsulfat getrocknet und im Vakuum bis zur Trockene eingeengt.
C₁₆H₂₄N₂O₆S (372.44)
[M+H]+ = 373

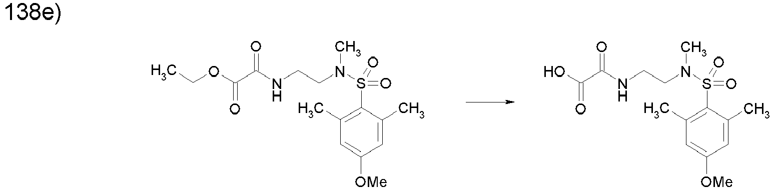

Eine Mischung aus 0.60 g (1.60 mMol) Produkt aus 138d, 5.6 ml 1 M Natronlauge und 6 ml Ethanol wird vier Stunden bei Raumtemperatur gerührt und danach im Vakuum bis zur Trockene eingeengt. Der Rückstand wird mit 8 ml 1 M Salzsäure versetzt und mit Ethylacetat extrahiert. Die vereinigten organischen Extrakte werden mit Wasser und gesättigter Natriumchlorid-Lösung gewaschen, über Natriumsulfat getrocknet und im Vakuum bis zur Trockene eingeengt.
C₁₄H₂₀N₂O₆S (344.38)
[M+H]+ = 345

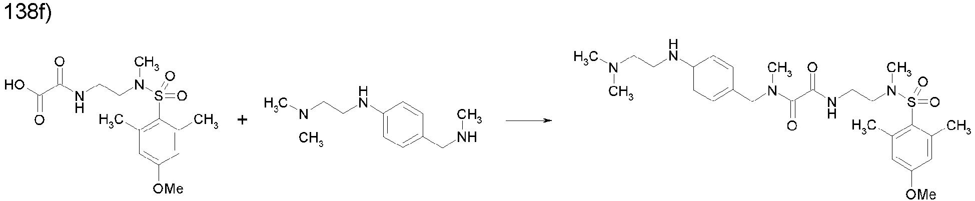

Beispiel 138 wird analog zu 1f aus 40.0 mg (0.12 mMol) Produkt aus 138e, 48.2 mg (0.12 mMol) N,N-Dimethyl-N'-(4-methylaminomethyl-phenyl)-ethan-1,2-diamin (analog J. Chem. Soc 1960, 3163-3165), 0.038 ml (0.29 mmol) DIPEA und 38.9 mg (0.12 mmol) TBTU in 1.5 ml DMF hergestellt.
C₂₆H₃₉N₅O₅S (533.68)
[M+H]+ = 534
HPLC (Methode 6): Retentionszeit = 1.61 min

### Beispiel 139 (Referenzbeispiel)

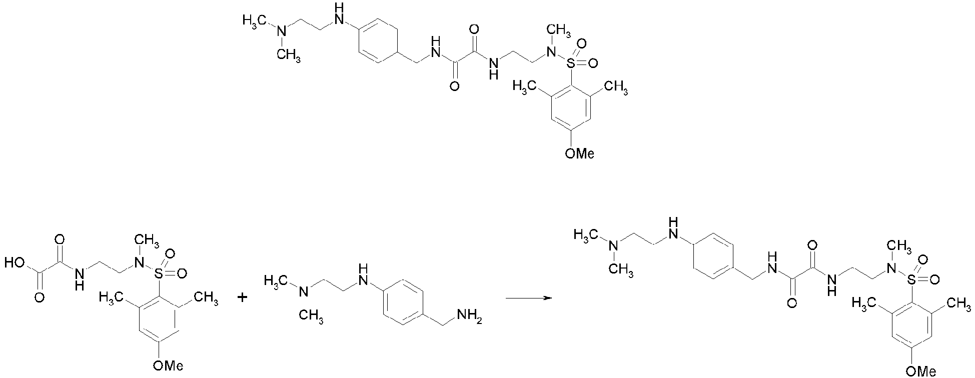

Beispiel 139 wird analog zu 1f aus 40.0 mg (0.12 mMol) Produkt aus 138e, 22.5 mg (0.12 mMol) N-(4-Aminomethyl-phenyl)-N',N'-dimethylethan-1,2-diamin (analog J. Chem. Soc 1960, 3163-3165), 0.038 ml (0.29 mmol) DIPEA und 38.9 mg (0.12 mmol) TBTU in 1.5 ml DMF hergestellt.
C₂₅H₃₇N₅O₅S (519.66)
[M+H]+ = 520
HPLC (Methode 6): Retentionszeit = 1.53 min

Die folgenden Verbindungen wurden analog zu Beispiel 22 hergestellt:

### Beispiel 140 (Referenzbeispiel)

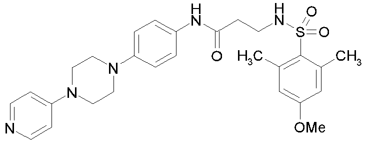

C₂₇H₃₃N₅O₄S x C₂HF₃O₂ (637.67)
[M+H]+ = 524
HPLC (Methode 6): Retentionszeit = 2.43 min

### Beispiel 141 (Referenzbeispiel)

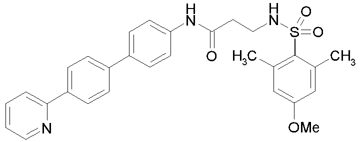

C₂₉H₂₉N₃O₄S x C₂HF₃O₂ (629.65)
[M+H]+ = 516
HPLC (Methode 6): Retentionszeit = 3.39 min

### Beispiel 142 (Referenzbeispiel)

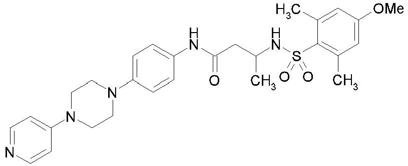

C₂₈H₃₅N₅O₄S (537.67)
[M+H]+ = 538
HPLC (Methode 6): Retentionszeit = 2.45 min

### Beispiel 143 (Referenzbeispiel)

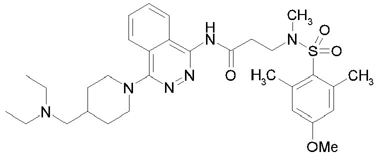

C₃₁H₄₄N₆O₄S x C₂HF₃O₂ (710.81)
[M+H]+ = 597
HPLC (Methode 6): Retentionszeit = 2.30 min

### Beispiel 144 (Referenzbeispiel)

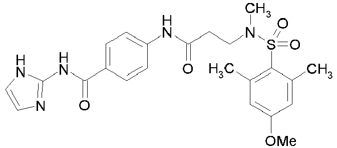

C₂₃H₂₇N₅O₅S x C₂HF₃O₂ (599.58)
[M+H]+ = 486
HPLC (Methode 6): Retentionszeit = 2.46 min

### Beispiel 145 (Referenzbeispiel)

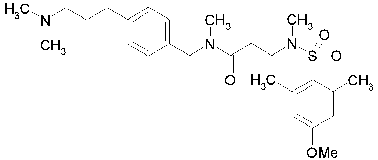

C₂₆H₃₉N₃O₄S (489.67)
[M+H]+ = 490
HPLC (Methode 6): Retentionszeit = 2.66 min

### Beispiel 146 (Referenzbeispiel)

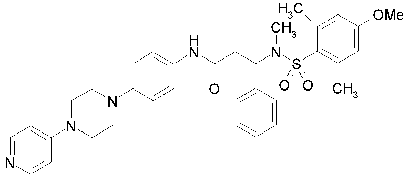

C₃₄H₃₉N₅O₄S (613.77)
[M+H]+ = 614
HPLC (Methode 6): Retentionszeit = 3.07 min

### Beispiel 147 (Referenzbeispiel)

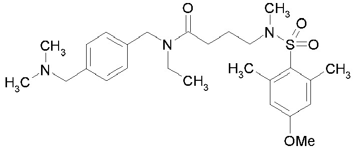

C₂₆H₃₉N₃O₄S x C₂HF₃O₂ (603.70)
[M+H]+ = 490
HPLC (Methode 6): Retentionszeit = 2.60 min

### Beispiel 148 (Referenzbeispiel)

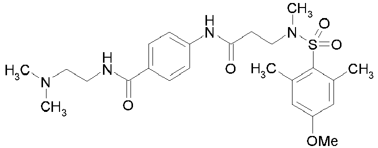

C₂₄H₃₄N₄O₅S x CH₂O₂ (536.64)
[M+H]+ = 491
HPLC (Methode 6): Retentionszeit = 2.29 min

### Beispiel 149 (Referenzbeispiel)

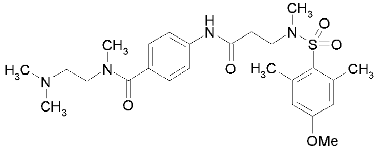

C₂₅H₃₆N₄O₅S x CH₂O₂ (550.67)
[M+H]+ = 505
HPLC (Methode 6): Retentionszeit = 2.32 min

### Beispiel 150 (Referenzbeispiel)

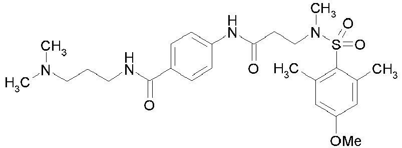

C₂₅H₃₆N₄O₅S (504.64)
[M+H]+ = 505
HPLC (Methode 6): Retentionszeit = 2.31 min

### Beispiel 151 (Referenzbeispiel)

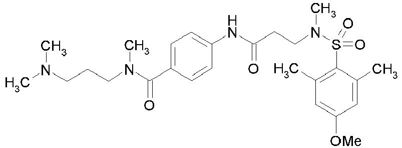

C₂₆H₃₈N₄O₅S x CH₂O₂ (564.70)
[M+H]+ = 519
HPLC (Methode 6): Retentionszeit = 2.34 min

### Beispiel 152 (Referenzbeispiel)

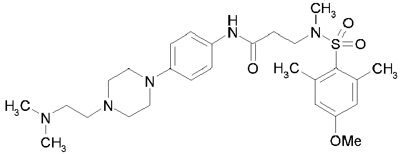

C₂₇H₄₁N₅O₄S x 2C₂HF₃O₂ (759.76)
[M+H]+ = 532
HPLC (Methode 5): Retentionszeit = 1.40 min

### Beispiel 153 (Referenzbeispiel)

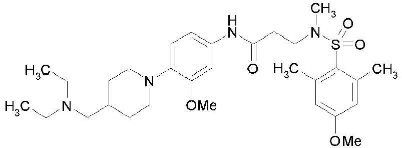

C₃₀H₄₆N₄O₅S x HCl (611.24)
[M+H]+ = 575
HPLC (Methode 1): Retentionszeit = 2.12 min

### Beispiel 154 (Referenzbeispiel)

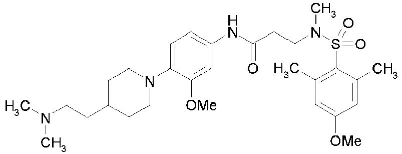

C₂₉H₄₄N₄O₅S x HCl (597.21)
[M+H]+ = 561
HPLC (Methode 8): Retentionszeit = 3.12 min

### Beispiel 155 (Referenzbeispiel)

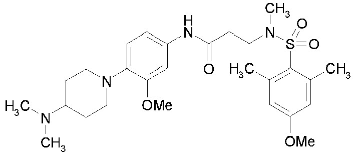

C₂₇H₄₀N₄O₅S x HCl (569.16)
[M+H]+ = 533
HPLC (Methode 11): Retentionszeit = 1.67 min

### Beispiel 156 (Referenzbeispiel)

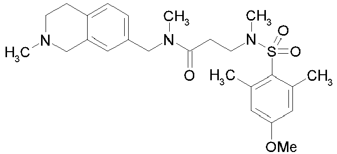

C₂₅H₃₅N₃O₄S x HCl (510.09)
[M+H]+ = 474
HPLC (Methode 7): Retentionszeit = 1.90 min

### Beispiel 157 (Referenzbeispiel)

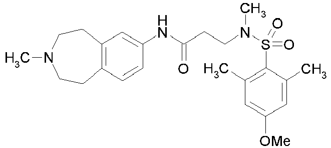

C₂₄H₃₃N₃O₄S x C₂HF₃O₂ (573.63)
[M+H]+ = 460
HPLC (Methode 5): Retentionszeit = 1.52 min

### Beispiel 158 (Referenzbeispiel)

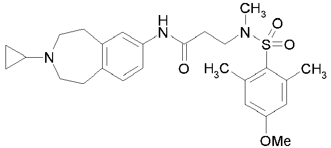

C₂₆H₃₅N₃O₄S x C₂HF₃O₂ (599.66)
[M+H]+ = 486
HPLC (Methode 5): Retentionszeit = 1.55 min

### Beispiel 159 (Referenzbeispiel)

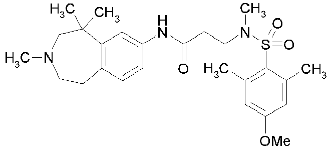

C₂₆H₃₇N₃O₄S x C₂HF₃O₂ (601.68)
[M+H]+ = 488
HPLC (Methode 5): Retentionszeit = 1.54 min

### Beispiel 160 (Referenzbeispiel)

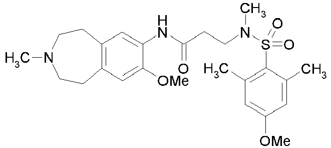

C₂₅H₃₅N₃O₅S x C₂HF₃O₂ (603.65)
[M+H]+ = 490
HPLC (Methode 5): Retentionszeit = 1.54 min

### Beispiel 161 (Referenzbeispiel)

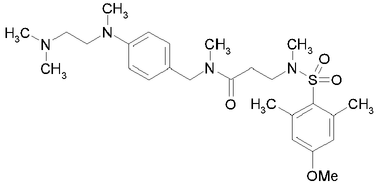

C₂₆H₄₀N₄O₄S x HCl (541.15)
[M+H]+ = 505
HPLC (Methode 5): Retentionszeit = 1.59 min

### Beispiel 162 (Referenzbeispiel)

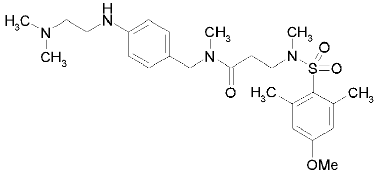

C₂₅H₃₈N₄O₄S x HCl (527.12)
[M+H]+ = 491
HPLC (Methode 5): Retentionszeit = 1.55 min

### Beispiel 163 (Referenzbeispiel)

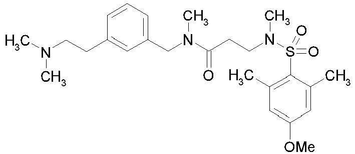

C₂₅H₃₇N₃O₄S x HCl (512.11)
[M+H]+ = 476
HPLC (Methode 5): Retentionszeit = 1.56 min

### Beispiel 164 (Referenzbeispiel)

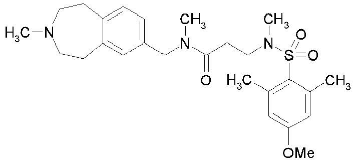

C₂₆H₃₇N₃O₄S x HCl (524.12)
[M+H]+ = 488
HPLC (Methode 5): Retentionszeit = 1.54 min

### Beispiel 165 (Referenzbeispiel)

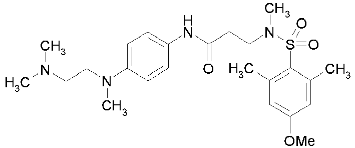

C₂₄H₃N₄O₄S x HCl (513.09)
[M+H]+ = 477
HPLC (Methode 7): Retentionszeit = 1.88 min

### Beispiel 166 (Referenzbeispiel)

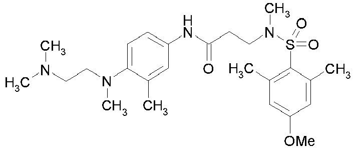

C₂₅H₃₈N₄O₄S x HCl (527.12)
[M+H]+ = 491
HPLC (Methode 7): Retentionszeit = 1.92 min

### Beispiel 167 (Referenzbeispiel)

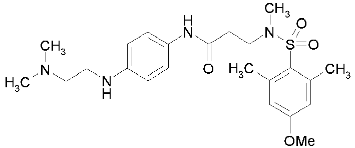

C₂₃H₃₄N₄O₄S x HCl (499.07)
[M+H]+ = 463
HPLC (Methode 7): Retentionszeit = 1.79 min

### Beispiel 168 (Referenzbeispiel)

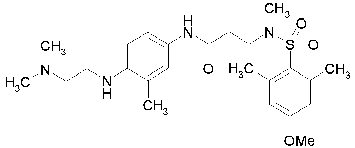

C₂₄H₃₆N₄O₄S x HCl (513.09)
[M+H]+ = 477
HPLC (Methode 7): Retentionszeit = 1.86 min

### Beispiel 169 (Referenzbeispiel)

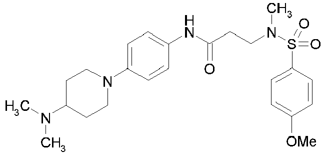

C₂₄H₃₄N₄O₄S x C₂HF₃O₂ (588.64)
[M+H]+ = 475
HPLC (Methode 5): Retentionszeit = 1.39 min

### Beispiel 170 (Referenzbeispiel)

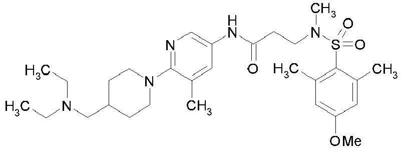

C₂₉H₄₅N₅O₄S x 2HCl (632.69)
[M+H]+ = 560
DC: Kieselgel, Dichlormethan / Ethanol 9:1, Rf-Wert = 0.22

### Beispiel 171 (Referenzbeispiel)

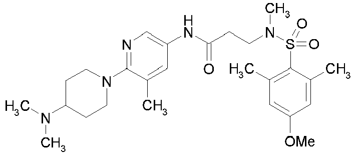

C₂₆H₃₉N₅O₄S x 2HCl (590.61)
[M+H]+ = 518
DC: Kieselgel, Dichlormethan / Ethanol 4:1, Rf-Wert = 0.68

### Beispiel 172 (Referenzbeispiel)

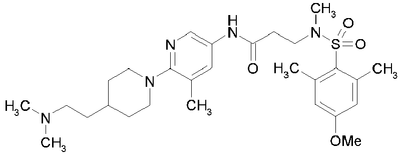

C₂₈H₄₃N₅O₄S x 2HCl (618.66)
[M+H]+ = 546
HPLC (Methode 5): Retentionszeit = 1.26 min

### Beispiel 173 (Referenzbeispiel)

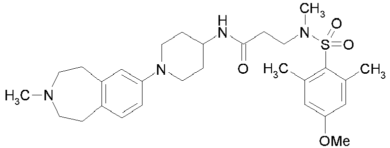

C₂₉H₄₂N₄O₄S (542.73)
[M+H]+ = 543
HPLC (Methode 4): Retentionszeit = 2.8 min

### Beispiel 174 (Referenzbeispiel)

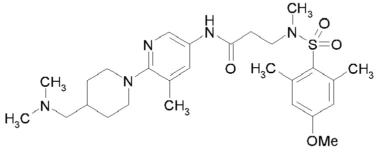

C₂₇H₄₁N₅O₄S x 2HCl (604.63)
[M+H]+ = 532
HPLC (Methode 5): Retentionszeit = 1.39 min

### Beispiel 175 (Referenzbeispiel)

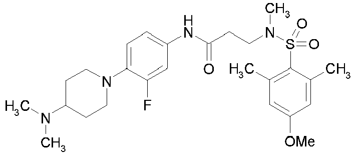

C₂₆H₃₇FN₄O₄S x HCl (557.12)
[M+H]+ = 521
DC: Kieselgel, Dichlormethan / Ethanol 9:1, Rf-Wert = 0.25

### Beispiel 176 (Referenzbeispiel)

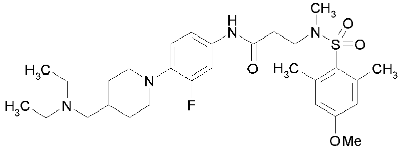

C₂₉H₄₃FN₄O₄S x HCl (599.20)
[M+H]+ = 563
DC: Kieselgel, Dichlormethan / Ethanol 9:1, Rf-Wert = 0.40

### Beispiel 177 (Referenzbeispiel)

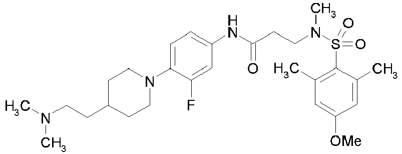

C₂₈H₄₁FN₄O₄S x HCl (585.17)
[M+H]+ = 549
DC: Kieselgel, Dichlormethan / Ethanol 9:1, Rf-Wert = 0.35

### Beispiel 178 (Referenzbeispiel)

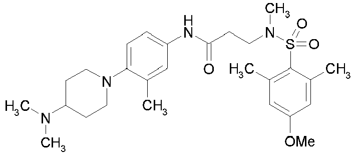

C₂₇H₄₀N₄O₄S (516.70)
[M+H]+ = 517
DC: Kieselgel, Dichlormethan / Ethanol / Ammoniak 8:2:0.01, Rf-Wert = 0.41

### Beispiel 179 (Referenzbeispiel)

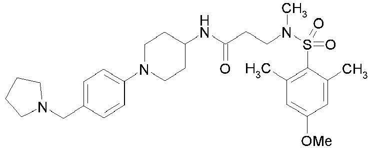

C₂₉H₄₂N₄O₄S x HCl (579.19)
[M+H]+ = 543
DC: Kieselgel, Dichlormethan / Ethanol / Ammoniak 8:2:0.01, Rf-Wert = 0.47

### Beispiel 180 (Referenzbeispiel)

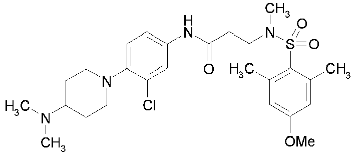

C₂₆H₃₇ClN₄O₄S x HCl (573.58)
[M+H]+ = 537/539
DC: Kieselgel, Dichlormethan / Ethanol 9:1, Rf-Wert = 0.26

### Beispiel 181 (Referenzbeispiel)

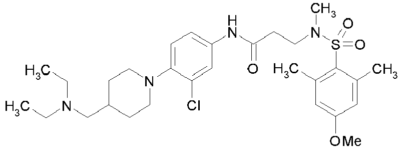

C₂₉H₄₃ClN₄O₄S x HCl (615.66)
[M+H]+ = 579/581
DC: Kieselgel, Dichlormethan / Ethanol 9:1, Rf-Wert = 0.45

### Beispiel 182 (Referenzbeispiel)

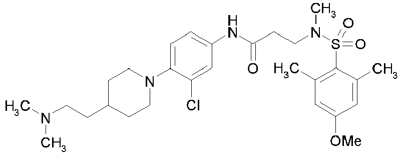

C₂₈H₄₁ClN₄O₄S x HCl (601.63)
[M+H]+ = 565/567
DC: Kieselgel, Dichlormethan / Ethanol 9:1, Rf-Wert = 0.30

### Beispiel 183 (Referenzbeispiel)

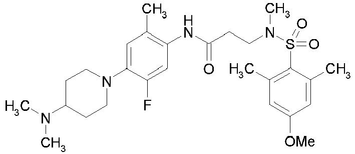

C₂₇H₃₉FN₄O₄S x HCl (571.15)
[M+H]+ = 535
DC: Kieselgel, Dichlormethan / Ethanol / Ammoniak 8:2:0.01, Rf-Wert = 0.26

### Beispiel 184 (Referenzbeispiel)

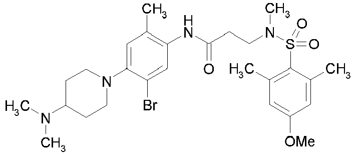

C₂₇H₃₉BrN₄O₄S x HCl (632.05)
[M+H]+ = 595/597
DC: Kieselgel, Dichlormethan / Ethanol / Ammoniak 8:2:0.01, Rf-Wert = 0.51

### Beispiel 185 (Referenzbeispiel)

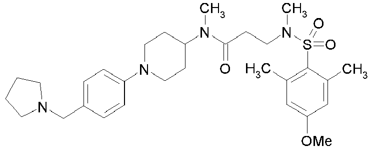

C₃₀H₄₄N₄O₄S x HCl (593.22)
[M+H]+ = 557
DC: Kieselgel, Dichlormethan / Ethanol / Ammoniak 8:2:0.01, Rf-Wert = 0.63

### Beispiel 186 (Referenzbeispiel)

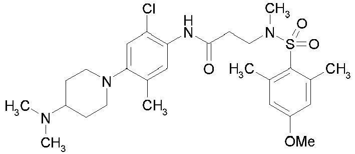

C₂₇H₃₉ClN₄O₄S x HCl (587.60)
[M+H]+ = 551
DC: Kieselgel, Dichlormethan / Ethanol / Ammoniak 8:2:0.01, Rf-Wert = 0.58

### Beispiel 187 (Referenzbeispiel)

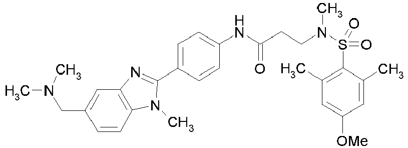

C₃₀H₃₇N₅O₄S x HCl (600.17)
[M+H]+ = 564
HPLC (Methode 4): Retentionszeit = 3.0 min

### Beispiel 188 (Referenzbeispiel)

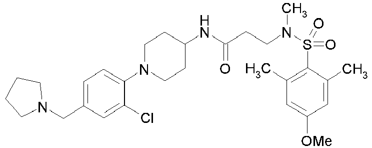

C₂₉H₄₁ClN₄O₄S x HCl (613.64)
[M+H]+ = 577/579
DC: Kieselgel, Dichlormethan / Methanol 9:1, Rf-Wert = 0.24

### Beispiel 189 (Referenzbeispiel)

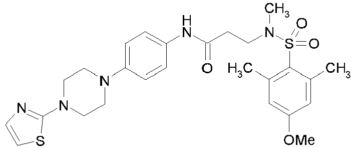

C₂₆H₃₃N₅O₄S₂ (543.70)
[M+H]+ = 544
HPLC (Methode 6): Retentionszeit = 3.11 min

### Beispiel 190 (Referenzbeispiel)

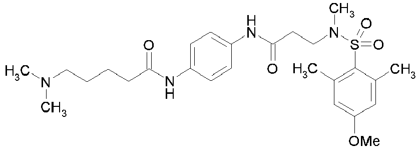

C₂₆H₃₈N₄O₅S (518.67)
[M+H]+ = 519
HPLC (Methode 6): Retentionszeit = 2.44 min

### Beispiel 191 (Referenzbeispiel)

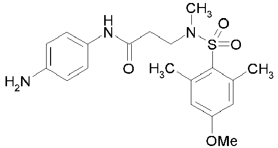

C₁₉H₂₅N₃O₄S x C₂HF₃O₂ (505.51)
[M+H]+ = 392
HPLC (Methode 6): Retentionszeit = 2.04 min

### Beispiel 192 (Referenzbeispiel)

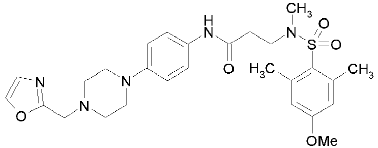

C₂₇H₃₅N₅O₅S (541.66)
[M+H]+ = 542
HPLC (Methode 6): Retentionszeit = 2.51 min

### Beispiel 193 (Referenzbeispiel)

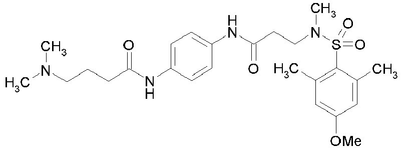

C₂₅H₃₆N₄O₅S (504.64)
[M+H]+ = 505
HPLC (Methode 6): Retentionszeit = 2.43 min

### Beispiel 194 (Referenzbeispiel)

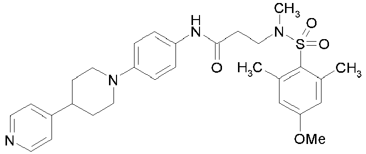

C₂₉H₃₆N₄O₄S (536.69)
[M+H]+ = 537
HPLC (Methode 6): Retentionszeit = 2.19 min

### Beispiel 195 (Referenzbeispiel)

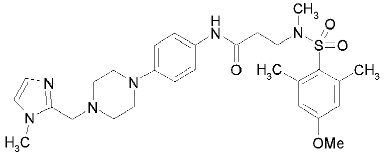

C₂₈H₃₈N₆O₄S (554.71)
[M+H]+ = 555
HPLC (Methode 6): Retentionszeit = 2.37 min

### Beispiel 196 (Referenzbeispiel)

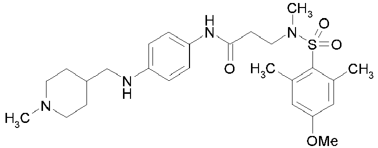

C₂₆H₃₈N₄O₄S (502.67)
[M+H]+ = 503
HPLC (Methode 6): Retentionszeit = 2.35 min

### Beispiel 197 (Referenzbeispiel)

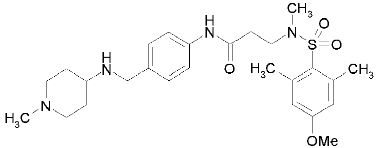

C₂₆H₃₈N₄O₄S (502.67)
[M+H]+ = 503
HPLC (Methode 6): Retentionszeit = 2.00 min

### Beispiel 198 (Referenzbeispiel)

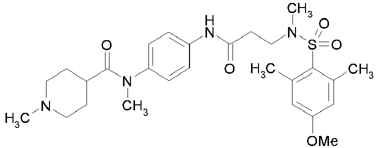

C₂₇H₃₈N₄O₅S x C₂HF₃O₂ (644.70)
[M+H]+ = 531
HPLC (Methode 6): Retentionszeit = 2.45 min

### Beispiel 199 (Referenzbeispiel)

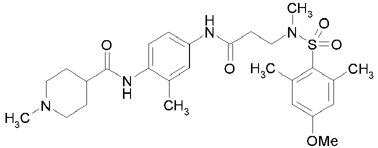

C₂₇H₃₈N₄O₅S (530.68)
[M+H]+ = 531
HPLC (Methode 6): Retentionszeit = 2.53 min

### Beispiel 200 (Referenzbeispiel)

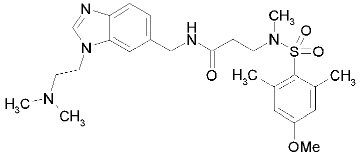

C₂₅H₃₅N₅O₄S (501.64)
[M+H]+ = 502
HPLC (Methode 6): Retentionszeit = 2.05 min

### Beispiel 201 (Referenzbeispiel)

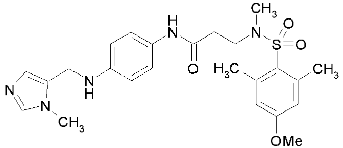

C₂₄H₃₁N₅O₄S (485.60)
[M+H]+ = 486
HPLC (Methode 6): Retentionszeit = 2.41 min

### Beispiel 202 (Referenzbeispiel)

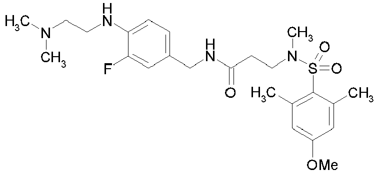

C₂₄H₃₅FN₄O₄S x CH₂O₂ (540.65)
[M+H]+ = 495
HPLC (Methode 6): Retentionszeit = 2.50 min

### Beispiel 203 (Referenzbeispiel)

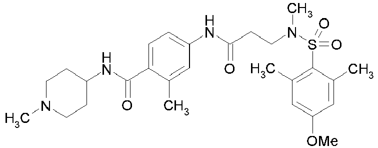

C₂₇H₃₈N₄O₅S (530.68)
[M+H]+ = 531
HPLC (Methode 6): Retentionszeit = 2.36 min

### Beispiel 204 (Referenzbeispiel)

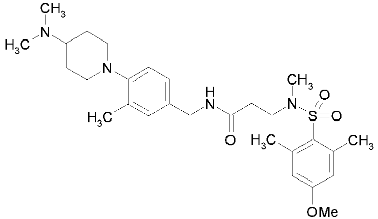

C₂₈H₄₂N₄O₄S (530.72)
[M+H]+ = 531
HPLC (Methode 6): Retentionszeit = 2.60 min

### Beispiel 205 (Referenzbeispiel)

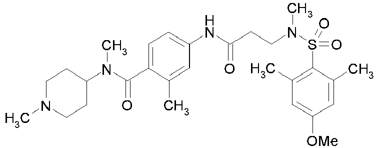

C₂₈H₄₀N₄O₅S (544.71)
[M+H]+ = 545
HPLC (Methode 6): Retentionszeit = 2.21 min

### Beispiel 206 (Referenzbeispiel)

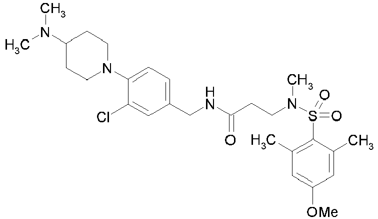

C₂₇H₃₉ClN₄O₄S (551.14)
[M+H]+ = 551/553
HPLC (Methode 6): Retentionszeit = 2.65 min

### Beispiel 207 (Referenzbeispiel)

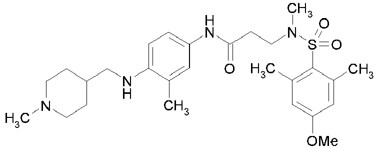

C₂₇H₄₀N₄O₄S x C₂HF₃O₂ (630.72)
[M+H]+ = 517
HPLC (Methode 6): Retentionszeit = 2.51 min

### Beispiel 208 (Referenzbeispiel)

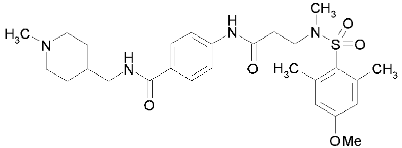

C₂₇H₃₈N₄O₅S x CH₂O₂ (576.71)
[M+H]+ = 531
HPLC (Methode 6): Retentionszeit = 2.33 min

### Beispiel 209 (Referenzbeispiel)

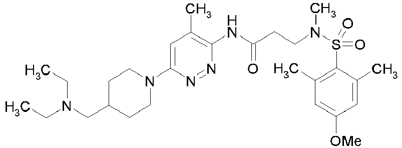

C₂₈H₄₄N₆O₄S x C₂HF₃O₂ (674.78)
[M+H]+ = 561
HPLC (Methode 6): Retentionszeit = 2.14 min

### Beispiel 210 (Referenzbeispiel)

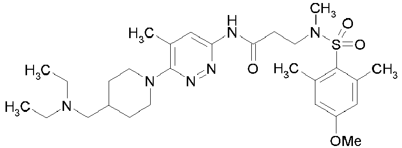

C₂₈H₄₄N₆O₄S x C₂HF₃O₂ (674.78)
[M+H]+ = 561
HPLC (Methode 6): Retentionszeit = 2.43 min

### Beispiel 211 (Referenzbeispiel)

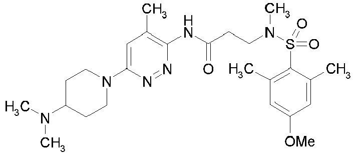

C₂₅H₃₈N₆O₄S x C₂HF₃O₂ (632.70)
[M+H]+ = 519
HPLC (Methode 6): Retentionszeit = 2.11 min

### Beispiel 212 (Referenzbeispiel)

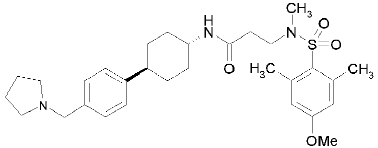

C₃₀H₄₃N₃O₄S x C₂HF₃O₂ (655.77)
[M+H]+ = 542
HPLC (Methode 6): Retentionszeit = 2.75 min

### Beispiel 213 (Referenzbeispiel)

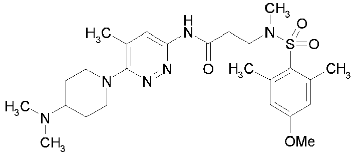

C₂₅H₃₈N₆O₄S x C₂HF₃O₂ (632.70)
[M+H]+ = 519
HPLC (Methode 6): Retentionszeit = 2.30 min

### Beispiel 214 (Referenzbeispiel)

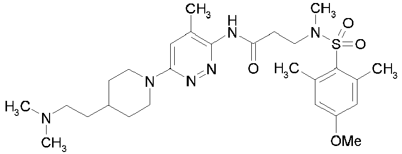

C₂₇H₄₂N₆O₄S x C₂HF₃O₂ (660.75)
[M+H]+ = 547
HPLC (Methode 6): Retentionszeit = 2.08 min

### Beispiel 215 (Referenzbeispiel)

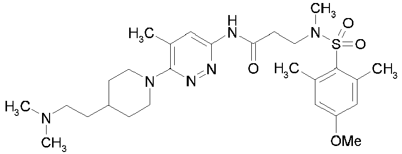

C₂₇H₄₂N₆O₄S x C₂HF₃O₂ (660.75)
[M+H]+ = 547
HPLC (Methode 6): Retentionszeit = 2.34 min

### Beispiel 216 (Referenzbeispiel)

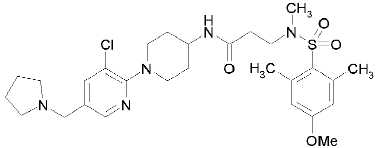

C₂₈H₄₀ClN₅O₄S x CH₂O₂ (624.19)
[M+H]+ = 578/580
HPLC (Methode 6): Retentionszeit = 2.64 min

### Beispiel 217 (Referenzbeispiel)

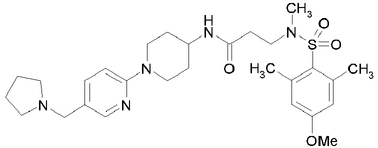

C₂₈H₄₁N₅O₄S x CH₂O₂ (589.75)
[M+H]+ = 544
HPLC (Methode 6): Retentionszeit = 2.09 min

### Beispiel 218 (Referenzbeispiel)

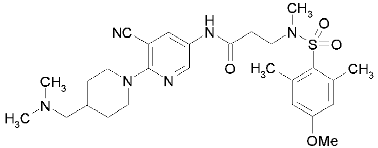

C₂₇H₃₈N₆O₄S x CH₂O₂ (588.72)
[M+H]+ = 543
HPLC (Methode 9): Retentionszeit = 1.67 min

### Beispiel 219 (Referenzbeispiel)

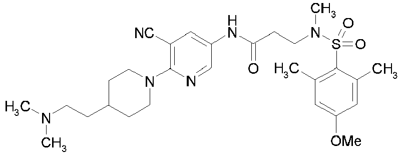

C₂₈H₄₀N₆O₄S (556.72)
[M+H]+ = 557
HPLC (Methode 9): Retentionszeit = 1.71 min

### Beispiel 220 (Referenzbeispiel)

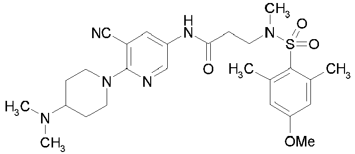

C₂₆H₃₆N₆O₄S x CH₂O₂ (574.69)
[M+H]+ = 529
HPLC (Methode 9): Retentionszeit = 1.61 min

### Beispiel 221 (Referenzbeispiel)

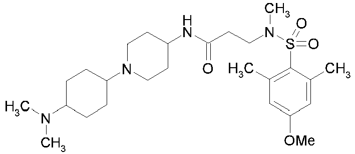

C₂₆H₄₄N₄O₄S (508.72)
[M+H]+ = 509
HPLC (Methode 9): Retentionszeit = 1.23 min

### Beispiel 222 (Referenzbeispiel)

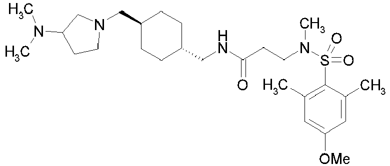

C₂₇H₄₆N₄O₄S x 2C₂HF₃O₂ (750.79)
[M+H]+ = 523
HPLC (Methode 9): Retentionszeit = 1.30 min

### Beispiel 223 (Referenzbeispiel)

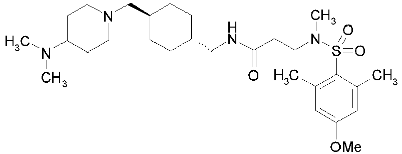

C₂₈H₄₈N₄O₄S x 2C₂HF₃O₂ (764.82)
[M+H]+ = 537
HPLC (Methode 9): Retentionszeit = 1.31 min

### Beispiel 224 (Referenzbeispiel)

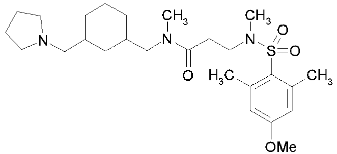

C₂₆H₄₃N₃O₄S (493.70)
[M+H]+ = 494
HPLC (Methode 9): Retentionszeit = 1.72 min

### Beispiel 225 (Referenzbeispiel)

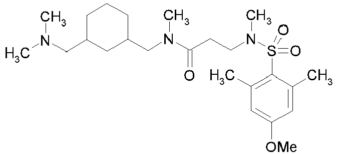

C₂₄H₄₁N₃O₄S x C₂HF₃O₂ (581.69)
[M+H]+ = 468
HPLC (Methode 9): Retentionszeit = 1.69 min

### Beispiel 226 (Referenzbeispiel)

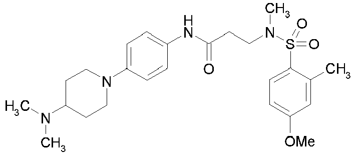

C₂₅H₃₆N₄O₄S x C₂HF₃O₂ (602.67)
[M+H]+ = 489
HPLC (Methode 5): Retentionszeit = 1.39 min

### Beispiel 227 (Referenzbeispiel)

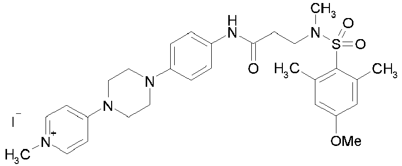

C₂₉H₃₈N₅O₄S x I (679.61)
[M+H]+ = 552
HPLC (Methode 5): Retentionszeit = 1.55 min

### Beispiel 228 (Referenzbeispiel)

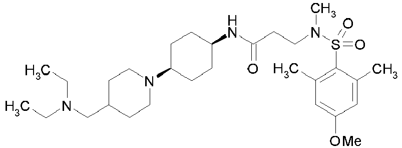

C₂₉H₅₀N₄O₄S (550.80)
[M+H]+ = 551
HPLC (Methode 5): Retentionszeit = 1.38 min

### Beispiel 229 (Referenzbeispiel)

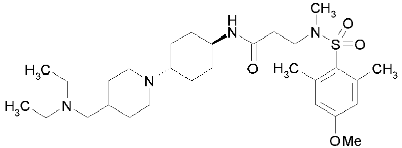

C₂₉H₅₀N₄O₄S (550.80)
[M+H]+ = 551
HPLC (Methode 5): Retentionszeit = 1.40 min

### Beispiel 230 (Referenzbeispiel)

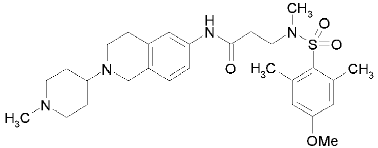

C₂₈H₄₀N₄O₄S x 2HCl (601.63)
[M+H]+ = 529
HPLC (Methode 5): Retentionszeit = 1.41 min

### Beispiel 231 (Referenzbeispiel)

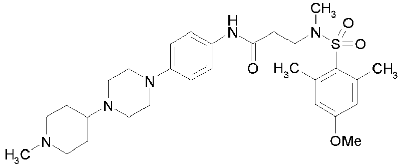

C₂₉H₄₃N₅O₄S (557.75)
[M+H]+ = 558
HPLC (Methode 1): Retentionszeit = 1.90 min

### Beispiel 232 (Referenzbeispiel)

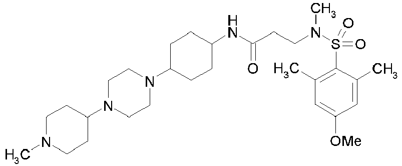

C₂₉H₄₉N₅O₄S (563.80)
[M+H]+ = 564
HPLC (Methode 5): Retentionszeit = 1.33 min

### Beispiel 233 (Referenzbeispiel)

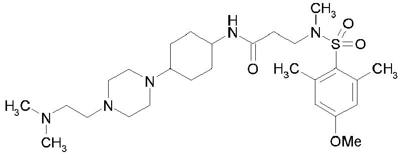

C₂₇H₄₇N₅O₄S x 2HCl (610.68)
[M+H]+ = 538
HPLC (Methode 7): Retentionszeit = 1.74 min

### Beispiel 234 (Referenzbeispiel)

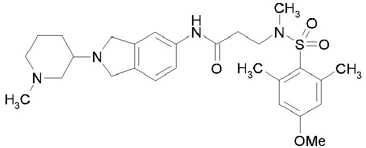

C₂₇H₃₈N₄O₄S (610.68)
[M+H]+ = 515
HPLC (Methode 5): Retentionszeit = 1.41 min

### Beispiel 235 (Referenzbeispiel)

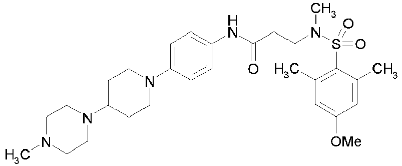

C₂₉H₄₃N₅O₄S (557.75)
[M+H]+ = 558
HPLC (Methode 5): Retentionszeit = 1.43 min

### Beispiel 236 (Referenzbeispiel)

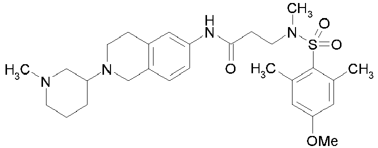

C₂₈H₄₀N₄O₄S x 2C₂HF₃O₂ (756.75)
[M+H]+ = 529
HPLC (Methode 5): Retentionszeit = 1.42 min

### Beispiel 237 (Referenzbeispiel)

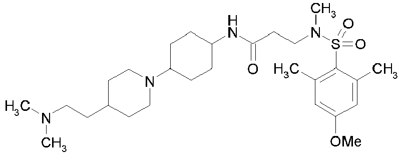

C₂₈H₄₈N₄O₄S x 2HCl (609.69)
[M+H]+ = 537
HPLC (Methode 7): Retentionszeit = 1.70 min

### Beispiel 238 (Referenzbeispiel)

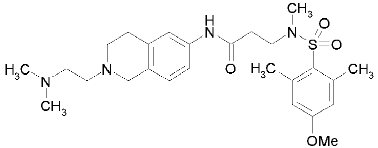

C₂₆H₃₈N₄O₄S x 2C₂HF₃O₂ (730.72)
[M+H]+ = 503
HPLC (Methode 5): Retentionszeit = 1.40 min

### Beispiel 239 (Referenzbeispiel)

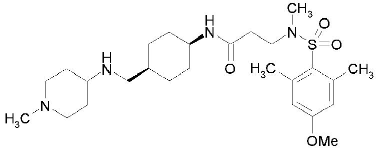

C₂₆H₄₄N₄O₄S x 2HCl (581.64)
[M+H]+ = 509
HPLC (Methode 5): Retentionszeit = 1.38 min

### Beispiel 240 (Referenzbeispiel)

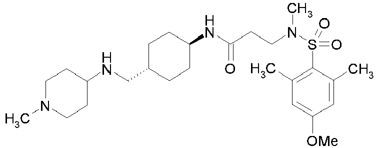

C₂₆H₄₄N₄O₄S x 2HCl (581.64)
[M+H]+ = 509
HPLC (Methode 5): Retentionszeit = 1.40 min

### Beispiel 241 (Referenzbeispiel)

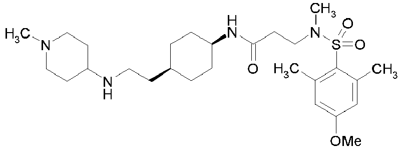

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 242 (Referenzbeispiel)

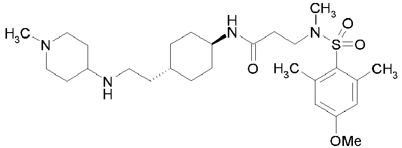

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 243 (Referenzbeispiel)

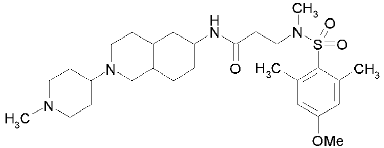

C₂₈H₄₆N₄O₄S x 2HCl (607.68)
[M+H]+ = 535
HPLC (Methode 5): Retentionszeit = 1.10 min

### Beispiel 244 (Referenzbeispiel)

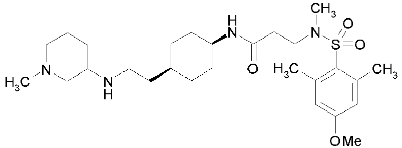

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 245 (Referenzbeispiel)

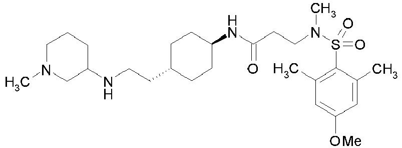

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 246 (Referenzbeispiel)

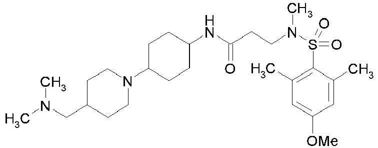

C₂₇H₄₆N₄O₄S x 2C₂HF₃O₂ (750.79)
[M+H]+ = 523
HPLC (Methode 5): Retentionszeit = 1.38 min

### Beispiel 247 (Referenzbeispiel)

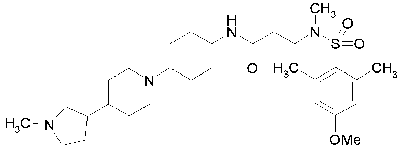

C₂₉H₄₈N₄O₄S x 2C₂HF₃O₂ (776.83)
[M+H]+ = 549
HPLC (Methode 5): Retentionszeit = 1.39 min

### Beispiel 248 (Referenzbeispiel)

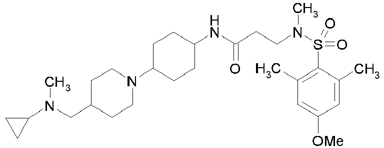

C₂₉H₄₈N₄O₄S x 2C₂HF₃O₂ (776.83)
[M+H]+ = 549
HPLC (Methode 5): Retentionszeit = 1.39 min

### Beispiel 249 (Referenzbeispiel)

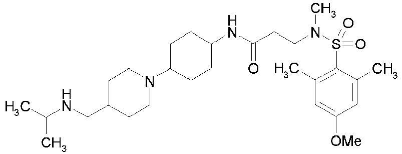

C₂₈H₄₈N₄O₄S x 2HCl (609.69)
[M+H]+ = 537
HPLC (Methode 5): Retentionszeit = 1.38 min

### Beispiel 250 (Referenzbeispiel)

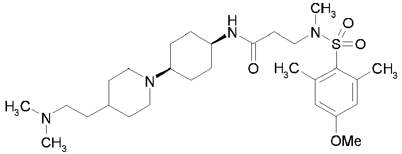

C₂₈H₄₈N₄O₄S x 2HCl (609.69)
[M+H]+ = 537
HPLC (Methode 11): Retentionszeit = 1.60 min

### Beispiel 251 (Referenzbeispiel)

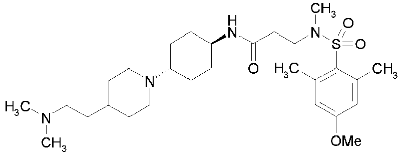

C₂₈H₄₈N₄O₄S x 2HCl (609.69)
[M+H]+ = 537
HPLC (Methode 7): Retentionszeit = 1.71 min

### Beispiel 252 (Referenzbeispiel)

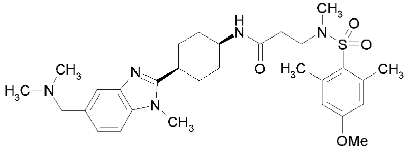

C₃₀H₄₃N₅O₄S x HCl (606.22)
[M+H]+ = 570
DC: Kieselgel, Dichlormethan / Methanol / Ammoniak 9:1:0.1, Rf-Wert = 0.29

### Beispiel 253 (Referenzbeispiel)

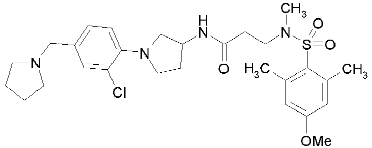

C₂₈H₃₉ClN₄O₄S x HCl (599.61)
[M+H]+ = 563/565
HPLC (Methode 5): Retentionszeit = 1.59 min

### Beispiel 254 (Referenzbeispiel)

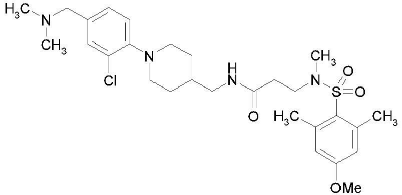

C₂₈H₄₁ClN₄O₄S x HCl (601.63)
[M+H]+ = 565/567
DC: Kieselgel, Dichlormethan / Methanol / Ammoniak 9:1:0.1, Rf-Wert = 0.46

### Beispiel 255 (Referenzbeispiel)

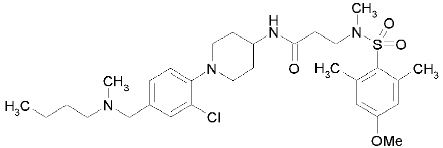

C₃₀H₄₅ClN₄O₄S x HCl (629.68)
[M+H]+ = 593/595
DC: Kieselgel, Dichlormethan / Methanol 9:1, Rf-Wert = 0.42

### Beispiel 256 (Referenzbeispiel)

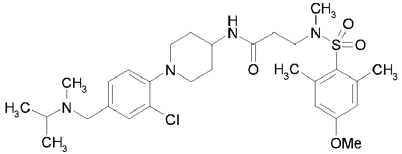

C₂₉H₄₃ClN₄O₄S x HCl (615.66)
[M+H]+ = 579/581
DC: Kieselgel, Dichlormethan / Methanol 9:1, Rf-Wert = 0.23

### Beispiel 257 (Referenzbeispiel)

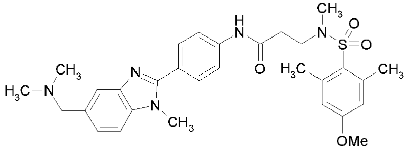

C₃₀H₃₇N₅O₄S x HCl (600.17)
[M+H]+ = 564
DC: Kieselgel, Dichlormethan / Methanol / Ammoniak 8:2:0.1, Rf-Wert = 0.67

### Beispiel 258 (Referenzbeispiel)

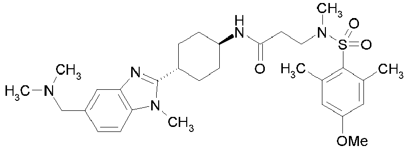

C₃₀H₄₃N₅O₄S (569.76)
[M+H]+ = 570
DC: Kieselgel, Dichlormethan / Methanol / Ammoniak 8:2:0.2, Rf-Wert = 0.65

### Beispiel 259 (Referenzbeispiel)

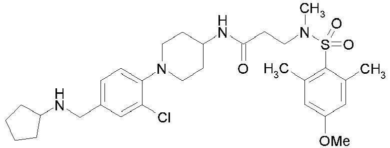

C₃₀H₄₃ClN₄O₄S x HCl (627.67)
[M+H]+ = 591/593
DC: Kieselgel, Dichlormethan / Methanol 9:1, Rf-Wert = 0.20

### Beispiel 260 (Referenzbeispiel)

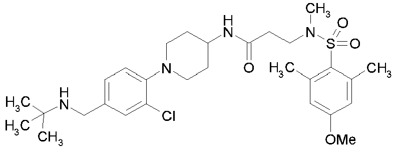

C₂₉H₄₃ClN₄O₄S x HCl (615.66)
[M+H]+ = 579/581
DC: Kieselgel, Dichlormethan / Methanol 9:1, Rf-Wert = 0.17

### Beispiel 261 (Referenzbeispiel)

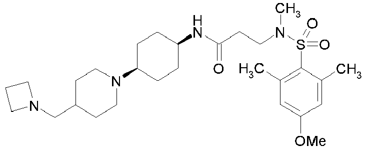

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.23

### Beispiel 262 (Referenzbeispiel)

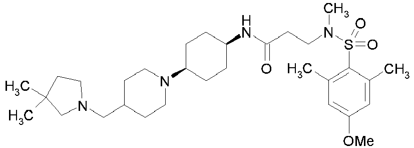

C₃₁H₅₂N₄O₄S x 2HCl (649.76)
[M+H]+ = 577
DC: Kieselgel, Dichlormethan / Methanol / Ammoniak 8:2:0.2, Rf-Wert = 0.69

### Beispiel 263 (Referenzbeispiel)

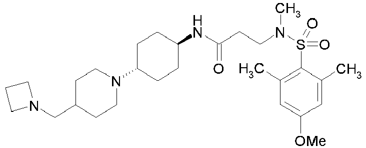

C₂₈H₄₆N₄O₄S (534.76)
[M+H]+ = 535
DC: Kieselgel, Dichlormethan / Methanol / Ammoniak 8:2:0.2, Rf-Wert = 0.58

### Beispiel 264 (Referenzbeispiel)

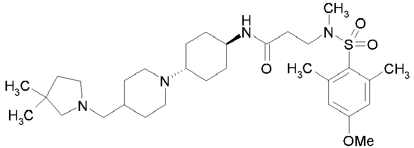

C₃₁H₅₂N₄O₄S (576.84)
[M+H]+ = 577
DC: Kieselgel, Dichlormethan / Methanol / Ammoniak 8:2:0.2, Rf-Wert = 0.56

### Beispiel 265 (Referenzbeispiel)

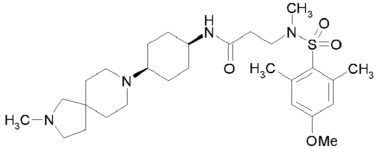

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.58

### Beispiel 266 (Referenzbeispiel)

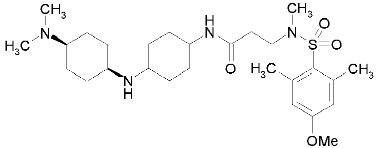

C₂₇H₄₆N₄O₄S (522.74)
[M+H]+ = 523
HPLC (Methode 9): Retentionszeit = 1.30 min

### Beispiel 267 (Referenzbeispiel)

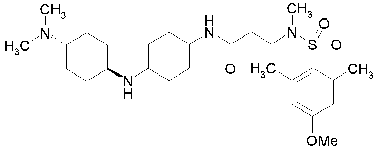

C₂₇H₄₆N₄O₄S (522.74)
[M+H]+ = 523
HPLC (Methode 9): Retentionszeit = 1.28 min

### Beispiel 268 (Referenzbeispiel)

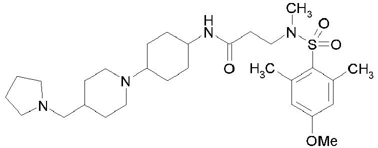

C₂₉H₄₈N₄O₄S X CH₂O₂ (594.81)
[M+H]+ = 549
HPLC (Methode 6): Retentionszeit = 1.96 min

### Beispiel 269 (Referenzbeispiel)

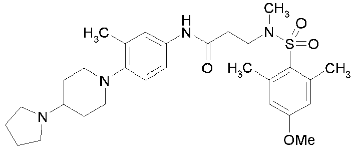

C₃₀₉H₄₄N₄O₄S (556.76)
[M+H]+ = 557
HPLC (Methode 9): Retentionszeit = 1.71 min

### Beispiel 270 (Referenzbeispiel)

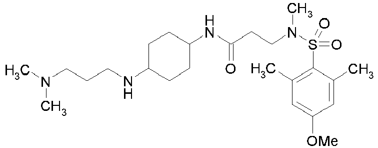

C₂₄H₄₂N₄O₄S x C₂HF₃O₂ (596.70)
[M+H]+ = 483
HPLC (Methode 9): Retentionszeit = 1.23 min

### Beispiel 271 (Referenzbeispiel)

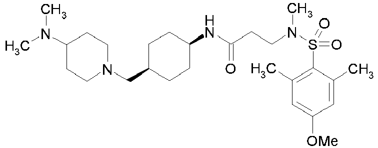

C₂₇H₄₆N₄O₄S (522.74)
[M+H]+ = 523
HPLC (Methode 9): Retentionszeit = 1.23 min

### Beispiel 272 (Referenzbeispiel)

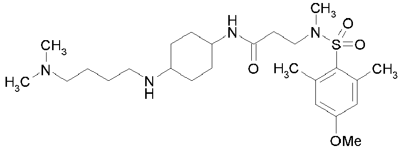

C₂₅H₄₄N₄O₄S (496.71)
[M+H]+ = 497
HPLC (Methode 9): Retentionszeit = 1.25 min

### Beispiel 273 (Referenzbeispiel)

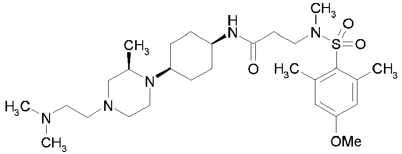

C₂₈H₄₉N₅O₄S x 2HCl (624.71)
[M+H]+ = 552
HPLC (Methode 10): Retentionszeit = 1.06 min

### Beispiel 274 (Referenzbeispiel)

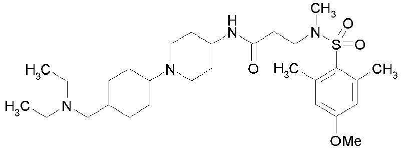

C₂₉H₅₀N₄O₄S x CH₂O₂ (596.82)
[M+H]+ = 551
HPLC (Methode 9): Retentionszeit = 1.31 min

### Beispiel 275 (Referenzbeispiel)

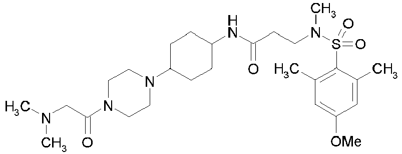

C₂₇H₄₅N₅O₅S x CH₂O₂ (597.77)
[M+H]+ = 552
HPLC (Methode 9): Retentionszeit = 1.20 min

### Beispiel 276 (Referenzbeispiel)

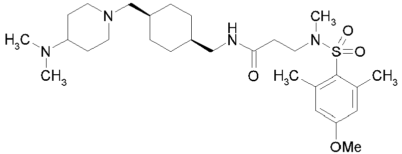

C₂₈H₄₈N₄O₄S (536.77)
[M+H]+ = 537
HPLC (Methode 6): Retentionszeit = 1.32 min

### Beispiel 277 (Referenzbeispiel)

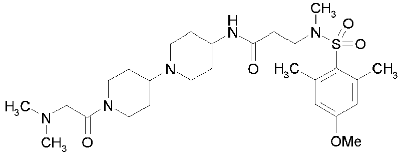

C₂₇H₄₅N₅O₅S x C₂HF₃O₂ (665.77)
[M+H]+ = 552
HPLC (Methode 9): Retentionszeit = 1.18 min

### Beispiel 278 (Referenzbeispiel)

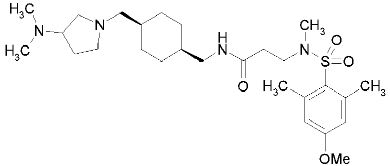

C₂₇H₄₆N₄O₄S x 2 C₂HF₃O₂ (750.79)
[M+H]+ = 523
HPLC (Methode 9): Retentionszeit = 1.29 min

### Beispiel 279 (Referenzbeispiel)

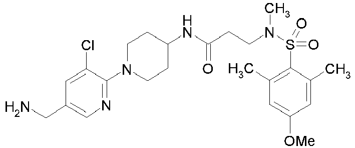

C₂₄H₃₄ClN₅O₄S (524.08)
[M+H]+ = 524/526
HPLC (Methode 9): Retentionszeit = 1.60 min

### Beispiel 280 (Referenzbeispiel)

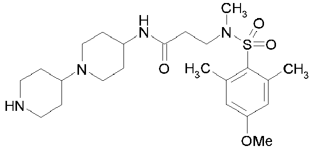

C₂₃H₃₈N₄O₄S x 2C₂HF₃O₂ (694.69)
[M+H]+ = 467
HPLC (Methode 9): Retentionszeit = 1.16 min

### Beispiel 281 (Referenzbeispiel)

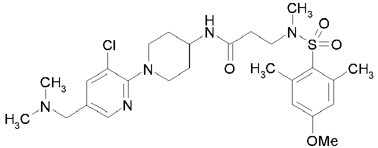

C₂₆H₃₈ClN₅O₄S x C₂HF₃O₂ (666.15)
[M+H]+ = 552/554
HPLC (Methode 9): Retentionszeit = 1.68 min

### Beispiel 282 (Referenzbeispiel)

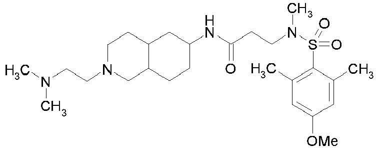

C₂₆H₄₄N₄O₄S x 2HCl (581.64)
[M+H]+ = 509
HPLC (Methode 5): Retentionszeit = 1.36 min

### Beispiel 283 (Referenzbeispiel)

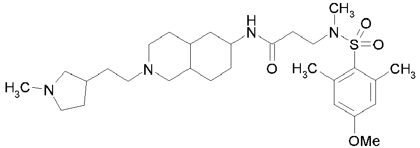

C₂₉H₄₈N₄O₄S x 2HCl (621.70)
[M+H]+ = 549
HPLC (Methode 5): Retentionszeit = 1.38 min

### Beispiel 284 (Referenzbeispiel)

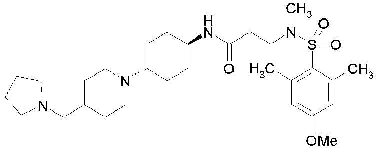

C₂₉H₄₈N₄O₄S (548.78)
[M+H]+ = 549
HPLC (Methode 5): Retentionszeit = 1.38 min

### Beispiel 285 (Referenzbeispiel)

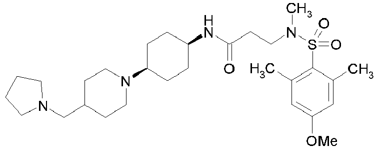

C₂₉H₄₈N₄O₄S (548.78)
[M+H]+ = 549
HPLC (Methode 5): Retentionszeit = 1.36 min

### Beispiel 286 (Referenzbeispiel)

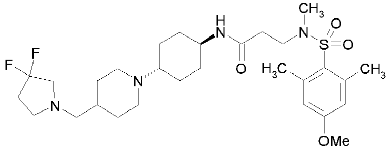

C₂₉H₄₆F₂N₄O₄S (584.76)
[M+H]+ = 585
HPLC (Methode 5): Retentionszeit = 1.38 min

### Beispiel 287 (Referenzbeispiel)

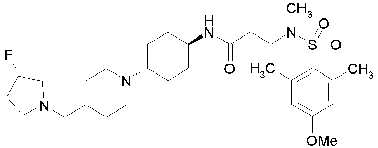

C₂₉H₄₇FN₄O₄S (566.77)
[M+H]+ = 567
HPLC (Methode 5): Retentionszeit = 1.38 min

### Beispiel 288 (Referenzbeispiel)

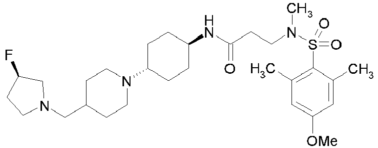

C₂₉H₄₇FN₄O₄S (566.77)
[M+H]+ = 567
HPLC (Methode 5): Retentionszeit = 1.36 min

### Beispiel 289 (Referenzbeispiel)

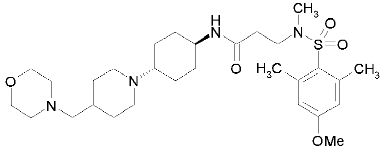

C₂₉H₄₈N₄O₅S (564.78)
[M+H]+ = 565
HPLC (Methode 5): Retentionszeit = 1.37 min

### Beispiel 290 (Referenzbeispiel)

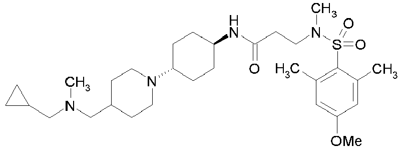

C₃₀H₅₀N₄O₄S (562.81)
[M+H]+ = 563
HPLC (Methode 5): Retentionszeit = 1.39 min

### Beispiel 291 (Referenzbeispiel)

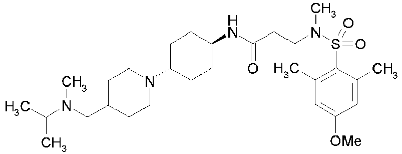

C₂₉H₅₀N₄O₄S (550.80)
[M+H]+ = 551
HPLC (Methode 5): Retentionszeit = 1.36 min

### Beispiel 292 (Referenzbeispiel)

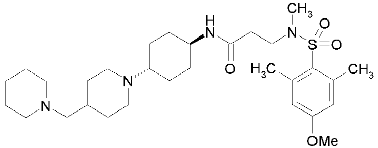

C₃₀H₅₀N₄O₄S (562.81)
[M+H]+ = 563
HPLC (Methode 5): Retentionszeit = 1.28 min

### Beispiel 293 (Referenzbeispiel)

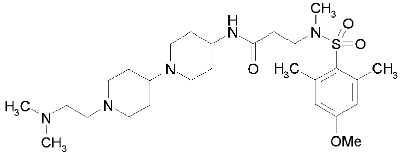

C₂₇H₄₇N₅O₄S x 3C₂HF₃O₂ (879.83)
[M+H]+ = 538
HPLC (Methode 5): Retentionszeit = 1.33 min

### Beispiel 294 (Referenzbeispiel)

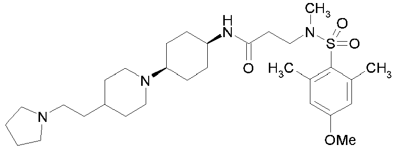

C₃₀H₅₀N₄O₄S x 2HCl (635.73)
[M+H]+ = 563
HPLC (Methode 5): Retentionszeit = 1.37 min

### Beispiel 295 (Referenzbeispiel)

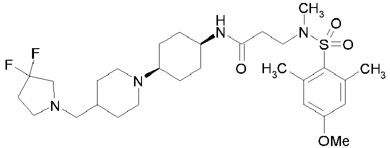

C₂₉H₄₆F₂N₄O₄S x 2C₂HF₃O₂ (584.76)
[M+H]+ = 585
HPLC (Methode 5): Retentionszeit = 1.38 min

### Beispiel 296 (Referenzbeispiel)

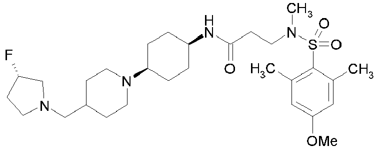

C₂₉H₄₇FN₄O₄S x 2C₂HF₃O₂ (794.82)
[M+H]+ = 567
HPLC (Methode 5): Retentionszeit = 1.35 min

### Beispiel 297 (Referenzbeispiel)

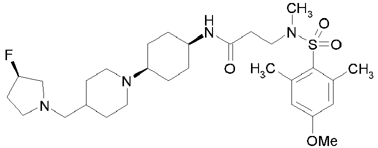

C₂₉H₄₇FN₄O₄S x 2C₂HF₃O₂ (794.82)
[M+H]+ = 567
HPLC (Methode 5): Retentionszeit = 1.36 min

### Beispiel 298 (Referenzbeispiel)

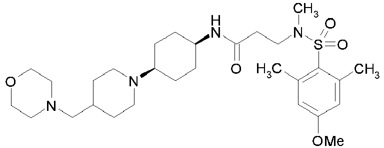

C₂₉H₄₈N₄O₅S (564.78)
[M+H]+ = 565
HPLC (Methode 5): Retentionszeit = 1.36 min

### Beispiel 299 (Referenzbeispiel)

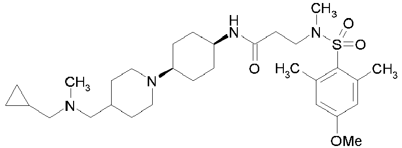

C₃₀H₅₀N₄O₄S (562.81)
[M+H]+ = 563
HPLC (Methode 5): Retentionszeit = 1.37 min

### Beispiel 300 (Referenzbeispiel)

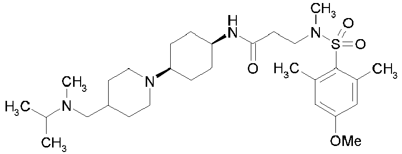

C₂₉H₅₀N₄O₄S (550.80)
[M+H]+ = 551
HPLC (Methode 5): Retentionszeit = 1.37 min

### Beispiel 301 (Referenzbeispiel)

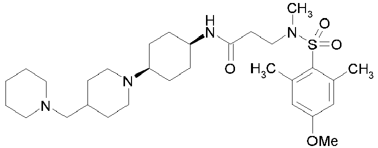

C₃₀H₅₀N₄O₄S (562.81)
[M+H]+ = 563
HPLC (Methode 5): Retentionszeit = 1.37 min

### Beispiel 302 (Referenzbeispiel)

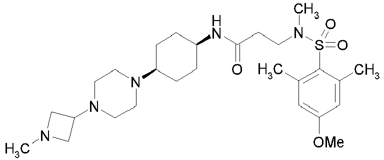

C₃₀H₅₀N₄O₄S x 3C₂HF₃O₂ (877.81)
[M+H]+ = 536
HPLC (Methode 5): Retentionszeit = 1.34 min

### Beispiel 303 (Referenzbeispiel)

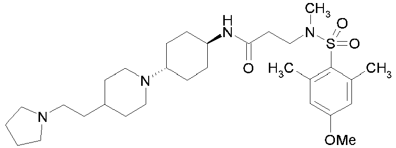

C₃₀H₅₀N₄O₄S (562.81)
[M+H]+ = 563
HPLC (Methode 5): Retentionszeit = 1.37 min

### Beispiel 304 (Referenzbeispiel)

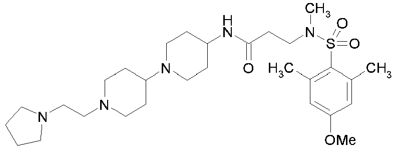

C₂₉H₄₉N₅O₄S x 3C₂HF₃O₂ (905.87)
[M+H]+ = 564
HPLC (Methode 5): Retentionszeit = 1.08 min

### Beispiel 305 (Referenzbeispiel)

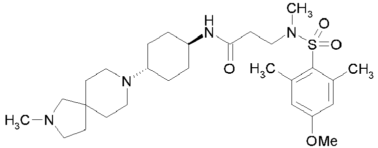

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.19

### Beispiel 306 (Referenzbeispiel)

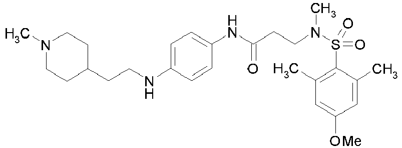

C₂₇H₄₀N₄O₄S x C₂HF₃O₄ (630.72)
[M+H]+ = 517
HPLC (Methode 9): Retentionszeit = 1.40 min

### Beispiel 307 (Referenzbeispiel)

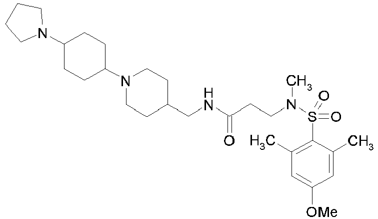

C₂₉H₄₈N₄O₄S (548.78)
[M+H]+ = 549
HPLC (Methode 6): Retentionszeit = 1.24 min

### Beispiel 308 (Referenzbeispiel)

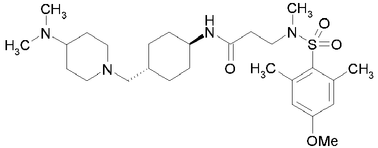

C₂₇H₄₆N₄O₄S (522.74)
[M+H]+ = 523
HPLC (Methode 9): Retentionszeit = 1.29 min

### Beispiel 309 (Referenzbeispiel)

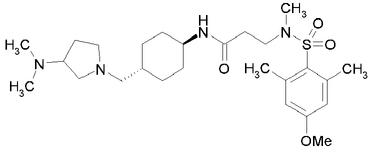

C₂₆H₄₄N₄O₄S (508.72)
[M+H]+ = 509
HPLC (Methode 9): Retentionszeit = 1.30 min

### Beispiel 310 (Referenzbeispiel)

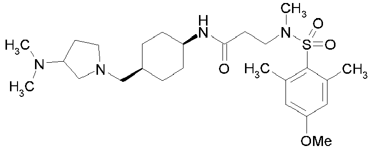

C₂₆H₄₄N₄O₄S (508.72)
[M+H]+ = 509
HPLC (Methode 9): Retentionszeit = 1.23 min

### Beispiel 311 (Referenzbeispiel)

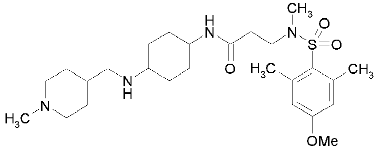

C₂₆H₄₄N₄O₄S (508.72)
[M+H]+ = 509
HPLC (Methode 6): Retentionszeit = 1.20 min

### Beispiel 312 (Referenzbeispiel)

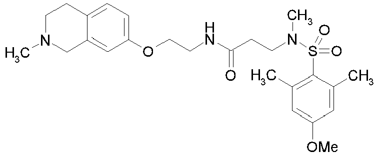

C₂₅H₃₅N₃O₅S x HCl (526.09)
[M+H]+ = 490
HPLC (Methode 10): Retentionszeit = 1.16 min

### Beispiel 313 (Referenzbeispiel)

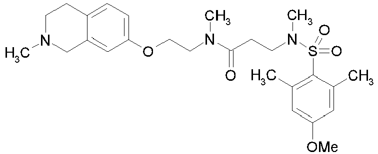

C₂₆H₃₇N₃O₅S x HCl (540.12)
[M+H]+ = 504
HPLC (Methode 10): Retentionszeit = 1.22 min

### Beispiel 314 (Referenzbeispiel)

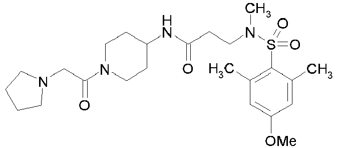

C₂₄H₃₈N₄O₅S x C₂HF₃O₂ (608.67)
[M+H]+ = 495
HPLC (Methode 9): Retentionszeit = 1.47 min

### Beispiel 315 (Referenzbeispiel)

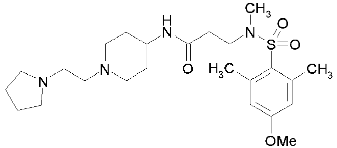

C₂₄H₄₀N₄O₄S (480.66)
[M+H]+ = 481
HPLC (Methode 9): Retentionszeit = 1.21 min

### Beispiel 316 (Referenzbeispiel)

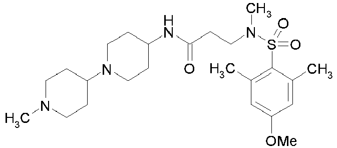

C₂₄H₄₀N₄O₄S x C₂HF₃O₂ (594.69)
[M+H]+ = 481
HPLC (Methode 9): Retentionszeit = 1.19 min

### Beispiel 317 (Referenzbeispiel)

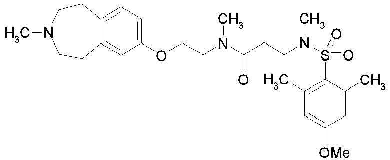

C₂₇H₃₉N₃O₅S x HCl (554.14)
[M+H]+ = 518
HPLC (Methode 5): Retentionszeit = 1.36 min

### Beispiel 593 (Referenzbeispiel)

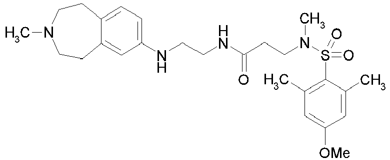

C₂₆H₃₈N₄O₄S x HCl (539.13)
[M+H]+ = 503
HPLC (Methode 5): Retentionszeit = 1.29 min

### Beispiel 594 (Referenzbeispiel)

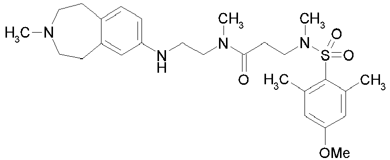

C₂₇H₄₀N₄O₄S x HCl (553.16)
[M+H]+ = 517
HPLC (Methode 5): Retentionszeit = 1.35 min

### Beispiel 595 (Referenzbeispiel)

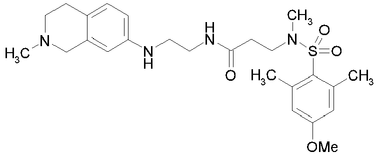

C₂₅H₃₆N₄O₄S x HCl (525.10)
[M+H]+ = 489
HPLC (Methode 5): Retentionszeit = 1.31 min

### Beispiel 596 (Referenzbeispiel)

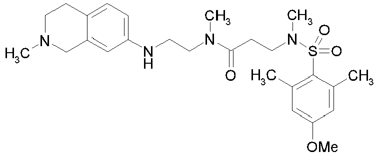

C₂₆H₃₈N₄O₄S x HCl (539.13)
[M+H]+ = 503
HPLC (Methode 5): Retentionszeit = 1.35 min

### Beispiel 597 (Referenzbeispiel)

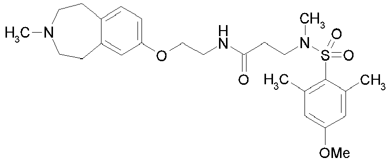

C₂₆H₃₇N₃O₅S x HCl (540.12)
[M+H]+ = 504
HPLC (Methode 10): Retentionszeit = 1.18 min

### Beispiel 598 (Referenzbeispiel)

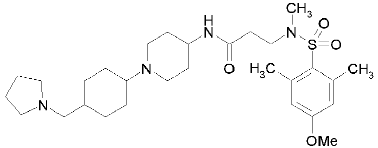

C₂₉H₄₈N₄O₄S x C₂HF₃O₂ (662.81)
[M+H]+ = 549
HPLC (Methode 9): Retentionszeit = 1.27 min

### Beispiel 599 (Referenzbeispiel)

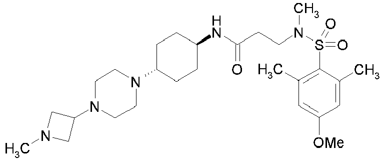

C₂₇H₄₅N₅O₄S x 3HCl (645.13)
[M+H]+ = 536
HPLC (Methode 5): Retentionszeit = 1.14 min

### Beispiel 600 (Referenzbeispiel)

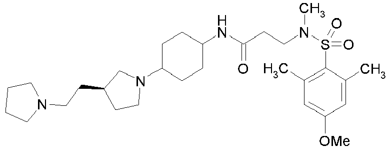

C₂₉H₄₈N₄O₄S x 2HCl (621.70)
[M+H]+ = 549
HPLC (Methode 5): Retentionszeit = 1.16 min

### Beispiel 601 (Referenzbeispiel)

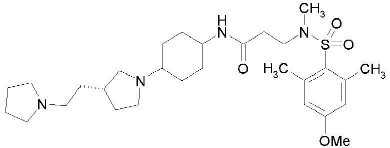

C₂₉H₄₈N₄O₄S (548.78)
[M+H]+ = 549
HPLC (Methode 5): Retentionszeit = 1.16 min

### Beispiel 602 (Referenzbeispiel)

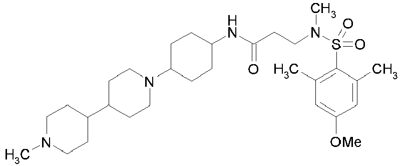

C₃₀H₅₀N₄O₄S x C₂HF₃O₂ (676.83)
[M+H]+ = 563
HPLC (Methode 9): Retentionszeit = 1.14 min

### Beispiel 608 (Referenzbeispiel)

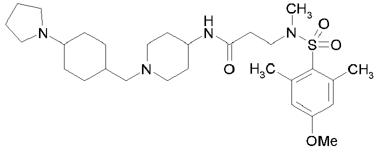

C₂₉H₄₈N₄O₄S x C₂HF₃O₂ (662.81)
[M+H]+ = 549
HPLC (Methode 9): Retentionszeit = 1.30 min

### Beispiel 609 (Referenzbeispiel)

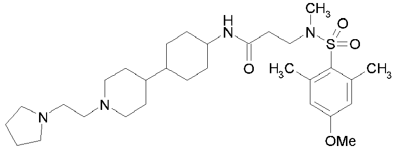

C₃₀H₅₀N₄O₄S x 2HCl (635.73)
[M+H]+ = 563
HPLC (Methode 11): Retentionszeit = 1.70 min

### Beispiel 610 (Referenzbeispiel)

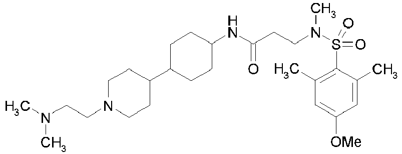

C₂₈H₄₈N₄O₄S x 2HCl (609.69)
[M+H]+ = 537
HPLC (Methode 11): Retentionszeit = 1.67 min

### Beispiel 611 (Referenzbeispiel)

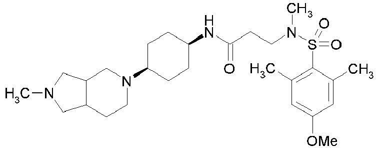

C₂₇H₄₄N₄O₄S x 2HCl (593.65)
[M+H]+ = 521
HPLC (Methode 11): Retentionszeit = 1.61 min

### Beispiel 636 (Referenzbeispiel)

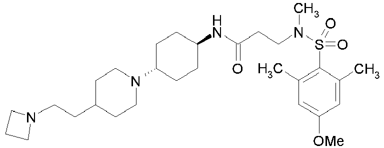

C₂₉H₄₈N₄O₄S x 2HCl (621.70)
[M+H]+ = 549
HPLC (Methode 4): Retentionszeit = 2.39 min

### Beispiel 637 (Referenzbeispiel)

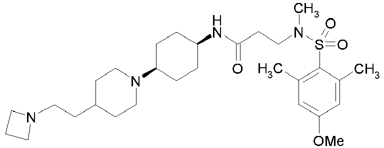

C₂₉H₄₈N₄O₄S x 2HCl (621.70)
[M+H]+ = 549
HPLC (Methode 4): Retentionszeit = 2.34 min

Die folgenden Verbindungen wurden analog zu Beispiel 53 hergestellt:

### Beispiel 318 (Referenzbeispiel)

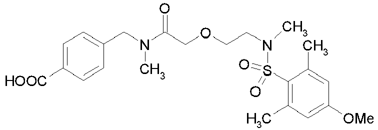

C₂₃H₃₀N₂O₇S (478.56)
[M+H]+ = 479
HPLC (Methode 6): Retentionszeit = 3.21 min

### Beispiel 319 (Referenzbeispiel)

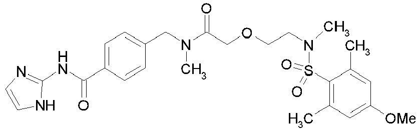

C₂₆H₃₃N₅O₆S (543.64)
[M+H]+ = 544
HPLC (Methode 6): Retentionszeit = 2.51 min

### Beispiel 320 (Referenzbeispiel)

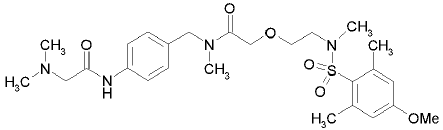

C₂₆H₃₈N₄O₆S (534.67)
[M+H]+ = 535
HPLC (Methode 6): Retentionszeit = 2.48 min

### Beispiel 321 (Referenzbeispiel)

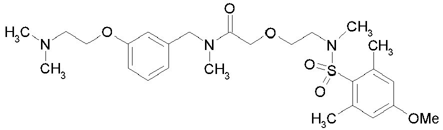

C₂₆H₃₉₈N₃O₆S (521.67)
[M+H]+ = 522
HPLC (Methode 6): Retentionszeit = 2.60 min

### Beispiel 322 (Referenzbeispiel)

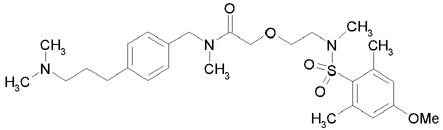

C₂₇H₄₁N₃O₅S (519.70)
[M+H]+ = 520
HPLC (Methode 6): Retentionszeit = 2.61 min

### Beispiel 323 (Referenzbeispiel)

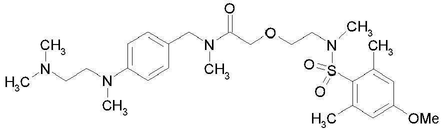

C₂₇H₄₂N₄O₅S x HCl (571.17)
[M+H]+ = 535
HPLC (Methode 5): Retentionszeit = 1.57 min

### Beispiel 324 (Referenzbeispiel)

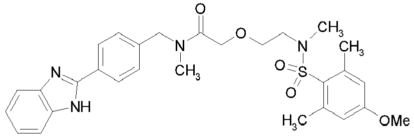

C₂₉H₃₄N₄O₅S x C₂HF₃O₂ (664.69)
[M+H]+ = 551
HPLC (Methode 5): Retentionszeit = 1.60 min

### Beispiel 325 (Referenzbeispiel)

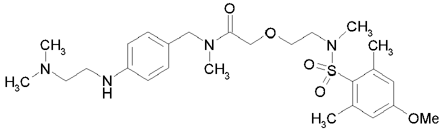

C₂₆H₄₀N₄O₅S x HCl (557.15)
[M+H]+ = 521
HPLC (Methode 5): Retentionszeit = 1.53 min

### Beispiel 326 (Referenzbeispiel)

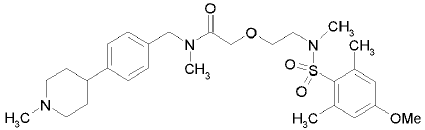

C₂₈H₄₁N₃O₅S x C₂HF₃O₂ (645.73)
[M+H]+ = 532
HPLC (Methode 5): Retentionszeit = 1.54 min

### Beispiel 327 (Referenzbeispiel)

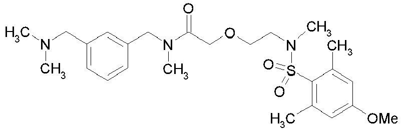

C₂₅H₃₇N₃O₅S x HCl (528.11)
[M+H]+ = 492
HPLC (Methode 5): Retentionszeit = 1.53 min

### Beispiel 328 (Referenzbeispiel)

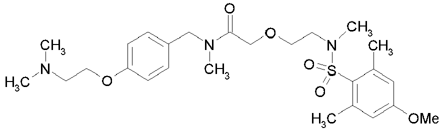

C₂₆H₃₉N₃O₆S x C₂HF₃O₂ (635.69)
[M+H]+ = 522
HPLC (Methode 5): Retentionszeit = 1.53 min

### Beispiel 329 (Referenzbeispiel)

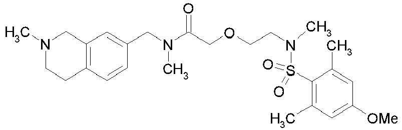

C₂₆H₃₇N₃O₅S x HCl (540.12)
[M+H]+ = 504
HPLC (Methode 7): Retentionszeit = 1.93 min

### Beispiel 330 (Referenzbeispiel)

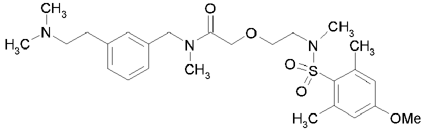

C₂₆H₃₉N₃O₅S x HCl (542.13)
[M+H]+ = 506
HPLC (Methode 5): Retentionszeit = 1.54 min

### Beispiel 331 (Referenzbeispiel)

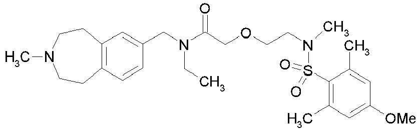

C₂₈H₄₁N₃OₛS x HCl (568.17)
[M+H]+ = 532
DC: Kieselgel, Dichlormethan / Methanol / Ammoniak 9:1:0.1, Rf-Wert = 0.30

### Beispiel 332 (Referenzbeispiel)

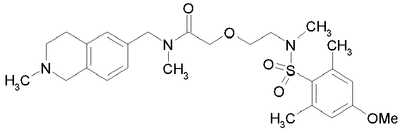

C₂₆H₃₇N₃O₅S x HCl (540.12)
[M+H]+ = 504
HPLC (Methode 5): Retentionszeit = 1.51 min

### Beispiel 333 (Referenzbeispiel)

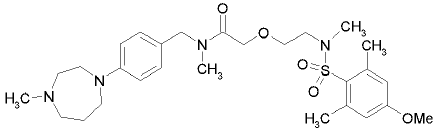

C₂₈H₄₂N₄O₅S x HCl (583.18)
[M+H]+ = 547
HPLC (Methode 5): Retentionszeit = 1.54 min

### Beispiel 334 (Referenzbeispiel)

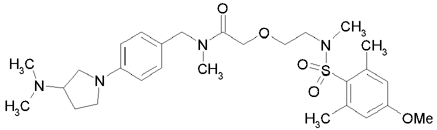

C₂₈H₄₂N₄O₅S x HCl (583.18)
[M+H]+ = 547
HPLC (Methode 10): Retentionszeit = 1.24 min

### Beispiel 335 (Referenzbeispiel)

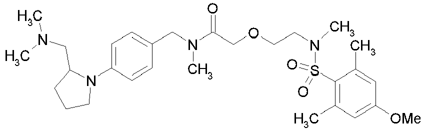

C₂₉H₄₄N₄O₅S x HCl (597.21)
[M+H]+ = 561
HPLC (Methode 5): Retentionszeit = 1.55 min

### Beispiel 336 (Referenzbeispiel)

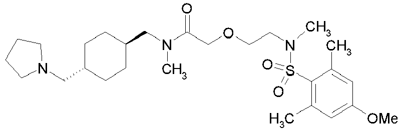

C₂₇H₄₅N₃O₅S x HCl (560.19)
[M+H]+ = 524
DC: Kieselgel, Dichlormethan / Methanol / Ammoniak 8:2:0.01, Rf-Wert = 0.39

### Beispiel 337 (Referenzbeispiel)

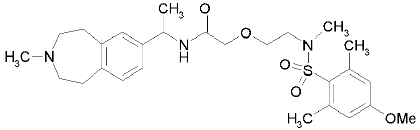

C₂₇H₃₉N₃O₅S x HCl (554.14)
[M+H]+ = 518
HPLC (Methode 4): Retentionszeit = 3.4 min

### Beispiel 338 (Referenzbeispiel)

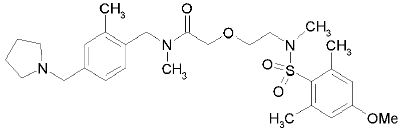

C₂₈H₄₁N₃O₅S x HCl (568.17)
[M+H]+ = 532
DC: Kieselgel, Dichlormethan / Methanol 9:1, Rf-Wert = 0.14

### Beispiel 339 (Referenzbeispiel)

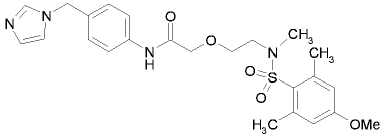

C₂₄H₃₀N₄O₅S X C₂HF₃O₂ (600.61)
[M+H]+ = 487
HPLC (Methode 6): Retentionszeit = 2.58 min

### Beispiel 340 (Referenzbeispiel)

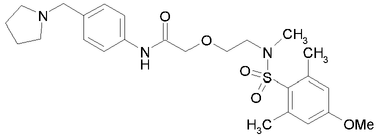

C₂₅H₃₅N₃O₅S x C₂HF₃O₂ (603.65)
[M+H]+ = 490
HPLC (Methode 6): Retentionszeit = 2.61 min

### Beispiel 341 (Referenzbeispiel)

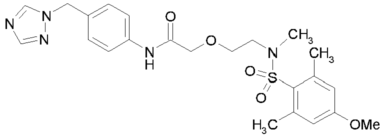

C₂₃H₂₉N₅O₅S X C₂HF₃O₂ (601.60)
[M+H]+ = 488
HPLC (Methode 6): Retentionszeit = 3.28 min

### Beispiel 342 (Referenzbeispiel)

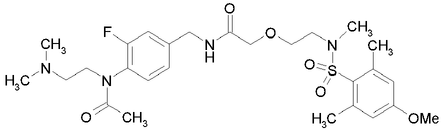

C₂₇H₃₉FN₄O₆S (566.69)
[M+H]+ = 567
HPLC (Methode 6): Retentionszeit = 2.59 min

### Beispiel 343 (Referenzbeispiel)

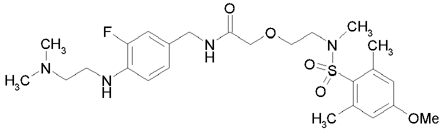

C₂₅H₃₇FN₄O₅S (524.65)
[M+H]+ = 525
HPLC (Methode 6): Retentionszeit = 2.59 min

### Beispiel 344 (Referenzbeispiel)

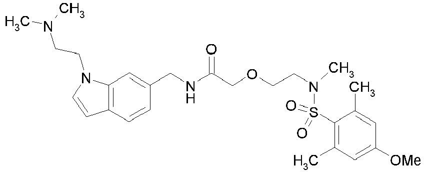

C₂₇H₃₈N₄O₅S (530.68)
[M+H]+ = ₅₃₁
HPLC (Methode 6): Retentionszeit = 2.65 min

### Beispiel 345 (Referenzbeispiel)

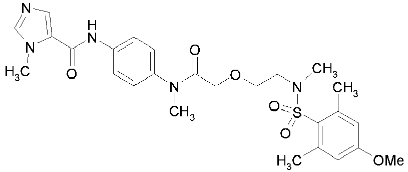

C₂₆H₃₃N₅O₆S (543.64)
[M+H]+ = 544
HPLC (Methode 6): Retentionszeit = 2.39 min

### Beispiel 346 (Referenzbeispiel)

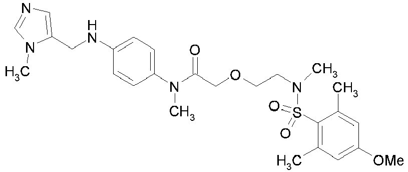

C₂₆H₃₅N₅O₅S (529.65)
[M+H]+ = 530
HPLC (Methode 6): Retentionszeit = 2.43 min

### Beispiel 347 (Referenzbeispiel)

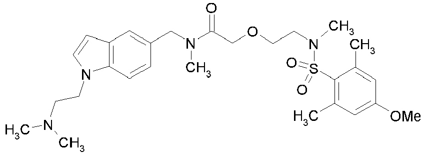

C₂₈H₄₀N₄O₅S (544.71)
[M+H]+ = 545
HPLC (Methode 6): Retentionszeit = 2.65 min

### Beispiel 348 (Referenzbeispiel)

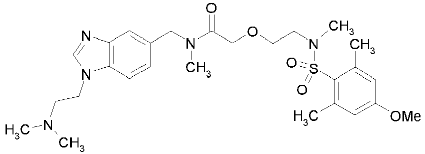

C₂₇H₃₉N₅O₅S (545.70)
[M+H]+ = 546
HPLC (Methode 6): Retentionszeit = 2.19 min

### Beispiel 349 (Referenzbeispiel)

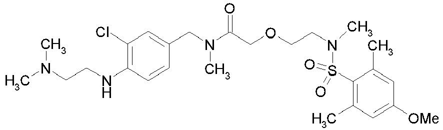

C₂₆H₃₉ClN₄O₅S (555.13)
[M+H]+ = 555/557
HPLC (Methode 6): Retentionszeit = 2.63 min

### Beispiel 350 (Referenzbeispiel)

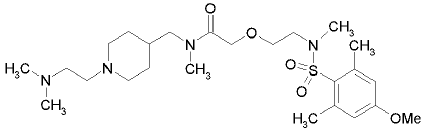

C₂₅H₄₄N₄O₅S (512.71)
[M+H]+ = 513
HPLC (Methode 6): Retentionszeit = 1.94 min

### Beispiel 351 (Referenzbeispiel)

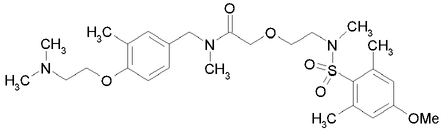

C₂₇H₄₁N₃O₆S (535.70)
[M+H]+ = 536
HPLC (Methode 6): Retentionszeit = 2.56 min

### Beispiel 352 (Referenzbeispiel)

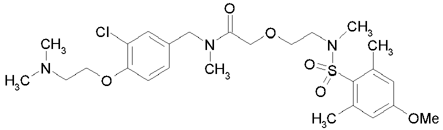

C₂₆H₃₈ClN₃O₆S x CH₂O₂ (602.14)
[M+H]+ = 556/558
HPLC (Methode 6): Retentionszeit = 2.65 min

### Beispiel 353 (Referenzbeispiel)

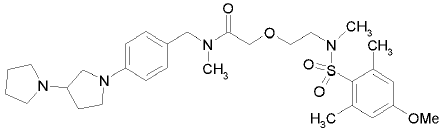

C₃₀H₄₄N₄O₅S (572.76)
[M+H]+ = 573
HPLC (Methode 6): Retentionszeit = 2.69 min

Beispiel 354 (Referenzbeispiel) 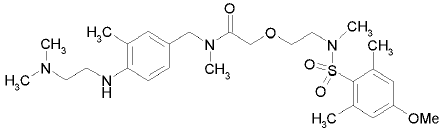 C₂₇H₄₂N₄O₅S (534.71)
[M+H]+ = 535
HPLC (Methode 6): Retentionszeit = 2.54 min

### Beispiel 355 (Referenzbeispiel)

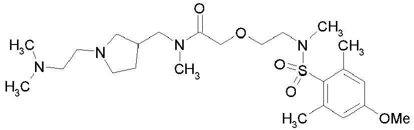

C₂₄H₄₂N₄O₅S (498.68)
[M+H]+ = 499
HPLC (Methode 6): Retentionszeit = 1.95 min

### Beispiel 356 (Referenzbeispiel)

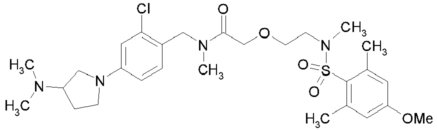

C₂₈H₄₁ClN₄O₅S (581.17)
[M+H]+ = 581/583
HPLC (Methode 6): Retentionszeit = 2.77 min

### Beispiel 357 (Referenzbeispiel)

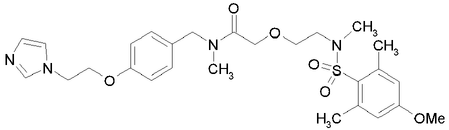

C₂₇H₃₆N₄O₆S (544.66)
[M+H]+ = 545
HPLC (Methode 6): Retentionszeit = 2.59 min

### Beispiel 358 (Referenzbeispiel)

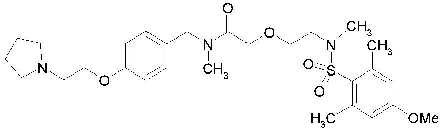

C₂₈H₄₁N₃O₆S (547.71)
[M+H]+ = 548
HPLC (Methode 6): Retentionszeit = 2.59 min

### Beispiel 359 (Referenzbeispiel)

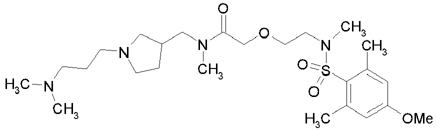

C₂₅H₄₄N₄O₅S (512.71)
[M+H]+ = 513
HPLC (Methode 6): Retentionszeit = 1.94 min

### Beispiel 360 (Referenzbeispiel)

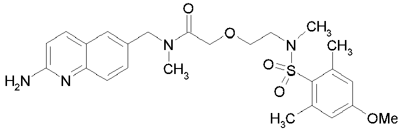

C₂₅H₃₂N₄O₅S (500.61)
[M+H]+ = 501
HPLC (Methode 6): Retentionszeit = 2.41 min

Beispiel 361 (Referenzbeispiel) 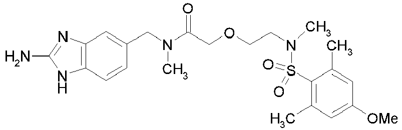 C₂₃H₃₁N₅O₅S (489.59)
[M+H]+ = 490
HPLC (Methode 6): Retentionszeit = 2.46 min

### Beispiel 362 (Referenzbeispiel)

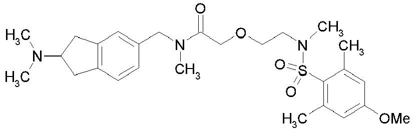

C₂₇H₃₉N₃O₅S X C₂HF₃O₂ (631.71)
[M+H]+ = 518
HPLC (Methode 6): Retentionszeit = 2.54 min

### Beispiel 363 (Referenzbeispiel)

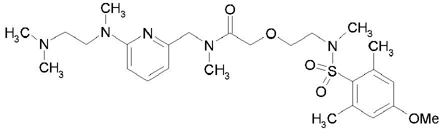

C₂₆H₄₁N₅O₅S x CH₂O₂ (581.73)
[M+H]+ = 536
HPLC (Methode 6): Retentionszeit = 2.58 min

### Beispiel 364 (Referenzbeispiel)

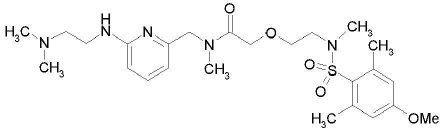

C₂₅H₃₉N₅O₅S x CH₂O₂ (567.70)
[M+H]+ = 522
HPLC (Methode 6): Retentionszeit = 2.31 min

### Beispiel 365 (Referenzbeispiel)

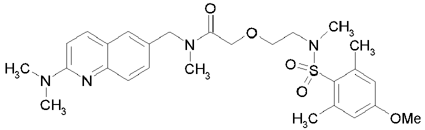

C₂₇H₃₆N₄O₅S x C₂HF₃O₂ (642.69)
[M+H]+ = 529
HPLC (Methode 6): Retentionszeit = 2.56 min

### Beispiel 366 (Referenzbeispiel)

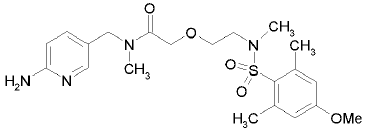

C₂₁H₃₀N₄O₅S (450.55)
[M+H]+ = 451
HPLC (Methode 6): Retentionszeit = 2.31 min

### Beispiel 367 (Referenzbeispiel)

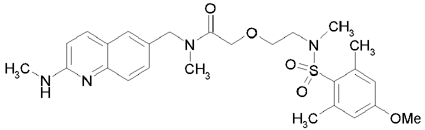

C₂₆H₃₄N₄O₅S (514.64)
[M+H]+ = 515
HPLC (Methode 6): Retentionszeit = 2.51 min

Beispiel 368 (Referenzbeispiel) 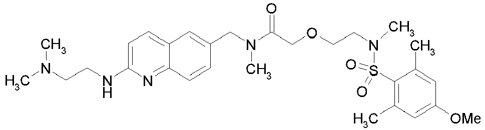 C₂₉H₄₁N₅O₅S x C₂HF₃O₂ (685.76)
[M+H]+ = 572
HPLC (Methode 6): Retentionszeit = 2.14 min

### Beispiel 369 (Referenzbeispiel)

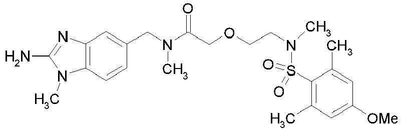

C₂₄H₃₃N₅O₅S x C₂HF₃O₂ (617.64)
[M+H]+ = 504
HPLC (Methode 9): Retentionszeit = 1.61 min

### Beispiel 370 (Referenzbeispiel)

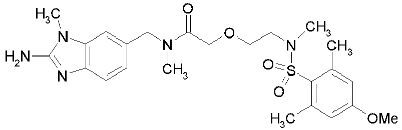

C₂₄H₃₃N₅O₅S x C₂HF₃O₂ (617.64)
[M+H]+ = 504
HPLC (Methode 9): Retentionszeit = 1.59 min

### Beispiel 371 (Referenzbeispiel)

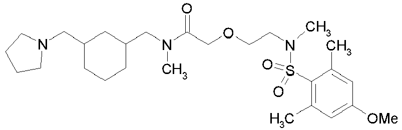

C₂₇H₄₅N₃O₅S x C₂HF₃O₂ (637.75)
[M+H]+ = 524
HPLC (Methode 9): Retentionszeit = 1.70 min

### Beispiel 372 (Referenzbeispiel)

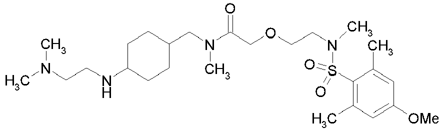

C₂₆H₄₆N₄O₅S (526.73)
[M+H]+ = 527
HPLC (Methode 5): Retentionszeit = 1.43 min

### Beispiel 373 (Referenzbeispiel)

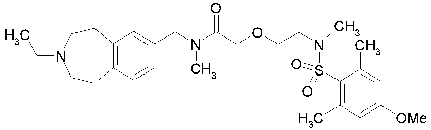

C₂₈H₄₁N₃O₅S x C₂HF₃O₂ (645.73)
[M+H]+ = 532
HPLC (Methode 5): Retentionszeit = 1.56 min

### Beispiel 374 (Referenzbeispiel)

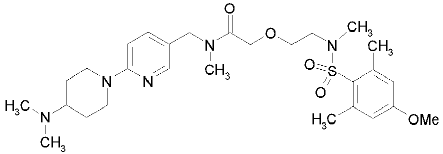

C₂₈H₄₃N₅O₅S x C₂HF₃O₂ (675.76)
[M+H]+ = 562
HPLC (Methode 5): Retentionszeit = 1.41 min

Beispiel 375 (Referenzbeispiel) 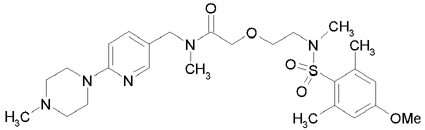 C₂₆H₃₉N₅O₅S x C₂HF₃O₂ (647.71)
[M+H]+ = 534
HPLC (Methode 5): Retentionszeit = 1.42 min

### Beispiel 376 (Referenzbeispiel)

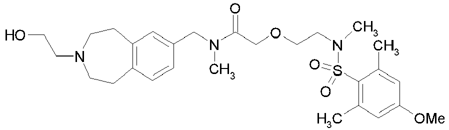

C₂₈H₄₁N₃O₆S (547.71)
[M+H]+ = 548
HPLC (Methode 5): Retentionszeit = 1.53 min

### Beispiel 377 (Referenzbeispiel)

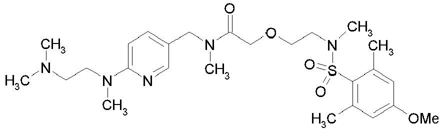

C₂₆H₄₁N₅O₅S (535.70)
[M+H]+ = 536
HPLC (Methode 5): Retentionszeit = 1.42 min

### Beispiel 378 (Referenzbeispiel)

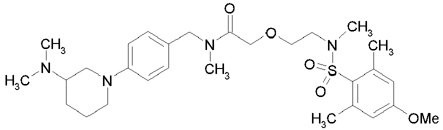

C₂₉H₄₄N₄O₅S x HCl (597.21)
[M+H]+ = 561
HPLC (Methode 7): Retentionszeit = 1.91 min

### Beispiel 379 (Referenzbeispiel)

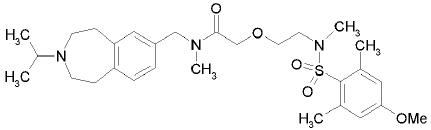

C₂₉H₄₃N₃O₅S x C₂HF₃O₂ (659.76)
[M+H]+ = 546
HPLC (Methode 5): Retentionszeit = 1.59 min

### Beispiel 380 (Referenzbeispiel)

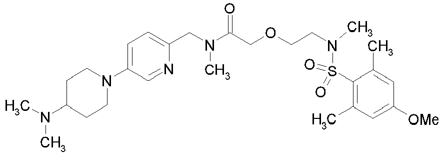

C₂₈H₄₃N₅O₅S x 2C₂HF₃O₂ (789.78)
[M+H]+ = 562
HPLC (Methode 5): Retentionszeit = 1.40 min

### Beispiel 381 (Referenzbeispiel)

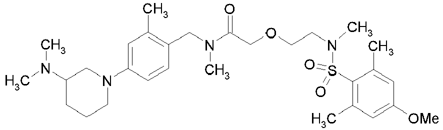

C₃₀H₄₆N₄O₅s x C₂HF₃O₂ (688.80)
[M+H]+ = 575
HPLC (Methode 5): Retentionszeit = 1.56 min

### Beispiel 382 (Referenzbeispiel)

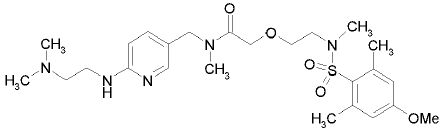

C₂₅H₃₉N₅O₅S x 2HCl (594.60)
[M+H]+ = 522
HPLC (Methode 5): Retentionszeit = 1.40 min

### Beispiel 383 (Referenzbeispiel)

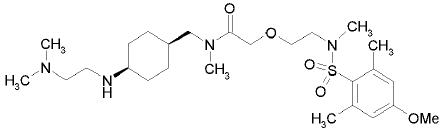

C₂₆H₄₆N₄O₅S x 2HCl (599.65)
[M+H]+ = 527
HPLC (Methode 5): Retentionszeit = 1.41 min

### Beispiel 384 (Referenzbeispiel)

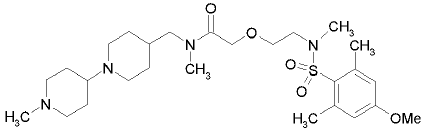

C₂₇H₄₆N₄O₅S x HCl (575.20)
[M+H]+ = 539
DC: Kieselgel, Dichlormethan / Methanol / Ammoniak 8:2:0.01, Rf-Wert = 0.35

### Beispiel 385 (Referenzbeispiel)

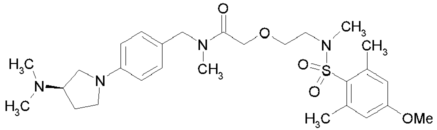

C₂₈H₄₂N₄O₅S x HCl (583.18)
[M+H]+ = 547
DC: Kieselgel, Dichlormethan / Methanol / Ammoniak 9:1:0.1, Rf-Wert = 0.32

### Beispiel 386 (Referenzbeispiel)

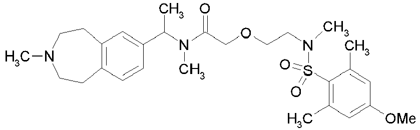

C₂₈H₄₁N₃OₛS x HCl (568.17)
[M+H]+ = 532
DC: Kieselgel, Dichlormethan / Methanol / Ammoniak 9:1:0.1, Rf-Wert = 0.35

### Beispiel 387 (Referenzbeispiel)

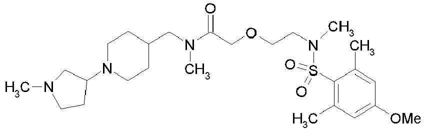

C₂₆H₄₄N₄O₅S x HCl (561.18)
[M+H]+ = 525
DC: Kieselgel, Dichlormethan / Methanol / Ammoniak 8:2:0.01, Rf-Wert = 0.12

### Beispiel 388 (Referenzbeispiel)

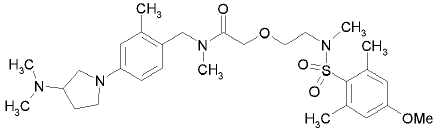

C₂₉H₄₄N₄O₅S x HCl (597.21)
[M+H]+ = 561
DC: Kieselgel, Dichlormethan / Methanol 9:1, Rf-Wert = 0.30

### Beispiel 389 (Referenzbeispiel)

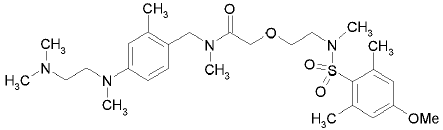

C₂₈H₄₄N₄O₅S x HCl (585.20)
[M+H]+ = 549
DC: Kieselgel, Dichlormethan / Methanol 9:1, Rf-Wert = 0.15

### Beispiel 390 (Referenzbeispiel)

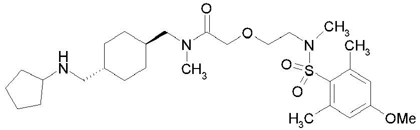

C₂₈H₄₇N₃O₅S x HCl (574.22)
[M+H]+ = 538
DC: Kieselgel, Dichlormethan / Methanol 9:1, Rf-Wert = 0.05

### Beispiel 391 (Referenzbeispiel)

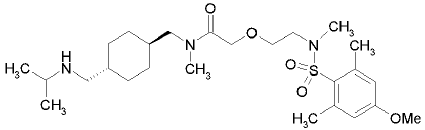

C₂₆H₄₅N₃O₅S x HCl (548.18)
[M+H]+ = 512
HPLC (Methode 5): Retentionszeit = 1.56 min

### Beispiel 392 (Referenzbeispiel)

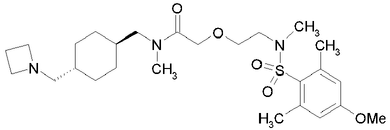

C₂₆H₄₃N₃O₅S x HCl (546.16)
[M+H]+ = 510
DC: Kieselgel, Dichlormethan / Methanol 9:1, Rf-Wert = 0.05

### Beispiel 393 (Referenzbeispiel)

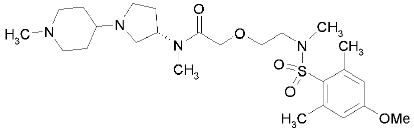

C₂₅H₄₂N₄O₅S x 2HCl (583.61)
[M+H]+ = 511
DC: Kieselgel, Dichlormethan / Ethanol /Ammoniak 9:1:0.1, Rf-Wert = 0.22

### Beispiel 394 (Referenzbeispiel)

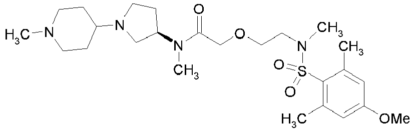

C₂₅H₄₂N₄O₅S x 2HCl (583.61)
[M+H]+ = 511
HPLC (Methode 5): Retentionszeit = 1.37 min

### Beispiel 395 (Referenzbeispiel)

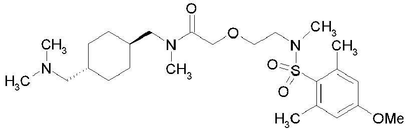

C₂₅H₄₃N₃O₅S x HCl (534.15)
[M+H]+ = 498
DC: Kieselgel, Dichlormethan / Methanol / Ammoniak 8:2:0.01, Rf-Wert = 0.58

### Beispiel 396 (Referenzbeispiel)

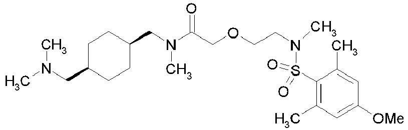

C₂₅H₄₃N₃O₅S x HCl (534.15)
[M+H]+ = 498
DC: Kieselgel, Dichlormethan / Methanol / Ammoniak 8:2:0.01, Rf-Wert = 0.57

### Beispiel 397 (Referenzbeispiel)

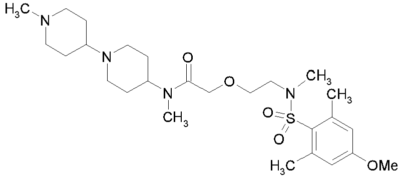

C₂₆H₄₄N₄O₅S x 2HCl (597.64)
[M+H]+ = 525
DC: Kieselgel, Dichlormethan / Methanol / Ammoniak 8:2:0.2, Rf-Wert = 0.52

### Beispiel 398 (Referenzbeispiel)

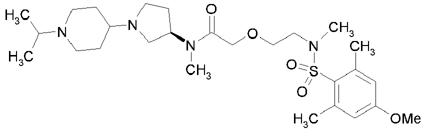

C₂₇H₄₆N₄O₅S x 2HCl (611.67)
[M+H]+ = 539
HPLC (Methode 12): Retentionszeit = 2.45 min

Beispiel 399 (Referenzbeispiel) 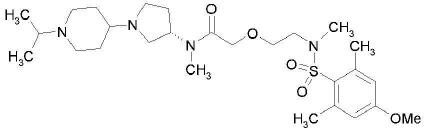 C₂₇H₄₆N₄O₅S x 2HCl (611.67)
[M+H]+ = 539
HPLC (Methode 12): Retentionszeit = 2.35 min

### Beispiel 400 (Referenzbeispiel)

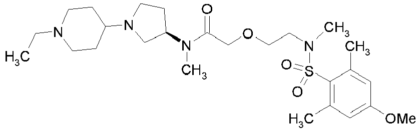

C₂₆H₄₄N₄O₅S x 2HCl (597.64)
[M+H]+ = 525
HPLC (Methode 12): Retentionszeit = 2.3 min

### Beispiel 401 (Referenzbeispiel)

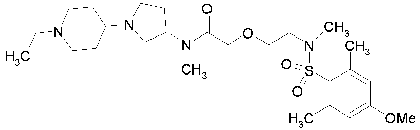

C₂₆H₄₄N₄O₅S x 2HCl (597.64)
[M+H]+ = 525
HPLC (Methode 12): Retentionszeit = 2.3 min

### Beispiel 402 (Referenzbeispiel)

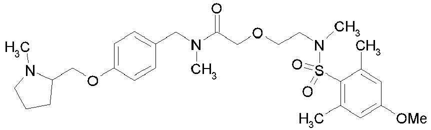

C₂₈H₄₁N₃O₆S (547.71)
[M+H]+ = 548
HPLC (Methode 9): Retentionszeit = 1.7 min

### Beispiel 403 (Referenzbeispiel)

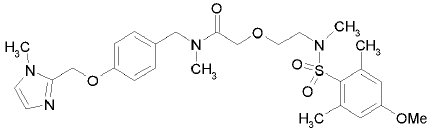

C₂₇H₃₆N₄O₆S (544.66)
[M+H]+ = 545
HPLC (Methode 9): Retentionszeit = 1.69 min

### Beispiel 404 (Referenzbeispiel)

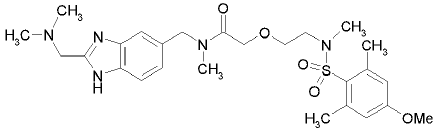

C₂₆H₃₇N₅O₅S (531.67)
[M+H]+ = 532
HPLC (Methode 6): Retentionszeit = 1.56 min

### Beispiel 405 (Referenzbeispiel)

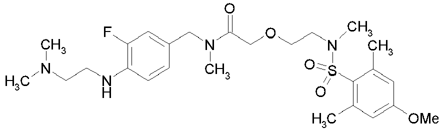

C₂₆H₃₉FN₄O₅S (538.68)
[M+H]+ = 539
HPLC (Methode 6): Retentionszeit = 2.60 min

Beispiel 406 (Referenzbeispiel) 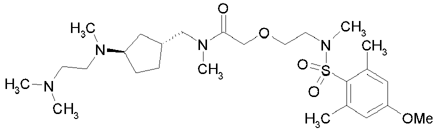 C₂₆H₄₆N₄O₅S x 2HCl (599.65)
[M+H]+ = 527
HPLC (Methode 7): Retentionszeit = 1.78 min

### Beispiel 407 (Referenzbeispiel)

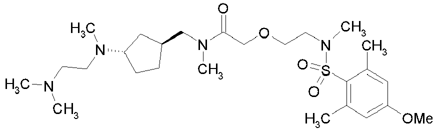

C₂₆H₄₆N₄O₅S x 2HCl (599.65)
[M+H]+ = 527
HPLC (Methode 7): Retentionszeit = 1.77 min

### Beispiel 408 (Referenzbeispiel)

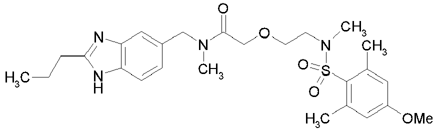

C₂₆H₃₆N₄O₅S (516.65)
[M+H]+ = 517
HPLC (Methode 9): Retentionszeit = 1.65 min

### Beispiel 409 (Referenzbeispiel)

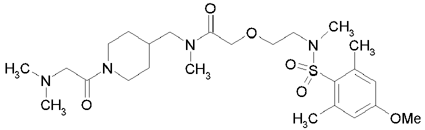

C₂₅H₄₂N₄O₆S x C₂HF₃O₂ (640.71)
[M+H]+ = 527
HPLC (Methode 9): Retentionszeit = 1.55 min

### Beispiel 410 (Referenzbeispiel)

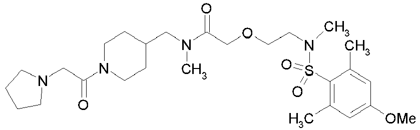

C₂₇H₄₄N₄O₆S x C₂HF₃O₂ (666.75)
[M+H]+ = 553
HPLC (Methode 9): Retentionszeit = 1.57 min

### Beispiel 411 (Referenzbeispiel)

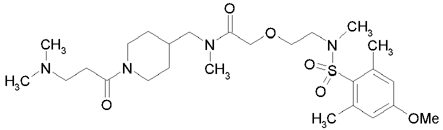

C₂₆H₄₄N₄O₆S x C₂HF₃O₂ (654.74)
[M+H]+ = 541
HPLC (Methode 9): Retentionszeit = 1.57 min

### Beispiel 412 (Referenzbeispiel)

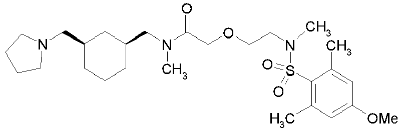

C₂₇H₄₅N₃O₅S x C₂HF₃O₂ (637.75)
[M+H]+ = 524
HPLC (Methode 6): Retentionszeit = 1.71 min

Beispiel 413 (Referenzbeispiel) 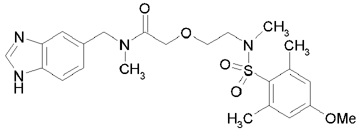 C₂₃H₃₀N₄O₅S (474.57)
[M+H]+ = 475
HPLC (Methode 9): Retentionszeit = 1.53 min

### Beispiel 414 (Referenzbeispiel)

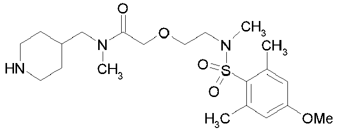

C₂₁H₃₅N₃O₅S (441.59)
[M+H]+ = 442
HPLC (Methode 9): Retentionszeit = 1.48 min

### Beispiel 415 (Referenzbeispiel)

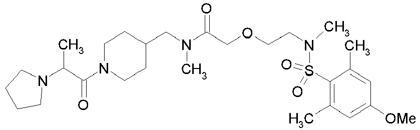

C₂₈H₄₆N₄O₆S x C₂HF₃O₂ (680.78)
[M+H]+ = 567
HPLC (Methode 9): Retentionszeit = 1.60 min

### Beispiel 416 (Referenzbeispiel)

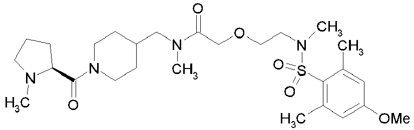

C₂₇H₄₄N₄O₆S x C₂HF₃O₂ (666.75)
[M+H]+ = 553
HPLC (Methode 9): Retentionszeit = 1.57 min

### Beispiel 417 (Referenzbeispiel)

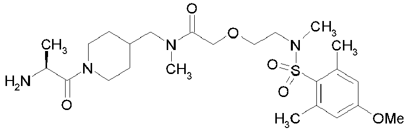

C₂₄H₄₀N₄O6S x C₂HF₃O₂ (626.69)
[M+H]+ = 513
HPLC (Methode 9): Retentionszeit = 1.53 min

### Beispiel 418 (Referenzbeispiel)

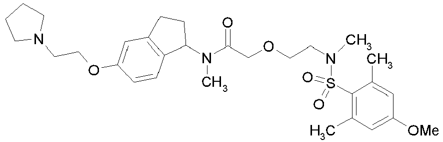

C₃₀H₄₃N₃O₆S x CH₂O₂ (619.77)
[M+H]+ = 574
HPLC (Methode 9): Retentionszeit = 1.73 min

### Beispiel 419 (Referenzbeispiel)

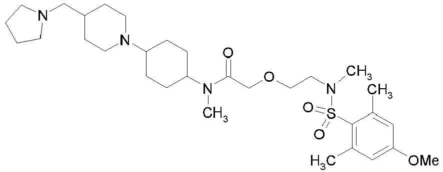

C₃₁H₅₂N₄O₅S x C₂HF₃O₂ (706.86)
[M+H]+ = 593
HPLC (Methode 9): Retentionszeit = 1.34 min

### Beispiel 420 (Referenzbeispiel)

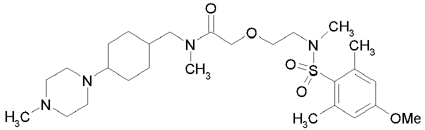

C₂₇H₄₆N₄O₅S (538.74)
[M+H]+ = 539
HPLC (Methode 9): Retentionszeit = 1.43 min

### Beispiel 421 (Referenzbeispiel)

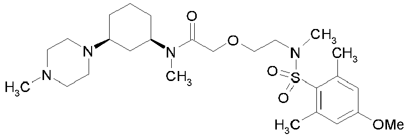

C₂₆H₄₄N₄O₅S x C₂HF₃O₂ (638.74)
[M+H]+ = 525
HPLC (Methode 6): Retentionszeit = 1.53 min

### Beispiel 422 (Referenzbeispiel)

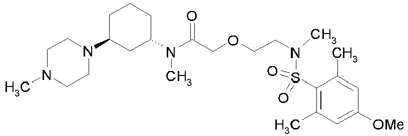

C₂₆H₄₄N₄O₅S x C₂HF₃O₂ (638.74)
[M+H]+ = 525
HPLC (Methode 6): Retentionszeit = 1.41 min

### Beispiel 423 (Referenzbeispiel)

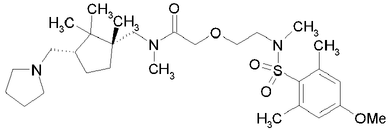

C₂₉H₄₉N₃O₅S x C₂HF₃O₂ (665.81)
[M+H]+ = 552
HPLC (Methode 9): Retentionszeit = 1.74 min

### Beispiel 424 (Referenzbeispiel)

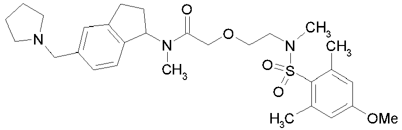

C₂₉H₄₁N₃O₅S (543.72)
[M+H]+ = 544
HPLC (Methode 6): Retentionszeit = 1.73 min

### Beispiel 425 (Referenzbeispiel)

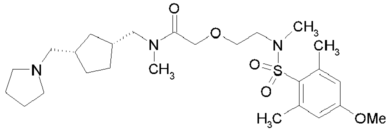

C₂₆H₄₃N₃O₅S x C₂HF₃O₂ (623.73)
[M+H]+ = 510
HPLC (Methode 9): Retentionszeit = 1.63 min

### Beispiel 426 (Referenzbeispiel)

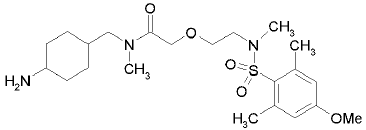

C₂₂H₃₇N₃O₅S (455.61)
[M+H]+ = 456
HPLC (Methode 6): Retentionszeit = 1.55 min

### Beispiel 427 (Referenzbeispiel)

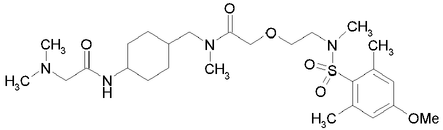

C₂₆H₄₄N₄O₆S (540.72)
[M+H]+ = 541
HPLC (Methode 6): Retentionszeit = 1.59 min

### Beispiel 428 (Referenzbeispiel)

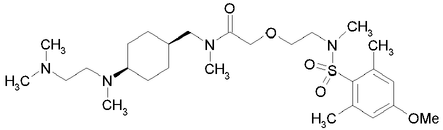

C₂₇H₄₈N₄O₅S x 2HCl (613.68)
[M+H]+ = 541
HPLC (Methode 7): Retentionszeit = 1.65 min

### Beispiel 429 (Referenzbeispiel)

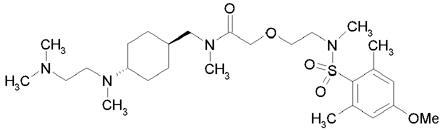

C₂₇H₄₈N₄O₅S x 2HCl (613.68)
[M+H]+ = 541
HPLC (Methode 11): Retentionszeit = 1.64 min

### Beispiel 430 (Referenzbeispiel)

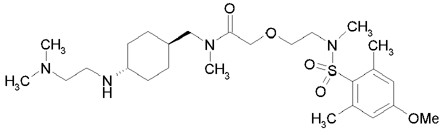

C₂₆H₄₆N₄O₅S x 2C₂HF₃O₂ (754.78)
[M+H]+ = 527
HPLC (Methode 5): Retentionszeit = 1.16 min

### Beispiel 431 (Referenzbeispiel)

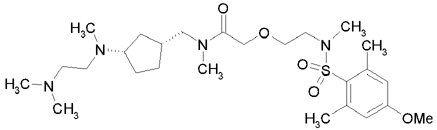

C₂₆H₄₆N₄O₅S x 2HCl (599.65)
[M+H]+ = 527
HPLC (Methode 7): Retentionszeit = 1.82 min

### Beispiel 432 (Referenzbeispiel)

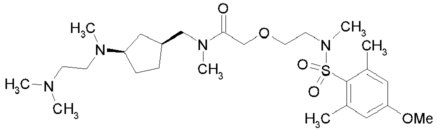

C₂₆H₄₆N₄O₅S x 2HCl (599.65)
[M+H]+ = 527
HPLC (Methode 7): Retentionszeit = 1.82 min

### Beispiel 433 (Referenzbeispiel)

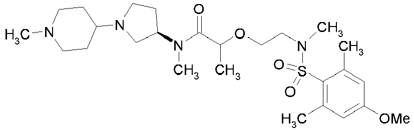

C₂₆H₄₄N₄O₅S x 2HCl (597.64)
[M+H]+ = 525
HPLC (Methode 12): Retentionszeit = 2.3 min

### Beispiel 434 (Referenzbeispiel)

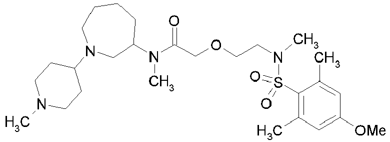

C₂₇H₄₆N₄O₅S x 2HCl (611.67)
[M+H]+ = 539
DC: Kieselgel, Dichlormethan / Methanol / Ammoniak 8:2:0.2, Rf-Wert = 0.65

### Beispiel 435 (Referenzbeispiel)

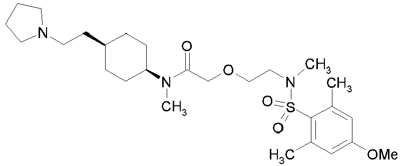

C₂₇H₄₅N₃O₅S (523.73)
[M+H]+ = 524
HPLC (Methode 6): Retentionszeit = 1.29 min

### Beispiel 436 (Referenzbeispiel)

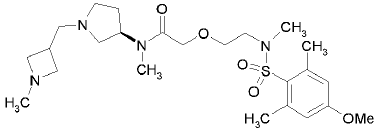

C₂₄H₄₀N₄O₅S x CH₂O₂ (542.69)
[M+H]+ = 497
HPLC (Methode 9): Retentionszeit = 1.25 min

### Beispiel 437 (Referenzbeispiel)

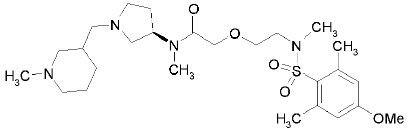

C₂₆H₄₄N₄O₅S x CH₂O₂ (570.74)
[M+H]+ = 525
HPLC (Methode 9): Retentionszeit = 1.31 min

### Beispiel 438 (Referenzbeispiel)

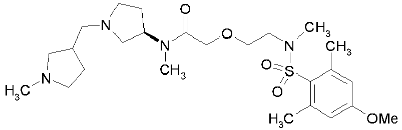

C₂₅H₄₂N₄O₅S x CH₂O₂ (556.72)
[M+H]+ = 511
HPLC (Methode 9): Retentionszeit = 1.31 min

### Beispiel 439 (Referenzbeispiel)

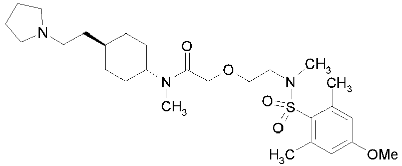

C₂₇H₄₅N₃O₅S (523.73)
[M+H]+ = 524
HPLC (Methode 6): Retentionszeit = 1.67 min

### Beispiel 440 (Referenzbeispiel)

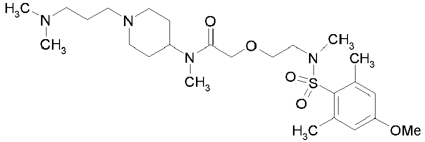

C₂₅H₄₄N₄O₅S x C₂HF₃O₂ (626.73)
[M+H]+ = 513
HPLC (Methode 9): Retentionszeit = 1.29 min

### Beispiel 441 (Referenzbeispiel)

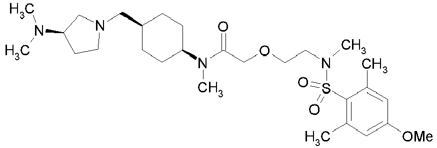

C₂₈H₄₈N₄O₅S x 2 HCl (625.69)
[M+H]+ = 553
HPLC (Methode 9): Retentionszeit = 1.35 min

### Beispiel 442 (Referenzbeispiel)

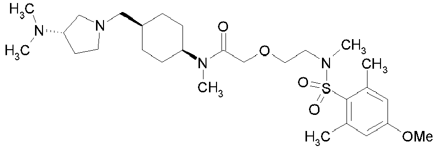

C₂₈H₄₈N₄O₅S x 2 HCl (625.69)
[M+H]+ = 553
HPLC (Methode 9): Retentionszeit = 1.32 min

Beispiel 443 (Referenzbeispiel) 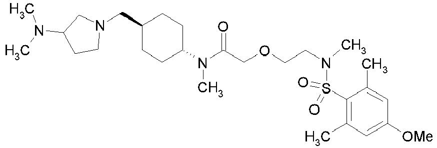 C₂₈H₄₈N₄O₅S (552.77)
[M+H]+ = 553
HPLC (Methode 9): Retentionszeit = 1.37 min

### Beispiel 444 (Referenzbeispiel)

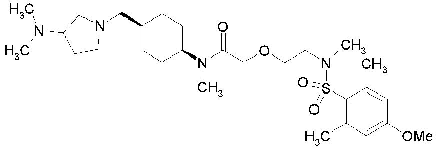

C₂₈H₄₈N₄O₅S x CH₂O₂ (598.80)
[M+H]+ = 553
HPLC (Methode 9): Retentionszeit = 1.35 min

### Beispiel 445 (Referenzbeispiel)

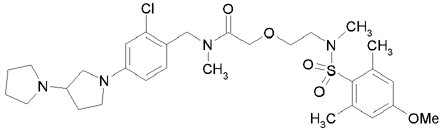

C₃₀H₄₃ClN₄O₅S (607.21)
[M+H]+ = 608
HPLC (Methode 6): Retentionszeit = 1.80 min

### Beispiel 446 (Referenzbeispiel)

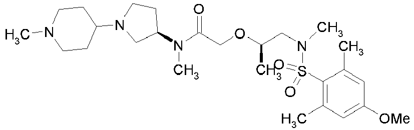

C₂₆H₄₄N₄O₅S x 2HCl (597.64)
[M+H]+ = 525
HPLC (Methode 12): Retentionszeit = 2.4 min

Beispiel 447 (Referenzbeispiel) 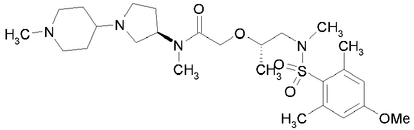 C₂₆H₄₄N₄O₅S x 2HCl (597.64)
[M+H]+ = 525
HPLC (Methode 12): Retentionszeit = 2.4 min

### Beispiel 448 (Referenzbeispiel)

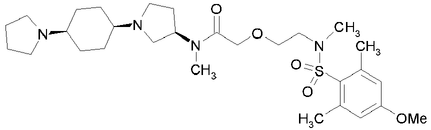

C₂₉H₄₈N₄O₅S x 2HCl (637.70)
[M+H]+ = 565
HPLC (Methode 12): Retentionszeit = 2.3 min

### Beispiel 449 (Referenzbeispiel)

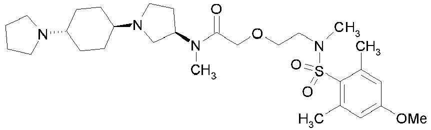

C₂₉H₄₈N₄O₅S x 2HCl (637.70)
[M+H]+ = 565
HPLC (Methode 12): Retentionszeit = 2.92 min

### Beispiel 450 (Referenzbeispiel)

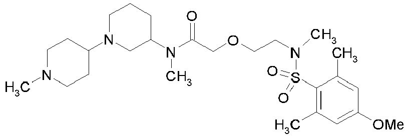

C₂₆H₄₄N₄O₅S x 2HCl (597.64)
[M+H]+ = 525
DC: Kieselgel, Dichlormethan / Methanol / Ammoniak 8:2:0.2, Rf-Wert = 0.60

### Beispiel 451

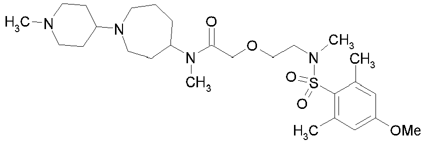

C₂₇H₄₆N₄O₅S x 2HCl (611.67)
[M+H]+ = 539
DC: Kieselgel, Dichlormethan / Methanol / Ammoniak 8:2:0.2, Rf-Wert = 0.62

### Beispiel 452

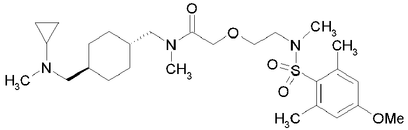

C₂₇H₄₅N₃O₅S x HCl (560.19)
[M+H]+ = 524
HPLC (Methode 12): Retentionszeit = 3.01 min

### Beispiel 453

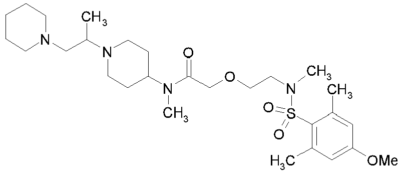

C₂₈H₄₈N₄O₅S x 2HCl (625.69)
[M+H]+ = 553
HPLC (Methode 12): Retentionszeit = 2.45 min

### Beispiel 454

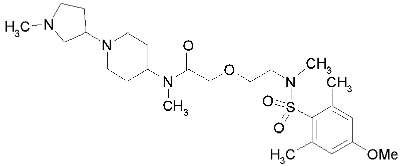

C₂₅H₄₂N₄O₅S x 2HCl (583.61)
[M+H]+ = 511
HPLC (Methode 12): Retentionszeit = 2.33 min

### Beispiel 455

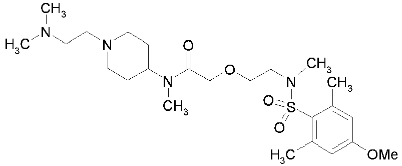

C₂₄H₄₂N₄O₅S x C₂HF₃O₂ (612.70)
[M+H]+ = 499
HPLC (Methode 9): Retentionszeit = 1.32 min

### Beispiel 456

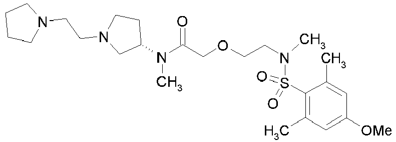

C₂₅H₄₂N₄O₅S x 2C₂HF₃O₂ (738.74)
[M+H]+ = 511
HPLC (Methode 9): Retentionszeit = 1.24 min

### Beispiel 457

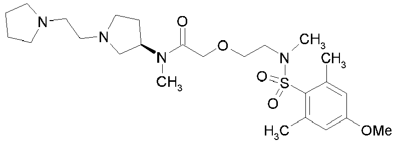

C₂₅H₄₂N₄O₅S x 2C₂HF₃O₂ (738.74)
[M+H]+ = 511
HPLC (Methode 9): Retentionszeit = 1.27 min

### Beispiel 458

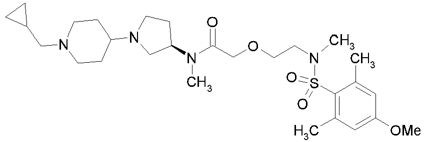

C₂₈H₄₆N₄O₅S x CH₂O₂ (596.78)
[M+H]+ = 551
HPLC (Methode 6): Retentionszeit = 1.31 min

### Beispiel 459

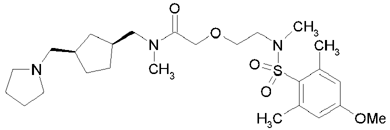

C₂₆H₄₃N₃O₅S x C₂HF₃O₂ (623.73)
[M+H]+ = 510
HPLC (Methode 9): Retentionszeit = 1.62 min

### Beispiel 460

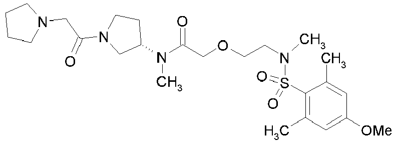

C₂₅H₄₀N₄O₆S x C₂HF₃O₂ (638.70)
[M+H]+ = 525
HPLC (Methode 9): Retentionszeit = 1.49 min

### Beispiel 461

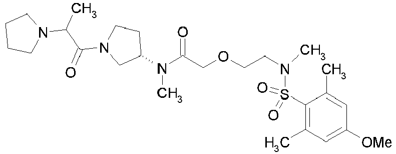

C₂₆H₄₂N₄O₆S x C₂HF₃O₂ (652.72)
[M+H]+ = 539
HPLC (Methode 9): Retentionszeit = 1.51 min

### Beispiel 462

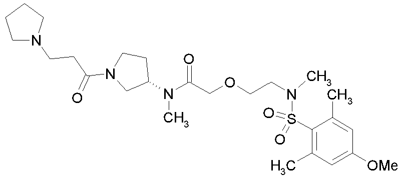

C₂₆H₄₂N₄O₆S x C₂HF₃O₂ (652.72)
[M+H]+ = 539
HPLC (Methode 9): Retentionszeit = 1.52 min

### Beispiel 463

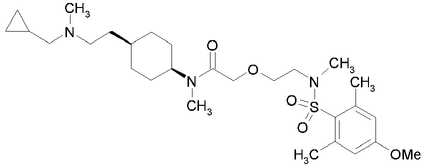

C₂₈H₄₇N₃O₅S (537.76)
[M+H]+ = 538
HPLC (Methode 9): Retentionszeit = 1.71 min

### Beispiel 464

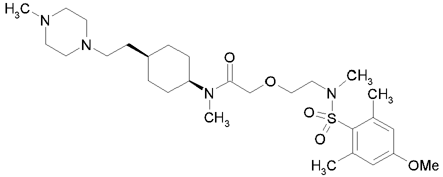

C₂₈H₄₈N₄O₅S (552.77)
[M+H]+ = 553
HPLC (Methode 9): Retentionszeit = 1.39 min

### Beispiel 465

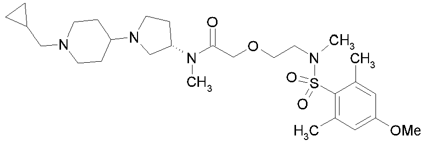

C₂₈H₄₆N₄O₅S x CH₂O₂ (596.78)
[M+H]+ = 551
HPLC (Methode 6): Retentionszeit = 1.32 min

### Beispiel 466

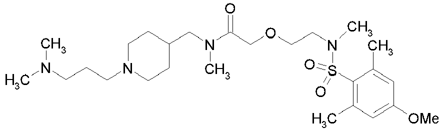

C₂₆H₄₆N₄O₅S (526.73)
[M+H]+ = 527
HPLC (Methode 9): Retentionszeit = 1.27 min

### Beispiel 467

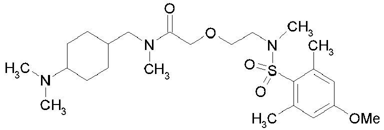

C₂₄H₄₁N₃O₅S (483.67)
[M+H]+ = 484
HPLC (Methode 6): Retentionszeit = 1.60 min

### Beispiel 468

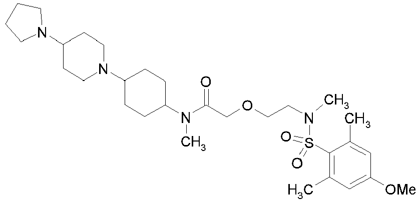

C₃₀H₅₀N₄O₅S x CH₂O₂ (624.83)
[M+H]+ = 579
HPLC (Methode 6): Retentionszeit = 1.31 min

### Beispiel 469

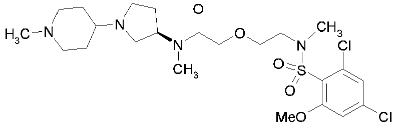

C₂₃H₃₆Cl₂N₄O₅S (551.53)
[M+H]+ = 551/553/555
HPLC (Methode 9): Retentionszeit = 1.30 min

### Beispiel 470

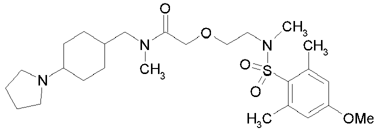

C₂₆H₄₃N₃O₅S (509.70)
[M+H]+ = 510
HPLC (Methode 6): Retentionszeit = 1.67 min

### Beispiel 471

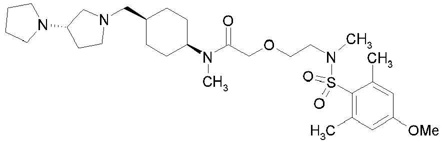

C₃₀H₅₀N₄O₅S x C₂HF₃O₂ (692.83)
[M+H]+ = 579
HPLC (Methode 9): Retentionszeit = 1.39 min

### Beispiel 472

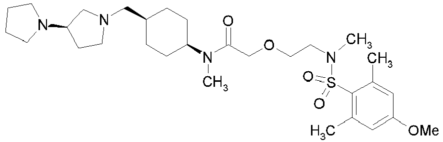

C₃₀H₅₀N₄O₅S x C₂HF₃O₂ (692.83)
[M+H]+ = 579
HPLC (Methode 9): Retentionszeit = 1.39 min

### Beispiel 473

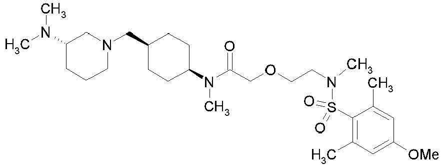

C₂₉H₅₀N₄O₅S x C₂HF₃O₂ (680.82)
[M+H]+ = 567
HPLC (Methode 9): Retentionszeit = 1.40 min

### Beispiel 474

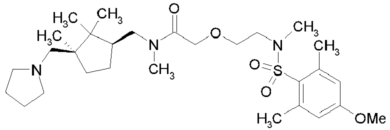

C₂₉H₄₉N₃O₅S (551.78)
[M+H]+ = 552
HPLC (Methode 9): Retentionszeit = 1.72 min

### Beispiel 475

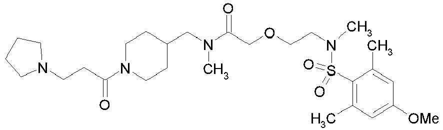

C₂₈H₄₆N₄O₆S x C₂HF₃O₂ (680.78)
[M+H]+ = 567
HPLC (Methode 9): Retentionszeit = 1.60 min

Beispiel 476 (Referenzbeispiel) 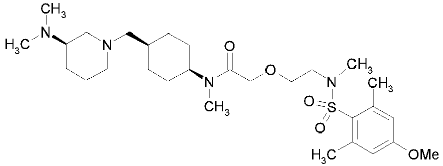 C₂₉H₅₀N₄O₅S x C₂HF₃O₂ (680.82)
[M+H]+ = 567
HPLC (Methode 9): Retentionszeit = 1.41 min

### Beispiel 477

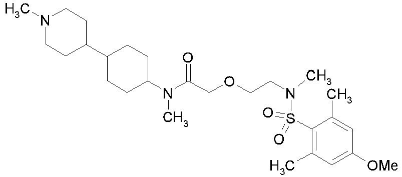

C₂₇H₄₅N₃O₅S x C₂HF₃O₂ (637.75)
[M+H]+ = 524
HPLC (Methode 9): Retentionszeit = 1.69 min

### Beispiel 478

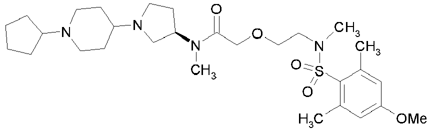

C₂₉H₄₈N₄O₅S x 2HCl (637.70)
[M+H]+ = 565
HPLC (Methode 12): Retentionszeit = 2.4 min

### Beispiel 479

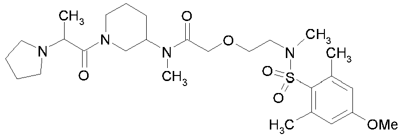

C₂₇H₄₄N₄O₆S x C₂HF₃O₂ (666.75)
[M+H]+ = 553
HPLC (Methode 9): Retentionszeit = 1.61 min

### Beispiel 480

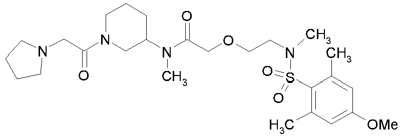

C₂₆H₄₂N₄O₆S x C₂HF₃O₂ (652.72)
[M+H]+ = 539
HPLC (Methode 9): Retentionszeit = 1.60 min

### Beispiel 481

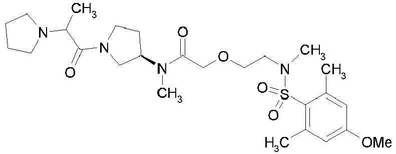

C₂₆H₄₂N₄O₆S (538.70)
[M+H]+ = 539
HPLC (Methode 9): Retentionszeit = 1.47 min

### Beispiel 482

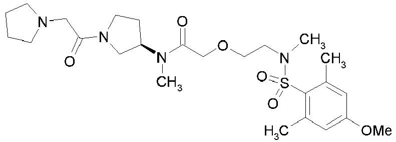

C₂₅H₄₀N₄O₆S (524.67)
[M+H]+ = 525
HPLC (Methode 9): Retentionszeit = 1.48 min

### Beispiel 575 (Referenzbeispiel)

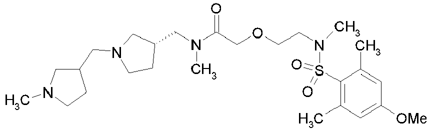

C₂₆H₄₄N₄O₅S (524.72)
[M+H]+ = 525
HPLC (Methode 9): Retentionszeit = 1.30 min

### Beispiel 576 (Referenzbeispiel)

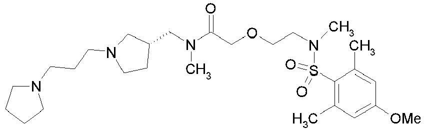

C₂₇H₄₆N₄O₅S (538.74)
[M+H]+ = 539
HPLC (Methode 9): Retentionszeit = 1.34 min

### Beispiel 577 (Referenzbeispiel)

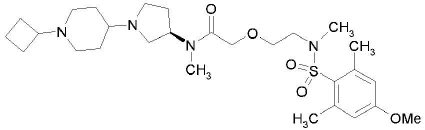

C₂₈H₄₆N₄O₅S x 2HCl (623.68)
[M+H]+ = 551
DC: Kieselgel, Dichlormethan / Methanol / Ammoniak 8:2:0.2, Rf-Wert = 0.71

Beispiel 578 (Referenzbeispiel) 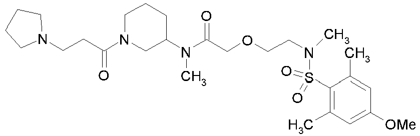 C₂₇H₄₄N₄O₆S x C₂HF₃O₂ (666.75)
[M+H]+ = 553
HPLC (Methode 9): Retentionszeit = 1.62 min

### Beispiel 579 (Referenzbeispiel)

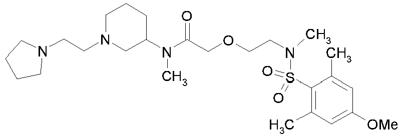

C₂₆H₄₄N₄O₅S x 2C₂HF₃O₂ (752.76)
[M+H]+ = 525
HPLC (Methode 9): Retentionszeit = 1.33 min

### Beispiel 580 (Referenzbeispiel)

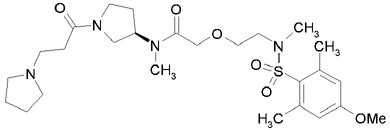

C₂₆H₄₂N₄O₆S x C₂HF₃O₂ (652.72)
[M+H]+ = 539
HPLC (Methode 9): Retentionszeit = 1.50 min

Beispiel 581 (Referenzbeispiel) 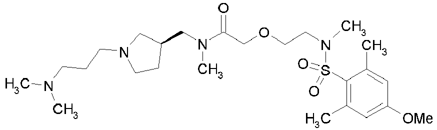 C₂₅H₄₄N₄O₅S (512.71)
[M+H]+ = 513
HPLC (Methode 9): Retentionszeit = 1.27 min

### Beispiel 582 (Referenzbeispiel)

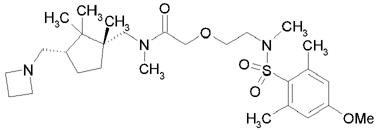

C₂₈H₄₇N₃O₅S x C₂HF₃O₂ (651.78)
[M+H]+ = 538
HPLC (Methode 9): Retentionszeit = 1.71 min

### Beispiel 583 (Referenzbeispiel)

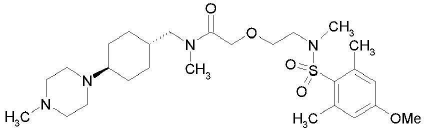

C₂₇H₄₆N₄O₅S (538.74)
[M+H]+ = 539
HPLC (Methode 9): Retentionszeit = 1.37 min

### Beispiel 584 (Referenzbeispiel)

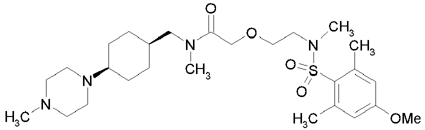

C₂₇H₄₆N₄O₅S (538.74)
[M+H]+ = 539
HPLC (Methode 9): Retentionszeit = 1.44 min

Beispiel 585 (Referenzbeispiel) 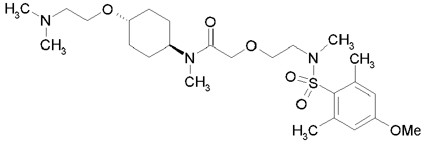 C₂₅H₄₃N₃O₆S x HCl (550.15)
[M+H]+ = 514
HPLC (Methode 5): Retentionszeit = 1.34 min

### Beispiel 586 (Referenzbeispiel)

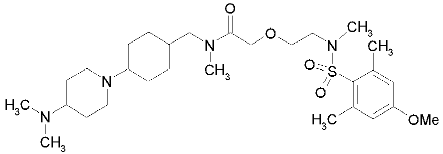

C₂₉H₅₀N₄O₅S x 2HCl (639.72)
[M+H]+ = 567
HPLC (Methode 5): Retentionszeit = 1.21 min

### Beispiel 587 (Referenzbeispiel)

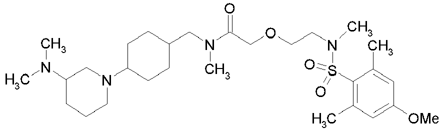

C₂₉H₅₀N₄O₅S x 2HCl (639.72)
[M+H]+ = 567
HPLC (Methode 5): Retentionszeit = 1.20 min

### Beispiel 588 (Referenzbeispiel)

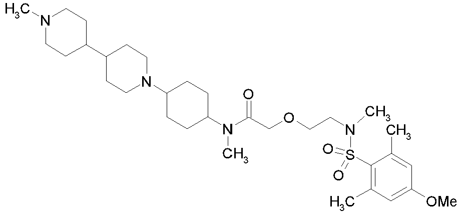

C₃₂H₅₄N₄O₅S x C₂HF₃O₂ (720.88)
[M+H]+ = 607
HPLC (Methode 9): Retentionszeit = 1.37 min

### Beispiel 589 (Referenzbeispiel)

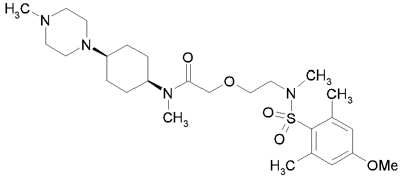

C₂₆H₄₄N₄O₅S x 2C₂HF₃O₂ (752.76)
[M+H]+ = 525
HPLC (Methode 9): Retentionszeit = 1.41 min

### Beispiel 590 (Referenzbeispiel)

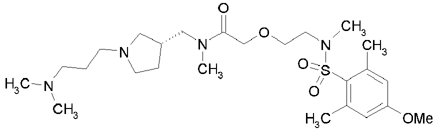

C₂₅H₄₄N₄O₅S (512.71)
[M+H]+ = 513
HPLC (Methode 9): Retentionszeit = 1.35 min

### Beispiel 591 (Referenzbeispiel)

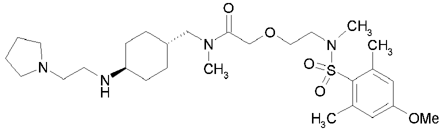

C₂₈H₄₈N₄O₅S x 2C₂HF₃O₂ (780.82)
[M+H]+ = 553
HPLC (Methode 9): Retentionszeit = 1.40 min

### Beispiel 592 (Referenzbeispiel)

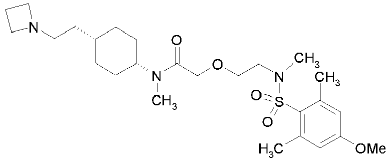

C₂₆H₄₃N₃O₅S (509.70)
[M+H]+ = 510
HPLC (Methode 9): Retentionszeit = 1.70 min

### Beispiel 612 (Referenzbeispiel)

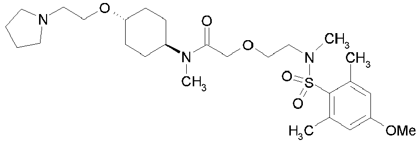

C₂₇H₄₅N₃O₆S x HCl (576.19)
[M+H]+ = 540
HPLC (Methode 5): Retentionszeit = 1.37 min

### Beispiel 613 (Referenzbeispiel)

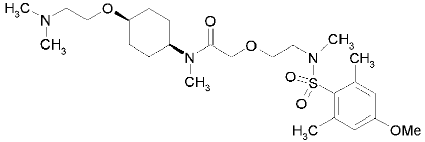

C₂₅H₄₃N₃O₆S x HCl (550.15)
[M+H]+ = 514
HPLC (Methode 5): Retentionszeit = 1.36 min

### Beispiel 614 (Referenzbeispiel)

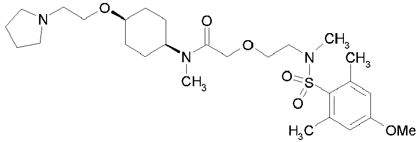

C₂₇H₄₅N₃O₆S x HCl (576.19)
[M+H]+ = 540
HPLC (Methode 5): Retentionszeit = 1.38 min

### Beispiel 615 (Referenzbeispiel)

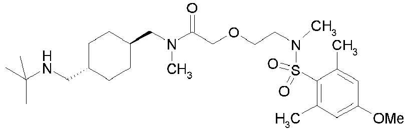

C₂₇H₄₇N₃O₅S x HCl (562.21)
[M+H]+ = 526
HPLC (Methode 4): Retentionszeit = 3.0 min

### Beispiel 616 (Referenzbeispiel)

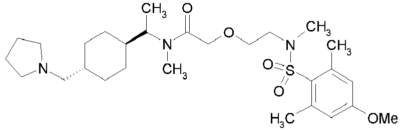

C₂₈H₄₇N₃O₅S x HCl (574.22)
[M+H]+ = 538
HPLC (Methode 4): Retentionszeit = 3.1 min

### Beispiel 617 (Referenzbeispiel)

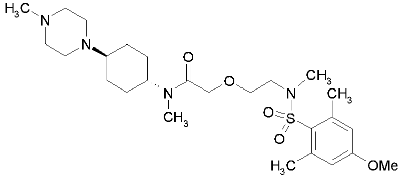

C₂₆H₄₄N₄O₅S (524.72)
[M+H]+ = 525
HPLC (Methode 9): Retentionszeit = 1.32 min

### Beispiel 618 (Referenzbeispiel)

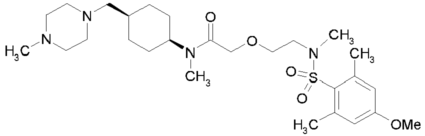

C₂₇H₄₆N₄O₅S (538.74)
[M+H]+ = 539
HPLC (Methode 9): Retentionszeit = 1.57 min

### Beispiel 619 (Referenzbeispiel)

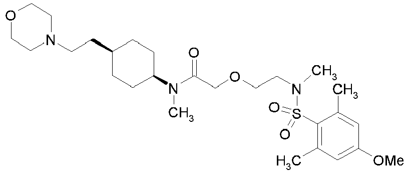

C₂₇H₄₅N₃O₆S (539.73)
[M+H]+ = 540
HPLC (Methode 9): Retentionszeit = 1.65 min

### Beispiel 620 (Referenzbeispiel)

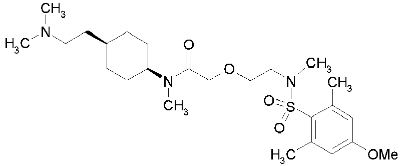

C₂₅H₄₃N₃O₅S (497.69)
[M+H]+ = 498
HPLC (Methode 9): Retentionszeit = 1.65 min

### Beispiel 621 (Referenzbeispiel)

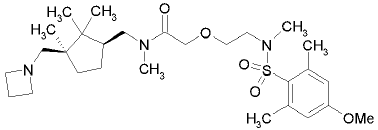

C₂₈H₄₇N₃O₅S x HCl (574.22)
[M+H]+ = 538
HPLC (Methode 9): Retentionszeit = 1.68 min

### Beispiel 622 (Referenzbeispiel)

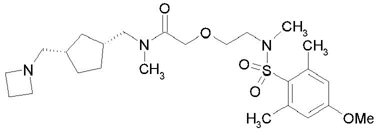

C₂₅H₄₁N₃O₅S x C₂HF₃O₂ (609.70)
[M+H]+ = 496
HPLC (Methode 9): Retentionszeit = 1.62 min

### Beispiel 623 (Referenzbeispiel)

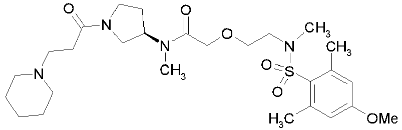

C₂₇H₄₄N₄O₆S x C₂HF₃O₂ (666.76)
[M+H]+ = 553
HPLC (Methode 9): Retentionszeit = 1.57 min

### Beispiel 624 (Referenzbeispiel)

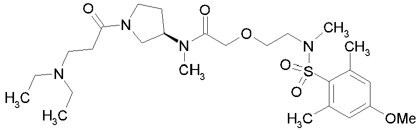

C₂₆H₄₄N₄O₆S x C₂HF₃O₂ (654.75)
[M+H]+ = 541
HPLC (Methode 9): Retentionszeit = 1.56 min

Beispiel 625 (Referenzbeispiel) 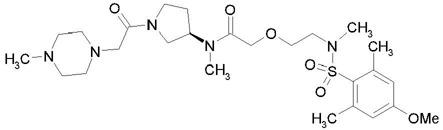 C₂₆H₄₃N₅O₆S x C₂HF₃O₂ (667.74)
[M+H]+ = 554
HPLC (Methode 9): Retentionszeit = 1.00 min

### Beispiel 626 (Referenzbeispiel)

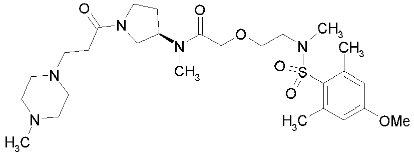

C₂₇H₄₅N₅O₆S x C₂HF₃O₂ (681.77)
[M+H]+ = 568
HPLC (Methode 9): Retentionszeit = 1.03 min

### Beispiel 627 (Referenzbeispiel)

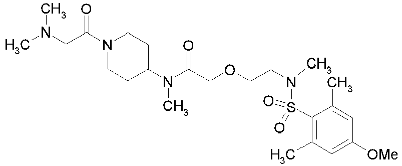

C₂₄H₄₀N₄O₆S x C₂HF₃O₂ (626.69)
[M+H]+ = 513
HPLC (Methode 9): Retentionszeit = 1.50 min

### Beispiel 628 (Referenzbeispiel)

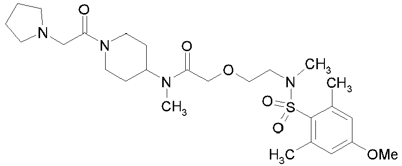

C₂₆H₄₂N₄O₆S x C₂HF₃O₂ (654.72)
[M+H]+ = 539
HPLC (Methode 9): Retentionszeit = 1.55 min

### Beispiel 629 (Referenzbeispiel)

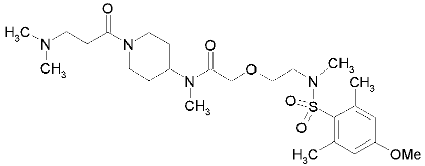

C₂₅H₄₂N₄O₆S x C₂HF₃O₂ (640.71)
[M+H]+ = 527
HPLC (Methode 9): Retentionszeit = 1.63 min

### Beispiel 630 (Referenzbeispiel)

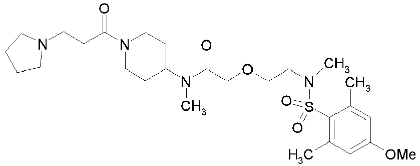

C₂₇H₄₄N₄O₆S (552.73)
[M+H]+ = 553
HPLC (Methode 9): Retentionszeit = 1.65 min

### Beispiel 638 (Referenzbeispiel)

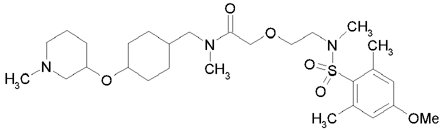

C₂₈H₄₇N₃O₆S x HCl (590.22)
[M+H]+ = 554
HPLC (Methode 5): Retentionszeit = 1.44 min

### Beispiel 639 (Referenzbeispiel)

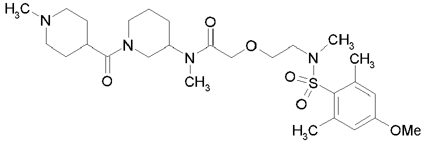

C₂₇H₄₄N₄O₆S x C₂HF₃O₂ (666.75)
[M+H]+ = 553
HPLC (Methode 9): Retentionszeit = 1.59 min

### Beispiel 640 (Referenzbeispiel)

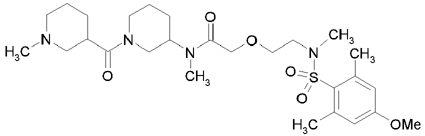

C₂₇H₄₄N₄O₆S x C₂HF₃O₂ (666.75)
[M+H]+ = 553
HPLC (Methode 9): Retentionszeit = 1.61 min

### Beispiel 641 (Referenzbeispiel)

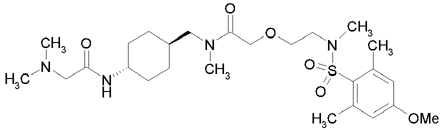

C₂₆H₄₄N₄O₆S x C₂HF₃O₂ (590.22)
[M+H]+ = 541
HPLC (Methode 9): Retentionszeit = 1.56 min

### Beispiel 642 (Referenzbeispiel)

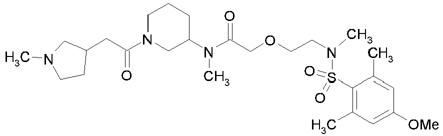

C₂₇H₄₄N₄O₆S x C₂HF₃O₂ (666.75)
[M+H]+ = 553
HPLC (Methode 9): Retentionszeit = 1.55 min

### Beispiel 643 (Referenzbeispiel)

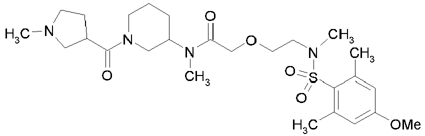

C₂₆H₄₂N₄O₆S x C₂HF₃O₂ (652.72)
[M+H]+ = 539
HPLC (Methode 9): Retentionszeit = 1.55 min

### Beispiel 644 (Referenzbeispiel)

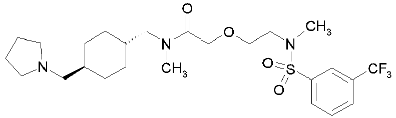

C₂₅H₃₈F₃N₃O₄S (533.66)
[M+H]+ = 534

### Beispiel 645 (Referenzbeispiel)

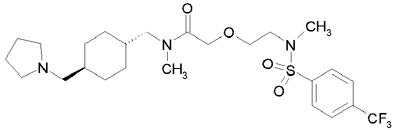

C₂₅H₃₈F₃N₃O₄S (533.66)
[M+H]+ = 534

### Beispiel 646 (Referenzbeispiel)

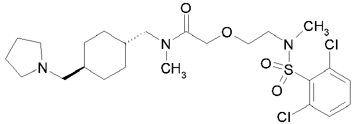

C₂₄H₃₇Cl₂N₃O₄S (534.55)
[M+H]+ = 535

### Beispiel 647 (Referenzbeispiel)

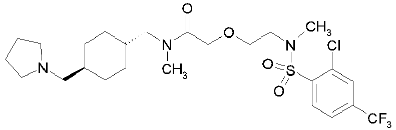

C₂₅H₃₇ClF₃N₃O₄S (568.10)
[M+H]+ = 569

Die folgenden Verbindungen wurden analog zu Beispiel 121 hergestellt:

### Beispiel 483 (Referenzbeispiel)

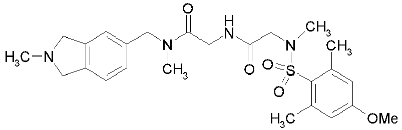

C₂₅H₃₄N₄O₅S x C₂HF₃O₂ (616.65)
[M+H]+ = 503
HPLC (Methode 6): Retentionszeit = 2.30 min

### Beispiel 484 (Referenzbeispiel)

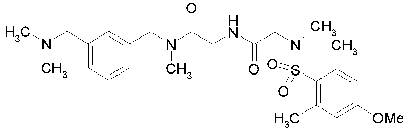

C₂₅H₃₆N₄O₅S x HCl (541.10)
[M+H]+ = 505
HPLC (Methode 5): Retentionszeit = 1.49 min

### Beispiel 485 (Referenzbeispiel)

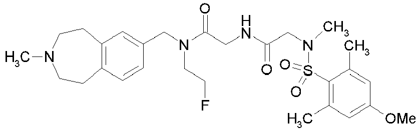

C₂₈H₃₉FN₄O₅S x C₂HF₃O₂ (676.72)
[M+H]+ = 563
HPLC (Methode 5): Retentionszeit = 1.55 min

### Beispiel 486 (Referenzbeispiel)

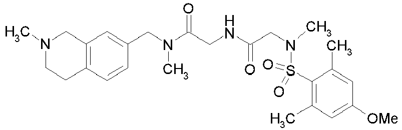

C₂₆H₃₆N₄O₅S x HCl (553.11)
[M+H]+ = 517
HPLC (Methode 11): Retentionszeit = 1.73 min

### Beispiel 487 (Referenzbeispiel)

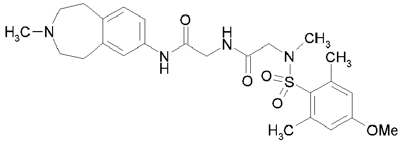

C₂₅H₃₄N₄O₅S x C₂HF₃O₂ (616.65)
[M+H]+ = 503
HPLC (Methode 5): Retentionszeit = 1.49 min

### Beispiel 488 (Referenzbeispiel)

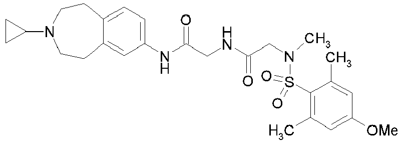

C₂₇H₃₆N₄O₅S x C₂HF₃O₂ (642.69)
[M+H]+ = 529
HPLC (Methode 5): Retentionszeit = 1.50 min

### Beispiel 489 (Referenzbeispiel)

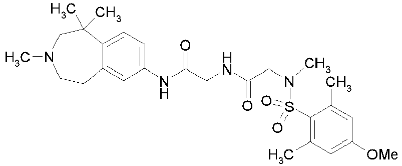

C₂₇H₃₈N₄O₅S x C₂HF₃O₂ (644.70)
[M+H]+ = 531
HPLC (Methode 5): Retentionszeit = 1.54 min

### Beispiel 490 (Referenzbeispiel)

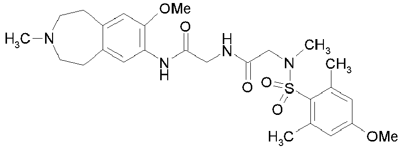

C₂₆H₃₆N₄O₆S x C₂HF₃O₂ (646.68)
[M+H]+ = 533
HPLC (Methode 5): Retentionszeit = 1.51 min

Beispiel 491 (Referenzbeispiel) 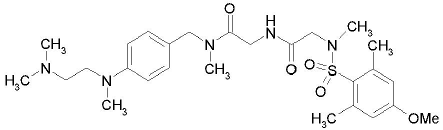 C₂₇H₄₁N₅O₅S x HCl (584.17)
[M+H]+ = 548
HPLC (Methode 5): Retentionszeit = 1.53 min

### Beispiel 492 (Referenzbeispiel)

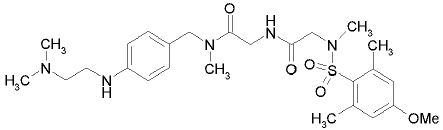

C₂₆H₃₉N₅O₅S x HCl (570.15)
[M+H]+ = 534
HPLC (Methode 5): Retentionszeit = 1.52 min

### Beispiel 493 (Referenzbeispiel)

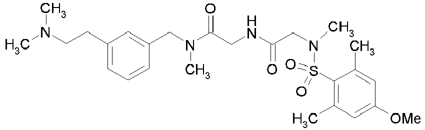

C₂₆H₃₈N₄O₅S x HCl (555.13)
[M+H]+ = 519
HPLC (Methode 5): Retentionszeit = 1.51 min

### Beispiel 494 (Referenzbeispiel)

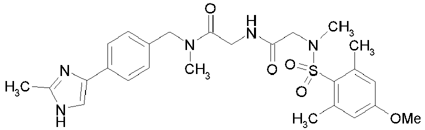

C₂₆H₃₃N₅O₅S (527.64)
[M+H]+ = 528
HPLC (Methode 4): Retentionszeit = 3.0 min

### Beispiel 495 (Referenzbeispiel)

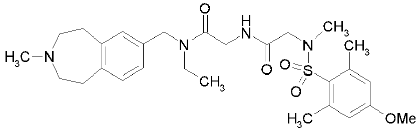

C₂₈H₄₀N₄O₅S x HCl (581.17)
[M+H]+ = 545
HPLC (Methode 4): Retentionszeit = 3.1 min

### Beispiel 496 (Referenzbeispiel)

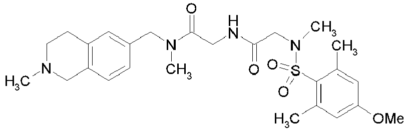

C₂₆H₃₆N₄O₅S x HCl (553.11)
[M+H]+ = 517
HPLC (Methode 5): Retentionszeit = 1.48 min

### Beispiel 497 (Referenzbeispiel)

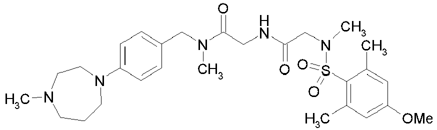

C₂₈H₄₁N₅O₅S x HCl (596.18)
[M+H]+ = 560
HPLC (Methode 5): Retentionszeit = 1.52 min

Beispiel 498 (Referenzbeispiel) 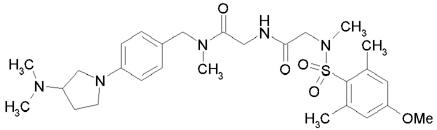 C₂₈H₄₁N₅O₅S x HCl (596.18)
[M+H]+ = 560
HPLC (Methode 5): Retentionszeit = 1.52 min

### Beispiel 499 (Referenzbeispiel)

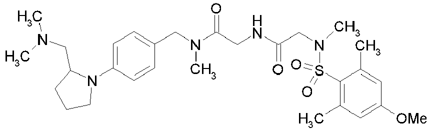

C₂₉H₄₃N₅O₅S x HCl (610.21)
[M+H]+ = 574
HPLC (Methode 5): Retentionszeit = 1.57 min

### Beispiel 500 (Referenzbeispiel)

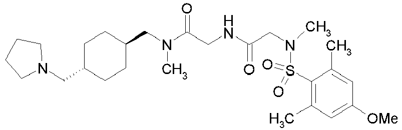

C₂₇H₄₄N₄O₅S x HCl (573.19)
[M+H]+ = 537
DC: Kieselgel, Dichlormethan / Methanol / Ammoniak 8:2:0.01, Rf-Wert = 0.66

### Beispiel 501 (Referenzbeispiel)

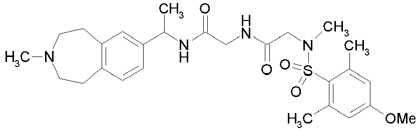

C₂₇H₃₈N₄O₅S x HCl (567.14)
[M+H]+ = 531
HPLC (Methode 4): Retentionszeit = 3.0 min

Beispiel 502 (Referenzbeispiel) 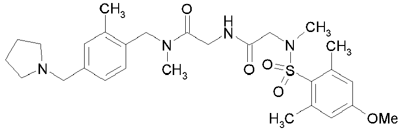 C₂₈H₄₀N₄O₅S x HCl (581.17)
[M+H]+ = 545
DC: Kieselgel, Dichlormethan / Methanol 9:1, Rf-Wert = 0.18

### Beispiel 503 (Referenzbeispiel)

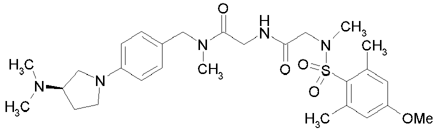

C₂₈H₄₁N₅O₅S x HCl (596.18)
[M+H]+ = 560
DC: Kieselgel, Ethylacetat / Methanol / Ammoniak 9:1:0.1, Rf-Wert = 0.42

### Beispiel 504 (Referenzbeispiel)

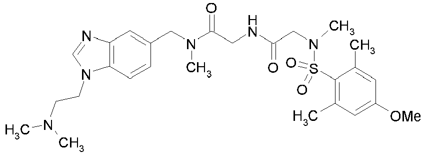

C₂₇H₃₈N₆O₅S (558.69)
[M+H]+ = 559
HPLC (Methode 6): Retentionszeit = 2.23 min

### Beispiel 505 (Referenzbeispiel)

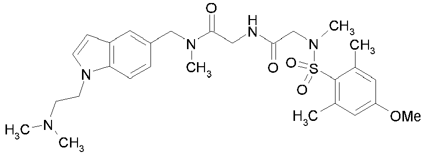

C₂₈H₃₉N₅O₅S (557.71)
[M+H]+ = 558
HPLC (Methode 6): Retentionszeit = 2.35 min

### Beispiel 506 (Referenzbeispiel)

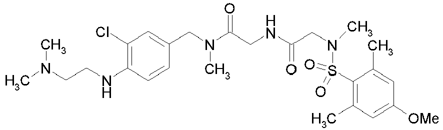

C₂₆H₃₈ClN₅O₅S x CH₂O₂ (614.16)
[M+H]+ = 568/570
HPLC (Methode 6): Retentionszeit = 2.55 min

### Beispiel 507 (Referenzbeispiel)

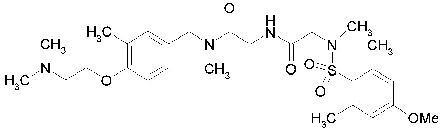

C₂₇H₄₀N₄O₆S (548.70)
[M+H]+ = 549
HPLC (Methode 6): Retentionszeit = 2.49 min

### Beispiel 508 (Referenzbeispiel)

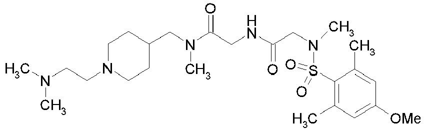

C₂₅H₄₃N₅O₅S (525.71)
[M+H]+ = 526
HPLC (Methode 6): Retentionszeit = 1.85 min

### Beispiel 509 (Referenzbeispiel)

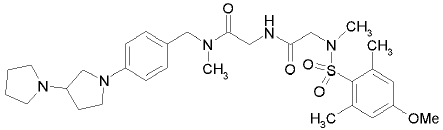

C₃₀H₄₃N₅O₅S (585.76)
[M+H]+ = 586
HPLC (Methode 6): Retentionszeit = 2.66 min

### Beispiel 510 (Referenzbeispiel)

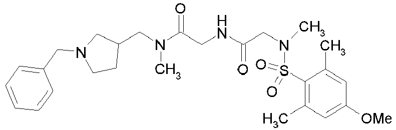

C₂₇H₃₈N₄O₅S (530.68)
[M+H]+ = 531
HPLC (Methode 6): Retentionszeit = 2.52 min

### Beispiel 511 (Referenzbeispiel)

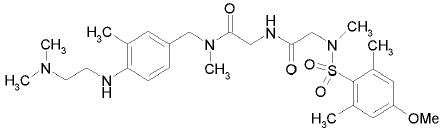

C₂₇H₄₁N₅O₅S (547.71)
[M+H]+ = 548
HPLC (Methode 6): Retentionszeit = 2.44 min

### Beispiel 512 (Referenzbeispiel)

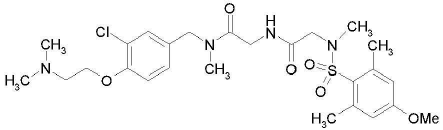

C₂₆H₃₇ClN₄O₆S (569.11)
[M+H]+ = 570
HPLC (Methode 6): Retentionszeit = 2.56 min

### Beispiel 513 (Referenzbeispiel)

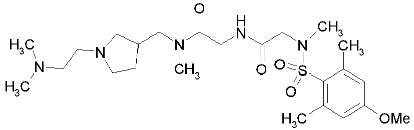

C₂₄H₄₁N₅O₅S (511.68)
[M+H]+ = 512
HPLC (Methode 6): Retentionszeit = 1.80 min

### Beispiel 514 (Referenzbeispiel)

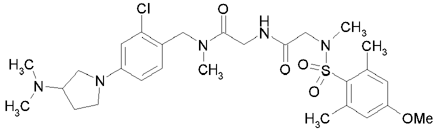

C₂₈H₄₀ClN₅O₅S (594.17)
[M+H]+ = 594/596
HPLC (Methode 6): Retentionszeit = 2.68 min

### Beispiel 515 (Referenzbeispiel)

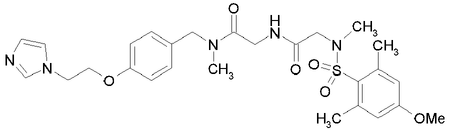

C₂₇H₃₅N₅O₆S (557.66)
[M+H]+ = 558
HPLC (Methode 6): Retentionszeit = 2.51 min

### Beispiel 516 (Referenzbeispiel)

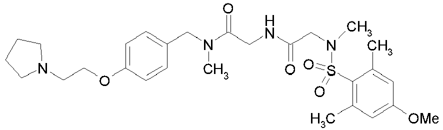

C₂₈H₄₀N₄O₆S (560.71)
[M+H]+ = 561
HPLC (Methode 6): Retentionszeit = 2.51 min

### Beispiel 517 (Referenzbeispiel)

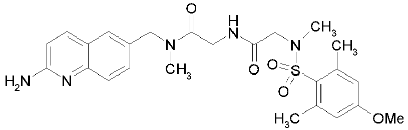

C₂₅H₃₁N₅O₅S (513.61)
[M+H]+ = 514
HPLC (Methode 6): Retentionszeit = 2.35 min

### Beispiel 518 (Referenzbeispiel)

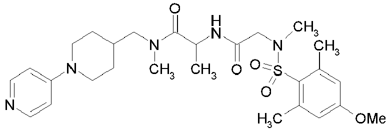

C₂₇H₃₉N₅O₅S x C₂HF₃O₂ (659.72)
[M+H]+ = 546
HPLC (Methode 6): Retentionszeit = 2.44 min

### Beispiel 519 (Referenzbeispiel)

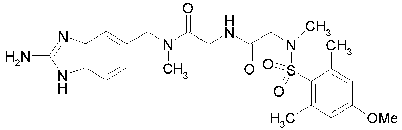

C₂₃H₃₀N₆O₅S (502.59)
[M+H]+ = 503
HPLC (Methode 6): Retentionszeit = 2.37 min

### Beispiel 520 (Referenzbeispiel)

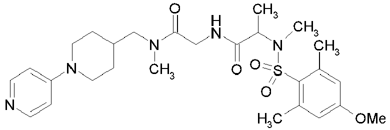

C₂₇H₃₉N₅O₅S x C₂HF₃O₂ (659.72)
[M+H]+ = 546
HPLC (Methode 6): Retentionszeit = 2.52 min

### Beispiel 521 (Referenzbeispiel)

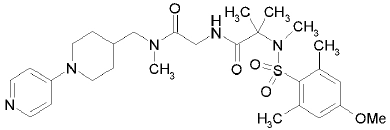

C₂₈H₄₁N₅O₅S x C₂HF₃O₂ (673.75)
[M+H]+ = 560
HPLC (Methode 6): Retentionszeit = 2.58 min

### Beispiel 522 (Referenzbeispiel)

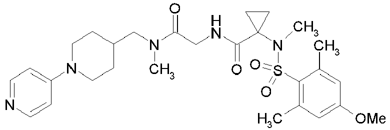

C₂₈H₃₉N₅O₅S x C₂HF₃O₂ (671.73)
[M+H]+ = 558
HPLC (Methode 6): Retentionszeit = 2.57 min

### Beispiel 523 (Referenzbeispiel)

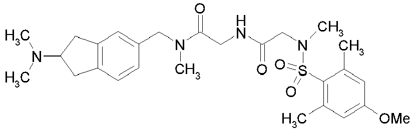

C₂₇H₃₈N₄O₅S x C₂HF₃O₂ (644.70)
[M+H]+ = 531
HPLC (Methode 6): Retentionszeit = 2.45 min

### Beispiel 524 (Referenzbeispiel)

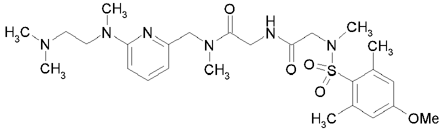

C₂₆H₄₀N₆O₅S (548.70)
[M+H]+ = 549
HPLC (Methode 6): Retentionszeit = 2.52 min

### Beispiel 525 (Referenzbeispiel)

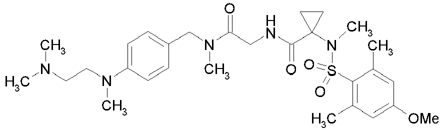

C₂₉H₄₃N₅O₅S x C₂HF₃O₂ (687.77)
[M+H]+ = 574
HPLC (Methode 6): Retentionszeit = 2.72 min

### Beispiel 526 (Referenzbeispiel)

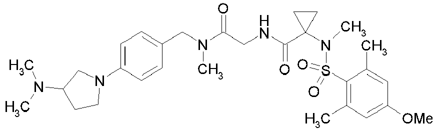

C₃₀H₄₃N₅O₅S (585.76)
[M+H]+ = 586
HPLC (Methode 6): Retentionszeit = 2.70 min

### Beispiel 527 (Referenzbeispiel)

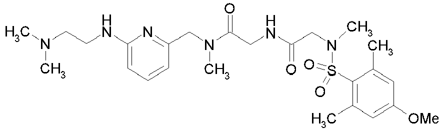

C₂₅H₃₈N₆O₅S x CH₂O₂ (580.70)
[M+H]+ = 535
HPLC (Methode 6): Retentionszeit = 2.16 min

### Beispiel 528 (Referenzbeispiel)

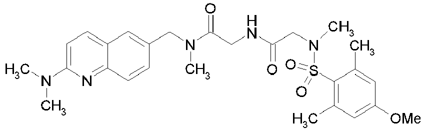

C₂₇H₃₅N₅O₅S x C₂HF₃O₂ (655.69)
[M+H]+ = 542
HPLC (Methode 6): Retentionszeit = 2.48 min

### Beispiel 529 (Referenzbeispiel)

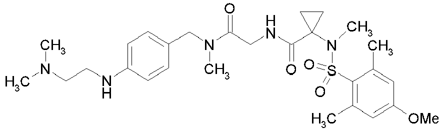

C₂₈H₄₁N₅O₅S x C₂HF₃O₂ (673.75)
[M+H]+ = 560
HPLC (Methode 6): Retentionszeit = 2.61 min

### Beispiel 530 (Referenzbeispiel)

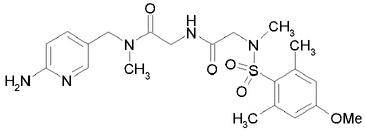

C₂₁H₂₉N₅O₅S (463.55)
[M+H]+ = 464
HPLC (Methode 9): Retentionszeit = 1.47 min

### Beispiel 531 (Referenzbeispiel)

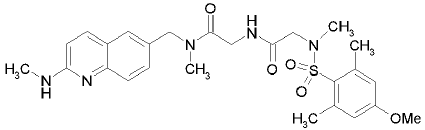

C₂₆H₃₃N₅O₅S (527.64)
[M+H]+ = 528
HPLC (Methode 9): Retentionszeit = 1.60 min

### Beispiel 532 (Referenzbeispiel)

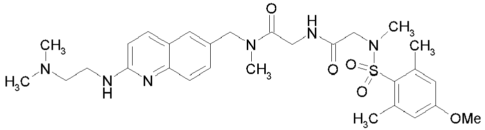

C₂₉H₄₀N₆O₅S (584.73)
[M+H]+ = 585
HPLC (Methode 9): Retentionszeit = 1.37 min

### Beispiel 533 (Referenzbeispiel)

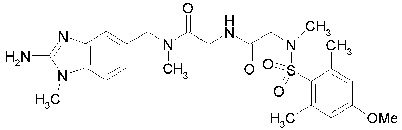

C₂₄H₃₂N₆O₅S x C₂HF₃O₂ (630.64)
[M+H]+ = 517
HPLC (Methode 9): Retentionszeit = 1.58 min

### Beispiel 534 (Referenzbeispiel)

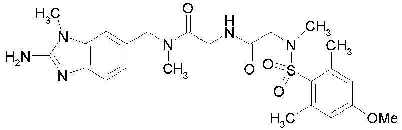

C₂₄H₃₂N₆O₅S x C₂HF₃O₂ (630.64)
[M+H]+ = 517
HPLC (Methode 9): Retentionszeit = 1.57 min

### Beispiel 535 (Referenzbeispiel)

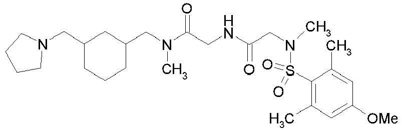

C₂₇H₄₄N₄O₅S x C₂HF₃O₂ (650.75)
[M+H]+ = 537
HPLC (Methode 9): Retentionszeit = 1.68 min

### Beispiel 536 (Referenzbeispiel)

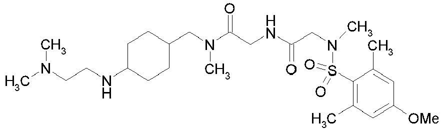

C₂₆H₄₅N₅O₅S x 2HCl (612.65)
[M+H]+ = 540
HPLC (Methode 5): Retentionszeit = 1.39 min

### Beispiel 537 (Referenzbeispiel)

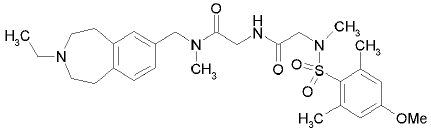

C₂₈H₄₀N₄O₅S x C₂HF₃O₂ (658.73)
[M+H]+ = 545
HPLC (Methode 5): Retentionszeit = 1.53 min

### Beispiel 538 (Referenzbeispiel)

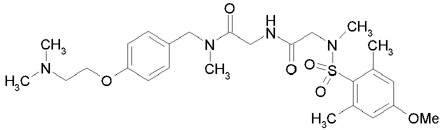

C₂₆H₃₈N₄O₆S x C₂HF₃O₂ (648.69)
[M+H]+ = 535
HPLC (Methode 5): Retentionszeit = 1.43 min

### Beispiel 539 (Referenzbeispiel)

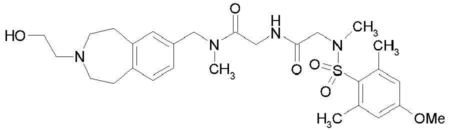

C₂₈H₄₀N₄O₆S (560.71)
[M+H]+ = 561
HPLC (Methode 5): Retentionszeit = 1.50 min

### Beispiel 540 (Referenzbeispiel)

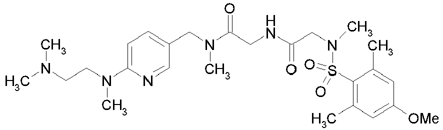

C₂₆H₄₀N₆O₅S (548.70)
[M+H]+ = 549
HPLC (Methode 5): Retentionszeit = 1.39 min

### Beispiel 541 (Referenzbeispiel)

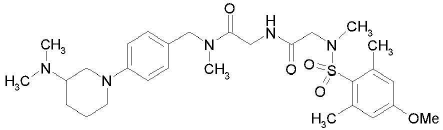

C₂₉H₄₃N₅O₅S x HCl (610.21)
[M+H]+ = 574
HPLC (Methode 7): Retentionszeit = 1.88 min

### Beispiel 542 (Referenzbeispiel)

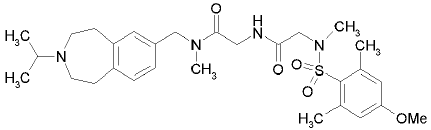

C₂₉H₄₂N₄O₅S x C₂HF₃O₂ (672.76)
[M+H]+ = 559
HPLC (Methode 5): Retentionszeit = 1.55 min

### Beispiel 543 (Referenzbeispiel)

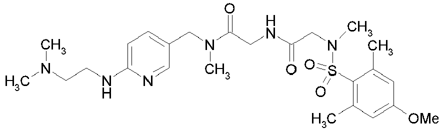

C₂₅H₃₈N₆O₅S x 2HCl (607.59)
[M+H]+ = 535
HPLC (Methode 5): Retentionszeit = 1.39 min

### Beispiel 544 (Referenzbeispiel)

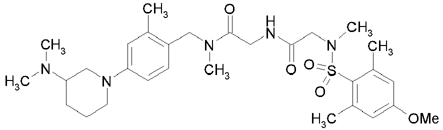

C₃₀H₄₅N₅O₅S x C₂HF₃O₂ (701.80)
[M+H]+ = 588
HPLC (Methode 5): Retentionszeit = 1.55 min

### Beispiel 545 (Referenzbeispiel)

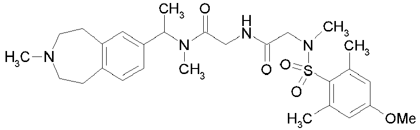

C₂₈H₄₀N₄O₅S x HCl (581.17)
[M+H]+ = 545
HPLC (Methode 4): Retentionszeit = 3.3 min

### Beispiel 546 (Referenzbeispiel)

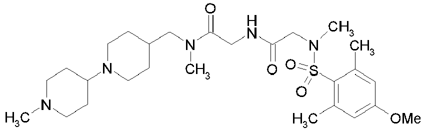

C₂₇H₄₅N₅O₅S x 2HCl (624.66)
[M+H]+ = 552
DC: Kieselgel, Dichlormethan / Methanol / Ammoniak 8:2:0.01, Rf-Wert = 0.25

### Beispiel 547 (Referenzbeispiel)

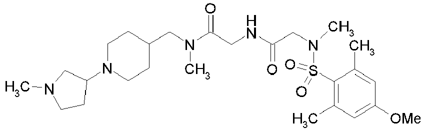

C₂₆H₄₃N₅O₅S x HCl (574.18)
[M+H]+ = 538
DC: Kieselgel, Dichlormethan / Methanol/Ammoniak 8:2:0.01, Rf-Wert = 0.10

### Beispiel 548 (Referenzbeispiel)

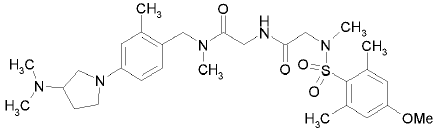

C₂₉H₄₃N₅O₅S x HCl (610.21)
[M+H]+ = 574
DC: Kieselgel, Dichlormethan / Methanol 9:1, Rf-Wert = 0.25

### Beispiel 549 (Referenzbeispiel)

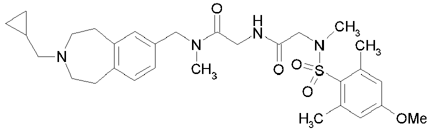

C₃₁H₄₄N₄O₅S x HCl (621.23)
[M+H]+ = 585
HPLC (Methode 12): Retentionszeit = 3.0 min

Beispiel 550 (Referenzbeispiel) 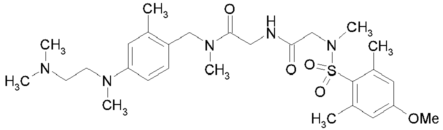 C₂₈H₄₃N₅O₅S x HCl (598.20)
[M+H]+ = 562
DC: Kieselgel, Dichlormethan / Methanol 9:1, Rf-Wert = 0.14

### Beispiel 551 (Referenzbeispiel)

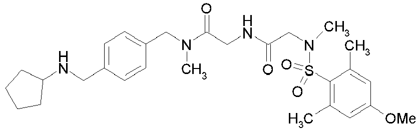

C₂₉H₄₂N₄O₅S x HCl (595.19)
[M+H]+ = 559
DC: Kieselgel, Dichlormethan / Methanol / Ammoniak 9:1:0.1, Rf-Wert = 0.32

### Beispiel 552 (Referenzbeispiel)

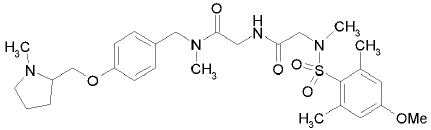

C₂₈H₄₀N₄O₆S (560.71)
[M+H]+ = 561
HPLC (Methode 9): Retentionszeit = 1.66 min

### Beispiel 553 (Referenzbeispiel)

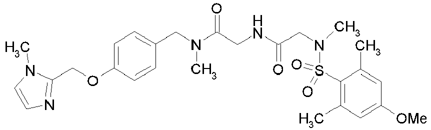

C₂₇H₃₅N₅O₆S (557.66)
[M+H]+ = 558
HPLC (Methode 9): Retentionszeit = 1.64 min

### Beispiel 554 (Referenzbeispiel)

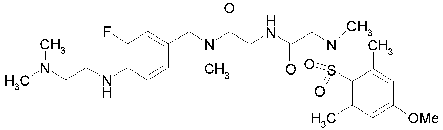

C₂₆H₃₈FN₅O₅S (551.68)
[M+H]+ = 552
HPLC (Methode 6): Retentionszeit = 2.30 min

### Beispiel 555 (Referenzbeispiel)

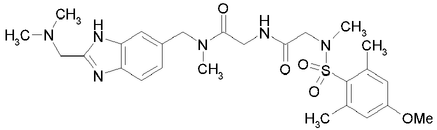

C₂₆H₃₆N₆O₅S (544.67)
[M+H]+ = 545
HPLC (Methode 6): Retentionszeit = 1.48 min

### Beispiel 556 (Referenzbeispiel)

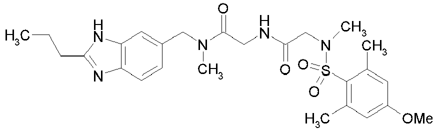

C₂₆H₃₅N₅O₅S (529.65)
[M+H]+ = 530
HPLC (Methode 9): Retentionszeit = 1.61 min

### Beispiel 557 (Referenzbeispiel)

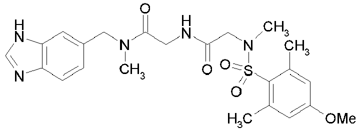

C₂₃H₂₉N₅O₅S (487.57)
[M+H]+ = 488
HPLC (Methode 9): Retentionszeit = 1.53 min

### Beispiel 558 (Referenzbeispiel)

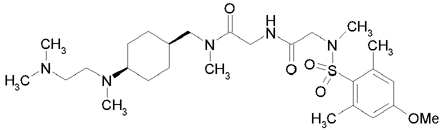

C₂₇H₄₇N₅O₅S x 2HCl (626.68)
[M+H]+ = 554
HPLC (Methode 7): Retentionszeit = 1.61 min

### Beispiel 559 (Referenzbeispiel)

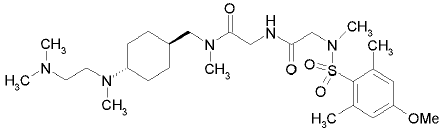

C₂₇H₄₇N₅O₅S x 2HCl (626.68)
[M+H]+ = 554
HPLC (Methode 7): Retentionszeit = 1.61 min

### Beispiel 560 (Referenzbeispiel)

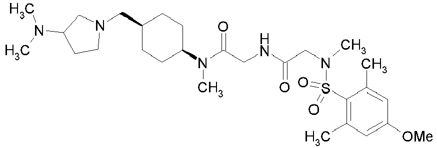

C₂₈H₄₇N₅O₅S x CH₂O₂ (611.80)
[M+H]+ = 566
HPLC (Methode 9): Retentionszeit = 1.33 min

### Beispiel 561 (Referenzbeispiel)

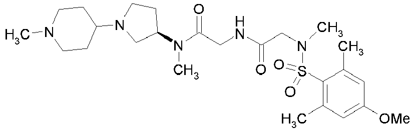

C₂₅H₄₁N₅O₅S x 2HCl (596.61)
[M+H]+ = 524
HPLC (Methode 12): Retentionszeit = 2.3 min

### Beispiel 562 (Referenzbeispiel)

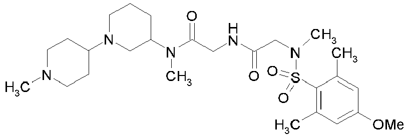

C₂₆H₄₃N₅O₅S x 2HCl (610.64)
[M+H]+ = 538
DC: Kieselgel, Dichlormethan / Methanol / Ammoniak 8:2:0.2, Rf-Wert = 0.53

### Beispiel 563 (Referenzbeispiel)

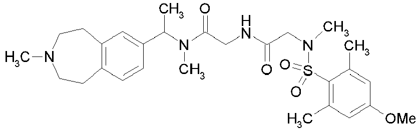

C₂₉H₄₂N₄O₅S x HCl (595.19)
[M+H]+ = 559
HPLC (Methode 12): Retentionszeit = 2.8 min

Beispiel 564 (Referenzbeispiel) 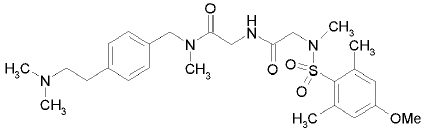 C₂₆H₃₈N₄O₅S x C₂HF₃O₂ (632.69)
[M+H]+ = 519
HPLC (Methode 9): Retentionszeit = 1.61 min

### Beispiel 565 (Referenzbeispiel)

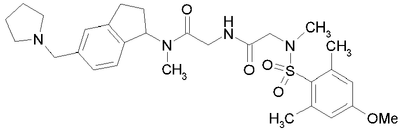

C₂₉H₄₀N₄O₅S (556.72)
[M+H]+ = 557
HPLC (Methode 9): Retentionszeit = 1.69 min

### Beispiel 566 (Referenzbeispiel)

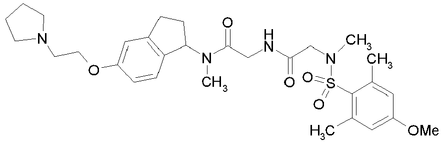

C₃₀H₄₂N₄O₆S (586.74)
[M+H]+ = 587
HPLC (Methode 9): Retentionszeit = 1.74 min

### Beispiel 603 (Referenzbeispiel)

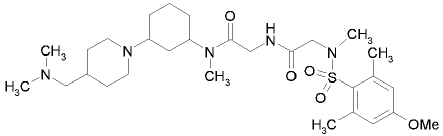

C₂₉H₄₉N₅O₅S x 2HCl (652.72)
[M+H]+ = 580
HPLC (Methode 10): Retentionszeit = 1.11 min

Beispiel 604 (Referenzbeispiel) 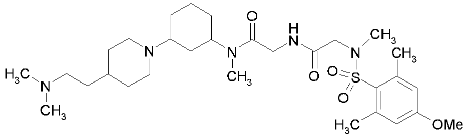 C₃₀H₅₁N₅O₅S x 2HCl (666.74)
[M+H]+ = 594
HPLC (Methode 10): Retentionszeit = 1.11 min

### Beispiel 605 (Referenzbeispiel)

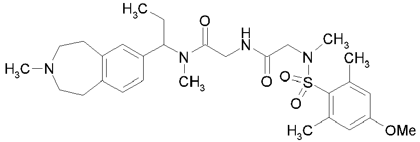

C₂₉H₄₂N₄O₅S x HCl (595.19)
[M+H]+ = 559
HPLC (Methode 12): Retentionszeit = 3.1 min

### Beispiel 606 (Referenzbeispiel)

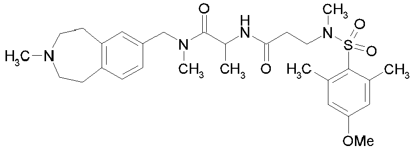

C₂₉H₄₂N₄O₅S x HCl (595.19)
[M+H]+ = 559
DC: Kieselgel, Dichlormethan / Methanol / Ammoniak 8:2:0.01, Rf-Wert = 0.68

### Beispiel 631 (Referenzbeispiel)

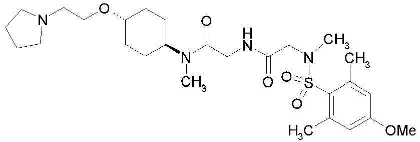

C₂₇H₄₄N₄O₅S x HCl (589.19)
[M+H]+ = 553
HPLC (Methode 5): Retentionszeit = 1.35 min

### Beispiel 632 (Referenzbeispiel)

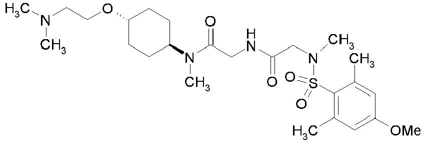

C₂₅H₄₂N₄O₆S x HCl (563.15)
[M+H]+ = 527
HPLC (Methode 5): Retentionszeit = 1.38 min

### Beispiel 633 (Referenzbeispiel)

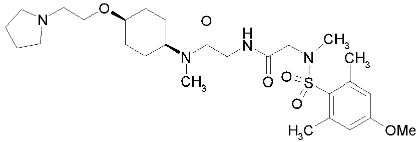

C₂₇H₄₄N₄O₆S x HCl (589.19)
[M+H]+ = 553
HPLC (Methode 5): Retentionszeit = 1.35 min

Beispiel 634 (Referenzbeispiel) 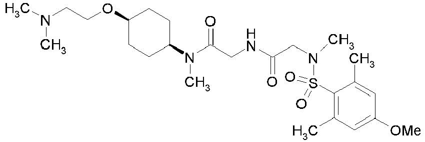 C₂₅H₄₂N₄O₆S x HCl (563.15)
[M+H]+ = 527
HPLC (Methode 5): Retentionszeit = 1.32 min

Die folgenden Verbindungen wurden analog zu Beispiel 130 hergestellt:

### Beispiel 567 (Referenzbeispiel)

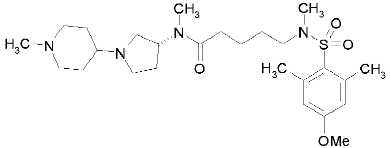

C₂₆H₄₄N₄O₄S x 2C₂HF₃O₂ (736.76)
[M+H]+ = 509
HPLC (Methode 6): Retentionszeit = 1.36 min

### Beispiel 568 (Referenzbeispiel)

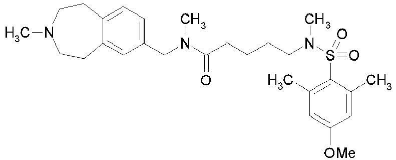

C₂₈H₄₁N₃O₄S x C₂HF₃O₂ (629.73)
[M+H]+ = 516
HPLC (Methode 6): Retentionszeit = 1.77 min

Die folgenden Verbindungen wurden analog zu Beispiel 136 hergestellt:

### Beispiel 569 (Referenzbeispiel)

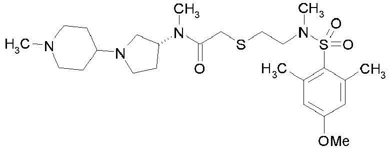

C₂₅H₄₂N₄O₄S₂ x C₂HF₃O₂ (640.78)
[M+H]+ = 527
HPLC (Methode 6): Retentionszeit = 1.35 min

### Beispiel 570 (Referenzbeispiel)

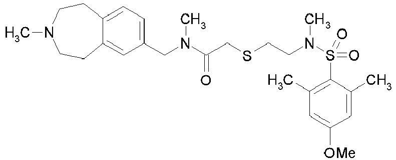

C₂₇H₃₉N₃O₄S₂ (533.75)
[M+H]+ = 534
HPLC (Methode 6): Retentionszeit = 1.74 min

Die folgende Verbindung wurde analog zu Beispiel 138 hergestellt:

### Beispiel 571 (Referenzbeispiel)

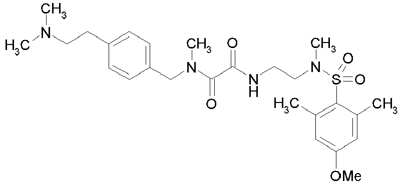

C₂₆H₃₈N₄O₅S x CH₂O₂ (564.70)
[M+H]+ = 519
HPLC (Methode 6): Retentionszeit = 2.33 min

### Beispiel 572 (Referenzbeispiel)

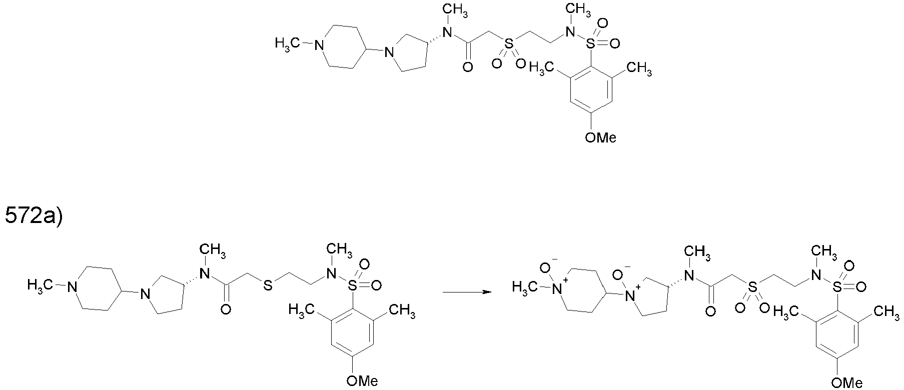

Eine Mischung aus 88.0 mg (0.167 mMol) 569, 0.15 g (0.61 mMol) 70 %ige m-Chlorperbenzoesäure (Fluka) und 3 ml Dichlormethan wird 30 Minuten bei Raumtemperatur gerührt und danach im Vakuum bis zur Trockene eingeengt. Der Rückstand wird in Methanol gelöst und membranfiltriert. Das Produkt wird anschließend durch präparative HPLC aus dem Filtrat gewonnen.
C₂₅H₄₂N₄O₈S₂ (590.76)
[M+H]+ = 591

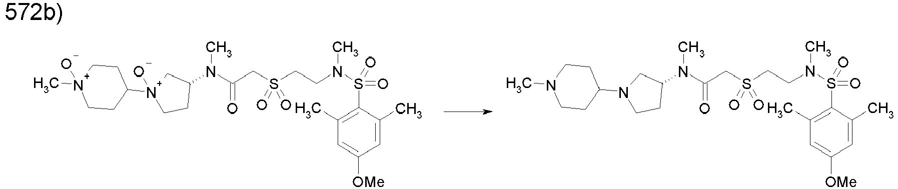

Eine Mischung aus 90.0 mg (0.15 mMol) Produkt aus 572a, 20.0 mg Raney-Nickel und 10 ml THF wird im Autoklaven eine Stunde bei Raumtemperatur gerührt. Anschließend wird der Katalysator abfiltriert und das Filtrat im Vakuum bis zur Trockene eingeengt. Das so erhaltene Rohpodukt wird durch präparative HPLC gereinigt.
C₂₅H₄₂N₄O₆S₂ x CH₂O₂ (604.78)
[M+H]+ = 559
HPLC (Methode 6): Retentionszeit = 1.33 min

### Beispiel 573 (Referenzbeispiel)

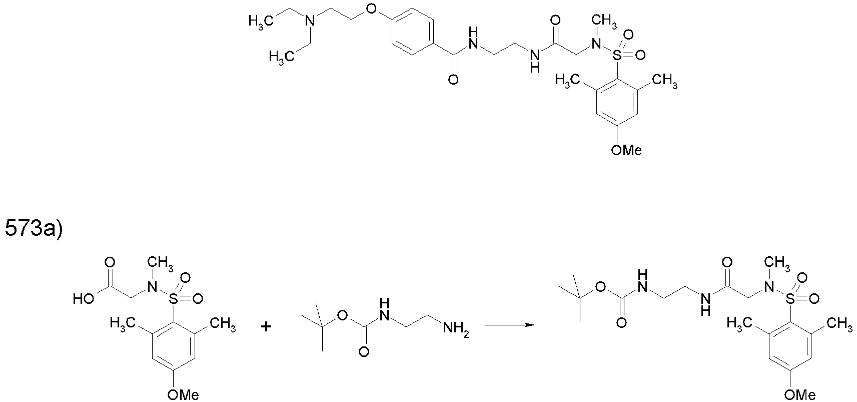

573a wird analog zu 1f aus 2.16 g (7.50 mMol) Produkt aus 121b, 1.20 g (7.50 mMol) N-Boc-ethylendiamin (Fluka), 3.14 ml (22.50 mMol) Triethylamin und 2.41 g (7.50 mMol) TBTU in 28 ml THF und 4 ml DMF hergestellt.
C₁₉H₃₁N₃O₆S (429.53)
DC: Kieselgel, Dichlormethan / Ethanol 19:1, Rf-Wert = 0.35

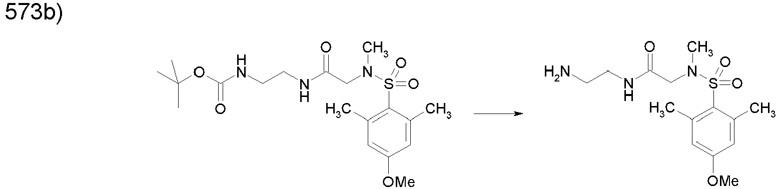

573b wird analog zu 28d aus 2.70 g (6.29 mMol) Produkt aus 573a und 7 ml TFA in 50 ml Dichlormethan hergestellt.
C₁₄H₂₃N₃O₄S (329.42)
DC: Kieselgel, Dichlormethan / Ethanol 9:1, Rf-Wert = 0.15

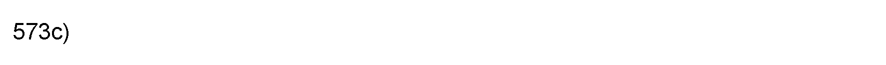

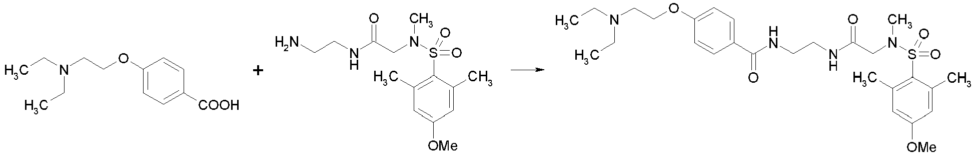

Beispiel 573 wird analog zu 1f aus 0.119 g (0.50 mMol) 4-(2-Diethylaminoethoxy)-benzoesäure (J. Med. Chem. 14, 1971, 836-842), 0.165 g (0.50 mmol) Produkt aus 573b, 0.21 ml (1.50 mMol) Triethylamin und 0.16 g (0.50 mMol) TBTU in 7 ml THF und 1 ml DMF hergestellt.
C₂₇H₄₀N₄O₆S x HCl (585.16)
HPLC (Methode 5): Retentionszeit = 1.44 min

Die folgenden Verbindungen wurde analog zu Beispiel 573 hergestellt:

### Beispiel 574 (Referenzbeispiel)

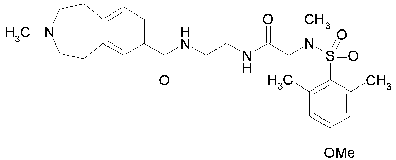

C₂₆H₃₆N₄O₅S x HCl (553.11)
[M+H]+ = 517
HPLC (Methode 5): Retentionszeit = 1.40 min

### Beispiel 607 (Referenzbeispiel)

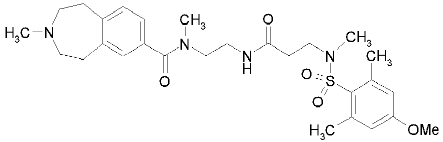

C₂₈H₄₀N₄O₅S x HCl (581.17)
[M+H]+ = 545
HPLC (Methode 12): Retentionszeit = 3.51 min

### Beispiel 635 (Referenzbeispiel)

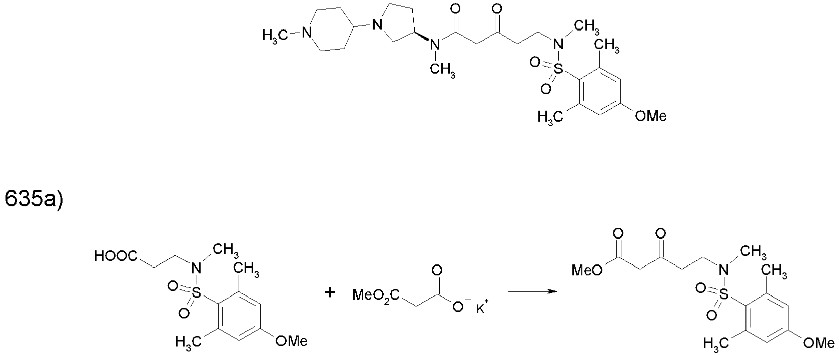

Eine Mischung aus 0.78 g (4.98 mMol) Monomethylmalonat Kaliumsalz (Fluka), 0.52 g (5.47 mMol) Magnesiumchlorid und 30 ml THF wird vier Stunden bei 50°C gerührt. Eine zweite Mischung aus 1.00 g (3.32 mMol) Produkt aus 22c, 0.65 g (3.98 mMol) CDI und 20 ml THF wird zuerst eine Stunde bei RT gerührt und anschließend zur ersten Mischung hinzu gegeben. Man lässt über Nacht bei RT rühren und filtriert danach den entstandenen Niederschlag ab. Das Filtrat wird im Vakuum bis zur Trockene eingeengt. Das so erhaltene Rohprodukt wird mit Wasser verrieben, abfiltriert und im Vakuumtrockenschrank bei 45°C getrocknet.
C₁₆H₂₃NO₆S (357.42)
[M+H]+ = 358
HPLC (Methode 9): Retentionszeit = 2.19 min

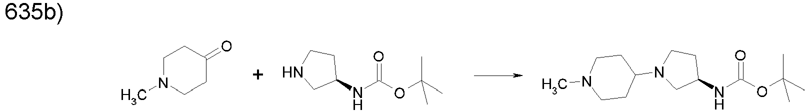

635b wird analog zu 60a aus 0.47 ml (4.07 mMol) 1-Methyl-4-piperidon (Fluka), 0.76 g .(4.07 mMol) (R)-3-(Boc-amino)-pyrrolidin (Fluka), 1.72 g (8.13 mMol) Natriumtriacetoxyborhydrid und 0.23 ml (4.07 mMol) Essigsäure in 10 ml Dichlormethan hergestellt.
C₁₅H₂₉N₃O₂ (283.41)
[M+H]+ = 284

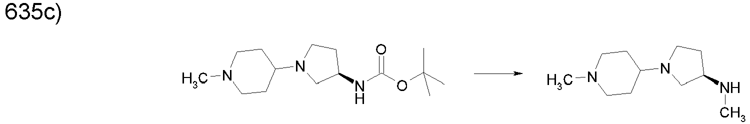

635c wird analog zu 38f aus 0.90 g (3.18 mMol) Produkt aus 635b und 5.0 ml (10.00 mMol) Lithiumaluminiumhydrid (2M in THF) in 15 ml THF hergestellt.
C₁₁H₂₃N₃ (197.32)
[M+H]+ = 198

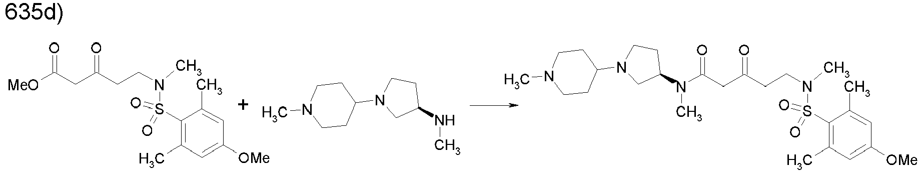

Eine Mischung aus 0.56 g (1.58 mMol) Produkt aus 635a, 0.54 g (2.73 mMol) Produkt aus 635c und 5 ml Toluol wird 24 Stunden auf 120°C erhitzt. Anschließend wird die Reaktionsmischung im Vakuum bis zur Trockene eingeengt. Das so erhaltene Rohprodukt wird durch präparative HPLC gereinigt.
C₂₆H₄₂N₄O₅S (522.70)
[M+H]+ = 523
HPLC (Methode 9): Retentionszeit = 1.33 min

Analog zu den voranstehend genannten Beispielen wurden erhalten:

### Beispiel 651

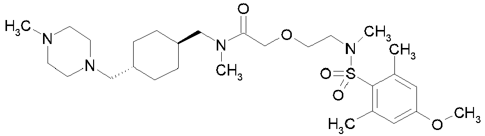

C₂₈H₄₈N₄O₅S (552.78)
[M+H]+ = 553
HPLC (Methode 5): Retentionszeit = 1.45 min

### Beispiel 652

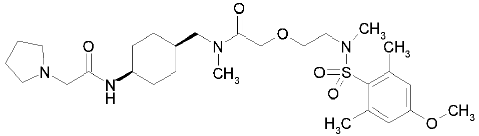

C₂₈H₄₆N₄O₆S (566.76)
[M+H]+ = 5567
HPLC (Methode 5): Retentionszeit = 1.65 min

### Beispiel 653

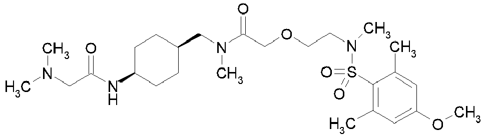

C₂₆H₄₄N₄O₆S (540.72)
[M+H]+ = 567
HPLC (Methode 5): Retentionszeit = 1.64 min

### Beispiel 654

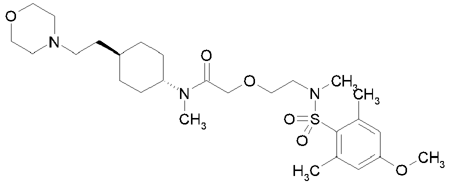

C₂₇H₄₅N₃O₆S (539.73)
[M+H]+ = 540
HPLC (Methode 5): Retentionszeit = 1.67 min

### Beispiel 655

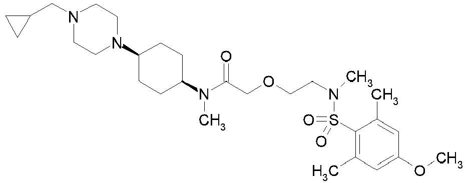

C₂₉H₄₈N₄O₅S (564.79)
[M+H]+ = 565
HPLC (Methode 5): Retentionszeit = 1.47 min

### Beispiel 656

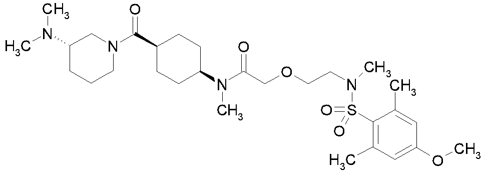

C₂₉H₄₈N₄O₆S (580.79)
[M+H]+ = 581
HPLC (Methode 5): Retentionszeit = 1.65 min

### Beispiel 657

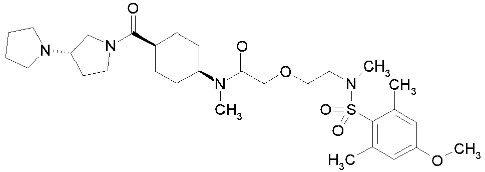

C₃₀H₄₈N₄O₆S (592.80)
[M+H]+ = 593
HPLC (Methode 5): Retentionszeit = 1.62 min

### Beispiel 658

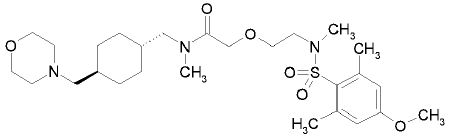

C₂₇H₄₅N₃O₆S (539.73)
[M+H]+ = 540
HPLC (Methode 5): Retentionszeit = 1.67 min

### Beispiel 659

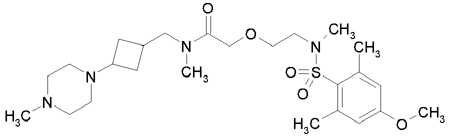

C₂₅H₄₂N₄O₅S (510.70)
[M+H]+ = 511
HPLC (Methode 5): Retentionszeit = 1.33 min

### Beispiel 660

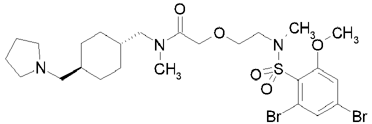

C₂₅H₃₉Br₂N₃O₅S (653.47)
[M+H]+ = 652/54/56
HPLC (Methode 5): Retentionszeit = 1.82 min

### Beispiel 661

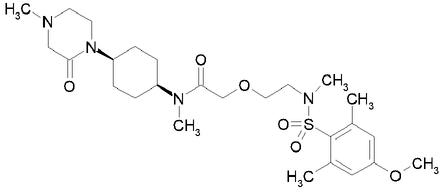

C₂₆H₄₂N₄O₆S (538.71)
[M+H]+ = 539
HPLC (Methode 5): Retentionszeit = 1.49 min

### Beispiel 662

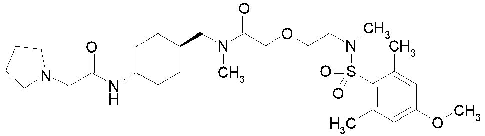

C₂₈H₄₆N₄O₆S (566.76)
[M+H]+ = 567
HPLC (Methode 5): Retentionszeit = 1.62 min

### Beispiel 663

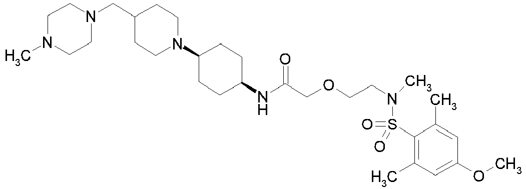

C₃₁H₅₃N₅O₅S (607.86)
[M+H]+ = 608
HPLC (Methode 7): Retentionszeit = 1.21 min

### Beispiel 664

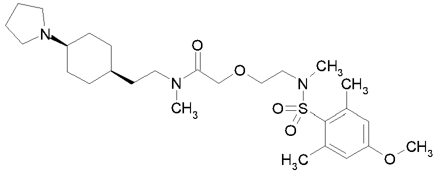

C₂₇H₄₅N₃O₅S (523.73)
[M+H]+ = 524
HPLC (Methode 5): Retentionszeit = 1.74 min

### Beispiel 665

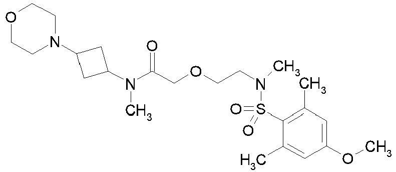

C₂₃H₃₇N₃O₆S (483.63)
[M+H]+ = 484
HPLC (Methode 5): Retentionszeit = 1.46 min

### Beispiel 666

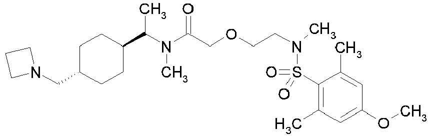

C₂₇H₄₅N₃O₅S (523.73)
[M+H]+ = 524
HPLC (Methode 12): Retentionszeit = 3.09 min

### Beispiel 667

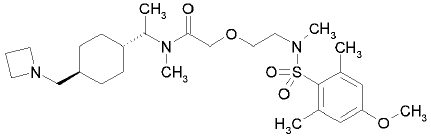

C₂₇H₄₅N₃O₅S (523.73)
[M+H]+ = 524
HPLC (Methode 12): Retentionszeit = 3.04 min

### Beispiel 668

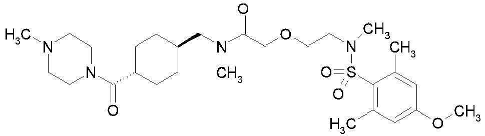

C₂₈H₄₆N₄O₆S (566.76)
[M+H]+ = 567
HPLC (Methode 5): Retentionszeit = 1.62 min

### Beispiel 669

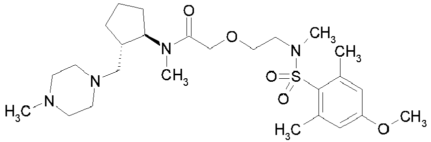

C₂₆H₄₄N₄O₅S (524.72)
[M+H]+ = 525
HPLC (Methode 4): Retentionszeit = 3.63 min

### Beispiel 670

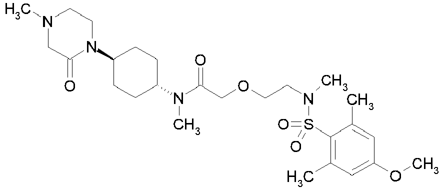

C₂₆H₄₂N₄O₆S (538.71)
[M+H]+ = 539
HPLC (Methode 5): Retentionszeit = 1.53 min

### Beispiel 671

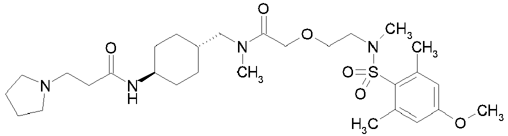

C₂₉H₄₈N₄O₆S (580.79)
[M+H]+ = 581
HPLC (Methode 5): Retentionszeit = 1.61 min

### Beispiel 672

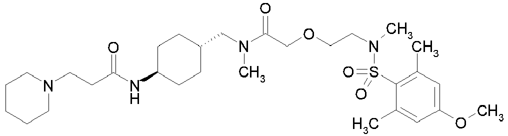

C₃₀H₅₀N₄O₆S (594.81)
[M+H]+ = 595
HPLC (Methode 5): Retentionszeit = 1.64 min

### Beispiel 673

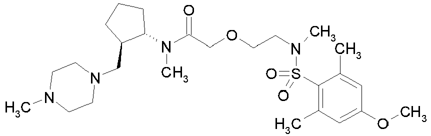

C₂₆H₄₄N₄O₅S (524.72)
[M+H]+ = 525
HPLC (Methode 4): Retentionszeit = 3.67 min

### Beispiel 674

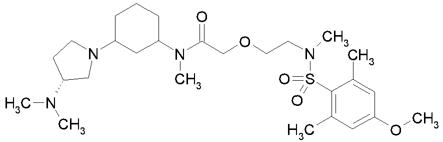

C₂₇H₄₆N₄O₅S (538.75)
[M+H]+ = 539
HPLC (Methode 5): Retentionszeit = 1.40 min

### Beispiel 675

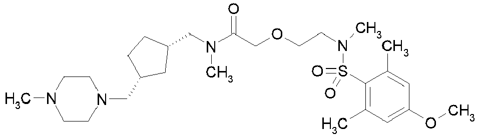

C₂₇H₄₆N₄O₅S (538.75)
[M+H]+ = 539
HPLC (Methode 5): Retentionszeit = 1.46 min

### Beispiel 676 (Referenzbeispiel)

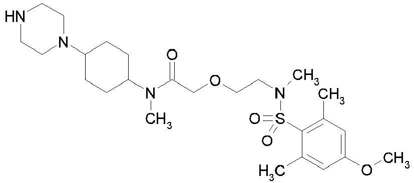

C₂₅H₄₂N₄O₅S (510.70)
[M+H]+ = 511
HPLC (Methode 8): Retentionszeit = 1.45 min

### Beispiel 677

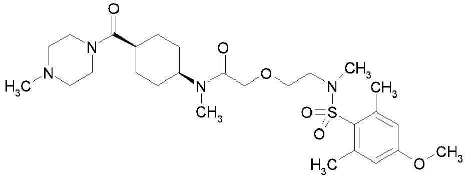

C₂₇H₄₄N₄O₆S (552.73)
[M+H]+ = 553
HPLC (Methode 5): Retentionszeit = 1.57 min

### Beispiel 678

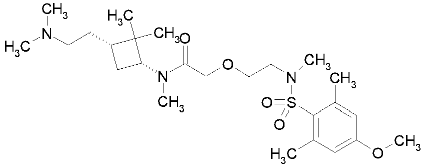

C₂₅H₄₃N₃O₅S (497.70)
[M+H]+ = 498
HPLC (Methode 5): Retentionszeit = 1.65 min

### Beispiel 679

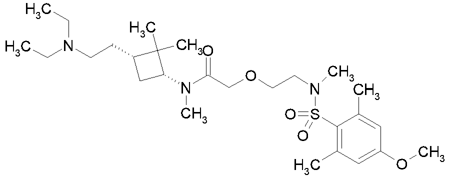

C₂₇H₄₇N₃O₅S (525.75)
[M+H]+ = 526
HPLC (Methode 5): Retentionszeit = 1.72 min

### Beispiel 680

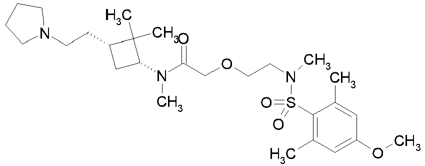

C₂₇H₄₅N₃O₅S (523.73)
[M+H]+ = 524
HPLC (Methode 5): Retentionszeit = 1.71 min

### Beispiel 681

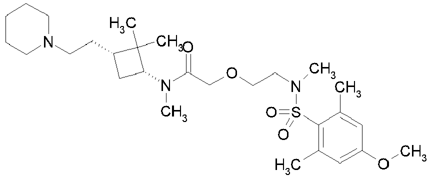

C₂₈H₄₇N₃O₅S (537.76)
[M+H]+ = 538
HPLC (Methode 5): Retentionszeit = 1.73 min

### Beispiel 682

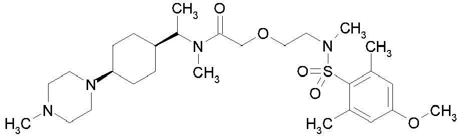

C₂₈H₄₈N₄O₅S (552.78)
[M+H]+ = 553
HPLC (Methode 8): Retentionszeit = 1.53 min

### Beispiel 683

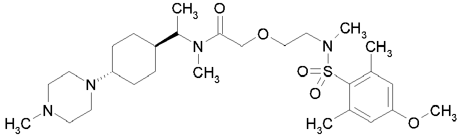

C₂₈H₄₈N₄O₅S (552.78)
[M+H]+ = 553
HPLC (Methode 8): Retentionszeit = 1.46 min

### Beispiel 684

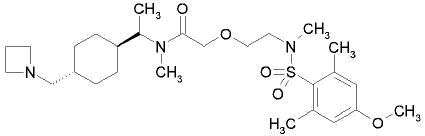

C₂₇H₄₅N₃O₅S (523.73)
[M+H]+ = 511
HPLC (Methode 12): Retentionszeit = 3.09 min

### Beispiel 685

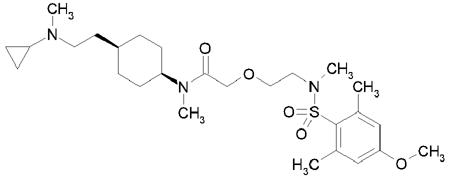

C₂₇H₄₅N₃O₅S (523.73)
[M+H]+ = 524
HPLC (Methode 5): Retentionszeit = 1.86 min

### Beispiel 686

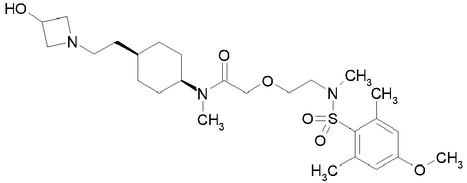

C₂₆H₄₃N₃O₆S (525.71)
[M+H]+ = 526
HPLC (Methode 5): Retentionszeit = 1.16 min

### Beispiel 687

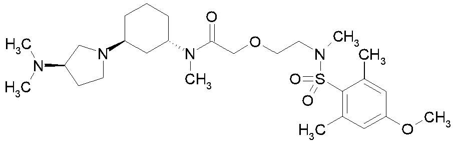

C₂₇H₄₆N₄O₅S (538.75)
[M+H]+ = 539
HPLC (Methode 14): Retentionszeit = 1.82 min

### Beispiel 688

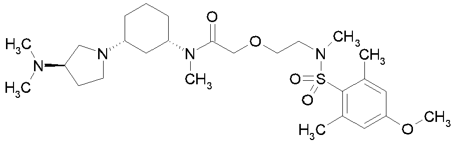

C₂₇H₄₆N₄O₅S (538.75)
[M+H]+ = 539
HPLC (Methode 5): Retentionszeit = 1.61 min

### Beispiel 689

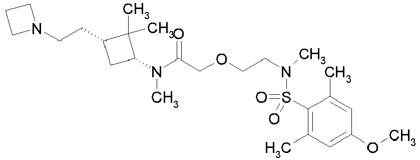

C₂₆H₄₃N₃O₅S (509.71)
[M+H]+ = 510
HPLC (Methode 5): Retentionszeit = 1.68 min

### Beispiel 690

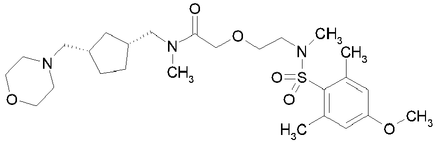

C₂₆H₄₃N₃O₆S (525.71)
[M+H]+ = 526
HPLC (Methode 5): Retentionszeit = 1.69 min

### Beispiel 691

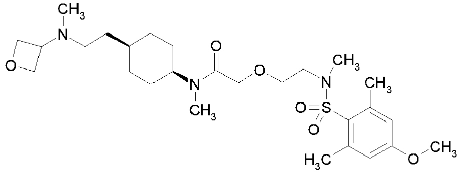

C₂₇H₄₅N₃O₆S (539.73)
[M+H]+ = 540
HPLC (Methode 5): Retentionszeit = 1.65 min

### Beispiel 692 (Referenzbeispiel)

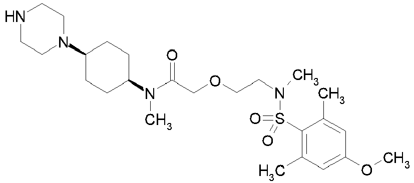

C₂₅H₄₂N₄O₅S (510.70)
[M+H]+ = 511
HPLC (Methode 14): Retentionszeit = 1.68 min

### Beispiel 693

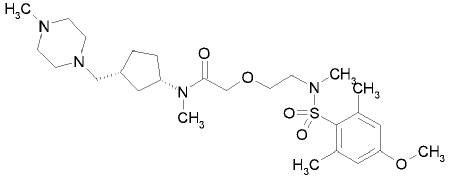

C₂₆H₄₄N₄O₅S (524.72)
[M+H]+ = 525
HPLC (Methode 5): Retentionszeit = 1.77 min

### Beispiel 694

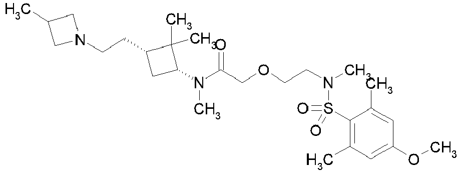

C₂₇H₄₅N₃O₅S (523.73)
[M+H]+ = 524
HPLC (Methode 5): Retentionszeit = 1.76 min

### Beispiel 695

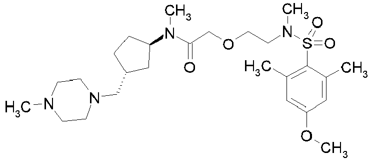

C₂₆H₄₄N₄O₅S (524.72)
[M+H]+ = 525
HPLC (Methode 5): Retentionszeit = 1.47 min

### Beispiel 696

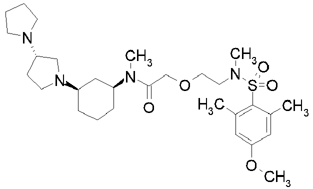

C₂₉H₄₈N₄O₅S (564.79)
[M+H]+ = 565
HPLC (Methode 14): Retentionszeit = 1.58 min

### Beispiel 697

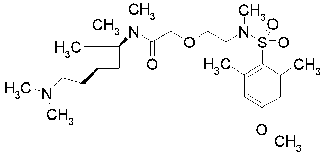

C₂₅H₄₃N₃O₅S (497.70)
[M+H]+ = 498
HPLC (Methode 5): Retentionszeit = 1.81 min

### Beispiel 698

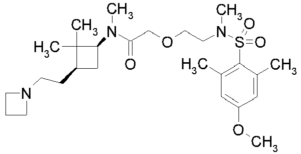

C₂₆H₄₃N₃O₅S (509.71)
[M+H]+ = 510
HPLC (Methode 5): Retentionszeit = 1.83 min

### Beispiel 699

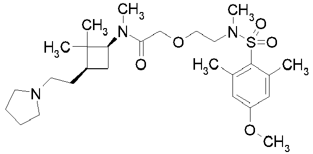

C₂₇H₄₅N₃O₅S (523.73)
[M+H]+ = 524
HPLC (Methode 5): Retentionszeit = 1.87 min

### Beispiel 700

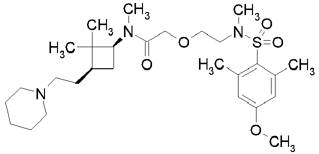

C₂₈H₄₇N₃O₅S (537.76)
[M+H]+ = 538
HPLC (Methode 5): Retentionszeit = 1.91 min

### Beispiel 701

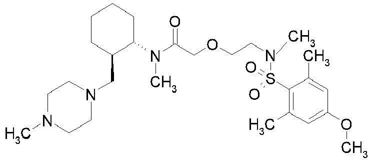

C₂₇H₄₆N₄O₅S (538.75)
[M+H]+ = 539
HPLC (Methode 5): Retentionszeit = 1.36 min

### Beispiel 702

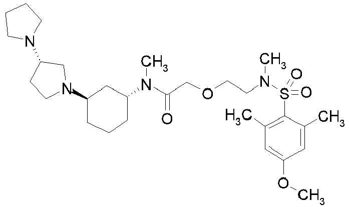

C₂₉H₄₈N₄O₅S (564.79)
[M+H]+ = 565
HPLC (Methode 14): Retentionszeit = 1.95 min

### Beispiel 703

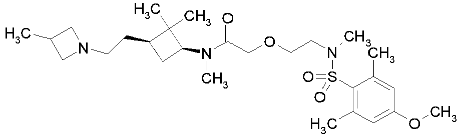

C₂₇H₄₅N₃O₅S (523.73)
[M+H]+ = 524
HPLC (Methode 5): Retentionszeit = 1.90 min

### Beispiel 704 (Referenzbeispiel)

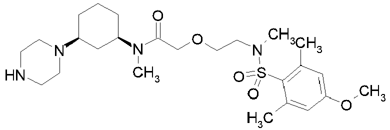

C₂₅H₄₂N₄O₅S (510.70)
[M+H]+ = 511
HPLC (Methode 5): Retentionszeit = 1.63 min

### Beispiel 705

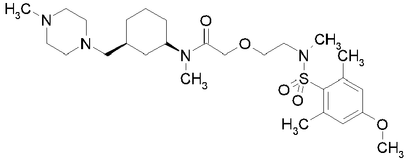

C₂₇H₄₆N₄O₅S (538.75)
[M+H]+ = 539
HPLC (Methode 5): Retentionszeit = 1.69 min

### Beispiel 706 (Referenzbeispiel)

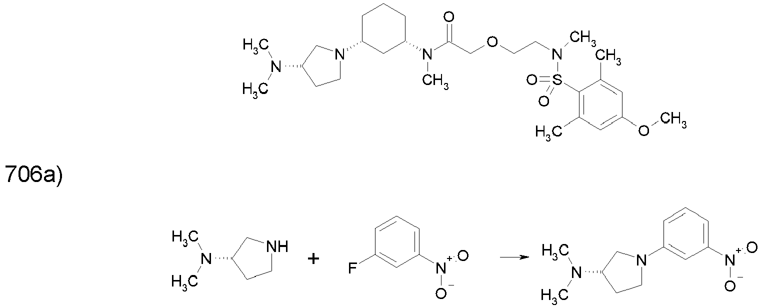

Eine Lösung von 800 mg (5.67 mMol) 3-Fluornitrobenzol, 647.4 mg (5.67 mMol) (3S)-(-)-(Dimethylamino)-pyrrolidin du 1.27 ml Diisopropylethylamin in 2 ml N-Methyl-pyrrolidinon wurde 14 Stunden bei 100°C gerührt. Das so entstandene Rohprodukt wurde chromatographisch isoliert und gereinigt.
C₁₂H₁₇N₃O₂ (235.3)
Ausbeute: 630 mg (47% der Theorie)
[M+H]⁺ = 236

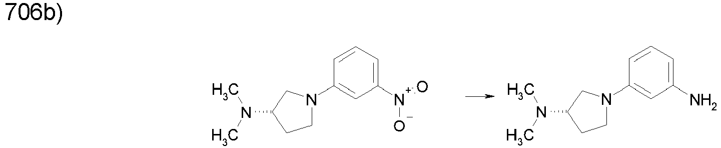

Das Produkt aus Beispiel 706a) wurde in 15 ml Ethylacetat gelöst, mit 200 mg Raney-Nickel versetzt und bei Raumtemperatur und 3 bar H₂-Druck hydriert.
C₁₂H₁₉N₃ (205.3)
Ausbeute: 104 mg (18% der Theorie)
[M+H]⁺ = 206

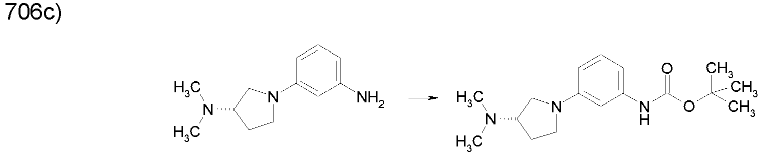

Das Produkt aus Beispiel 706b) (100 mg, 0.49 mMol) wurde in 2 ml Ethylacetat gelöst und mit 159 mg (0.73 mMol) Di-tert-butyl-dicarbonat versetzt und zwei Stunden bei Raumtemperatur gerührt. Anschließend wurde die Lösung eingedampft und das so erhaltene Rohprodukt chromatographisch gereinigt.
C₁₇H₂₇N₃O₂ (305.4)
Ausbeute: 134 mg (90% der Theorie)
[M+H]⁺ = 306

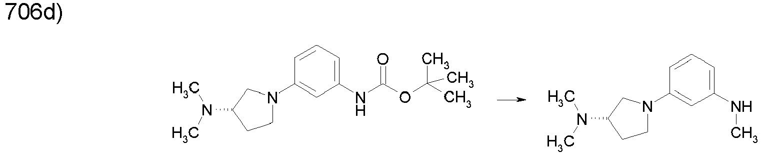

Zu einer Lösung des Produkts aus Beispiel 706c) (132 mg, 0.44mMol) in 3 ml wasserfreiem THF wurde vorsichtig tropfenweise eine Lösung von Lithiumaluminiumhydrid (2N in THF), die zuvor mit 2 ml wasserfreiem THF verdünnt wurde, unter Kühlung zugegeben. Anschließend wurde langsam erwärmt und dann das Reaktionsgemisch 4 Stunden unter Rückfluß erhitzt. Danach wurde auf -5°C gekühlt und tropfenweise 1 ml einer 1 N Natronlauge zugegeben. Der entstandene Niederschlag wurde abfiltriert und mit ca. 5 ml THF und dann mit Methanol nachgewaschen. Die vereinigten organischen Lösungen wurden über Natriumsulfat getrocknet und dann eingedampft. Das so erhaltene Produkt wurde ohne Reinigung weiter umgesetzt.
C₁₃H₂₁N₃ (219.3)
Ausbeute: 80 mg (83% der Theorie)
[M+H]⁺ = 220
Analytisch HPLC (Methode 10): Retentionszeit: 0.31 min.

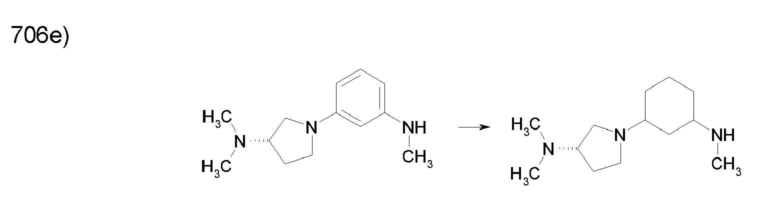

Das Produkt aus Beispiel 706d) wurde in 5 ml Methanol gelöst und nach Zusatz von 10 mg eines Nishimura-Katalysators bei Raumtemperatur und 3 bar H₂-Druck hydriert. Das so erhaltene Rohprodukt wurde ohne Reinigung weiter umgesetzt.
C₁₃H₂₇N₃ (225.4)
Ausbeute: 80 mg (97% der Theorie)
[M+H]⁺ = 226
Analytische HPLC (Methode 10): Retentionszeit: 0.28 min.

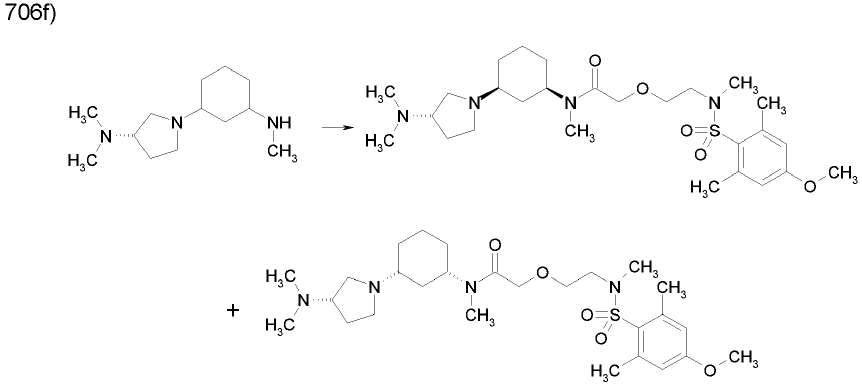

Das Produkt aus Beispiel 706e) wurde analog Beispiel 53 weiter umgesetzt. Das so erhaltene rohe Diastereomeren-Gemisch wurde chromatographisch in zwei Diastereomeren-Paare getrennt (HPLC-Methode 13). Man erhielt so das Diastereomeren-Paar mit cis-ständigen Substituenten am Cyclohexanring.
C₂₇H₄₆N₄O₅S (538.7)
Ausbeute: 14 mg (7.2% der Theorie)
[M+H]⁺ = 539
HPLC (Methode 13): Retentionszeit: 1.79 min.

### Beispiel 707 (Referenzbeispiel)

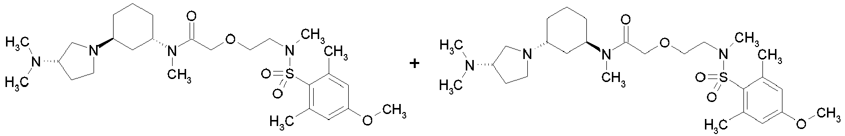

Das Produkt aus Beispiel 706e) wurde analog Beispiel 53 weiter umgesetzt. Das so erhaltene rohe Diastereomeren-Gemisch wurde chromatographisch in zwei Diastereomeren-Paare getrennt (HPLC-Methode 13). Man erhielt so das Diastereomeren-Paar mit trans-ständigen Substituenten am Cyclohexanring.
C₂₇H₄₆N₄O₅S (538.7)
Ausbeute: 3 mg (1.5% der Theorie)
[M+H]⁺ = 539
HPLC (Methode 13): Retentionszeit: 1.91 min

### Beispiel 708 (Referenzbeispiel)

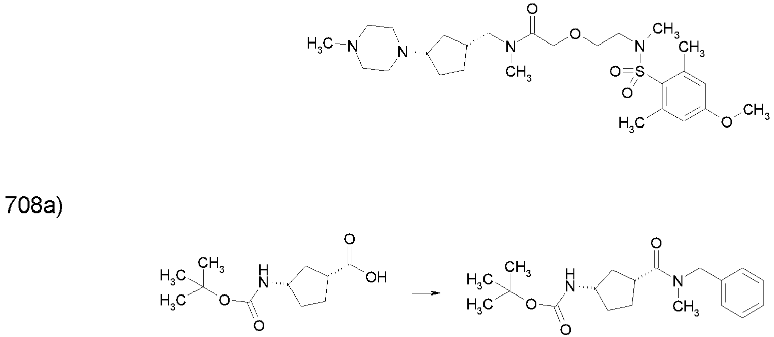

Analog Beispiel (55f) wurde (1*R*,3*S*)-3-tert-Butyloxycarbonylamino-cyclopentancarbonsäure mit *N*-Methyl-benzylamin umgesetzt.
Ausbeute: 82% der Theorie
C₁₉H₂₈N₂O₃ (332.44)
[M+H]⁺ = 333
HPLC (Methode 5): Retentionszeit: 2.41 min

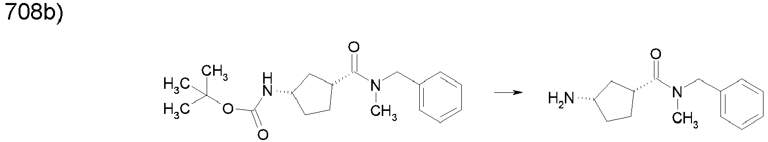

Die *tert*-Butoxycarbonyl-Schutzgruppe wurde unter Standard-Bedingungen mit Trifluoressigsäure in Dichlormethan abgespalten.
Ausbeute: 92% der Theorie
C₁₄H₂₀N₂O₃ (232.32)

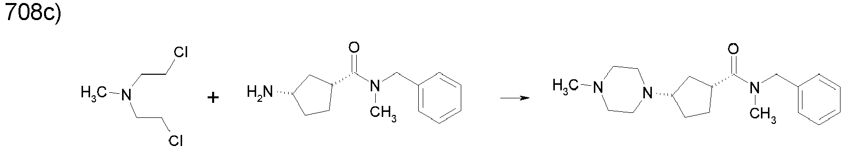

Eine Lösung des Produktes aus Beispiel (708b) (3.0 g, 12.91 mMol) in 45 ml Acetonitril wurde mit 8.92 g (65 mMol) Kaliumcarbonat, 0.10 g (0.6 mMol) Kaliumjodid und 2.49 g (12.91 mMol) Bis-chlorethyl-methylamin Hydrochlorid versetzt und vier Stunden unter Rückfluß erhitzt. Nach dem Abkühlen wurde das Reaktionsgemisch mit ca. 50 ml Dichlormethan verdünnt und mit 50 ml Wasser extrahiert. Die organische Phase wurde über Natriumsulfat getrocknet und eingedampft. Das so erhaltene rohe Produkt wurde ohne Reinigung weiter umgesetzt.
Ausbeute: 65% der Theorie
C₁₉H₂₉N₃O (315.45)

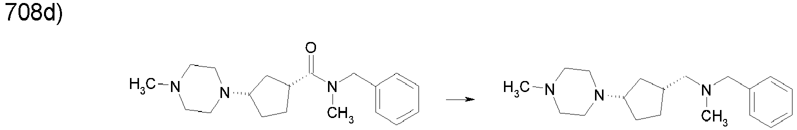

1.0 g (3.17 mMol) des Produktes aus Beispiel 708c) wurde unter Standardbedingungen mit Lithiumaluminiumhydrid in THF reduziert.
Ausbeute: 880 mg (91 % der Theorie)
C₁₉H₃₁N₃ (301.47)

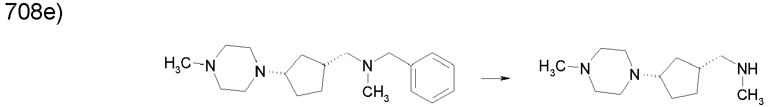

Das Produkt aus Beispiel 708d) (880 mg, 2.92 mMol) wurden in Methanol unter Zusatz von Pd/Kohle (10%) bei Raumtemperatur und 3 bar H₂-Druck hydriert.
Ausbeute: (94% der Theorie)
C₁₂H₂₅N₃ (211.35)

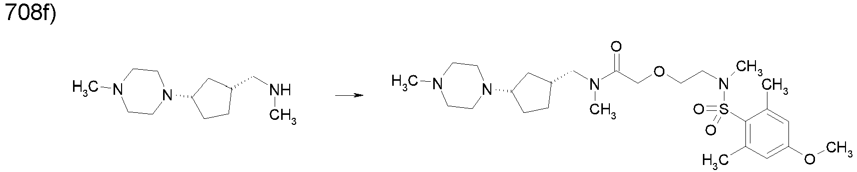

Das Produkt aus Beispiel 708e) wurde analog Beispiel (53) weiter umgesetzt.
Ausbeute: 67% der Theorie
C₂₆H₄₄N₄O₅S (524.72)
[M+H]+ = 525
HPLC (Methode 5): Retentionszeit = 1.44 min

### Beispiel 709 (Referenzbeispiel)

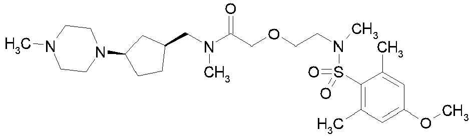

C₂₆H₄₄N₄O₅S (524.72)
[M+H]+ = 525
HPLC (Methode 5): Retentionszeit = 1.43 min

Die nachfolgenden Beispiele beschreiben pharmazeutische Darreichungsformen, die als Wirkstoff eine beliebige Verbindung der allgemeinen Formel I enthalten:

### Beispiel I

Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

### Beispiel III

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

### Beispiel IV

Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel V

Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel I 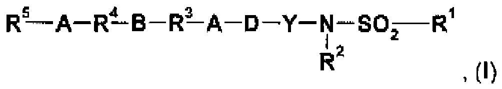 die ausgewählt sind aus der Gruppe bestehend aus:
| **Beispiel** | **Struktur** |
|---|---|
| (651) | 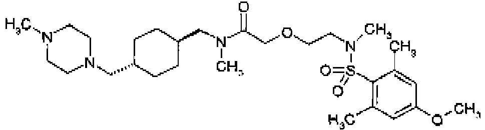 |
| (652) | 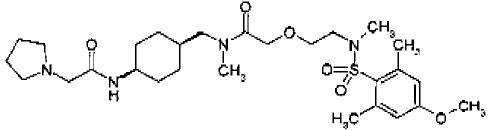 |
| (653) | 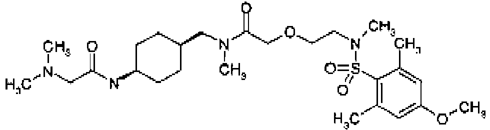 |
| (654) | 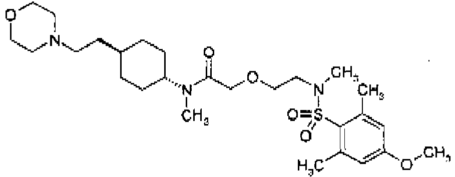 |
| (655) | 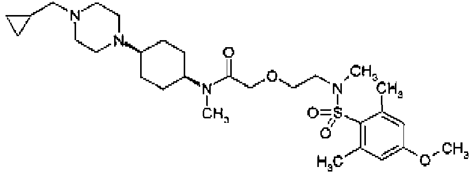 |
| (656) | 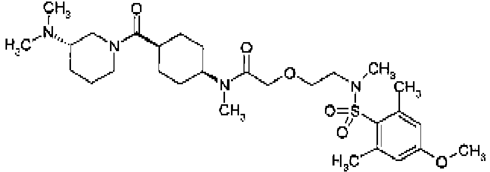 |
| (657) | 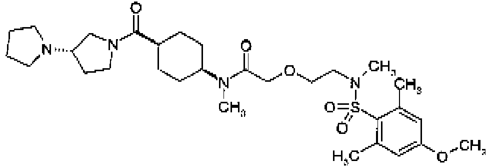 |
| (658) | 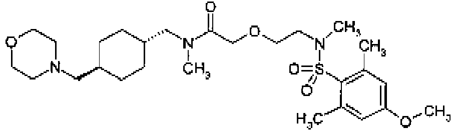 |
| (659) | 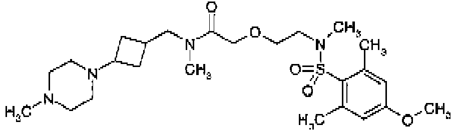 |
| (660) | 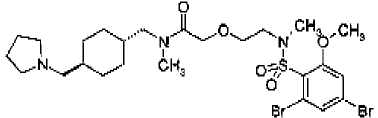 |
| (661) | 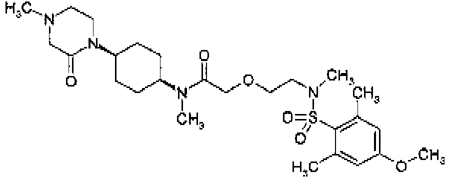 |
| (662) | 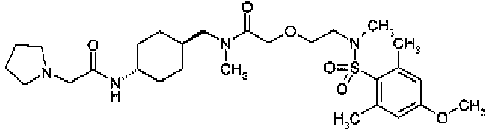 |
| (663) | 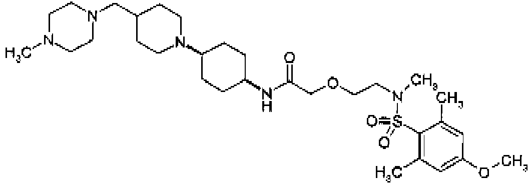 |
| (664) | 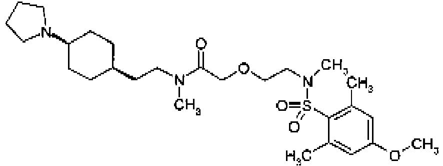 |
| (665) | 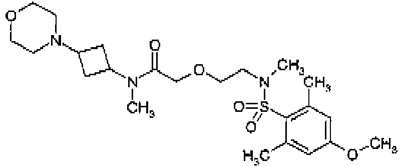 |
| (666) | 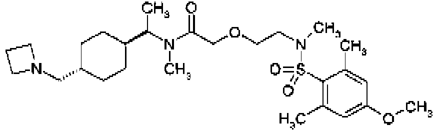 |
| (667) | 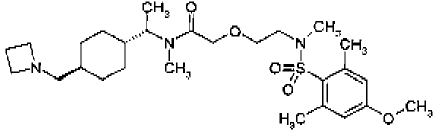 |
| (668) | 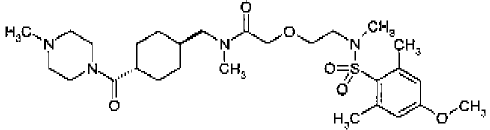 |
| (669) | 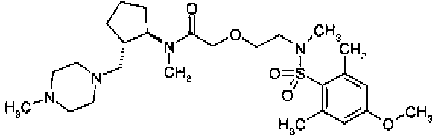 |
| (670) | 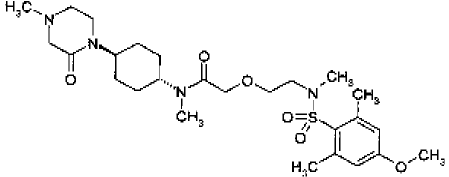 |
| (671) | 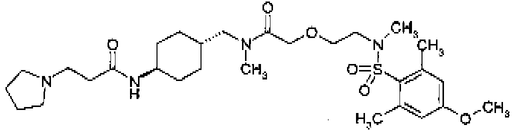 |
| (672) | 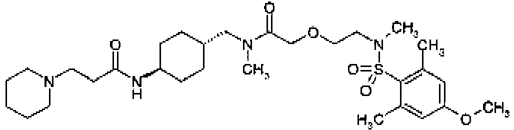 |
| (673) | 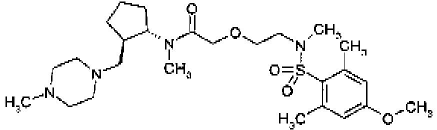 |
| (674) | 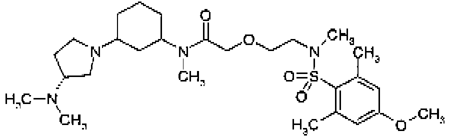 |
| (675) | 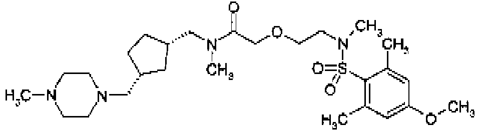 |
| (677) | 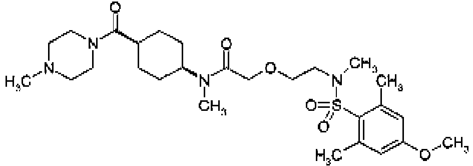 |
| (678) | 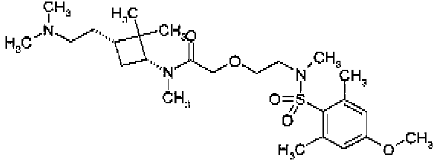 |
| (679) | 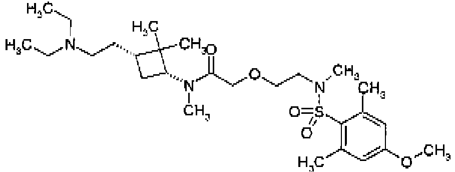 |
| (680) | 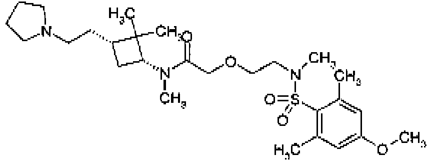 |
| (681) | 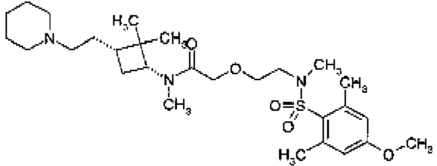 |
| (682) | 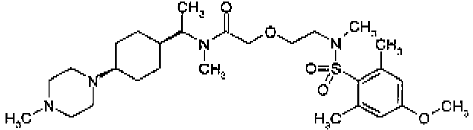 |
| (683) | 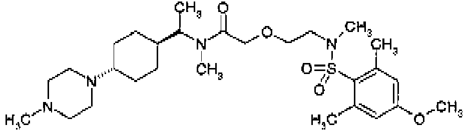 |
| (684) | 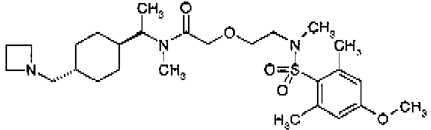 |
| (685) | 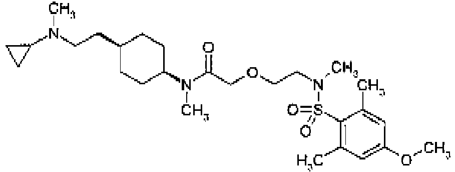 |
| (686) | 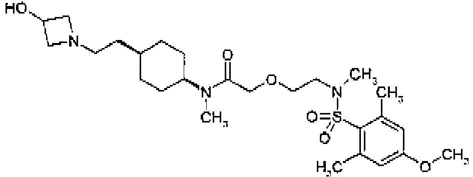 |
| (687) | 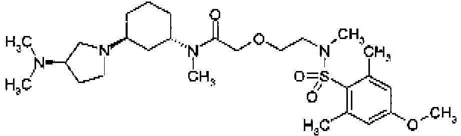 |
| (688) | 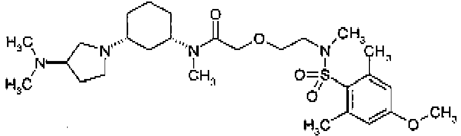 |
| (689) | 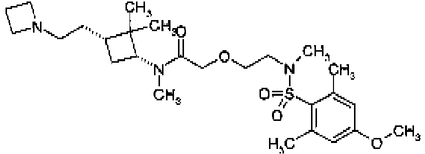 |
| (690) | 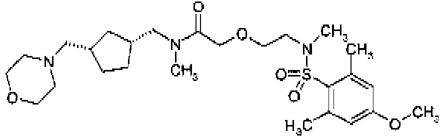 |
| (691) | 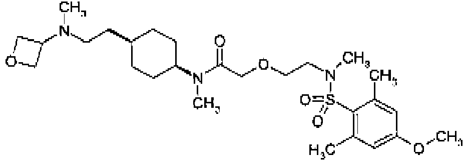 |
| (693) | 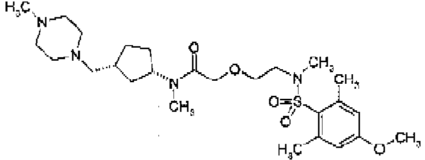 |
| (694) | 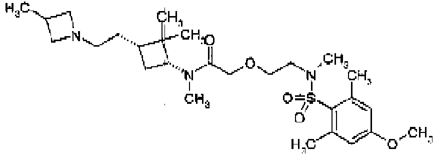 |
| (695) | 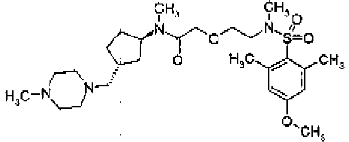 |
| (696) | 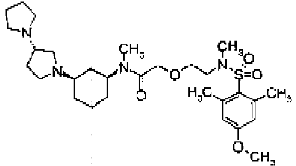 |
| (697) | 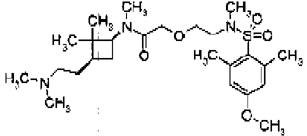 |
| (698) | 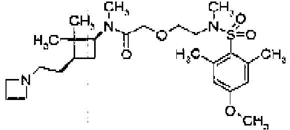 |
| (699) | 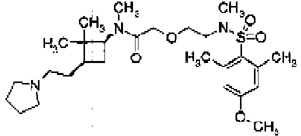 |
| (700) | 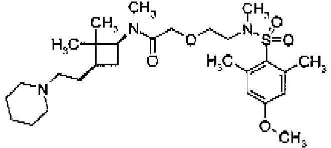 |
| (701) | 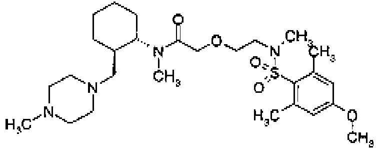 |
| (702) | 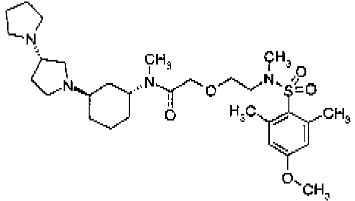 |
| (703) | 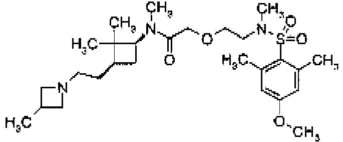 |
| (705) | 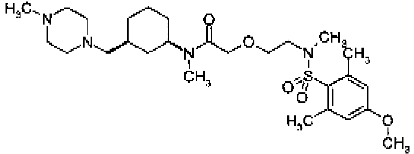 |
deren Enantiomere, deren Diastereomere, deren Gemische und deren Salze, insbesondere deren physiologisch verträgliche Salze mit organischen oder anorganischen Säuren oder Basen.

2. Physiologisch verträgliche Salze der Verbindungen nach Anspruch 1 mit anorganischen oder organischen Säuren oder Basen.

3. Arzneimittel, enthaltend eine Verbindung nach Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach mindestens einem der Ansprüche 1 bis 2 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

5. Verfahren zur Herstellung eines Arzneimittels gemäß Anspruch 3, **dadurch gekennzeichnet, dass** auf nichtchemischem Weg eine Verbindung nach mindestens einem der Ansprüche 1 bis 2 in einen oder mehrere inerte Trägerstoffe und/oder Verdünnungsmittel eingearbeitet wird.

## Claims

1. Compounds of general formula I 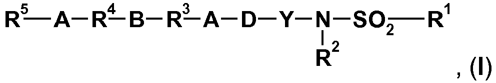 which are selected from among:
| **Example** | **Structure** |
|---|---|
| (651) | 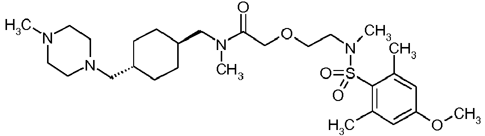 |
| (652) | 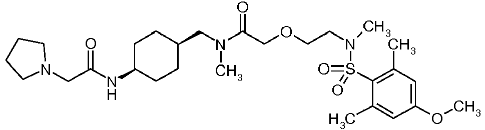 |
| (653) | 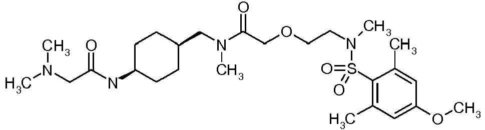 |
| (654) | 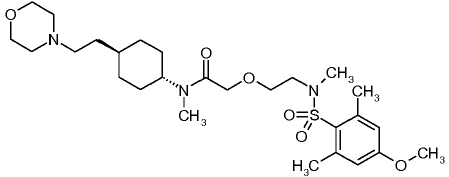 |
| (655) | 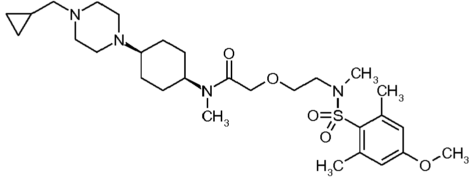 |
| (656) | 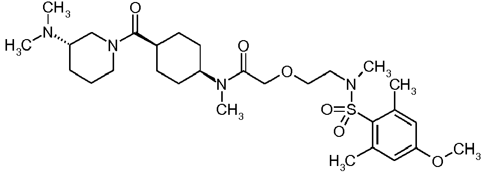 |
| (657) | 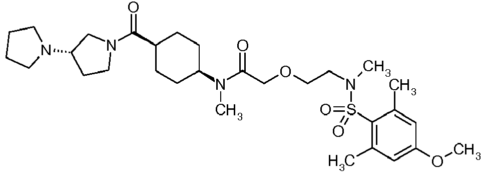 |
| (658) | 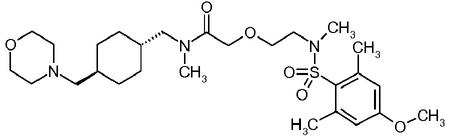 |
| (659) | 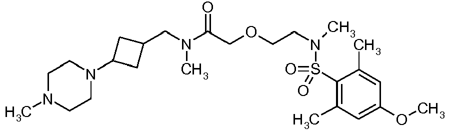 |
| (660) | 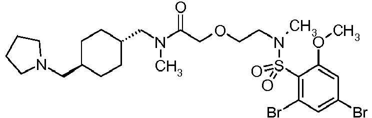 |
| (661) | 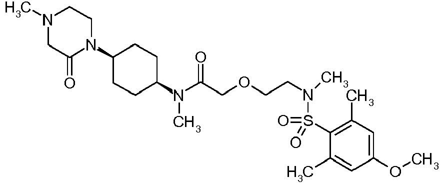 |
| (662) | 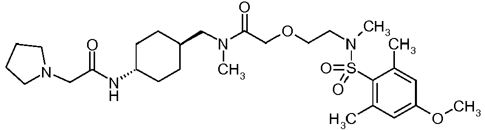 |
| (663) | 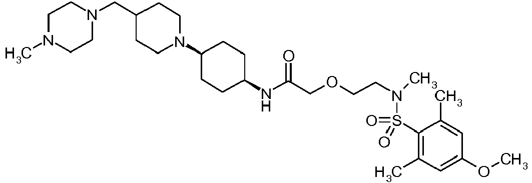 |
| (664) | 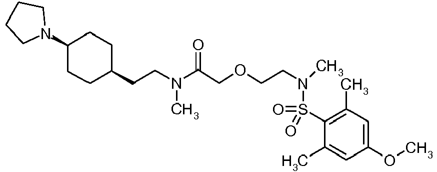 |
| (665) | 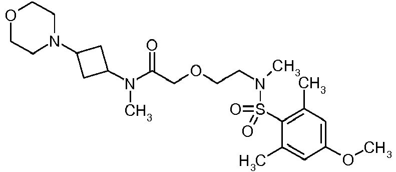 |
| (666) | 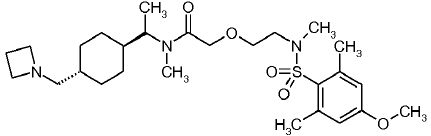 |
| (667) | 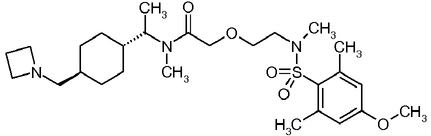 |
| (668) | 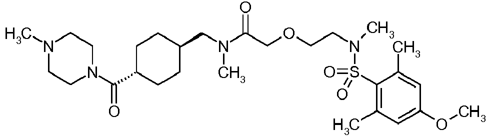 |
| (669) | 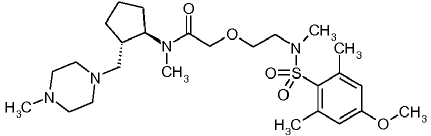 |
| (670) | 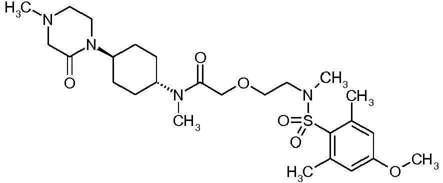 |
| (671) | 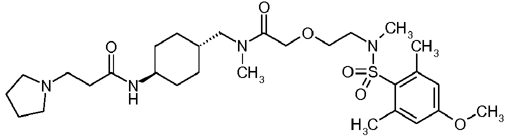 |
| (672) | 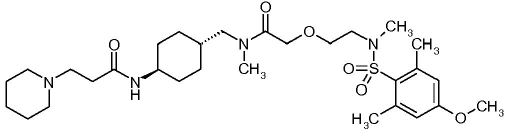 |
| (673) | 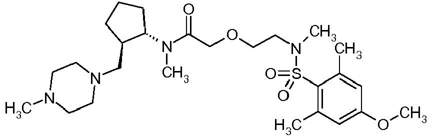 |
| (674) | 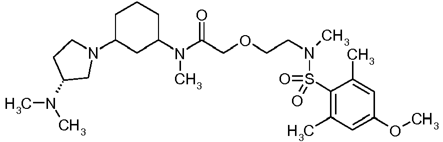 |
| (675) | 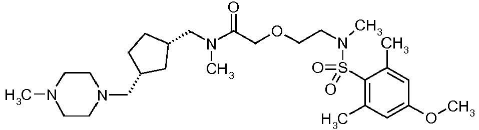 |
| (677) | 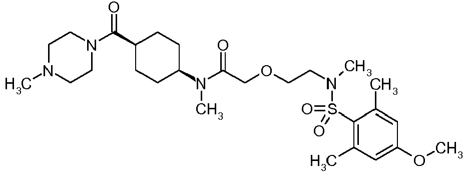 |
| (678) | 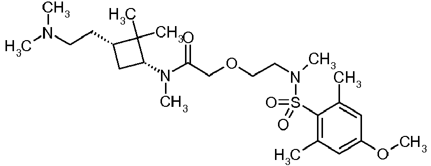 |
| (679) | 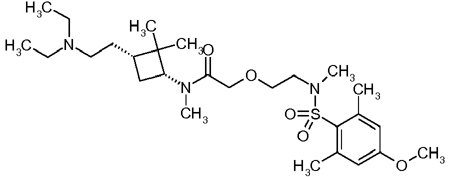 |
| (680) | 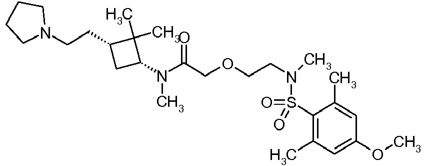 |
| (681) | 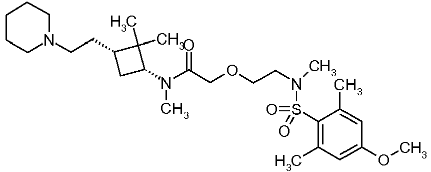 |
| (682) | 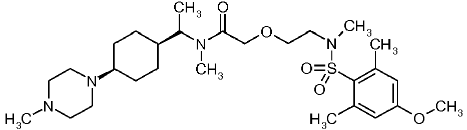 |
| (683) | 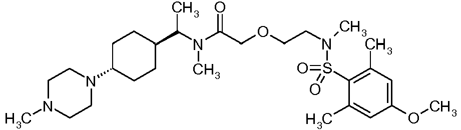 |
| (684) | 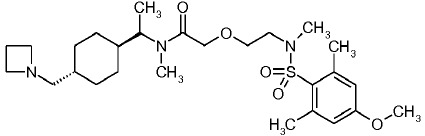 |
| (685) | 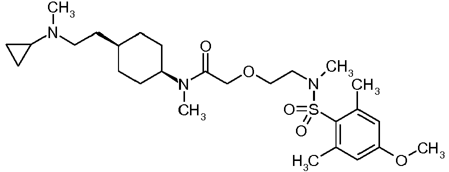 |
| (686) | 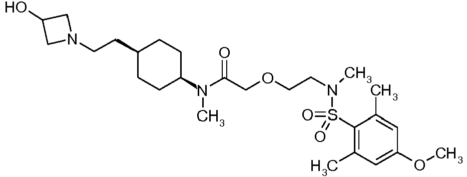 |
| (687) | 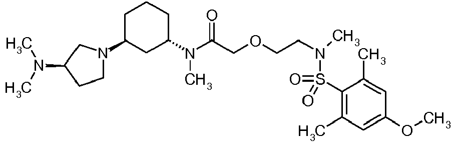 |
| (688) | 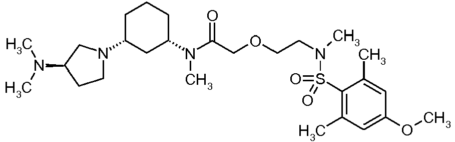 |
| (689) | 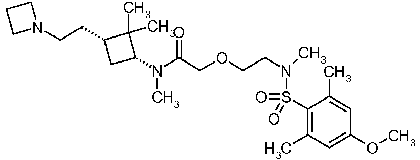 |
| (690) | 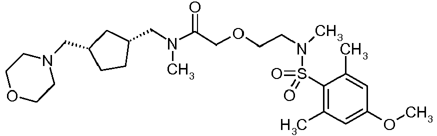 |
| (691) | 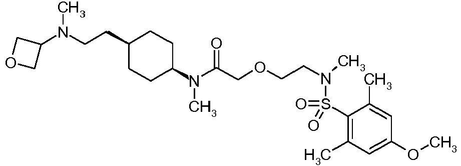 |
| (693) | 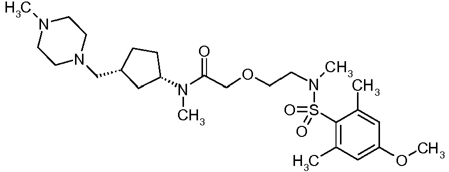 |
| (694) | 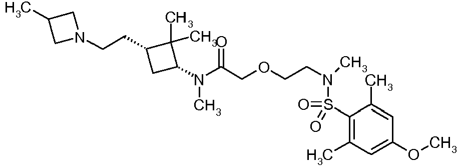 |
| (695) | 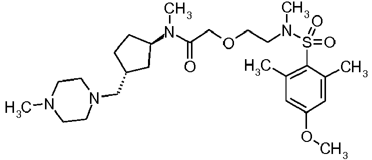 |
| (696) | 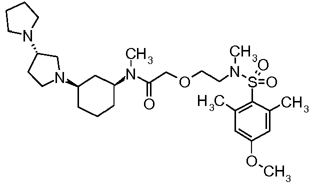 |
| (697) | 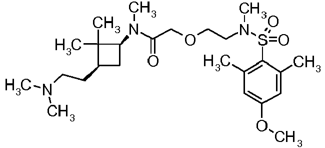 |
| (698) | 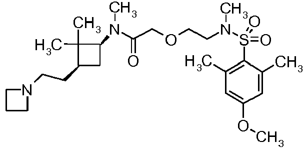 |
| (699) | 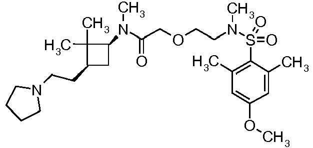 |
| (700) | 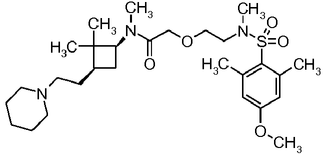 |
| (701) | 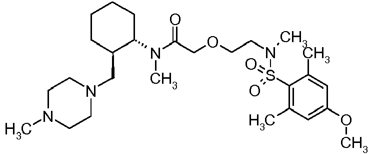 |
| (702) | 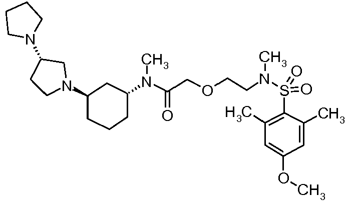 |
| (703) | 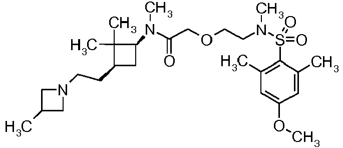 |
| (705) | 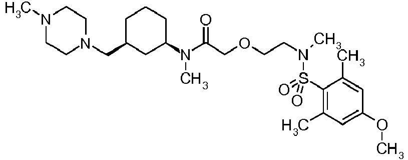 |
the enantiomers, the diastereomers, the mixtures and the salts thereof, particularly the physiologically acceptable salts thereof with organic or inorganic acids or bases.

2. Physiologically acceptable salts of the compounds according to claim 1 with inorganic or organic acids or bases.

3. Medicament, containing a compound according to claim 1 or a physiologically acceptable salt according to claim 2 optionally together with one or more inert carriers and/or diluents.

4. Use of a compound according to at least one of claims 1 to 2 for preparing a medicament for the acute and prophylactic treatment of acute pain, visceral pain, neuropathic pain, inflammatory/pain receptor-mediated pain, tumour pain and headache diseases.

5. Process for preparing a medicament according to claim 3, **characterised in that** by a non-chemical method a compound according to at least one of claims 1 to 2 is incorporated in one or more inert carriers and/or diluents.

## Revendications

1. Composés de formule générale I 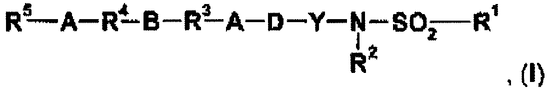 qui sont choisis dans le groupe comprenant :
| Exemple | Structure |
|---|---|
| (651) | 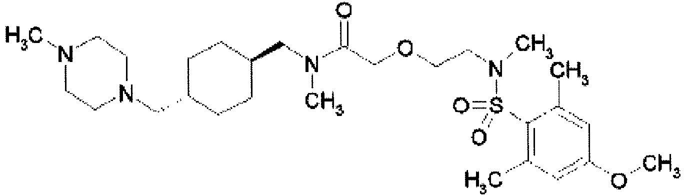 |
| (652) | 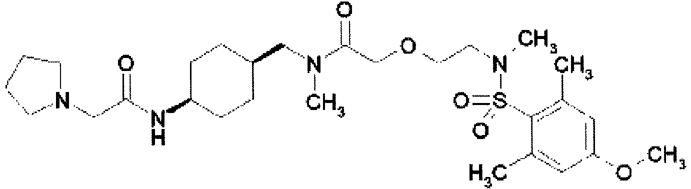 |
| (653) | 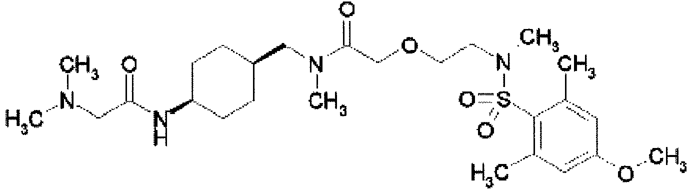 |
| (654) | 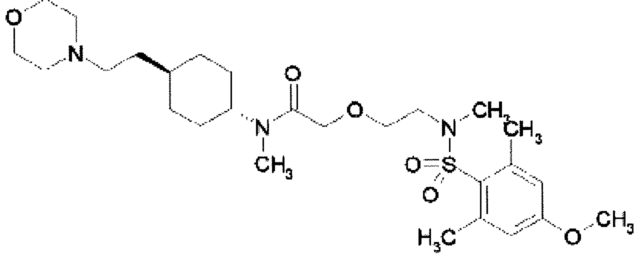 |
| (655) | 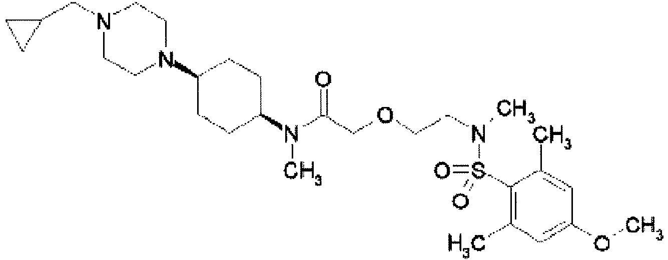 |
| (656) | 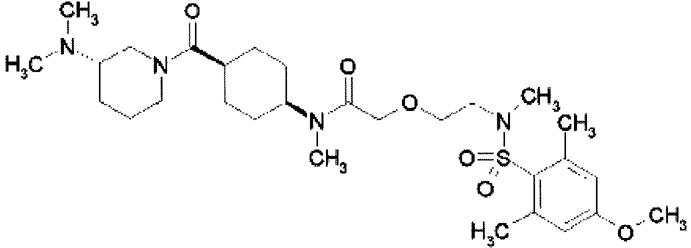 |
| (657) | 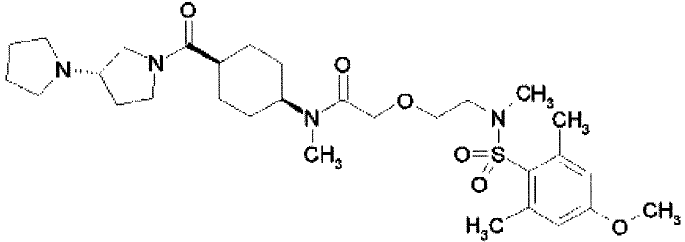 |
| (658) | 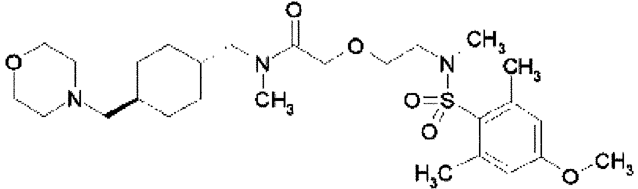 |
| (659) | 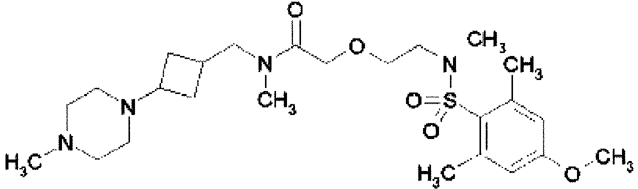 |
| (660) | 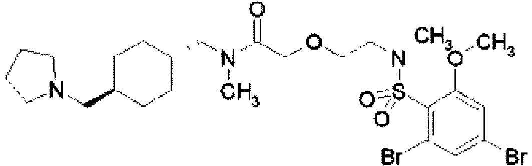 |
| (661) | 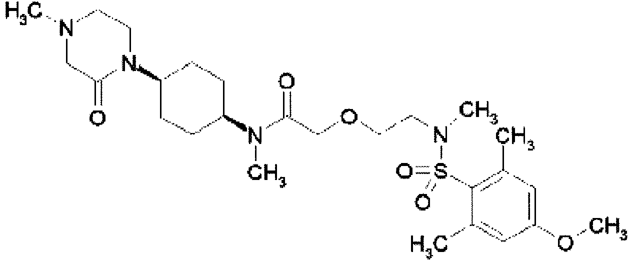 |
| (662) | 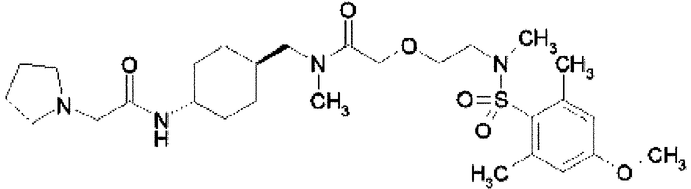 |
| (663) | 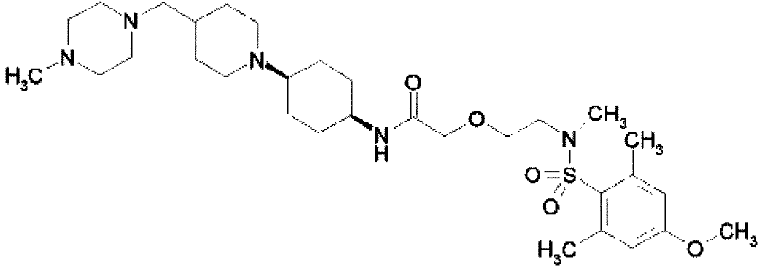 |
| (664) | 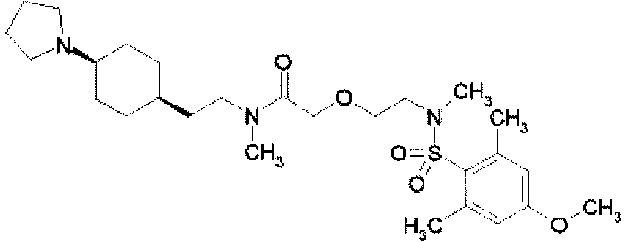 |
| (665) | 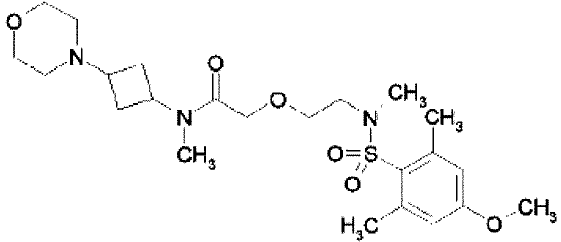 |
| (666) | 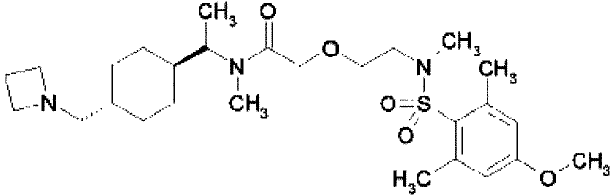 |
| (667) | 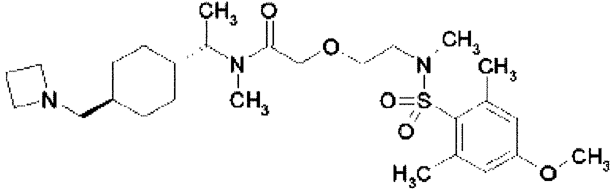 |
| (668) | 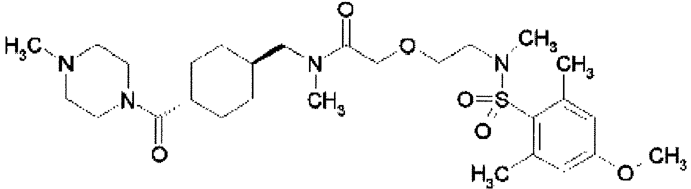 |
| (669) | 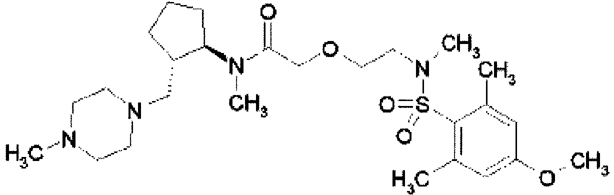 |
| (670) | 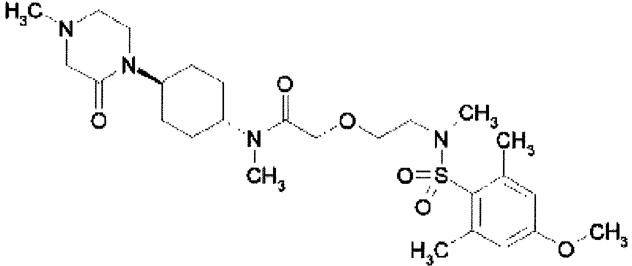 |
| (671) | 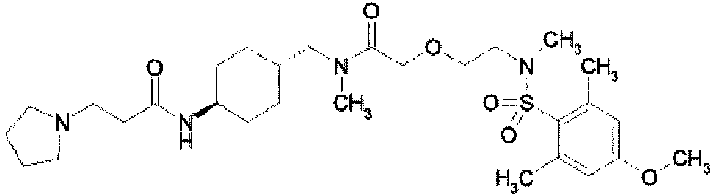 |
| (672) | 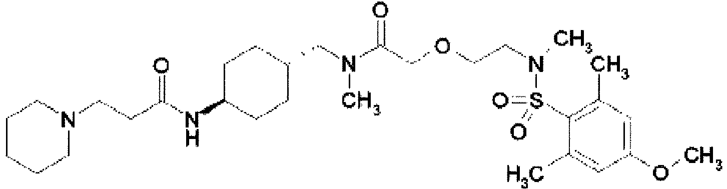 |
| (673) | 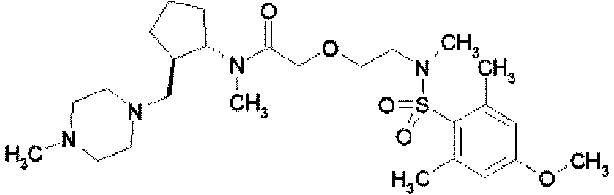 |
| (674) | 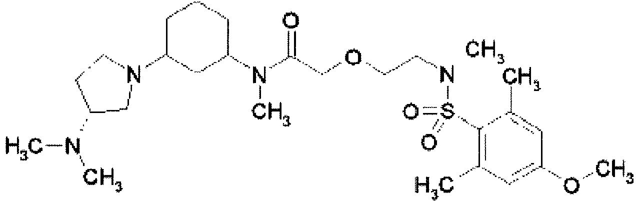 |
| (675) | 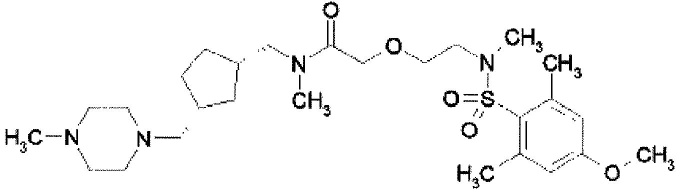 |
| (677) | 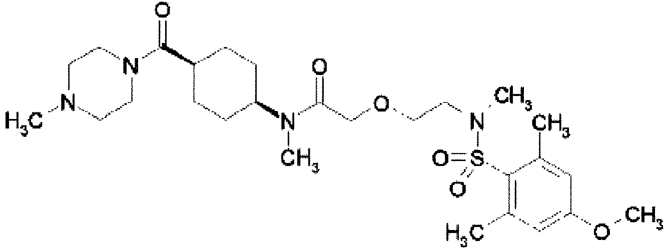 |
| (678) | 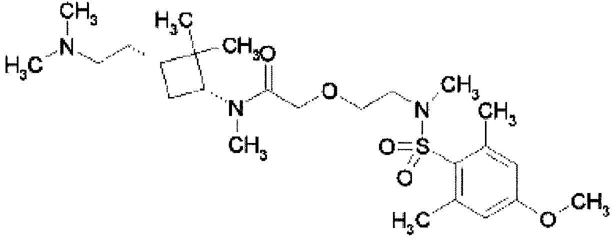 |
| (679) | 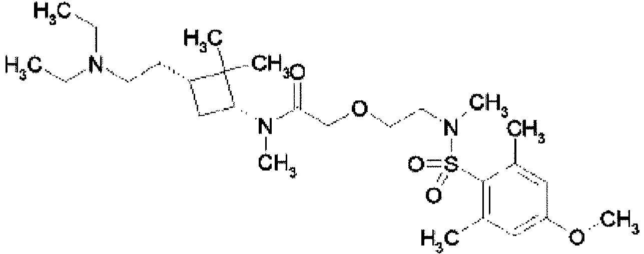 |
| (680) | 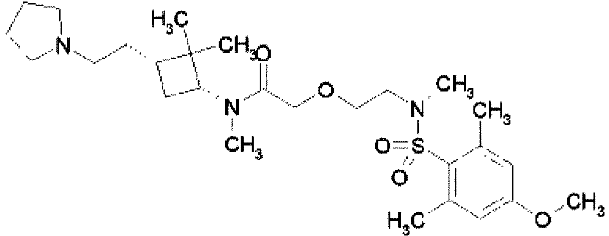 |
| (681) | 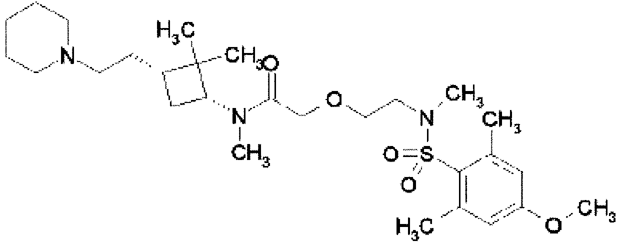 |
| (682) | 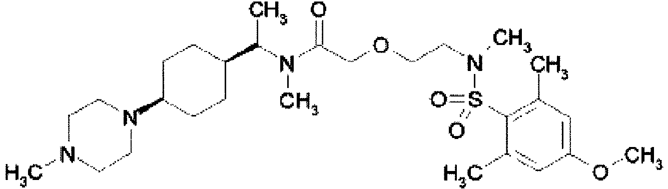 |
| (683) | 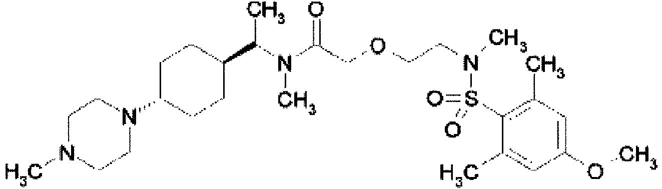 |
| (684) | 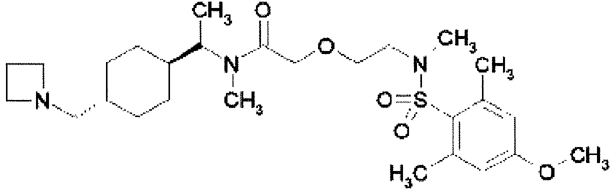 |
| (685) | 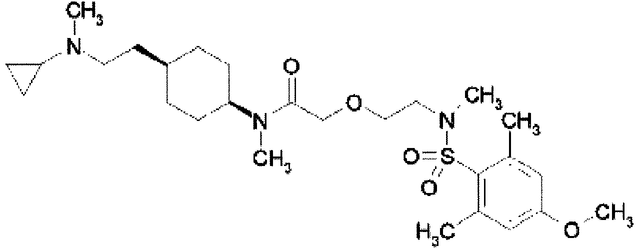 |
| (686) | 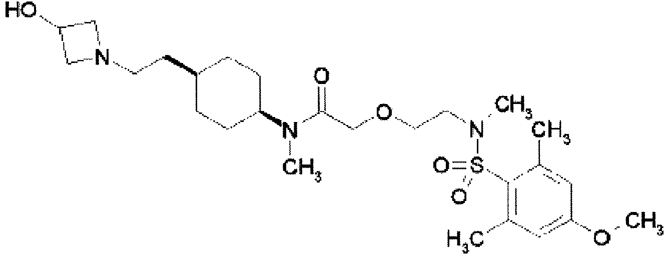 |
| (687) | 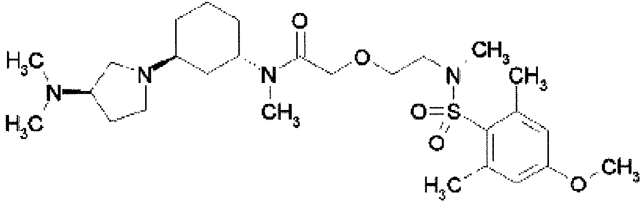 |
| (688) | 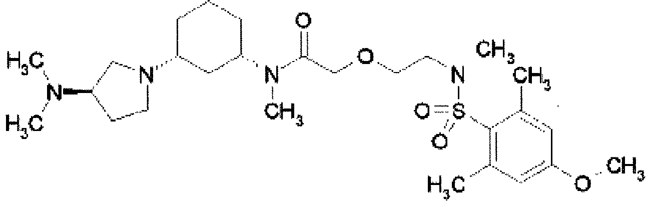 |
| (689) | 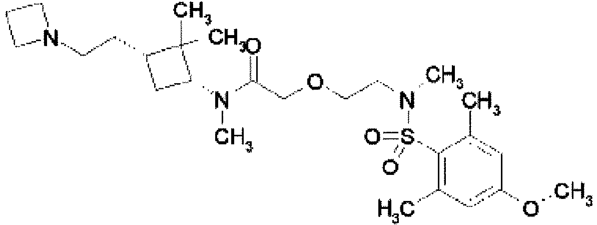 |
| (690) | 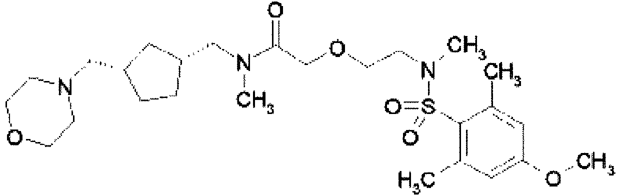 |
| (691) | 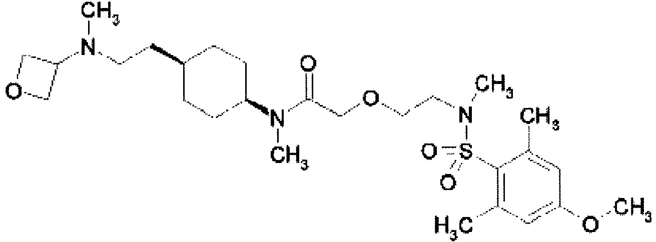 |
| (693) | 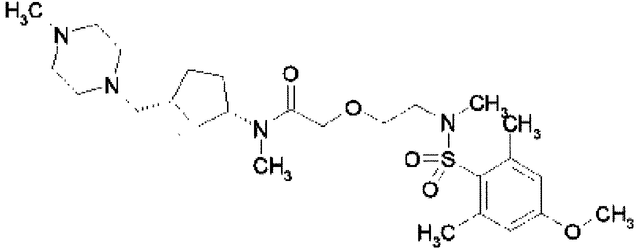 |
| (694) | 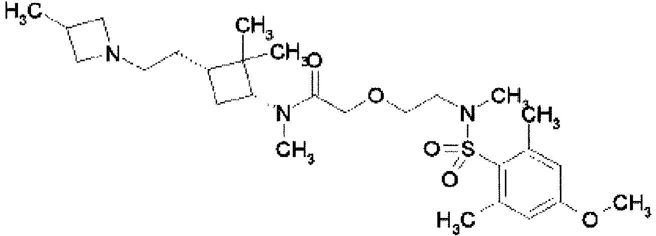 |
| (695) | 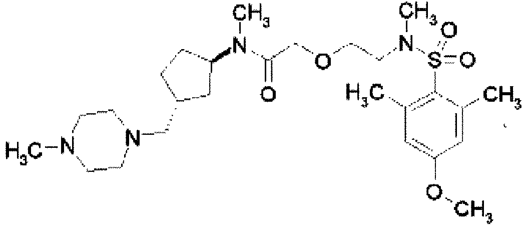 |
| (696) | 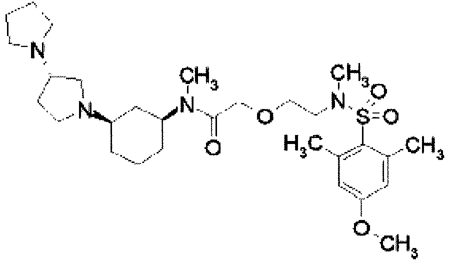 |
| (697) | 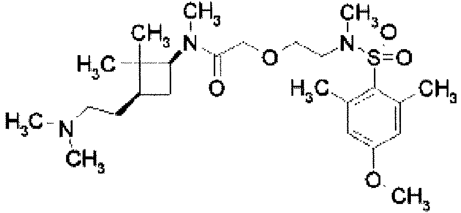 |
| (698) | 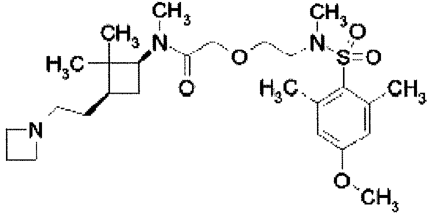 |
| (699) | 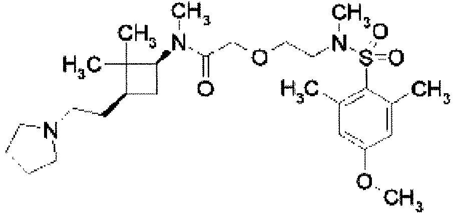 |
| (700) | 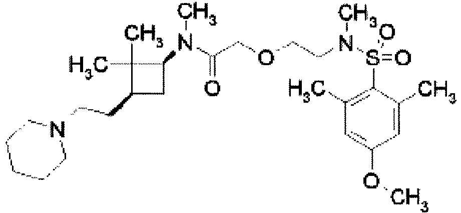 |
| (701) | 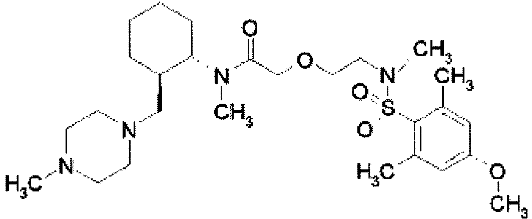 |
| (702) | 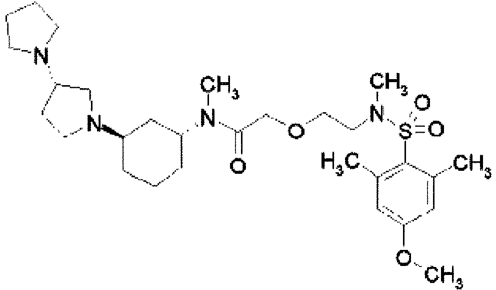 |
| (703) | 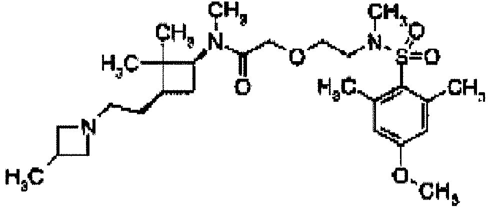 |
| (705) | 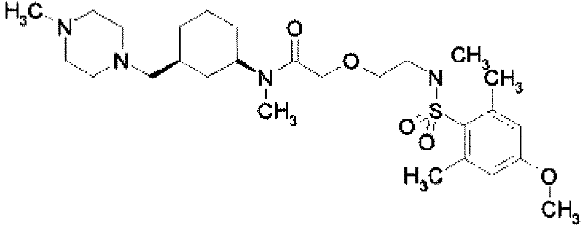 |
leurs énantiomères, leurs diastéréomères, leurs mélanges et leurs sels, en particulier leurs sels physiologiquement acceptables avec des acides ou des bases organiques ou inorganiques.

2. Sels physiologiquement acceptables des composés selon la revendication 1 avec des acides ou des bases inorganiques ou organiques.

3. Médicament, contenant un composé selon la revendication 1 ou un sel physiologiquement acceptable selon la revendication 2 parallèlement éventuellement à un ou plusieurs véhicules et/ou diluants inertes.

4. Utilisation d'un composé selon au moins l'une des revendications 1 à 2, pour la production d'un médicament pour le traitement aigu et prophylactique des douleurs aiguës, des douleurs viscérales, des douleurs neuropathiques, des douleurs inflammatoires/induites par un récepteur nociceptif, des douleurs tumorales et des céphalées.

5. Procédé de production d'un médicament selon la revendication 3, **caractérisé en ce que**, de façon non chimique, un composé selon au moins l'une des revendications 1 à 2 est incorporé dans un ou plusieurs véhicules et/ou diluants inertes.
